# FASCICULE DE BREVET EUROPEEN

(11) **EP 2 018 165 B1**
(45) Date de publication et mention de la délivrance du brevet: **13.04.2011**
(21) Numéro de dépôt: 07717861.4
(22) Date de dépôt: 12.01.2007
(51) Int. Cl.: A61K 31/435, A61P 9/00, A61P 11/00, C07D 519/00

(54) **COMPOSES DIMERES AGONISTES DES RECEPTEURS DES FGFs**
DIMERE VERBINDUNGEN FÜR EINEN FGF-REZEPTOR-AGONISTEN
FGF-PECEPTOR AGONIST DIMERIC COMPOUNDS

(30) Priorité: 13.01.2006 FR 0600317
(43) Date de publication de la demande: 28.01.2009
(73) Titulaire: Sanofi-Aventis, 75013 Paris (FR)
(72) Inventeur: BONO, Françoise, 92160 Antony (FR); GUILLO, Nathalie, 92160 Antony (FR); MAFFRAND, Jean-Pierre, 92160 Antony (FR); FONS, Pierre, 92160 Antony (FR); OLSEN, Jacob-Alsboek, 65926 Frankfurt (DE); ANNE-ARCHARD, Gilles, 92160 Antony (FR)
(74) Mandataire: Le Coupanec, Pascale A.M.P.
(86) Numéro de dépôt international: PCT/FR2007/000051
(87) Numéro de publication internationale: WO 2007/080325

(56) Documents cités:
- WO-A-03/084956
- WO-A-2005/028476
- ROTARU ET AL.: "Synthesis of New Non-Symmetrical Substituted 7,7'-Bisindolizines by the Direct Reaction of 4,4'-Bipyidinium-Ylides with Dimethyl Acetylenedicarboxylate" J. HETEROCYCL. CHEM., vol. 41, 2004, pages 893-897, XP002395674
- SEED B: "MAKING AGONISTS OF ANTAGONISTS" CHEMISTRY AND BIOLOGY, CURRENT BIOLOGY, LONDON, GB, vol. 1, no. 1, 1994, pages 125-129, XP008007292 ISSN: 1074-5521 cité dans la demande

## Description

La présente invention a pour objet de nouveaux composés hétérocycliques inducteurs de dimérisation des récepteurs des Fibroblast Growth Factors (ou FGFRs), leur procédé de préparation et leurs applications en thérapeutique. La présente invention a pour objet notamment de nouveaux composés à structure dimérique en tant qu'agonistes des FGFRs.

L'angiogenèse est un processus de génération de nouveaux vaisseaux capillaires. Lors de l'obstruction d'un vaisseau sanguin, l'angiogenèse, associée à une dilatation des capillaires (artériogenèse), améliore la revascularisation de la zone obstruée. Il a été montré *in vitro* et *in vivo* que plusieurs facteurs de croissance (tels que les Fibroblast Growth Factors ou FGFs) stimulent ce processus.
Les FGFs sont une famille de polypeptides synthétisés par un grand nombre de cellules lors du développement embryonnaire et par des cellules des tissus adultes dans diverses conditions pathologiques.
Le FGF2 (ou b-FGF) est le premier et le mieux caractérisé de ces facteurs de croissance. Le FGF2 est une protéine de 18 kD qui induit la prolifération, la migration et la production de protéases par les cellules endothéliales en culture et la néovascularisation *in vivo.* Le FGF2 interagit avec les cellules endothéliales par l'intermédiaire de deux classes de récepteurs, les récepteurs de haute affinité à activité tyrosine kinase (FGFRs) et les récepteurs de basse affinité de type héparane sulfate protéoglycane (HSPG) situés à la surface des cellules et dans les matrices extracellulaires. Ainsi, le FGF2 et ses récepteurs représentent des cibles très pertinentes pour les thérapies visant à activer ou à inhiber les processus d'angiogenèse.
De ce fait, de puissants antagonistes de la liaison des FGFs à leurs récepteurs à activité tyrosine kinase (FGFRs) tels que des dérivés d'indolizine sont décrits dans les demandes de brevet internationales W02003084956 et W02005028476, et des dérivés d'imidazo[1,5-a]pyridine dans la demande de brevet internationale WO2006097625.

Par ailleurs il est connu que les récepteurs de la surface cellulaire à activité tyrosine kinase transmettent les informations à travers la membrane plasmatique notamment par des mécanismes de dimérisation des domaines extracellulaires de ces récepteurs. Des ligands connus capables d'activer ces mécanismes de dimérisation sont typiquement des composés naturels comme les FGFs, le PDGF (Platelet-Derived Growth Factor), le VEGF (Vascular Endothelial Growth Factor), l'EPO (Erythropoietin), le G-CSF (Granulocyte-Colony Stimulating Factor) ou la TPO (Thrombopoietin), les cytokines ou l'insuline.

Une fois dimérisés, certains de ces récepteurs provoquent une cascade de signaux qui aboutit *via* la prolifération et la migration cellulaire à la formation de nouveaux vaisseaux et donc à une activation de l'angiogenèse.

B. Seed (Chemistry and Biology, November, 1994, 1, 125-129) pose le principe général qu'il serait possible de construire des agonistes des récepteurs cellulaires par dimérisation de composés naturels ou légèrement modifiés ayant une action antagoniste de ces mêmes récepteurs cellulaires. Ces préconisations conceptuelles ne sont soutenues ou illustrées par aucune molécule spécifique notamment des molécules de synthèse de faible poids moléculaire. Des articles tels que S A. Qureshi (PNAS, 1999, vol 96, no 21, 12156-12161), B E. Welm (The Journal of cell biology, 2002, vol 157, 4, 703-714), K. Koide (J. Am. Chem. Soc., 2001, 123, 398-408) décrivent des composés non peptidiques ou inducteurs chimiques de dimérisation (CID), ces composés agissent sur des récepteurs chimériques et non sur des récepteurs endogènes. Ils ne présentent pas de résultats montrant qu'un CID permet l'activation de la voie de signalisation d'un récepteur endogène.

D'autre part, Ariad (WO96/13613, WO97/31898 et WO97/31899) a développé des dimères de dérivés de la Rapamycine qui sont capables de multimériser des protéines chimériques de FKBP (FK506 binding protein) contenant des domaines dérivés d'immunophilines.

La demanderesse a maintenant trouvé de nouvelles molécules de synthèse capables d'activer la formation de néo-vaisseaux sanguins (ou angiogenèse) en induisant la dimérisation des récepteurs FGFs.

L'invention a pour but de proposer de nouveaux composés à structure dimérique agonistes des récepteurs FGFs.

Ces composés entraînent une dimérisation des récepteurs FGFs qui provoque leur activation et au final une activation de l'angiogenèse.

La présente invention a pour objet des composés agonistes des récepteurs FGFs répondant à la formule générale :

M₁-L-M₂

dans laquelle M₁ ou M₂ identiques ou différents représentent chacun indépendamment l'un de l'autre une unité monomère M et L représente un groupe de liaison qui lie M₁ et M₂ de façon covalente,
ladite unité monomère répondant à la formule générale M qui suit : 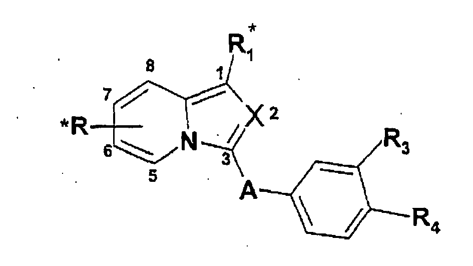 dans laquelle,
X représente N ou C-R₂*,
A représente un radical -CO-,
* indique le site de liaison entre L avec l'unité monomère M₁ d'une part et avec l'unité monomère M₂ d'autre part; ledit site de liaison de chaque unité monomère M₁ ou M₂ étant situé sur l'un des substituants R, R₁ ou R₂ ;
R représente un atome d'hydrogène, un atome d'halogène, un radical alkyle linéaire ou ramifié de 1 à 5 atomes de carbone, un radical hydroxy, ou un radical de formule :
- -CO₂R₅
- -CO-NR₆R₇
- -O-Alk
- -O-Alk-CO₂R₆
- -O-Alk-CO-NR₆R₇
- -O-Alk-NR₆R₇
- -O-Alk-Ph
- NR₆R₇
- -NH-SO₂-Alk
- -NH-CO-Alk, ou
- -NH-CO₂-Alk
   dans lesquels :
   - R₅ représente un atome d'hydrogène, un radical alkyle linéaire ou ramifié de 1 à 5 atomes de carbone ou un radical benzyle ;
   - R₆ et R₇ identiques ou différents représentent chacun un atome d'hydrogène, un radical alkyle linéaire ou ramifié de 1 à 5 atomes de carbone ou un radical benzyle,
   - Alk représente un radical alkyle linéaire ou ramifié de 1 à 5 atomes de carbone ou un radical alkylène linéaire ou ramifié de 1 à 5 atomes de carbone ;
   - Ph représente un radical phényle éventuellement substitué par un ou plusieurs atomes d'halogène, par un ou plusieurs radicaux hydroxy, par un ou plusieurs radicaux alcoxy linéaires ou ramifiés de 1 à 5 atomes de carbone, ou par un ou plusieurs radicaux -COOR₅;

R₁ représente un atome d'hydrogène, un atome d'halogène, un radical hydroxy, un radical cyano, un radical alkyle linéaire ou ramifié de 1 à 5 atomes de carbones ou un radical de formule :
- -CO₂R₅
- -CO-NR₆R₇
- -CO-NH-Alk-CO₂R₅
- -CO-NH-Ph
- -O-Alk
- -O-Alk-CO₂R₅
- -O-Alk-CO-NR₆R₇
- -O-Alk-NR₆R₇
- -O-Alk-OR₅
- -O-Alk-Ph
- -O-Ph
- -NR₆R₇
- -NH-SO₂-Alk
- -NH-CO-Alk
- -NH-CO-Alk-CO₂R₅
- -NH-CO-Alk-CO-NR₆R₇
- -NH-CO₂-Alk
- -NH-CO-Ph, ou
- un radical aryle ou hétéroaryle de 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, ou par un ou plusieurs radicaux -CO₂R₅ ou -CO-NR₆R₇ dans lesquels Alk, Ph, R₅, R₆ et R₇ sont tels que définis au niveau du groupe R;

R₂ représente un radical alkyle linéaire ou ramifié de 1 à 5 atomes de carbone, un radical cycloalkyle de 3 à 6 atomes de carbone ou un radical phényle éventuellement substitué par un ou plusieurs atomes d'halogène, par un ou plusieurs radicaux carboxy, par un ou plusieurs radicaux alcoxy linéaires ou ramifiés de 1 à 5 atomes de carbone, par un ou plusieurs radicaux hydroxy, par un ou plusieurs radicaux benzyloxy, ou par un ou plusieurs radicaux alcoxycarbonyle de 2 à 6 atomes de carbone ; .

R₃ et R₄ représentent indépendamment l'un de l'autre un atome d'hydrogène, un radical hydroxy, un radical amino, un radical nitro ou un radical de formule :
- -CO₂R₅
- -CO-NR₆R₇
- -CO-NHOH
- -O-Alk
- -O-Alk-NR₆R₇
- -O-Alk-CO-NR₆R₇
- -NHOH
- NR₆R₇
- -N(R₆)-CO-Alk
- -N(R₈)-CO-CF₃
- -N(R₈)-CO-Ph
- -N(R₈)-CO₂-Alk
- -N(R₈)-SO₂-Alk
- -N(R₈)-CO-Alk-NR₈R₇
- -N(R₈)-SO₂-Alk-NR₈R₇, ou
- -NH-Alk-R₆R₇
dans lesquels R₈ représente un atome d'hydrogène ou un radical -Alk-COOR₅ et Alk, R₅, R₆ et R₇ sont tels que définis au niveau du groupe R;
ou bien R₃ et R₄ forment ensemble, avec les atomes de carbone du noyau phényle auquel ils sont attachés, un cycle à 6 chaînons comportant un atome d'azote et un autre hétéroatome tel que l'oxygène et
L représente :
   un radical alkylène linéaire ou ramifié de 2 à 25 atomes de carbone, un radical alkényle linéaire ou ramifié de 2 à 25 atomes de carbone, ou un groupe alkynyle linéaire ou ramifié de 2 à 25 atomes de carbone ;
   ou un radical choisi parmi les formules: 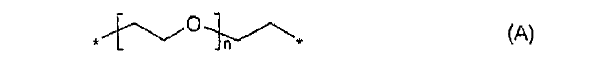 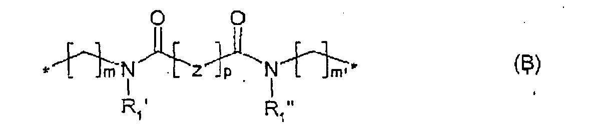 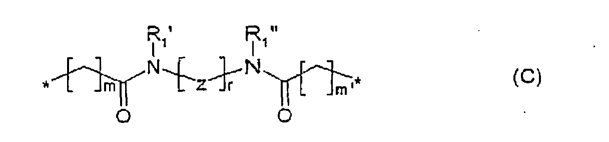 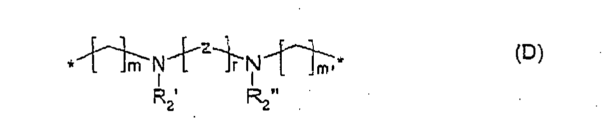 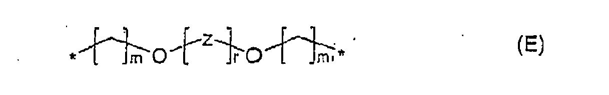 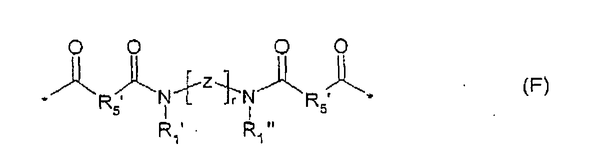 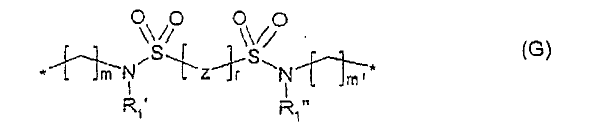 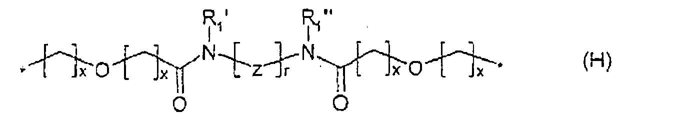
dans lesquelles
   * indique l'atome de connexion de L avec l'unité monomère M sur l'un des substituants R, R₁ ou R₂ ;
Z représente une liaison ou un radical carbonyle ou un radical alkylène linéaire, ramifié ou cyclique de 1 à 6 atomes de carbone, éventuellement subsituté par 1 ou 2 radicaux carbonyl, ou bien un radical
   - -[CH₂]ₛ-[-CH-(CH₂)_{q}-OR₃']-[CH₂]ₛ-
   - -[CH₂]ₛ-[-CH-(CH₂)_{q}-N R₃'R₄']-[CH₂]ₛ-
   - -[CH₂-CH₂-O]ₜ-CH₂-CH₂-
   - phényle ou alkylphénylalkyle, le groupe phényle étant éventuellement substitué par un ou plusieurs radicaux alcoxy de 1 à 5 atomes de carbone, ou
   - hétéroaryle ou alkylhétéroarylalkyle, le groupe hétéroaryle étant éventuellement substitué par un ou plusieurs radicaux alcoxy de 1 à 5 atomes de carbone,
n représente un nombre entier de 1 à 7
m et m' sont identiques ou différents et représentent un nombre entier de 0 à 8
p représente un nombre entier de 0 à 11
r représente un nombre entier de 1 à 11
q représente un nombre entier de 0 à 5
s représente un nombre entier de 0 à 5
t représente un nombre entier de 0 à 5
x représente un nombre entier de 1 à 5
m, m', n, p, r, s, t, x étant tels que le nombre de chaînons du groupe de liaison L n'excède pas 25,
R₁' et R₁" identiques ou différents représentent un atome d'hydrogène, un radical alkyle linéaire ou ramifié de 1 à 5 atomes de carbone,
R₂' et R₂" identiques ou différents représentent un atome d'hydrogène ou un radical alkyle linéaire ou ramifié de 1 à 5 atomes de carbone ou un radical benzyle ou un groupe sulfate,
R₁' et R₁" ainsi que R₂' et R₂" pouvant être éventuellement liés pour former un cycle,
R₃' et R₄' identiques ou différents représentent chacun un atome d'hydrogène, un radical alkyle linéaire ou ramifié de 1 à 5 atomes de carbone ou un radical benzyle, ou un groupe sulfate,
R₅' représente un radical alkyle linéaire ou ramifié de 1 à 5 atomes de carbone.
à l'état de base ou sel d'addition à un acide ou à une base, ainsi qu'à l'état d'hydrate ou de solvat.

La présente invention a particulièrement pour objet des composés tels que définis plus haut, comprenant l'unité monomère de formule M dans laquelle :

X = C-R₂ ;

R sur les positions 6, 7 ou 8 de l'indolizine représente un atome d'hydrogène, un atome d'halogène, un radical hydroxy ou un radical de formule :
- -COOR₅
- -CO-NR₆R₇
- -O-Alk
- -O-Alk-CO₂R₅
- -O-Alk-NR₆R₇
- -NR₆R₇
- -NH-SO₂-Alk
- -NH-CO-Alk, ou
- -NH-CO₂-Alk
   dans lesquels :
   - R₅ représente un atome d'hydrogène, un radical alkyle linéaire ou ramifié de 1 à 5 atomes de carbone ou un radical benzyle ;
   - R₆ et R₇ identiques ou différents représentent chacun un atome d'hydrogène, un radical alkyle linéaire ou ramifié de 1 à 5 atomes de carbone ou un radical benzyle,
   - Alk représente un radical alkyle linéaire ou ramifié de 1 à 5 atomes de carbone ou un radical alkylène linéaire ou ramifié de 1 à 5 atomes de carbone ;
   - Ph représente un radical phényle éventuellement substitué par un ou plusieurs atomes d'halogène, par un ou plusieurs radicaux hydroxy, par un ou plusieurs radicaux alcoxy linéaire ou ramifié de 1 à 5 atomes de carbone, ou par un ou plusieurs radicaux -COOR₅;

R₁ représente un atome d'hydrogène, un atome d'halogène, un radical hydroxy, un radical cyano ou un radical de formule :
- -CO₂R₅
- -CO-NR₆R₇
- -CO-NH-Alk-CO₂R₅
- -CO-NH-Ph
- -O-Alk
- -O-Alk-CO₂R₅
- -O-Alk-CO-NR₆R₇
- -O-Alk-NR₆R₇
- -O-Alk-OR₅
- -O-Alk-Ph
- -O-Ph
- -NR₆R₇
- -NH-SO₂-Alk
- -NH-CO-Alk
- -NH-CO-Alk-CO₂R₅
- -NH-CO-Alk-CO-NR₆R₇
- -NH-CO₂-Alk
- -NH-CO-Ph, ou
- un radical aryle ou hétéroaryle de 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, ou par un ou plusieurs radicaux -CO₂R₅ ou -CO-NR₆R₇ dans lesquels Alk, Ph, R₅, R₆ et R₇ sont tels que définis au niveau du groupe R;

R₂ représente un radical alkyle linéaire ou ramifié de 1 à 5 atomes de carbone, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone ;

R₃ et R₄ identiques ou différents représentent chacun un atome d'hydrogène, un radical hydroxy, un radical alcoxy de 1 à 5 atomes de carbone, un radical amino, un radical nitro, un radical de formule :
- -NR₆R₇
- -NH-CO-Alk
- -NH-SO₂-Alk
- -CO₂R₅
- -CO-NR₆R₇, ou
- -CO-NHOH
dans lesquels Alk, Ph, R₅, R₆ et R₇ sont tels que définis au niveau du groupe R;

La présente invention a plus particulièrement pour objet des composés tels que définis plus haut, comprenant l'unité monomère de formule M dans laquelle :

X=C-R₂;

R sur les positions 6, 7 ou 8 de l'indolizine représente un atome d'hydrogène, un radical hydroxy, un radical carboxy ou un radical de formule :
- -O-Alk
- -O-Alk-CO₂R₅
- -O-Alk-CO-NR₆R₇
- -O-Alk-NR₆R₇
- -O-Alk-Ph
- -NR₆R₇
- -NH-SO₂-Alk, ou
- -NH-CO-Alk
   dans lesquels :
   - R₅ représente un atome d'hydrogène, un radical alkyle de 1 à 5 atomes de carbone ou un radical benzyle ;
   - R₆ et R₇ identiques ou différents représentent chacun un atome d'hydrogène, un radical alkyle linéaire ou ramifié de 1 à 5 atomes de carbone ou un radical benzyle ;
   - Alk représente un radical alkyle ou un radical alkylène linéaire ou ramifié de 1 à 5 atomes de carbone ;
   - Ph représente un radical phényle éventuellement substitué par un ou plusieurs atomes d'halogène, par un ou plusieurs radicaux hydroxy, par un ou plusieurs radicaux alcoxy de 1 à 5 atomes de carbone, ou par un ou plusieurs radicaux -COOR₅;

R₁ représente un atome d'halogène, un radical hydroxy, un radical carboxy ou un radical de formule :
- -O-Alk
- -O-Alk-CO₂R₅
- -O-Alk-Ph
- -O-Ph
- -NR₆R₇
- -NH-CO-Ph, ou
- un radical aryle ou hétéroaryle à 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, par un ou plusieurs radicaux -CO₂R₅ ou -CO-NR₆R₇ dans lesquels Alk, Ph, R₅, R₆ et R₇ sont tels que définis au niveau du groupe R;

R₂ représente un radical alkyle de 1 à 5 atomes de carbone ou un radical cycloalkyle de 3 à 6 atomes de carbone;

R₃ et R₄ identiques ou différents représentent chacun un radical alcoxy linéaire ou ramifié de 1 à 5 atomes de carbone, un radical amino, un radical carboxy, un radical hydroxy, un radical de formule CO-NR₆R₇, ou un radical de formule -NH-SO₂-Alk, dans lesquels Alk, R₆ et R₇ sont tels que définis au niveau du groupe R.

La présente invention a plus particulièrement pour objet des composés tels que définis plus haut, comprenant l'unité monomère de formule M dans laquelle ;

X = C-R₂ ;

R sur les positions 6 ou 8 de l'indolizine représente un atome d'hydrogène ou un radical hydroxy ;
R₁ représente un radical hydroxy, ou un radical de formule :
- -O-Alk
- -O-Alk-Ph
- -NR₆R₇, ou
- -NH-CO-Ph
dans lesquels Alk, Ph, R₆ et R₇ sont tels que définis plus haut,
R₂ représente un radical alkyle de 1 à 5 atomes de carbone ;
R₃ représente un radical alcoxy linéaire ou ramifié de 1 à 5 atomes de carbone ou un radical carboxy ;
R₄ représente un radical amino.

La présente invention a particulièrement pour objet des composés tels que définis plus haut, comprenant l'unité monomère de formule M dans laquelle :

X=N;

R sur les positions 5, 6, 7 ou 8 de l'imidazo[1,5-a]pyridine représente un atome d'hydrogène, un atome d'halogène, un radical alkyle linéaire ou ramifié de 1 à 5 atomes de carbone, un radical hydroxy ou un radical de formule :
- -CO₂R₅
- -CO-NR₆R₇
- -O-Alk
- -O-Alk-CO₂R₅
- -O-Alk-CO-NR₆R₇
- -O-Alk-NR₆R₇
- -O-Alk-Ph
- -NR₆R₇
- -NH-SO₂-Alk
- -NH-CO-Alk, ou
- -NH-CO₂-Alk
   dans lesquels :
   - R₅ représente un atome d'hydrogène, un radical alkyle linéaire ou ramifié de 1 à 5 atomes de carbone ou un radical benzyle ;
   - R₆ et R₇ identiques ou différents représentent chacun un atome d'hydrogène, un radical alkyle linéaire ou ramifié de 1 à 5 atomes de carbone ou un radical benzyle,
   - Alk représente un radical alkyle linéaire ou ramifié de 1 à 5 atomes de carbone ou un radical alkylène linéaire ou ramifié de 1 à 5 atomes de carbone ;
   - Ph représente un radical phényle éventuellement substitué par un ou plusieurs atomes d'halogène, par un ou plusieurs radicaux hydroxy, par un ou plusieurs radicaux alcoxy linéaires ou ramifiés de 1 à 5 atomes de carbone, ou par un ou plusieurs radicaux -COOR₅;

R₁ représente un atome d'hydrogène, un atome d'halogène, un radical cyano ou un radical de formule :
- -CO₂R5
- -CO-NH-Ph
- -NR₆R₇
- -NH-SO₂-Alk
- -NH-CO-Alk
- -NH-CO₂-Alk
- -NH-CO-Ph, ou
- un radical aryle ou hétéroaryle à 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, par un ou plusieurs radicaux -CO₂R₅ ou -CO-NR₆R₇, dans lesquels Alk, Ph, R₅, R₆ et R₇ sont tels que définis au niveau du groupe R;

R₃ et R₄ identiques ou différents représentent chacun un atome d'hydrogène, un radical hydroxy, un radical alcoxy linéaire ou ramifié de 1 à 5 atomes de carbone, un radical amino, un radical nitro, ou un radical de formule :
- -NR₆R₇
- -NH-CO-Alk
- -NH-SO₂-Alk
- -CO₂R₅
- -CO-NR₆R₇, ou
- -CO-NHOH
dans lesquels Alk, Ph, R5, R6 et R7 sont tels que définis au niveau du groupe R;
ou R3 et R4 forment ensemble, avec les atomes de carbone du noyau phényle auquel ils sont rattachés, un cycle carboné à 6 chaînons, comportant un atome d'azote et un autre hétéroatome tel que l'oxygène.

La présente invention a plus particulièrement pour objet des composés tels que définis plus haut, comprenant l'unité monomère de formule M dans laquelle :

X=N;

R sur les positions 6, 7 ou 8 de l'imidazo[1,5-a]pyridine représente un atome d'hydrogène, un atome d'halogène, un radical hydroxy, un radical carboxy ou un radical de formule :
- -CO-NR₆R₇
- -O-Alk
- -O-Alk-CO₂R₅
- -O-Alk-Ph
- -NR₆R₇
- NH-SO₂-Alk, ou
- -NH-CO-Alk
   dans lesquels :
   - R₅ représente un atome d'hydrogène, un radical alkyle linéaire ou ramifié de 1 à 5 atomes de carbone ou un radical benzyle ;
   - R₆ et R₇ identiques ou différents représentent chacun un atome d'hydrogène, un radical alkyle linéaire ou ramifié de 1 à 5 atomes de carbone ou un radical benzyle,
   - Alk représente un radical alkyle linéaire ou ramifié de 1 à 5 atomes de carbone ou un radical alkylène linéaire ou ramifié de 1 à 5 atomes de carbone ;
   - Ph représente un radical phényle éventuellement substitué par un ou plusieurs atomes d'halogène, par un ou plusieurs radicaux hydroxy, par un ou plusieurs radicaux alcoxy linéaire ou ramifié de 1 à 5 atomes de carbone, ou par un ou plusieurs radicaux -COOR₅;

R₁ représente un atome d'hydrogène, un atome d'halogène, un radical carboxy ou un radical de formule :
- NR₆R₇
- -NH-SO₂-Alk
- -NH-CO-Alk
- -NH-CO-Ph, ou
- un radical aryle ou hétéroaryle à 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaire ou ramifié de 1 à 5 atomes de carbone, ou par un ou plusieurs radicaux -CO₂R₅ ou -CO-NR₆R₇,
dans lesquels Alk, Ph, R₅, R₆ et R₇ sont tels que définis au niveau du groupe R;

R₃ et R₄ identiques ou différents représentent chacun un radical alcoxy linéaire ou ramifié de 1 à 5 atomes de carbone, un radical amino, un radical carboxy, un radical hydroxy, ou un radical de formule CO-NR₆R₇ ou -NH-SO₂-Alk.

La présente invention a plus particulièrement pour objet des composés tels que définis plus haut, comprenant l'unité monomère de formule M dans laquelle :

X=N,

R sur la position 8 de l'imidazo[1,5-a]pyridine représente un atome d'hydrogène, un radical hydroxy, ou un radical carboxy,
R₁ représente un atome d'hydrogène, un radical de formule -NH-CO-Ph, ou un radical aryle ou hétéroaryle à 5 ou 6 atomes choisis parmi C, N, O, S, éventuellement substitué par un ou plusieurs radicaux -CO₂R₅,
dans lesquels Alk, Ph, R₅ sont tels que définis plus haut,
R₃ représente un radical alcoxy linéaire ou ramifié de 1 à 5 atomes de carbone ou un radical carboxy,
R₄ représente un radical amino.

Dans le cadre de la présente invention on entend par :
- un atome d'halogène : un fluor, un chlore, un brome ou un iode ;
- un hétéroatome : un atome d'azote, d'oxygène ou de soufre ;
- un groupe alkyle linéaire ou ramifié: un groupe aliphatique saturé linéaire ou ramifié. A titre d'exemples, on peut citer les groupes méthyle, éthyle, propyle, isopropyle, butyle, iso-butyle, méthyl-cyclopropyle, pentyle, 2,2-diméthylpropyle, sec-butyle, tert-butyle ;
- un groupe alkylène linéaire ou ramifié: un groupe alkyle tel que précédemment, qui est divalent saturé, linéaire ou ramifié. A titre d'exemple, on peut citer les radicaux méthylène, éthylène, propylène ;
- un groupe alkèlyne linéaire ou ramifié : un groupe aliphatique linéaire ou ramifié contenant une ou plusieurs insaturations éthyléniques ;
- un groupe alkynyle linéaire ou ramifié : un groupe aliphatique linéaire ou ramifié contenant une ou plusieurs insaturations acétyléniques ;
- un groupe alcoxy linéaire ou ramifié: un groupe -O-alkyle, où le groupe alkyle est tel que défini ci-dessus ;
- un cycle ou un groupe cycloalkyle : un groupe alkyle cyclique comprenant de 3 à 6 atomes de carbone et dont tous les atomes de carbones sont engagés dans le cycle. On peut citer par exemple les groupes cyclopropyle, cyclobutyle, cyclopentyle, cyclohexyle ;
- un polycycle : groupe comprenant deux ou plusieurs groupes cycloalkyles tels que définis précédemment
- un hétérocycle : un groupe cycloalkyle tel que défini précédemment et comprenant un ou plusieurs hétéroatomes tels que O, N et/ou S ;
- un groupe aryle : un groupe aromatique monocyclique, par exemple un groupe phényle;
- un groupe hétéroaryle : un groupe aromatique cyclique comprenant 5 ou 6 atomes et comprenant un ou plusieurs hétéroatomes tels que précédemment définis. A titre d'exemple de groupes hétéroaryles, on peut citer un thiényle, furyle, pyrrolyle, imidazolyle, pyridinyle.

### Les dimères agonistes.

Les agonistes de formule M₁-L-M₂ selon l'invention comprennent deux unités monomères de formule générale M, appelées M₁ et M₂ qui peuvent être identiques ou différentes, choisies comme ayant chacune une activité antagoniste des FGFRs. Les composés de formule M₁-L-M₂ peuvent exister à l'état de bases ou salifiés par des acides ou des bases pharmaceutiquement acceptables. De tels sels d'addition font également partie de l'invention.
Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

### Le groupe de liaison.

L représente un groupe de liaison qui lie M₁ et M₂ de façon covalente de telle façon que la distance entre les deux monomères M₁ et M₂ permette la dimérisation de deux récepteurs FGFs. Ledit groupe de liaison comprend de préférence de 1 à 25 chaînons. Ledit groupe de liaison L comprend plus particulièrement de 8 à 20 chaînons. On entend par chaînons seulement les liaisons entre atomes qui permettent de relier les unités monomères M₁ et M₂.

Le groupe de liaison L est caractérisé par une flexibilité qui permet à chaque unité monomère du composé de formule M₁-L-M₂ d'établir le contact avec les sites de liaison extracellulaires des récepteurs transmembranaires FGFRs.

L est attaché d'une part à une unité monomère de formule M₁ par un atome placé sur l'un quelconque des substituants R, R₁ ou R₂ et attaché d'autre part à l'autre unité monomère de formule M₂ par un atome placé sur l'un quelconque des substituants R, R₁ ou R₂, avec M₁ et M₂ identiques ou différents.

La présente invention a plus particulièrement pour objet des composés tels que définis plus haut caractérisés en ce que ;
L relie les 2 unités monomères M₁ et M₂ par le radical R₁ ou ;
L relie les 2 unités monomères M₁ et M₂ par le radical R₂ ou ;
L relie les 2 unités monomères M₁ et M₂ par le radical R en sa position 8 ou ;
L relie les 2 unités monomères M₁ et M₂ par le radical R en sa position 7 ou ;
L relie les 2 unités monomères M₁ et M₂ par le radical R en sa position 6 ou ;
L relie les 2 unités monomères M₁ et M₂ d'une part par le radical R en sa position 8 et d'autre part par le radical R en sa position 7 ou 6, ou;
L relie les 2 unités monomères M₁ et M₂ d'une part par le radical R en sa position 7 et d'autre part par le radical R en sa position 6, ou ;
L relie les 2 unités monomères M₁ et M₂ d'une part par le radical R₂ et d'autre part par le radical R₁ ou;
L relie les 2 unités monomères M₁ et M₂ d'une part par le radical R₁ et d'autre part par le radical R en sa position 8.

Les atomes de connexion qui sont situés sur l'un quelconque des substituants R, R₁ ou R₂ de l'unité monomère de formule M peuvent être représentés par des atomes O, N, C, S.

Les jonctions entre L et les unités monomères peuvent être représentées par des liaisons C-O, C-N, C-C ou C-S. Ces liaisons peuvent être choisis parmi des groupes fonctionnels tels que les esters, les amides, les éthers, les carbamates, les urées, les sulfonamides, les thioéthers, les sulfones, les thiourées ou encore par des liaisons C-C alkyl-alkyl, alkyl-aryl ou aryl-aryl.

Les groupes de liaison L adaptés à l'invention peuvent être choisis parmi les structures du type des radicaux aliphatiques pouvant être linéaires ou ramifiés et éventuellement interrompus par un ou plusieurs hétéroatomes tels que O, N et/ou S, un ou plusieurs cycles, un ou plusieurs polycycles, un ou plusieurs hétérocycles (tels que pipérazine), un ou plusieurs aryles (tels que phényle) ou hétéroaryles (tels que pyridine).
Les groupes de liaison L peuvent éventuellement comprendre une ou plusieurs fonctions telles qu'amide, urée, thiourée, carbamate, carbonate, sulfonamide, thiocarbamate, ester, thioester, cétone, *N-*sulfamate, guanidine, sulfone, et/ou sulfoxide.
Les ramifications dans le groupe de liaison L peuvent comprendre elles-même des radicaux aliphatiques pouvant être linéaires ou ramifiés et éventuellement interrompus par un ou plusieurs hétéroatomes tels que O, N et/ou S, un ou plusieurs cycles, un ou plusieurs polycycles, un ou plusieurs hétérocycles, un ou plusieurs aryles ou hétéroaryles, et/ou éventuellement une ou plusieurs fonctions telles qu'amide, urée, thiourée, carbamate, sulfonamide, thiocarbamate, ester, thioester, cétone, hydroxyle, *O-*sulfate, *N-*sulfamate, guanidine, sulfone, et/ou sulfoxide.

Les groupes de liaison L peuvent être plus particulièrement choisis parmi les radicaux suivants :
un radical alkylène linéaire ou ramifié de 2 à 25 atomes de carbone, un groupe alkényle linéaire ou ramifié de 2 à 25 atomes de carbone, ou un groupe alkynyle linéaire ou ramifié de 2 à 25 atomes de carbone ;
ou un radical choisi parmi les formules : 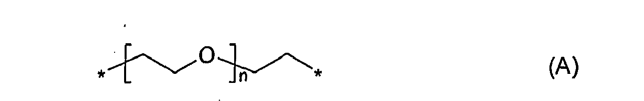 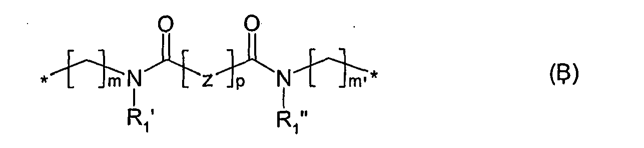 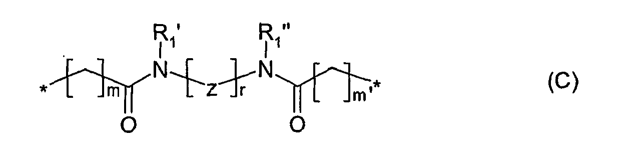 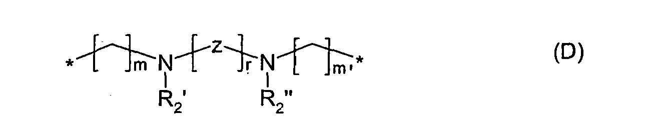 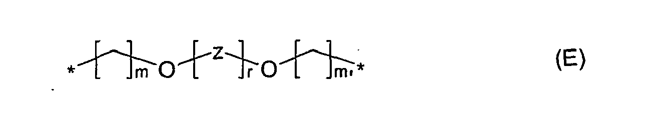 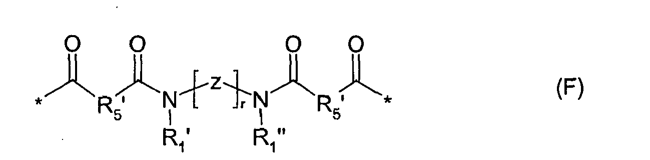 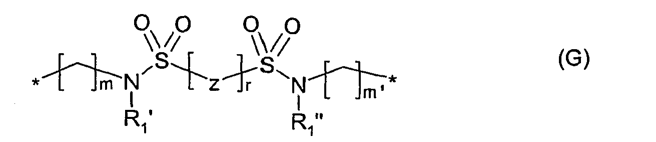 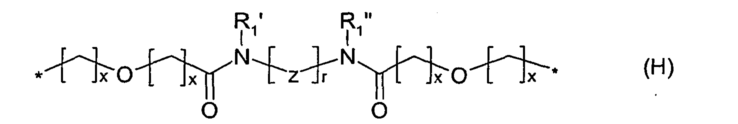 dans lesquelles
* indique l'atome de connexion de L avec l'unité monomère M sur l'un des substituants R, R₁ ou R₂ ;
   Z représente une liaison ou un radical carbonyle ou un radical alkylène linéaire, ramifié ou cyclique, saturé, insaturé ou partiellement insaturé, de 1 à 6 atomes de carbone, éventuellement subsituté par 1 ou 2 radicaux carbonyl, ou bien un radical
   - -[CH₂]ₛ-[-CH-(CH₂)_{q}-OR₃']-[CH₂]ₛ-
   - -[CH₂]ₛ-[-CH-(CH₂)_{q}-N R₃'R₄']-[CH₂]ₛ-
   - -[CH₂-CH₂-O]ₜ-CH₂-CH₂-
   - phényle ou alkylphénylalkyle, le groupe phényle étant éventuellement substitué par un ou plusieurs radicaux alcoxy de 1 à 5 atomes de carbone, ou
   - hétéroaryle ou alkylhétéroarylalkyle, le groupe hétéroaryle étant éventuellement substitué par un ou plusieurs radicaux alcoxy de 1 à 5 atomes de carbone,

   n représente un nombre entier de 1 à 7
   m et m' sont identiques ou différents et représentent un nombre entier de 0 à 8
   p représente un nombre entier de 0 à 11
   r représente un nombre entier de 1 à 11
   q représente un nombre entier de 0 à 5
   s représente un nombre entier de 0 à 5
   t représente un nombre entier de 0 à 5
   x représente un nombre entier de 1 à 5
   m, m', n, p, r, s, t, x étant tels que le nombre de chaînons du groupe de liaison L n'excède pas 25,
   R₁' et R₁" identiques ou différents représentent un atome d'hydrogène, un radical alkyle linéaire ou ramifié de 1 à 5 atomes de carbone,
   R₂' et R₂" identiques ou différents représentent un atome d'hydrogène ou un radical alkyle linéaire ou ramifié de 1 à 5 atomes de carbone ou un radical benzyle ou un groupe sulfate,
   R₁' et R₁" ainsi que R₂' et R₂" pouvant être éventuellement liés pour former un cycle,
   R₃' et R₄' identiques ou différents représentent chacun un atome d'hydrogène, un radical alkyle linéaire ou ramifié de 1 à 5 atomes de carbone ou un radical benzyle, ou un groupe sulfate,
   R₅' représente un radical alkyle linéaire ou ramifié de 1 à 5 atomes de carbone.

### Les unités monomères

Les unités monomères de formule M telle que définie plus haut sont des ligands des récepteurs FGFs possédant une activité antagoniste vis à vis du FGF2.

On se réfèrera pour la préparation des monomères de formule M dans laquelle A est - CO- ou -SO₂-, X est C-R₂ à WO2003084956 et WO2005028476, et X est N à WO2006097625.

La synthèse des dimères s'effectue par dimérisation de composés monomères de formule générale M, M étant un composé antagoniste lui-même possédant un atome de connexion libre ou un composé antagoniste dont l'un des atomes de connexion possible est libéré soit par une réaction chimique à partir de l'antagoniste lui-même soit parce que c'est un intermédiaire de synthèse du composé antagoniste.

La présente invention concerne également un procédé de préparation des dimères de formule M₁-L-M₂ comprenant la réaction d'au moins un réactif d'une unité monomère de formule M-W avec un réactif de formule U-L-U' où M et L ont la même signification que précédemment,
U et U' pouvant être identiques ou différents,
W étant situé sur l'un des substituants R, R₁ ou R₂ tels que définis plus haut,

W et U ainsi que W et U' représentent chacun un groupe fonctionnel capable de réagir l'un sur l'autre pour former une liaison covalente de type C-C, C-O, C-N, C-C ou C-S. Ces liaisons peuvent être choisis parmi des groupes fonctionnels tels que les esters, les amides, les éthers, les carbamates, les urées, les sulfonamides, les thioéthers, les sulfones, les thiourées ou encore par des liaisons C-C alkyl-alkyl, alkyl-aryl ou aryl-aryl.
W, U et U' peuvent être par exemple un groupe amino, hydroxyle, carboxy, chloroformiate, isocyanate, thiol, thioisocyanate, amido, carbamate, halogène, chlorure de sulfonyle, chlorure d'acide, fluorure d'acide, alcène, alcyne ou un réactif organométallique tel qu'un ester boronique ou un acide boronique.

Les réactifs U-L-U' décrits ci-dessus sont disponibles commercialement ou peuvent être préparés par des méthodes décrites dans la littérature ou par des méthodes choisies parmi celles connues de l'homme de l'art. Les préparations des réactifs U-L-U' utilisés dans la préparation des dimères de la présente invention sont citées ou décrites dans la partie expérimentale (exemples R1 à R66).

Les réactifs U-L-U' qui peuvent être utilisés dans la présente invention sont des agents alkylants connus de l'homme du métier tels que des dérivés dihalogénés, des agents acylants tels que diacides carboxyliques activés en présence d'un agent de couplage, dichlorures d'acide, difluorures d'acide, dichloroformiates, diisocyanates, dithisocyanates, des réactifs organométalliques tels que des diesters boroniques ou diacides boroniques, ou des agents sulfonylants tels que des dichlorures de sulfonyle. Lesdits agents de couplage peuvent être notamment des agents de couplage de type phosphonium tels que l'hexafluorophosphate de (benzotriazol-1-yloxy) tripyrrolidinophosphonium (PyBOP), l'hexafluorophosphate de benzotriazol-1-yloxy-tris(diméthylamino)phosphonium (BOP), le tétrafluorborate de *O-*benzotriazol-1-yl-*N*,*N*,*N*',*N*'-tétraméthyluronium (TBTU), ou des agents de couplage de type carbodiimide tel que le chlorhydrate de 1-(3-diméthylaminopropyl)-3-éthylcarbodiimide (EDCI).

Par exemple, on prépare les diacides carboxyliques à partir d'un acide carboxylique m étant tel que défini ci-dessus, tel que de l'acide {4-[(benzyloxy)carbonyl]phénoxy}acétique (décrit dans la demande de brevet WO2001060813) ou de l'acide [3-(éthoxycarbonyl)phénoxy]acétique (A. Banerjee, M. M. Adak, S. Das, S. Banerjee, S. Sengupta, Indian Chem. Soc., 1987, 64, 1, 34-37) que l'on active sous forme de chlorure d'acide ou avec un agent de couplage tel que BOP en présence d'une base faible telle que la triéthylamine, puis que l'on fait réagir avec une diamine R₁', R₁", Z et r étant tels que définis plus haut. Les diacides carboxyliques cibles sont obtenus après saponification.

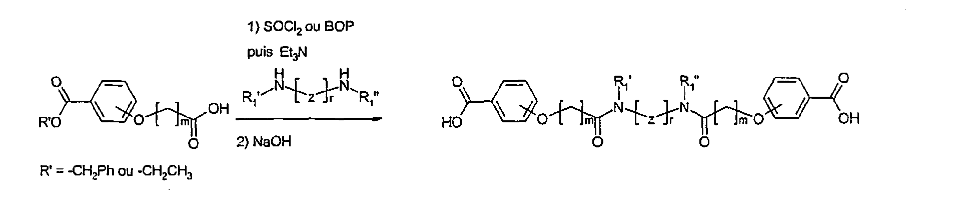

Les diacides carboxyliques sont aussi obtenus par réaction d'une amine avec R₁' et m étant tels que définis ci-dessus, telle que le 3-(2-aminoéthoxy)benzoate d'éthyle avec soit un diacide carboxylique avec Z, p étant tels que définis plus haut, en présence d'un agent de couplage tel que BOP ou EDCI et d'une base faible telle que la triéthylamine, soit avec un dichlorure de sulfonyle avec Z, r étant tels que définis plus haut, en présence d'une base telle que la triéthylamine. Les diacides carboxyliques cibles sont obtenus après saponification.

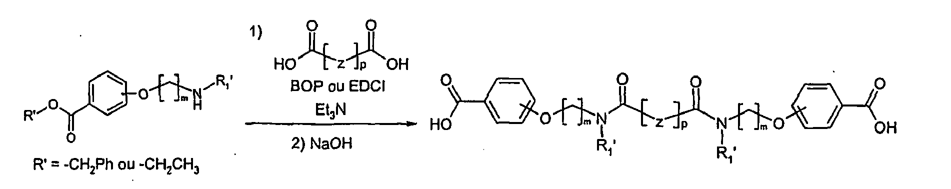

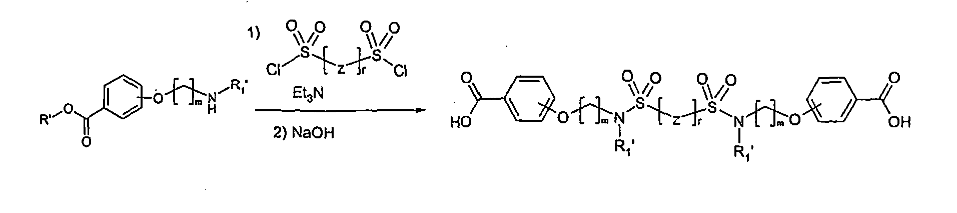

Les diacides carboxyliques sont aussi obtenus par alkylation d'une diamine avec R₂', R₂", Z et r étant tels que définis plus haut, avec un dérivé halogéné avec m étant tel que défini ci-dessus, tel que le 3-(2-iodoéthoxy)benzoate d'éthyle ou le 4-(2-iodoéthoxy)benzoate d'éthyle [CAS 56703-36-7] en présence d'une base telle que le carbonate de potassium. Les diacides carboxyliques cibles sont obtenus après saponification.

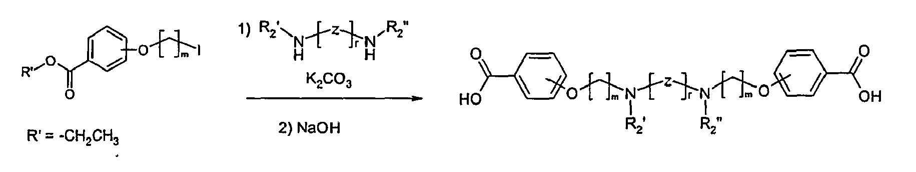

Les diacides carboxyliques sont aussi obtenus par réaction d'une diamine avec R₁', R₁", Z, r étant tels que définis plus haut, sur un anhydride avec R₅', x tels que défini plus haut, selon le protocole décrit dans R.E. Asay et al., J. Heterocyclic Chem., 1977, 14(1), 85-90).

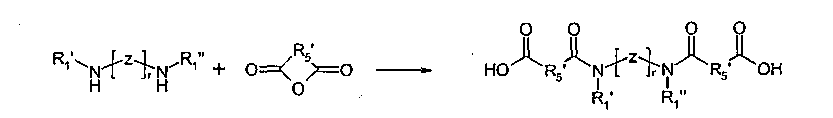

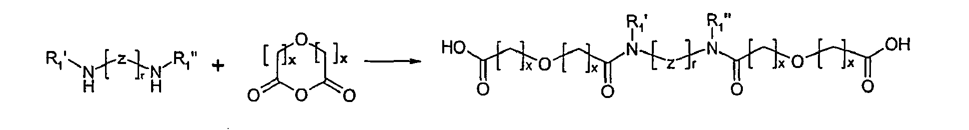

En jouant sur la stochiométrie des réactifs, on peut également préparer les diacides carboxyliques tels que ci-dessus avec un linker de formule (B), (C), (D), (G) dans lesquels m et m' sont différents.

Les diesters boroniques utilisés dans les réactions de dimérisation *via* un couplage de Suzuki (Synth.Commun., 1981, 11, 513) sont obtenus par alkylation d'un dérivé hydroxy tel que le 3-(4,4,5,5-tétraméthyl-1,3,2-dioxaborolan-2-yl)phénol ou le 4-(4,4,5,5-tétraméthyl-1,3,2-dioxaborolan-2-yl)phénol avec un dérivé dihalogéné en présence d'une base telle que le carbonate de potassium.

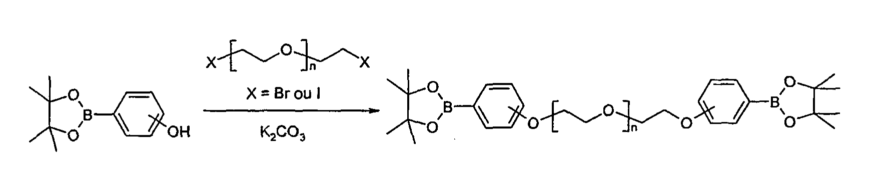

Les monomères M peuvent être dimérisés pour donner :
- des homodimères dans le cas où M₁ et M₂ sont identiques ainsi que leurs atomes de connexion avec le groupe de liaison L
- des hétérodimères dans le cas où M₁ et M₂ sont identiques et leurs atomes de connexion avec le groupe de liaison L sont différents, ainsi que dans le cas où M₁ et M₂ sont différents.

Les monomères M peuvent être dimérisés pour donner des homodimères ou des hétérodimères de formule générale M₁-L-M₂ *via* différentes voies de synthèse comme illustré dans les schémas qui suivent.

Les méthodes et schémas décrits ci-dessous s'appliquent et peuvent être généralisés pour les composés M-W avec A, X, R, R₁, R₂, R₃ et R₄ -tels que définis plus haut et le groupe de liaison L tels ques définis ci-dessus, sauf mention contraire.

**Méthode I)** Lorsque l'on veut dimériser un monomère M-W, dans lequel R ou R₁ représente ou possède une fonction W amino, on peut utiliser un tel monomère M-W pour réaliser une acylation en utilisant un agent diacylant tel qu'un diacide carboxylique activé en présence d'un agent de couplage adéquat tel que BOP ou PyBOP, ou un dichlorure d'acide ou un difluorure d'acide, et une base faible telle que la triéthylamine ou la pyridine.

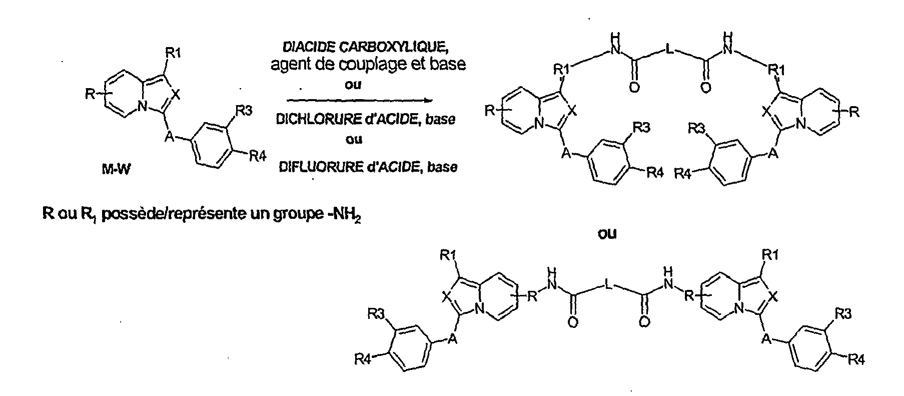

Illustrations de cas où R₁ = NH₂ :

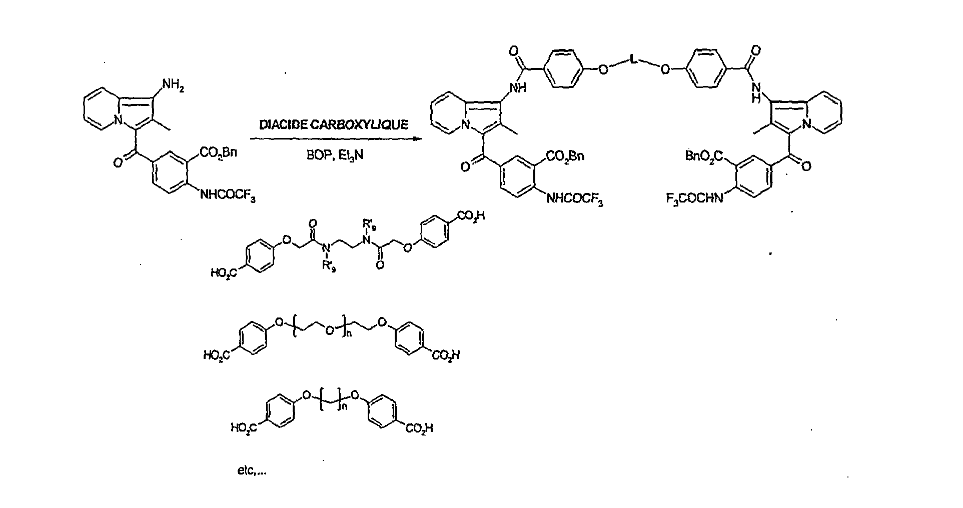

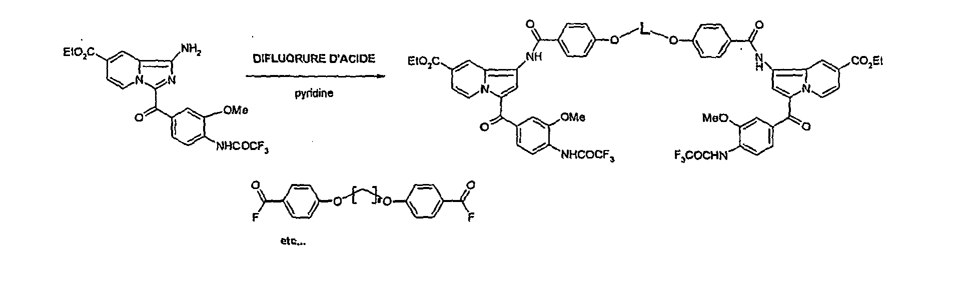

Illustration de cas où R₁ possède NH₂:

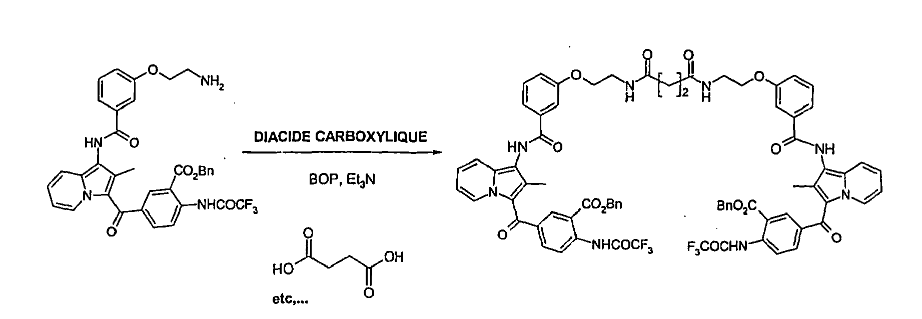

**Méthode II)** Lorsque l'on veut dimériser des unités monomères M-W, avec R, R₁, R₂, R₃ ou R₄ représentant ou posédant un acide carboxylique, et dans lequel R ou R₁ représente ou possède une fonction W amino, on peut faire réagir un tel monomère M-W pour avec un agent de silylation tel que le chlorure de triméthylsilyle et une base faible telle que la triéthylamine afin de protéger la fonction acide carboxylique sous forme d'ester de silyle et d'activer la fonction W amino sous forme d'amine silylée, puis réaliser un réaction d'acylation en utilisant un agent diacylant, tel qu'un diacide carboxylique activé en présence d'un agent de couplage adéquat tel que BOP ou PyBOP, ou un dichlorure d'acide ou un difluorure d'acide, et une base faible telle que la triéthylamine, suivie d'une hydrolyse en milieu acide.

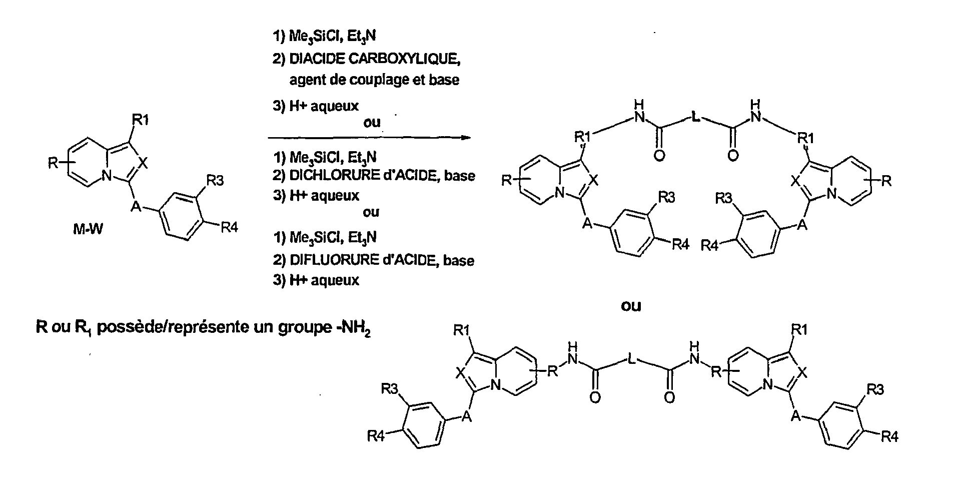

Illustrations de cas où M possède une fonction acide carboxylique et où R₁ = NH₂ :

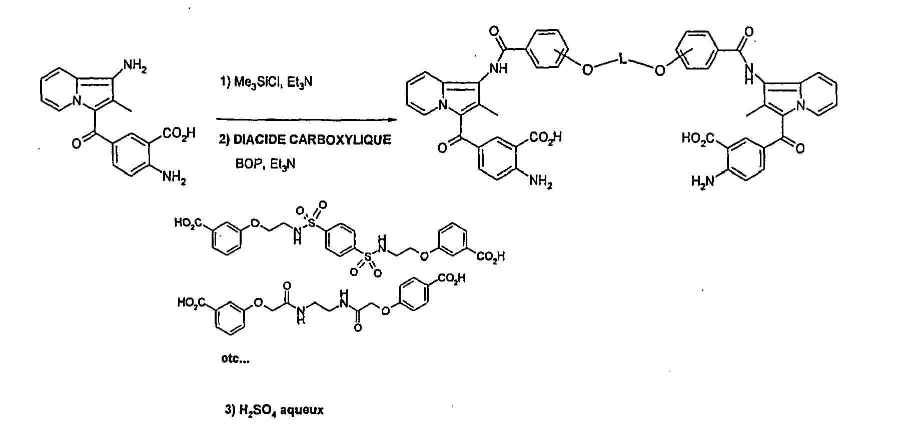

**Méthode III)** Lorsque l'on veut dimériser un monomère M-W, dans lequel R, R₁ ou R₂ représente ou possède une fonction W acide carboxylique, on peut utiliser un tel monomère M-W pour réaliser une acylation en utilisant, par exemple une diamine, un agent de couplage adéquat tel que BOP ou PyBOP ou TBTU, et une base faible telle que la triéthylamine : 1

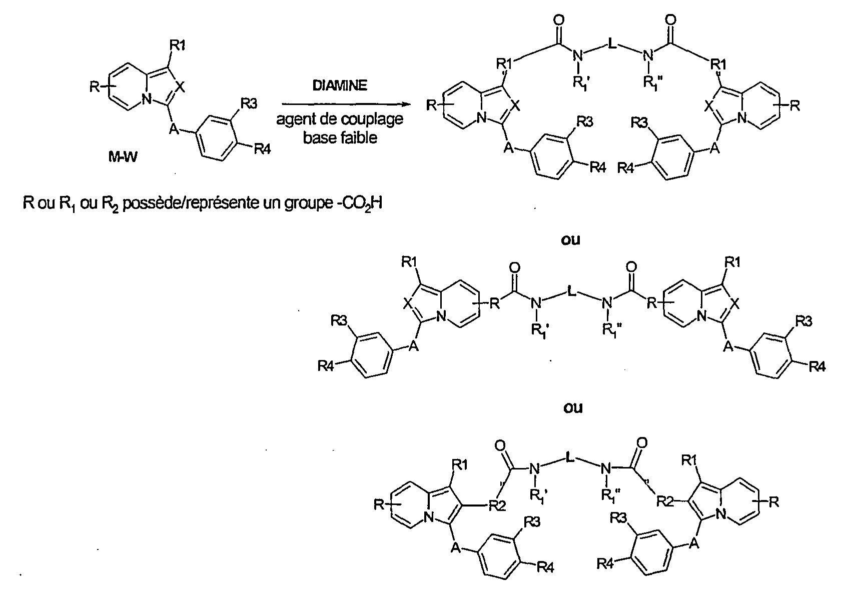

Illustrations de cas où R₁ possède -CO₂H :

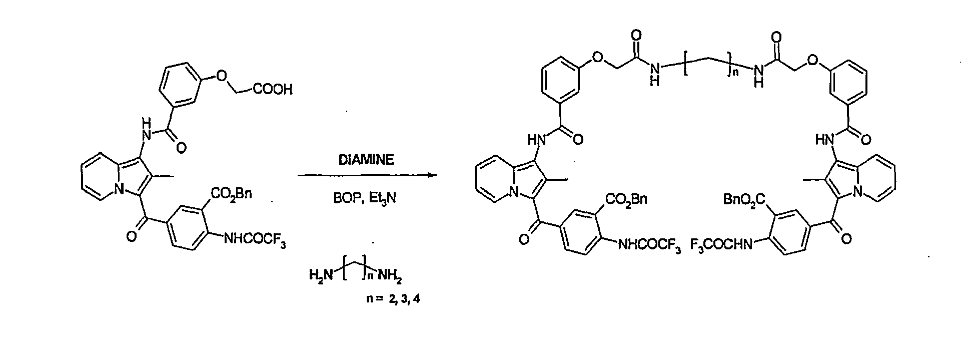

Illustration de cas où R possède -CO₂H :

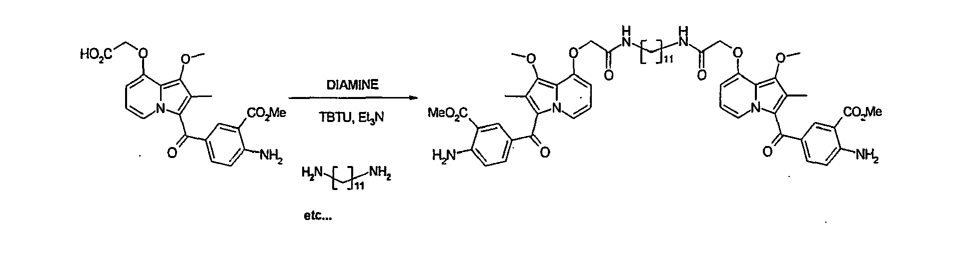

Illustration de cas où R = CO₂H :

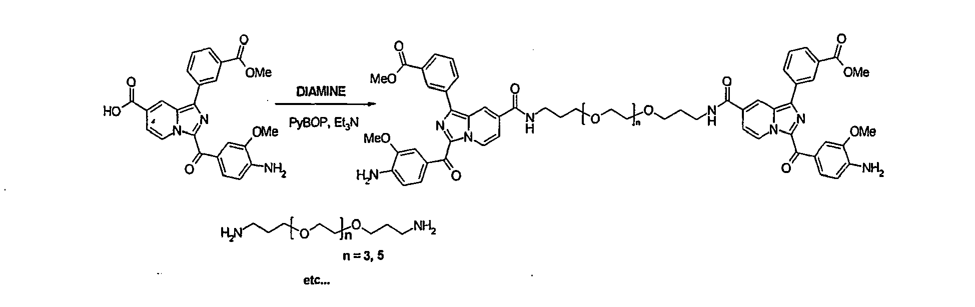

**Méthode IV)** Lorsque l'on veut dimériser un monomère M-W, dans lequel R, R₁ ou R₂ représente ou possède une fonction W hydroxy, amino, amido, carbamate ou sulfonamido, on peut utiliser un tel monomère M-W pour réaliser une alkylation en utilisant un dérivé dihalogéné et une base telle que l'hydrure de sodium ou l'hexaméthyldisilylamidure de potassium :

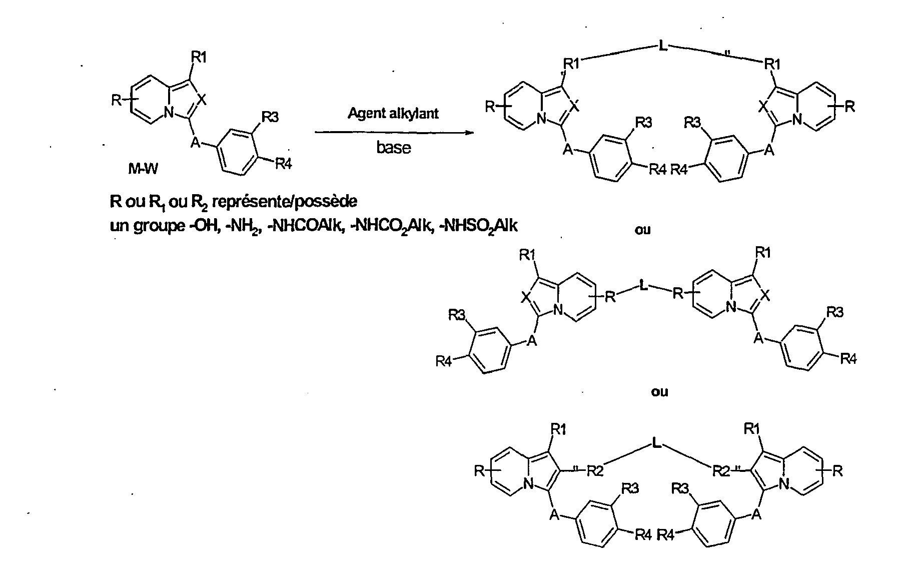

Ilustrations de cas où R₁ possède -OH :

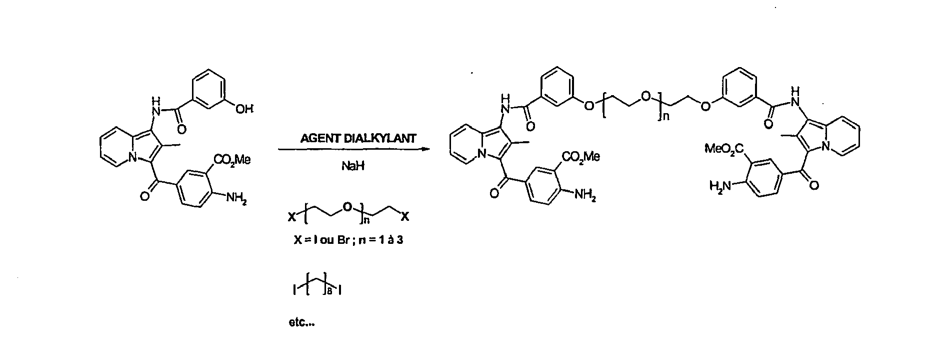

Illustrations de cas où R = -OH :

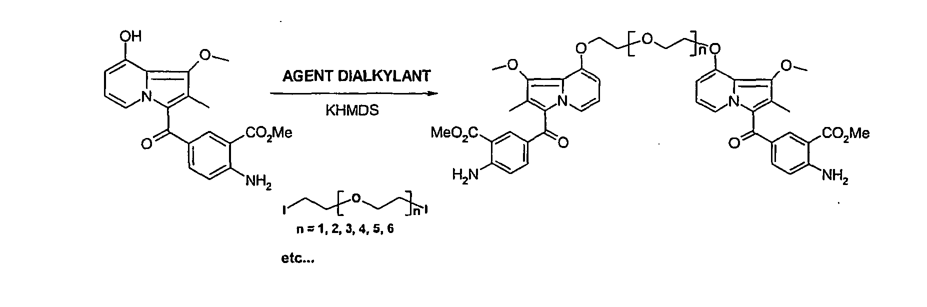

**Méthode V)** Lorsque l'on veut dimériser un monomère M-W, dans lequel R, R₁ ou R₂ représente ou possède un halogène, on peut utiliser un tel monomère M-W pour réaliser une réaction de couplage avec un catalyseur tel que le palladium, par exemple un couplage de SUZUKI (Synth.Commun., 1891, 11, 513) avec un diacide boronique ou un diester boronique adéquat en présence d'un catalyseur tel que le chlorure de palladium (dppf) et d'une base telle que le phosphate de potassium :

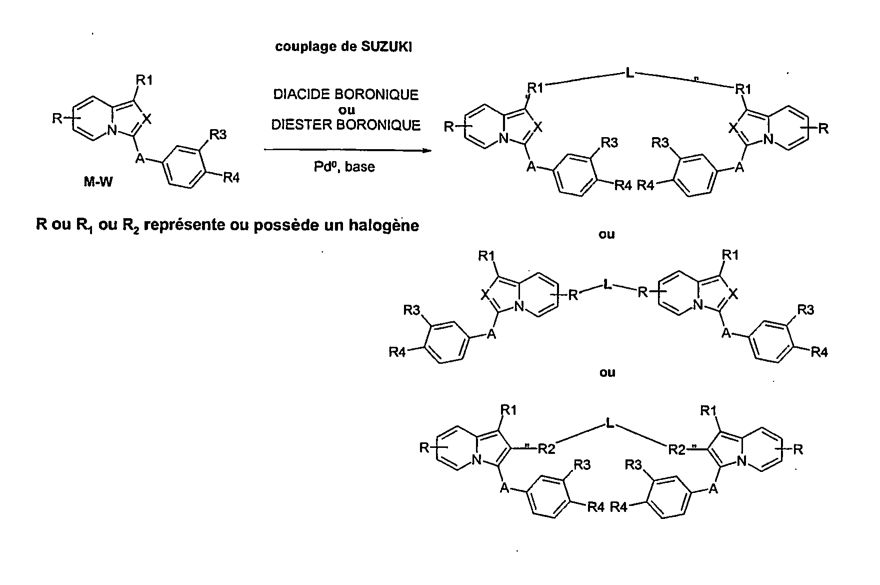

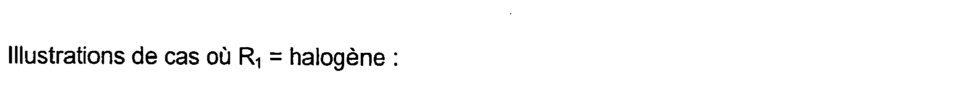

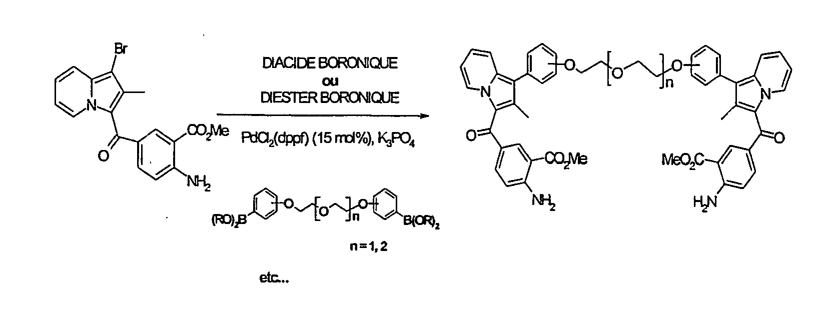

**Méthode VI)** On peut aussi obtenir un homodimère en réalisant les réactions décrites dans les méthodes ci-dessus sur un seul composé M₁-W (en jouant sur la stoechiométrie des réactifs M₁-W et U-L-U'), puis faire réagir l'intermédiaire M₁-L-U' obtenu avec un deuxième composé M₂-W (M₂ identique à M₁) :

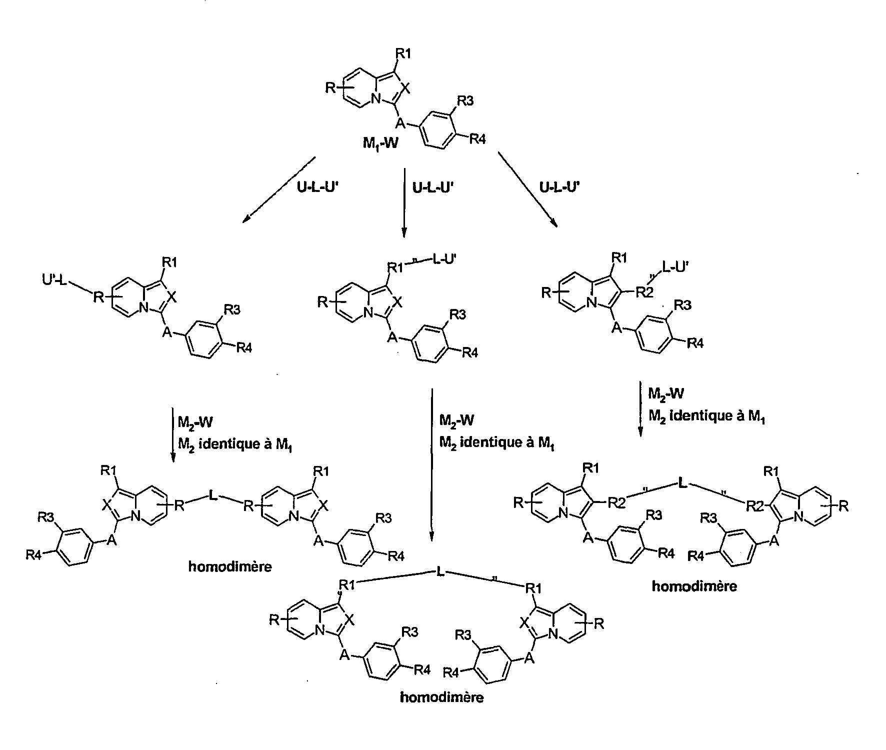

Ilustration de cas d'homodimérisation en deux étapes :

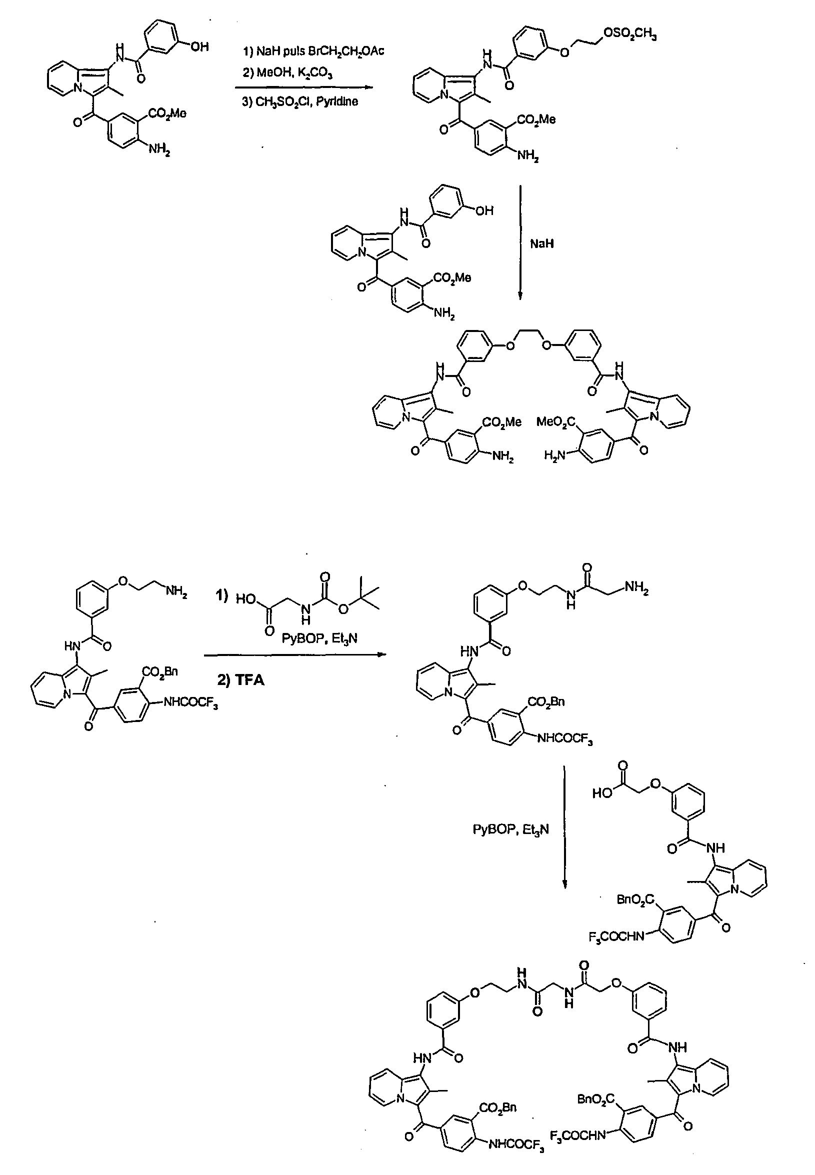

**Méthode VII)** On peut obtenir un hétérodimère en réalisant les réactions décrites dans les méthodes ci-dessus sur un seul composé de formule M₁-W (en jouant sur la stoechiométrie des réactifs M₁-W et U-L-U') puis faire réagir l'intermédiaire obtenu M₁-L-U' avec un deuxième composé de formule M₂-W (différent du premier) pour obtenir un hétérodimère soit de type homogène (chaque unité monomère appartient à la même famille indolizine X = C-R₂ ou imidazo[1,5-a]pyridine X = N) soit de type hétérogène (une unité monomère appartient à la famille indolizine X = C-R₂ et l'autre à la famille imidazo[1,5-a]pyridine X = N).

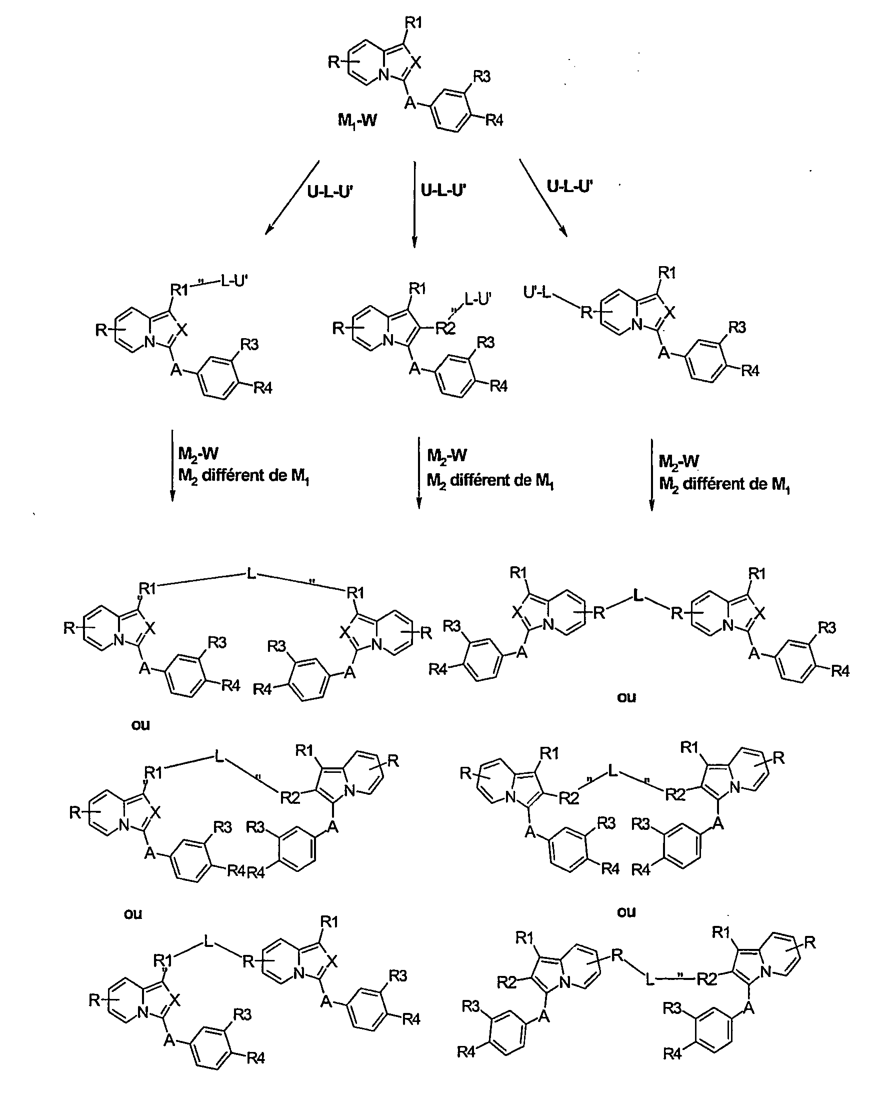

Ilustration de préparation d'hétérodimères :
Cas d'un hétérodimère homogène : 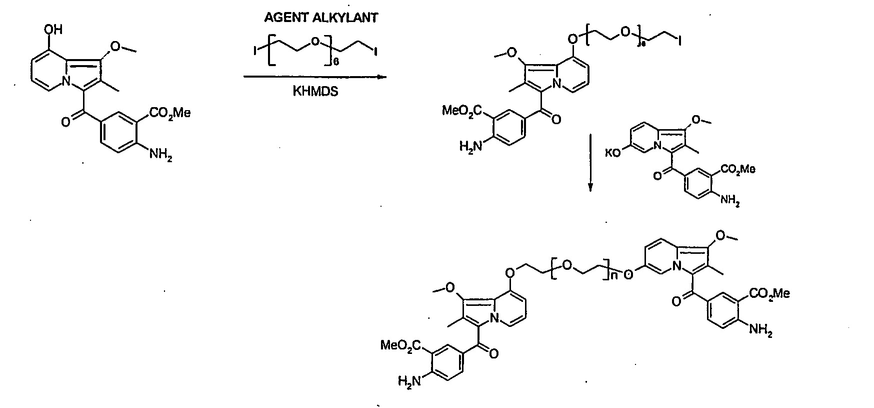
Cas d'un hétérodimère hétérogène :

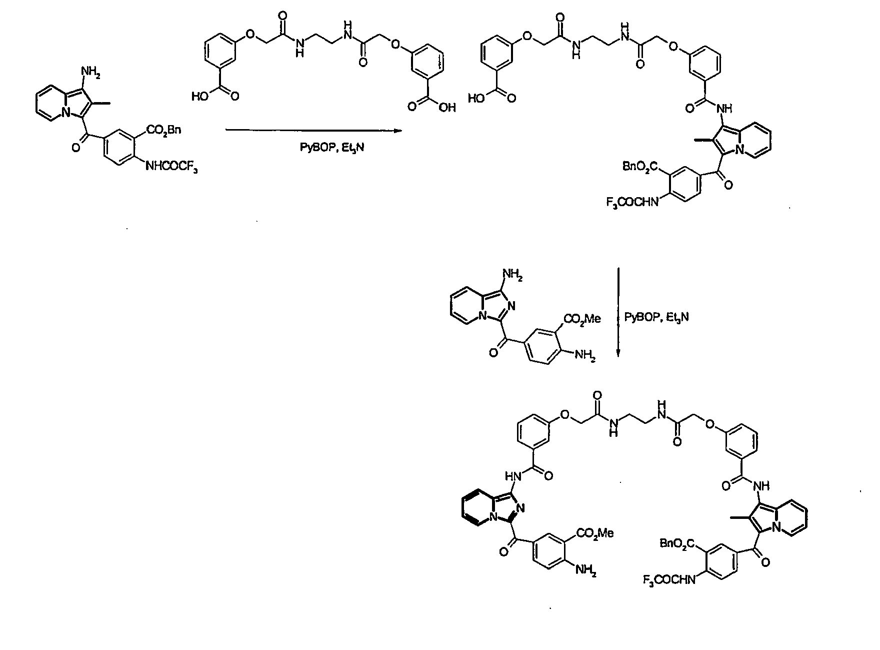

Après l'étape de dimérisation réalisée selon une des méthodes décrites ci-dessus, on réalise une ou plusieurs étapes de déprotections adéquates pour obtenir l'agoniste cible.

Lorsque R, R₁, R₃ et/ou R₄ possèdent une fonction ester de benzyle, une réduction avec du palladium sur charbon en présence d'un donneur d'hydrogène tel que de formiate d'ammonium permet d'obtenir l'acide carboxylique tel qu'illustré ci-dessous :

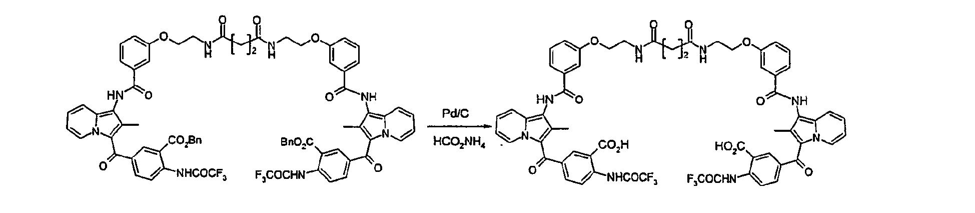

Lorsque R, R_{1'} R₃ et/ou R₄ possèdent une fonction carbamate de *tert-*butyle, une déprotection avec un acide tel que l'acide trifluoroacétique fournit l'amine tel qu'illustré ci-dessous :

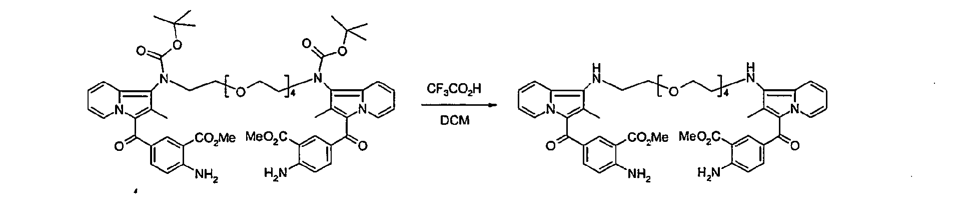

Lorsque R, R₁, R₃ et/ou R₄ possèdent une fonction ester et/ou une fonction amido (telle qu'acétamido, trifluoroacétamido), une hydrolyse en milieu basique avec par exemple de la soude ou de l'hydroxyde de lithium suivie d'une acidification fournit les dimères cibles, comme illustré ci-dessous :

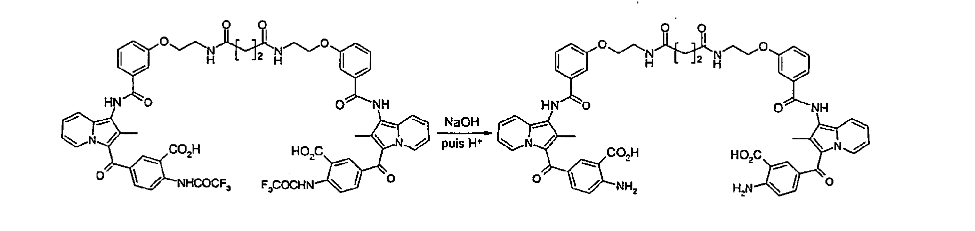

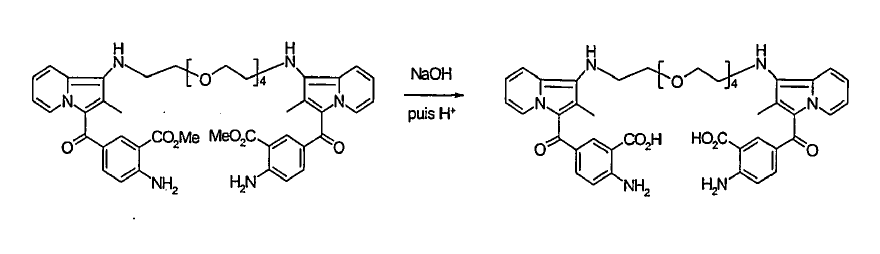

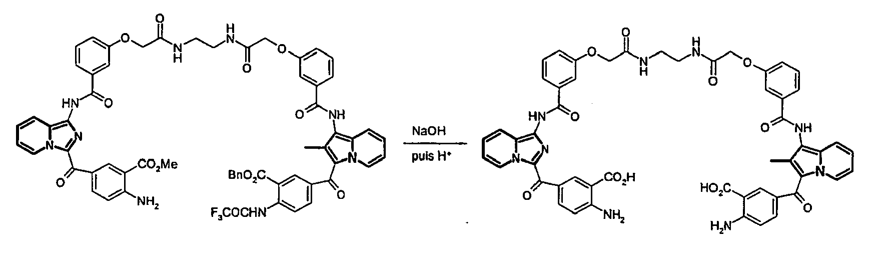

Parmi les composés objet de l'invention on peut citer :
- Sel disodique du 3,3'-{3,6,9,12,15-pentaoxaheptadécane-1,17-diylbis[oxy(1-méthoxy-2-méthylindolizine-8,3-diyl)carbonyl]}bis(acide 6-aminobenzoïque)
- Sel disodique du diacide 3,3'-(3,6,9,12,15-pentaoxaheptadécane-1,17-diylbis{oxy[3-(4-amino-3-méthoxybenzoyl)imidazo[1,5-a]pyridine-8,1-diyl]})benzoïque
- Sel disodique du 3,3'-{3,6,9,12,15,18-hexaoxaicosane-1,20-diylbis[oxy(1-méthoxy-2-méthylindolizine-6,3-diyl)carbonyl]}bis(acide 6-aminobenzoïque)
- Sel disodique du 3,3'-{3,6,9,12,15-pentaoxaheptadécane-1,17-diylbis[oxy(1-methoxy-2-méthylindolizine-6,3-diyl)carbonyl]}bis(acide 6-aminobenzoïque)
- Sel disodique du 3,3'-{3,6,9,12,15,18-hexaoxaicosane-1,20-diylbis[oxy(2-méthylindolizine-1,3-diyl)carbonyl]}bis(acide 6-aminobenzoïque)
- Sel disodique du 3,3'-{éthane-1,2-diylbis[oxyéthane-2,1-diyloxy-3,1-phénylèneméthylèneoxy(2-méthylindolizine-1,3-diyl)carbonyl]}bis(acide 6-aminobenzoïque)
- Sel disodique de 3,3'-{octane-1,8-diylbis[oxy-3,1-phénylènecarbonylimino(2-méthylindolizine-1,3-diyl)carbonyl]}bis(acide 6-aminobenzoïque)
- Sel disodique du 3,3'-{éthane-1,2-diylbis[oxyéthane-2,1-diyloxy-3,1-phénylènecarbonylimino(2-méthylindolizine-1,3-diyl)carbonyl]}bis(acide 6-aminobenzoïque)
- Sel disodique de 3,3'-{(1,4-dioxobutane-1,4-diyl)bis[iminoéthane-2,1-diyloxy-3,1-phénylènecarbonylimino(2-méthylindolizine-1,3-diyl)carbonyl]}bis(acide 6-aminobenzoïque)
- Sel disodique de 3,3'-{carbonyl bis[iminoéthane-2,1-diyloxy-3,1-phenylènecarbonylimino(2-méthylindolizine-1,3-diyl)carbonyl]}bis(acide 6-aminobenzoïque)
- Sel disodique du 3,3'-{éthane-1,2-diylbis[imino(2-oxoéthane-2,1-diyl)oxy-3,1-phénylènecarbonylimino(2-méthylindolizine-1,3-diyl)carbonyl]}bis(acide 6-aminobenzoïque)
- Sel disodique du 3,3'-{propane-1,3-diylbis[imino(2-oxoéthane-2,1-diyl)oxy-3,1-phénylènecarbonylimino(2-méthylindolizine-1,3-diyl)carbonyl]}bis(acide 6-aminobenzoïque)
- Sel disodique du 3,3'-{butane-1,4-diylbis[imino(2-oxoéthane-2,1-diyl)oxy-3,1-phénylènecarbonylimino(2-méthylindolizine-1,3-diyl)carbonyl]}bis(acide 6-aminobenzoïque)
- Sel disodique du 3,3'-{éthane-1,2-diylbis[imino(2-oxoéthane-2,1-diyl)oxy-4,1-phenylènecarbonylimino(2-méthylindolizine-1,3-diyl)carbonyl]}bis(acide 6-aminobenzoïque)
- Sel disodique du 3,3'-{éthane-1,2-diylbis[oxyéthane-2,1-diyloxy-4,1-phénylènecarbonylimino(2-méthylindolizine-1,3-diyl)carbonyl]}bis(acide 6-aminobenzoïque)
- Sel disodique du 3,3'-{oxybis[éthane-2,1-diyloxyéthane-2,1-diyloxy-4,1-phénylène(2-méthylindolizine-1,3-diyl)carbonyl]}bis(acide 6-aminobenzoïque)
- Sel disodique du 3,3'-{éthane-1,2-diylbis[oxyéthane-2,1-diyloxy-3,1-phénylène(2-méthylindolizine-1,3-diyl)carbonyl]}bis(acide 6-aminobenzoïque)
- Sel disodique du 3,3'-{oxybis[éthane-2,1-diyloxyéthane-2,1-diyloxy-3,1-phénylène(2-méthylindolizine-1,3-diyl)carbonyl]}bis(acide 6-aminobenzoïque)
- Sel disodique du 3,3'-{hexane-1,6-diylbis[imino(2-oxoéthane-2,1-diyl)oxy-3,1-phénylène(2-méthylindolizine-1,3-diyl)carbonyl]}bis(acide 6-aminobenzoïque)
- Sel disodique du 3,3'-{hexane-1,6-diylbis[imino(2-oxoéthane-2,1-diyl)oxy-4,1-phénylène(2-méthylindolizine-1,3-diyl)carbonyl]}bis(acide 6-aminobenzoïque)
- Sel disodique du diacide 2-amino-5-[(1-{[3-(2-{[2-({[3-({[3-(4-amino-3-carboxybenzoyl)imidazo[1,5-a]pyridin-1-yl]amino}carbonyl)phénoxy] acétyl}amino)éthyl]amino}-2-oxoéthoxy)benzoyl]amino}-2-méthylindolizin-3-yl)carbonyl]benzoïque

Ainsi les composés de l'invention présentent une activité agoniste des récepteurs des FGFs. Ils provoquent ainsi la dimérisation du récepteur et grâce à leur faible toxicité et leurs propriétés pharmacologiques et biologiques, les composés de la présente invention représentent une thérapie de choix dans les pathologies reliées à l'activation de l'angiogenèse.

L'ischémie est une diminution de la circulation artérielle dans un organe entraînant une baisse de la concentration en oxygène dans les tissus lésés. Dans les mécanismes de revascularisation post-ischémiques, deux mécanismes principaux entrent en jeu : l'angiogenèse et l'artériogenèse. L'angiogenèse est le processus de génération de nouveaux vaisseaux capillaires à partir de vaisseaux pré-existants. L'artériogenèse contribue au développement (augmentation en taille et en calibre) des vaisseaux collatéraux autour de la zone ischémiée ou avasculaire.
Parmi les facteurs de croissance impliqués dans ces processus de revascularisation, la famille des FGF et notamment le FGF-2 a été la plus largement décrite (Post, M. J., Laham, R., Sellke, F. W. & Simons, M. Therapeutic angiogenesis in cardiology using protein formulations. Cardiovasc Res 49, 522-31, 2001). Le FGF2 est une protéine de 18 KDalton qui induit la prolifération, la migration et la production de protéases par les cellules endothéliales en culture. Le FGF2 induit également la néovascularisation *in vivo* ainsi que le développement des vaisseaux collatéraux après ligature d'un vaisseau dans les modèles pharmacologiques.

Le FGF2 intèragit avec les cellules endothéliales par l'intermédiaire de deux classes de récepteurs, les récepteurs de haute affinité à activité tyrosine kinase (FGF-R1, -R2, -R3, -R4) et les récepteurs de basse affinité de type héparane sulfate proteoglycane situés à la surface des cellules et faisant partie intégrantes des matrices extracellulaires. Alors que le rôle paracrine du FGF2 sur les cellules endothéliales est largement décrit, le FGF2 pourrait également intervenir sur ces cellules à travers un processus autocrine : le FGF2 stimule la production d'autres facteurs angiogéniques (notamment le VEGF) (Janowska-Wieczorek, A., Majka, M., Ratajczak, J. & Ratajczak, M. Z. Autocrine/paracrine mechanisms in human hematopoiesis. Stem Cells 19, 99-107, 2001) la synthèse de protéases sur les cellules endothéliales (incluant le plasminogen activator et les métalloproteinases) nécessaires à la digestion de la matrice extracellulaire lors des processus d'angiogenèse (Carmeliet, P. Mechanisms of angiogenesis and arteriogenesis. Nat Med 6, 389-95, 2000). Par ailleurs, le FGF2 présente une activité proliférative et de migration sur d'autres types cellulaires impliqués dans la maturation des vaisseaux : cellules musculaires lisses, fibroblastes et péricytes. Ainsi, le FGF2 et ses récepteurs représentent des cibles très pertinentes pour les thérapies visant à induire les processus d'angiogenèse et d'artériogenèse (Khurana, R. & Simons, M. Insights from angiogenesis trials using fibroblast growth factor for advanced arteriosclerotic disease. Trends Cardiovasc Med 13, 116-22, 2003). Plusieurs évidences démontrent que le FGF2 est également impliqué dans la différenciation des angioblastes en cellules progénitrices endothéliales et participent ainsi à la revascularisation après une occlusion (Burger, P. E. et al. Fibroblast growth factor receptor-1 is expressed by endothelial progenitor cells. Blood 100, 3527-35, 2002).
Bien que dans la zone ischémiée les phénomènes d'hypoxie consécutifs induisent l'expression de facteur angiogéniques (notamment le VEGF et le FGF2), les processus naturels de compensation d'angiogenèse et d'artériogenèse ne sont pas souvent suffisants. Deux explications sont possibles : la production de cytokines angiogéniques est inadéquate ou leurs réponses sont atténuées. Par ailleurs, les patients nécessitant une revascularisation post-ischémique sont souvent âgés et présentent des caractéristiques pathologiques (diabètes, hypercholesterolémie...) qui limitent le rôle des cytokines angiogéniques après ischémie. Il a été montré que ces patients répondent à l'administration de cytokines angiogéniques et artériogéniques exogènes. Ainsi, les stratégies visant à augmenter la réponse des cellules de l'arbre vasculaire sont des stratégies adaptées pour augmenter la revascularisation post-ischémique et notamment cardiaque ou des artères coronaires (Freedman, S. B. & Isner, J. M. Therapeutic angiogenesis for ischemic cardiovascular disease. J Mol Cell Cardiol 33, 379-93, 2001; Freedman, S. B. & Isner, J. M. Therapeutic angiogenesis for coronary artery disease. Ann Intern Med 136, 54-71, 2002).
Les composés présentés dans cette invention sont des agonistes puissants et sélectifs des FGFRs. Leurs capacités d'induire l'angiogenèse a été démontré *in vitro* et *in vivo.*

Une des applications des composés de l'invention est le traitement post-ischémique après occlusion au niveau cardiaque ou des artères périphériques. En ce qui concerne le traitement de l'ischémie cardiaque, un des essais cliniques les plus prometteurs est un essai où du FGF-2 était séquestré dans des micro-sphères d'alginate en présence d'héparine (Laham, R. J. et al. Local perivascular delivery of basic fibroblast growth factor in patients undergoing coronary bypass surgery: results of a phase I randomized, double-blind, placebo-controlled trial. Circulation 100, 1865-71, 1999). Ces micro-sphères étaient implantées proche du foyer ischémique au niveau du myocarde. Après 90 jours, tous les patients traités au FGF2 ne présentaient aucun symptôme cardiaque ischémique. En comparaison, dans le groupe contrôle, 3 des 7 patients possédaient à 90 jours des symptômes persistants et 2 patients ont eu recours à de la chirurgie vasculaire. De façon intéressante, le bénéfice de la thérapie était maintenu après 3 ans de suivi. Ces observations suggèrent que des composés mimant le FGF2 peuvent représenter une thérapie de choix pour le traitement des conséquences de l'ischémie cardiaque.
Trois essais cliniques sur l'injection de FGF2 dans l'artère coronaire ont été réalisés lors de traitement du rétrécissement des artères coronaires (Laham, R. J. et al. Intracoronary basic fibroblast growth factor (FGF-2) in patients with severe ischemic heart disease: results of a phase I open-label dose escalation study. J Am Coll Cardiol 36, 2132-9, 2000; Simons, M. et al. Pharmacological treatment of coronary artery disease with recombinant fibroblast growth factor-2: double-blind, randomized, controlled clinical trial. Circulation 105, 788-93, 2002 ; Unger, E. F. et al. Effects of a single intracoronary injection of basic fibroblast growth factor in stable angina pectoris. Am J Cardiol 85, 1414-9, 2000). Le résultat de ces trois essais montre que les infusions intra-coronaires de FGF2 sont bien tolérées et améliorent significativement l'état des patients. Ainsi, les composés décrits dans l'invention peuvent trouver une application dans le traitement des maladies associées à un rétrécissement des artères coronaires et notamment dans le traitement des angines de poitrine.

Les maladies des artères distales et notamment les artérites des membres inférieurs sont dues à l'obstruction chronique des artérioles irriguant les extrémités. Les symptômes les plus courant sont l'engourdissement, la faiblesse et des raideurs douloureuses dues à une fatigue des groupes musculaires distaux. Ces phénomènes sont la résultante d'une diminution des calibres des artères au niveau des extrémités provoquée par l'athérosclérose. Ces pathologies touchent principalement les membres inférieurs. Dans un essai clinique de phase 1, des patients ayant des pathologies des artères périphériques entraînant la claudication ont reçu des injections de FGF2 (Lazarous, D. F. et al. Basic fibroblast growth factor in patients with intermittent claudication: results of a phase 1 trial. J Am Coll Cardiol 36, 1239-44, 2000). Dans ce contexte, le FGF2 était bien toléré chez ces patients et les données cliniques suggèrent un effet bénéfique du FGF2 notamment sur l'amélioration de la marche. Ces données cliniques suggèrent que les composés de l'invention représentent un outil thérapeutique de choix pour le traitement des maladies associées à une obstruction des artères distales.

La maladie de Buerger ou thromboangéite oblitérante affecte les structures vasculaires distales et se caractérise par une artérite distale des jambes avec douleurs et ulcérations. Dans ce contexte, une induction de l'angiogenèse et de la vasculogenèse représenterait une thérapie pour cette pathologie. Les composés de ladite invention représentent une thérapie de choix pour la thromboangéite oblitérante.

Le glutamate est un transmetteur putatif des neurones des ganglions dorsaux et la bradykinine est une molécule produite durant l'inflammation pouvant activer et sensibiliser les fibres nociceptives. Dans ce contexte, le FGF2 pourrait moduler la douleur inflammatoire malgré qu'aucun effet régulateur du FGF2 sur les fibres nociceptives n'ait été démontré *in vivo.* Cependant, il a été montré que le FGF2 bloquait totalement la libération de glutamate stimulée par la bradykinine *in vitro* (Rydh-Rinder et al. (2001) Regul Pept 102:69-79). De par l'activité agoniste des récepteurs du FGF, les composés de ladite invention représenteraient un traitement de choix dans le traitement de la nociception et ainsi dans le traitement de la douleur chronique.

La neuropathie périphérique est une atteinte axonale ou démyélinisante du nerf périphérique moteur et/ou sensitif qui entraîne une désensibilisation des membres distaux. Une des conséquences de l'atteinte des nerfs peut être un mal perforant, qui est particulièrement à craindre lorsqu'il y a une atteinte importante de la sensibilité profonde car dans ce cas le poids du corps a tendance à porter toujours sur les mêmes points d'appui. Une des complications secondaires majeures du diabète est le développement chronique d'une neuropathie périphérique. Dans ce contexte, Il a été démontré que le FGF2 induisait une régénération axonale qui pourrait être une thérapie de choix dans dans le traitement de la lésion des nerfs périphériques et donc dans la neuropathie périphérique (Basic fibroblast growth factor isoforms promote axonal elongation and branching of adult sensory neurons in vitro. Klimaschewski L, Nindl W, Feurle J, Kavakebi P, Kostron H. Neuroscience. 2004;126(2):347-53). De par l'activité agoniste des récepteurs du FGF, les composés de ladite invention représenteraient un traitement de choix dans la neuropathie périphérique chez le patient sain ou diabétique.

La prolifération et la migration des cellules musculaires lisses vasculaires contribuent à l'hypertrophie intimale des artères et joue ainsi un rôle prépondérant dans l'athérosclérose et dans la resténose après angioplastie et endoarterectomie. Il a été démontré qu'un facteur angiogène, le VEGF, réduisait significativement l'épaississement de l'intima en accélérant la ré-endothélialisation (Van Belle, E., Maillard, L., Tio, F. O. & Isner, J. M. Accelerated endothelialization by local delivery of recombinant human vascular endothelial growth factor reduces in-stent intimal formation. Biochem Biophys Res Commun 235, 311-6, 1997). Ainsi, un composé comme les composés de la présente invention possédant une activité pro-angiogène représente une thérapie de choix dans le traitement de l'athérosclérose et dans l'inhibition de la resténose après angioplastie ou endoartérectomie.

Le réseau vasculaire est essentiel au développement et à la préservation des tissus. En promouvant la délivrance des nutriments, de l'oxygène et des cellules, les vaisseaux sanguins aident à maintenir l'intégrité fonctionnelle et structurale des tissus. Dans ce contexte, l'angiogenèse et la vasculogenèse permettent de préserver et de perfuser les tissus après une ischémie. Les facteurs de croissance angiogéniques comme le VEGF et le FGF2 favorisent ainsi la revascularisation pour la régénération des tissus. Les composés présentés dans l'invention pourraient représenter un traitement de choix dans le traitement pour la régénération des muscles.

Les processus de régénération musculaire sur les muscles dystrophiques ou normaux dépendent de l'apport de cytokines et de facteurs de croissance angiogéniques au niveau local (Fibbi, G., D'Alessio, S., Pucci, M., Cerletti, M. & Del Rosso, M. Growth factor-dependent proliferation and invasion of muscle satellite cells require the cell-associated fibrinolytic system. Biol Chem 383, 127-36, 2002). Il a été proposé que le système FGF était un système critique de la régénération musculaire, de la survie et de la prolifération des myoblastes (Neuhaus, P. et al. Reduced mobility of fibroblast growth factor (FGF)-deficient myoblasts might contribute to dystrophic changes in the musculature of FGF2/FGF6/mdx triple-mutant mice. Mol Cell Biol 23, 6037-48, 2003). Le FGF2 ainsi que les composés de ladite invention pourraient être exploités afin de promouvoir la régénération cardiaque. Ils amélioreraient ainsi la perfusion du myocarde après ischémie (Hendel, R. C. et al. Effect of intracoronary recombinant human vascular endothelial growth factor on myocardial perfusion: evidence for a dose-dependent effect. Circulation 101, 118-21, 2000) ainsi que la survie et la progression de myoblastes transplantés et notamment dans la dystrophie musculaire de Duchenne.

L'angiogenèse est un phénomène essentiel durant la cicatrisation cutanée. Les nouveaux vaisseaux formés apportent l'oxygène et les nutriments nécessaires à la réparation tissulaire. Dans le cas de patient diabétique, la cicatrisation est un processus lent et difficile présentant des défauts d'angiogenèse. Les FGFS font partie des facteurs de croissance les plus impliqués dans les processus d'angiogenèse durant la phase de cicatrisation. Certains FGFs sont fortements sur-régulés dans les cellules du derme après une blessure cutanée. Par leurs activités agonistes des récepteurs du FGF, les composés de ladite invention représenteraient une thérapie de choix pour le traitement de la cicatrisation chez le patient sain ou diabétique.

La transplantation de pancréas bio-artificiel est une technique très prometteuse pour le traitement de certains types de diabète. Il vient d'être clairement démontré, chez le rat diabétique, que la vascularisation dans les pancréas bio-artificiel était beaucoup plus importante quand les pancréas étaient imprégnés de microsphères portant du FGF2 (Sakurai, Tomonori; Satake, Akira, Sumi, Shoichiro, Inoue, Kazutomo, Nagata, Natsuki, Tabata, Yasuhiko. The Efficient Prevascularization Induced by Fibroblast Growth Factor 2 With a Collagen-Coated Device Improves the Cell Survival of a Bioartificial Pancreas. Pancreas. 28(3):e70-e79, April 2004). Cette revascularisation améliore ainsi la survie des pancréas bio-artificiel implantés et par conséquent la survie du greffon. Par leurs activités agonistes des récepteurs du FGF, les composés de ladite invention représenteraient une thérapie de choix dans l'amélioration de la survie du greffon de pancréas bioartificiel chez le patient diabétique et de façon plus générale dans l'amélioration de la revascularisation des greffons et par conséquent dans la survie des greffes.

La rétinite pigmentaire est une pathologie impliquant la dégénérescence progressive de la rétine caractérisée par une dégénérescence des photorécepteurs et une oblitération des vaisseaux rétiniens. Récemment Lahdenranta & al. An anti-angiogenic state in mice and humans with retinal photoreceptor cell degeneration. Proc Natl Acad Sci U S A 98, 10368-73, 2001) ont proposé que des facteurs de croissance angiogéniques régulent la coordination neurale et la vascularisation associée de la rétine en fonctionnant simultanément comme facteurs de survie des photorécepteurs et comme régulateur des cellules endothéliales. Dans ce contexte, l'injection intra-vitréenne de FGF2 retarde la dégénérescence des photorécepteurs en agissant sur la survie et sur l'angiogenèse rétinienne (Faktorovich, E. G., Steinberg, R. H., Yasumura, D., Matthes, M. T. & LaVail, M. M. Basic fibroblast growth factor and local injury protect photoreceptors from light damage in the rat. J Neurosci 12, 3554-67,1992). Ces observations démontrent l'intérêt des composés décrits dans l'invention comme thérapie appropriée dans la dégénérescence rétinienne et notamment dans la rétinite pigmentaire.

Dans le domaine de la réparation osseuse, un des besoins essentiels est de trouver des agents qui stimule la formation de l'os. Parmi les principaux facteurs de croissance, il est maintenant clairement établi que l'administration systémique de FGF2 facilite la réparation de l'os (Acceleration of fracture healing in nonhuman primates by fibroblast growth factor-2. Kawaguchi H, Nakamura K, Tabata Y, Ikada Y, Aoyama 1, Anzai J, Nakamura T, Hiyama Y, Tamura M. J Clin Endocrinol Metab. 2001 Feb;86(2), 875-880). L'application locale de FGF2 dans des matrices de gélatine accélère la réparation osseuse chez des primates suggérant l'utilité clinique du FGF2 dans le traitement des fractures. Par les propriétés agonistes pour les récepteurs du FGF, les composés de ladite invention pourrait représenter un traitement de choix dans la réparation osseuse.

La pré-eclampsie est une pathologie du placenta associée à un défaut de vascularisation (Sherer, D. M. & Abulafia, O. Angiogenesis during implantation, and placental and early embryonic development. Placenta 22, 1-13, 2001). Ces défauts de vascularisation seraient dus à un défaut d'angiogenèse et entraîneraient des perturbations au niveau du placenta pouvant aboutir à la mort du foetus. Les composés de ladite invention pourraient être un traitement de choix pour pallier à un défaut d'angiogenèse dans les placentas pré-éclamptiques.

En plus des effets inducteurs de l'angiogenèse, les facteurs de croissance comme le VEGF ou le FGF2 protègent les cellules endothéliales des inducteurs intrinsèques et extrinsèques de l'apoptose. La voie de signalisation intrinsèque est activée par les mitochondries en réponse à un stress comme la déprivation ou les dommages sur l'ADN, alors que la voie de signalisation extrinsèque est induite par la liaison de facteurs pro-apoptotique comme le TNF-α ou Fas. Il est maintenant clairement décrit que le VEGF et le FGF2 sont deux facteurs de survie des cellules endothéliales (Role of Raf in Vascular Protection from Distinct Apoptotic Stimuli : A Alavi, J.D. Hood, R. Frausto, D. G. Stupack, D.A. Cheresh : Science 4 July 2003: Vol. 301. no. 5629, pp. 94-96). Le Syndrome de Détresse Respiratoire Aiguë (ARDS) se caractérise par des problèmes cardio-vasculaires et neuropsychiatriques. Dans le cadre des problèmes cardio-vasculaires les patients présentent des lésions vasculaires importantes et notamment une induction de l'apoptose des cellules endothéliales élevée. Récemment, Hamacher & al. ont démontré que les fluides de lavages bronchoalvéolaires de patients atteint d'ARDS présentaient une activité pro-apoptotique contre les cellules endothéliales micro-vasculaire de poumon (Tumor necrosis factor-alpha and angiostatin are mediators of endothelial cytotoxicity in bronchoalveolar lavages of patients with acute respiratory distress syndrome. Am J Respir Crit Care Med. 2002 Sep 1;166(5):651-6 : Hamacher J, Lucas R, Lijnen HR, Buschke S, Dunant Y, Wendel A, Grau GE, Suter PM, Ricou B.). Par leur activité anti-apoptotique sur les cellules endothéliales, les produits de ladite invention pourraient présenter un traitement de choix dans l'amélioration vasculaire des patients atteints de lésions vasculaires et notamment des patients atteints d'ARDS.

La surrégulation endogène de FGF7 (ou KGF) et de FGF18 semble être un important mécanisme pour favoriser la prolifération, la migration et la protection des follicules pileux dans des cas pathologiques ou suite à un traitement tumoral (Comprehensive Analysis of FGF and FGFR Expression in Skin: FGF18 is Highly Expressed in Hair Follicles and Capable of Inducing Anagen from Telogen Stage Hair Follicles. Mitsuko Kawano, Akiko Komi-Kuramochi, Masahiro Asada, Masashi Suzuki, Junko Oki, Ju Jiang and Toru Imamura). Par leur activité agoniste sur les récepteurs du FGF, les composés de ladite invention pourraient présenter un traitement de choix pour la réparation et la protection des follicules pileux et dans la protection et la régulation de la croissance capillaire.

L'incidence de l'obésité et du diabète de type II est en constante augmentation et est associée à une mortalité et une morbidité accrue. Il est clairement montré que cette adiposité est stockée principalement sous forme de triglycérides. Dans ce contexte, il a été rapporté que des souris transgéniques surexprimant le FGF19 (activateur du FGFR4) possédaient un taux de métabolisme augmenté associé à à une diminution de l'adiposité (E. Tomlinson, Transgenic mice expressing human fibroblast growth factor-19 display increased metabolic rate and decreased adiposity. Endocrinology. 2002 May;143(5):1741-7). De plus, des souris n'exprimant pas FGFR-4 montrait une augmentation de l'expression de l'acide biliaire associée à une augmentation de CYP7A1, une enzyme associée à la production de cet acide. Par leur activité agoniste sur les récepteurs du FGF et notamment du FGFR4, les composés de ladite invention pourraient favoriser la diminution du cholesterol associée à une diminution de l'adiposité.

Selon un autre de ses aspects, la présente invention a donc pour objet l'utilisation d'un composé tel que défini plus haut pour la préparation d'un médicament utile dans le traitement des maladies nécessitant une activation des récepteurs FGFs.
La présente invention a plus particulièrement pour objet l'utilisation d'un composé tel que défini plus haut pour la préparation d'un médicament utile dans le traitement de l'ischémie cardiaque, le traitement des maladies associées à un rétrécissement ou à une obstruction des artères ou des artérites, le traitement des angines de poitrine, le traitement de la thromboangéite oblitérante, le traitement de l'athérosclérose, le traitement de l'inhibition de la resténose après angioplastie ou endoartérectomie, le traitement de la cicatrisation, le traitement pour la régénération musculaire, le traitement pour la survie des myoblastes, le traitement de la nociception et le traitement de la douleur chronique, le traitement de la neuropathie périphérique, le traitement pour l'amélioration de la survie du greffon de pancréas bioartificiel chez le patient diabétique, le traitement de la diminution du cholesterol associée à une diminution de l'adiposité, le traitement de l'amélioration de la la revascularisation des greffons et de la survie des greffes, le traitement de la dégénérescence rétinienne, le traitement de la rétinite pigmentaire, le traitement de l'ostéoarthrite, le traitement de la pré-éclampsie, le traitement des lésions vasculaire et du syndrome de détresse respiratoire aiguë, le traitement de la protection osseuse, ou le traitement de la protection des follicules pileux.

Selon un autre de ses aspects, la présente invention a donc pour objet une composition pharmaceutique contenant, en tant que principe actif, un composé de formule M₁-L-M₂ selon l'invention ou un de ses sels pharmaceutiquement acceptables, éventuellement en association avec un ou plusieurs excipients inertes et appropriés.
Lesdits excipients sont choisis selon la forme pharmaceutique et le mode d'administration souhaitée : orale, sublinguale, sous-cutanée, intramusculaire, intraveineuse, transdermique, transmuqueux, locale ou rectale.

Les compositions pharmaceutiques selon la présente invention peuvent être administrées notamment par voie orale, sublinguale, sous-cutanée, intramusculaire, intraveineuse, transdermique, transmuqueux, locale ou rectale.

Dans les compositions pharmaceutiques de la présente invention pour l'administration orale, les principes actifs peuvent être administrés sous formes unitaires d'administration, en mélange avec des supports pharmaceutiques classiques. Les formes unitaires d'administration appropriées comprennent par exemple les comprimés éventuellement sécables, les gélules, les poudres, les granules et les solutions ou suspensions orales.
Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi sous forme de complexe d'inclusion dans des cyclodextrines, leurs éthers ou leurs esters.

La quantité de principe actif à administrer dépend, comme toujours, du degré de progression de la maladie, de l'âge et du poids du patient ainsi que de la voie d'administration.

La présente invention a pour objet une composition pharmaceutique telle que définie plus haut utile dans le traitement de l'ischémie cardiaque, le traitement des maladies associées à un rétrécissement ou à une obstruction des artères ou des artérites, le traitement des angines de poitrine, le traitement de la thromboangéite oblitérante, le traitement de l'athérosclérose, le traitement de l'inhibition de la resténose après angioplastie ou endoartérectomie, le traitement de la cicatrisation, le traitement pour la régénération musculaire, le traitement pour la survie des myoblastes, le traitement de la nociception et le traitement de la douleur chronique, le traitement de la neuropathie périphérique, le traitement pour l'amélioration de la survie du greffon de pancréas bioartificiel chez le patient diabétique, le traitement de la diminution du cholesterol associée à une diminution de l'adiposité, le traitement de l'amélioration de la la revascularisation des greffons et de la survie des greffes, le traitement de la dégénérescence rétinienne, le traitement de la rétinite pigmentaire, le traitement de l'ostéoarthrite, le traitement de la pré-éclampsie, le traitement des lésions vasculaire et du syndrome de détresse respiratoire aiguë, le traitement de la protection osseuse, ou le traitement de la protection des follicules pileux.

Tableau des exemples :
M₁-L-M₂
avec M de formule générale comme ci-dessous :

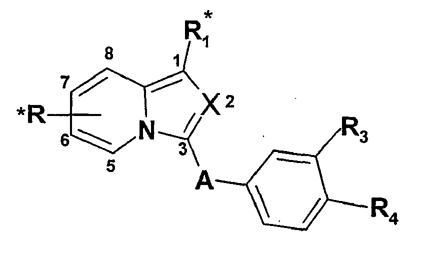

Pour les exemples décrits ci-dessous, A représente un radical CO.

Lorsque R est défini comme étant un radical 8-O*, 6-O*, cela signifie que R représente un radical hydroxyle en position 8 ou 6 de l'hétérocycle. Lorsque R est défini comme étant un radical 7-CONH* cela signifie que R représente un radical amido en position 7 de l'hétérocycle.

### Homodimères

| **Ex** | **R** | **R1** | **X** | **R₃** | **R₄** | **L** | **Sel** | **PF(°C) ou MH⁺/RT (min)** |
|---|---|---|---|---|---|---|---|---|
| 1 | 8-O^{*} | OCH₃ | CCH₃ | CO₂H | NH₂ | -(CH₂CH₂O)₆CH₂CH₂- | 2 Na, 1,5 H₂O | 265°C |
| 2 | 8-O^{*} | OCH₃ | CCH₃ | CO₂H | NH₂ | -(CH₂CH₂O)₅CH₂CH₂- | 2 Na, 3,6 H₂O | 201°C |
| 3 | 8-O^{*} | OCH₃ | CCH₃ | CO₂H | NH₂ | -(CH₂CH₂O)₄CH₂CH₂- | 2 Na, 5,45 H₂O | 162°C |
| 4 | 8-O^{*} | OCH₃ | CCH₃ | CO₂H | NH₂ | -(CH₂CH₂O)₃CH₂CH₂- | 2 Na, 4,8 H₂O | 239°C |
| 5 | 8-O^{*} | OCH₃ | CCH₃ | CO₂H | NH₂ | -(CH₂CH₂O)₂CH₂CH₂- | 2 Na, 2,5 H₂O | 252°C |
| 6 | 8-O^{*} | OCH₃ | CCH₃ | CO₂H | NH₂ | -CH₂CH₂OCH₂CH₂- | 2 Na, 7,45 H₂O | 258°C |
| **7** | 8-O* | OCH₃ | CCH₃ | CO₂H | NH₂ | -CH₂CONH(CH₂)₁₁NHCOCH₂- | 2 Na, 3,5 H₂O | 220°C |
| **8** | 8-O* | Ph-3-CO₂H | N | -OCH₃ | NH₂ | -(CH₂CH₂O)₅CH₂CH₂- | 2 Na, 5 H₂O | 209°C |
| **9** | 8-O* | 4-pyridine | N | -OCH₃ | NH₂ | -(CH₂CH₂O)₅CH₂CH₂- | 2,4 HCl 8,6 H₂O | 184°C |
| **10** | 8-O* | H | N | -OCH₃ | NH₂ | -(CH₂CH₂O)₅CH₂CH₂- | 2 HCl, 5 H₂O | 80°C |
| **11** | 6-O* | OCH₃ | CCH₃ | CO₂H | NH₂ | -(CH₂CH₂0)₆CH₂CH₂₇ | 2 Na, 7,4 H₂O | 181°C |
| **12** | 6-O* | OCH₃ | CCH₃ | CO₂H | NH₂ | -(CH₂CH₂O)₅CH₂CH₂- | 2 Na, 6 H₂O | 261-270°C |
| **13** | 6-O* | OCH₃ | CCH₃ | CO₂H | NH₂ | -(CH₂CH₂O)₄CH₂CH₂- | 2 Na, 5 H₂O | 255-260°C |
| **14** | 6-O* | OCH₃ | CCH₃ | CO₂H | NH₂ | -(CH₂CH₂O)₃CH₂CH₂- | 2 Na, 5 H₂O | 232°C |
| **15** | 6-O* | OCH₃ | CCH₃ | CO₂H | NH₂ | -(CH₂CH₂O)₂CH₂CH₂- | 2 Na, 6,95 H₂O | 297-300°C |
| **16** | 6-O* | OCH₃ | CCH₃ | CO₂H | NH₂ | -CH₂CH₂OCH₂CH₂- | 2 Na, 5 H₂O | 294-300°C |
| **17** | H | OCH₃ | CPh-4-O* | CO₂H | NH₂ | -(CH₂CH₂O)₅CH₂CH₂- | 2 Na, 7,5 H₂O | 174°C |
| **18** | H | OCH₃ | CPh-4-O* | CO₂H | NH₂ | -(CH₂CH₂O)₅CH₂CH₂- | 2 Na, 6,5 H₂O | 168°C |
| **19** | H | OCH₃ | CPh-3-O* | CO₂H | NH₂ | -(CH₂CH₂O)₅CH₂CH₂- | 2 Na, 2,65 H₂O | 197°C |
| **20** | H | OCH₃ | CPh-3-O* | CO₂H | NH₂ | -(CH₂CH₂O)₅CH₂CH₂- | 2 Na, 4,5 H₂O | 198°C |
| **21** | H | O* | CCH₃ | CO₂H | NH₂ | -(CH₂CH₂O)₅CH₂CH₂- | 2 Na, 5,5 H₂O | 224°C |
| **22** | H | O* | CCH₃ | CO₂H | NH₂ | -(CH₂CH₂O)_{S}CH₂CH₂- | 2 Na, 4 H₂O | 165°C |
| **23** | H | O* | CCH₃ | CO₂H | NH₂ | -(CH₂CH₂O)₄CH₂CH₂₋ | 2 Na, 4 H₂O | 186°C |
| **24** | H | O* | CCH₃ | CO₂H | NH₂ | -(CH₂CH₂O)₃CH₂CH₂- | 2 Na, 5 H₂O | 226°C |
| **25** | H | O* | CCH₃ | CO₂H | NH₂ | -(CH₂CH₂O)₂CH₂CH₂- | 2 Na, 4 H₂O | 244°C |
| **26** | H | O* | CCH₃ | CO₂H | NH₂ | -CH₂CH₂OCH₂CH₂- | 2 Na, 4 H₂O | 277°C |
| **27** | H | -OCH₂Ph-3-O* | CCH₃ | CO₂H | NH₂ | -(CH₂CH₂O)₂CH₂CH₂- | 2 Na, 2,65 H₂O | 188°C |
| **28** | H | -OCH₂Ph-4-O* | CCH₃ | CO₂H | NH₂ | -(CH₂CH₂O)₂CH₂CH₂ - | 2 Na, 3 H₂O | 247°C |
| **29** | H | -OCH₂Ph-4-O* | CCH₃ | CO₂H | NH₂ | -(CH₂)₈- | 2 Na, 3,5 H₂O | 239°C |
| **30** | H | -OCH₂Ph-3-O* | CCH₃ | CO₂H | NH₂ | -CH₂CONH(CH₂)₂NHCOCH₂- | 2 Na, 3 H₂O, + 1 acétone | 296°C |
| **31** | H | O* | CCH₃ | CO₂H | NH₂ | -CH₂CONH(CH₂)₂NHCOCH₂- | 2 Na, 3 H₂O | 320°C |
| **32** | H | O* | CCH₃ | CO₂H | NH₂ | -CH₂CONH(CH₂)₃NHCOCH₂- | 2 Na, 5 H₂O | 264°C |
| **33** | H | NH* | CCH₃ | CO₂H | NH₂ | -(CH₂CH₂O)4CH₂CH₂- | 2 Na, 4 H₂O | 193°C |
| **34** | H | -NHCOPh-3-O* | CCH₃ | CO₂H | NH₂ | -(CH₂)₈- | 2 Na, 5 H₂O + 0,35 DMF | 263°C |
| **35** | H | -NHCOPh-3-O* | CCH₃ | CO₂H | NH₂ | -(CH₂CH₂O)₅CH₂CH₂- | 2 Na, 5 H₂O, + 0,5 acétone | 252°C |
| **36** | H | -NHCOPh-3-O* | CCH₃ | CO₂H | NH₂ | -(CH₂CH₂O)₄CH_{Z}CH₂- | 2 Na, 5 H₂O, + 0,6 acétone | 267-269°C |
| **37** | H | -NHCOPh-3-O* | CCH₃ | CO₂H | NH₂ | -(CH₂CH₂O)₃CH₂CH₂- | 2 Na, 4,95 H₂O | 312-314°C |
| **38** | H | -NHCOPh-3-O* | CCH₃ | CO₂H | NH₂ | -(CH₂CH₂O)₂CH₂CH₂- | 2 Na, 5 H₂O, + 0,5 acétone | 300-310°C |
| **39** | H | -NHCOPh-3-O* | CCH₃ | CO₂H | NH₂ | -CH₂CH₂OCH₂CH₂- | 2 Na, 5 H₂O, + 0,3 acétone | 334-337°C |
| **40** | H | -NHCOPh-3-O* | CCH₃ | CO₂H | NH₂ | 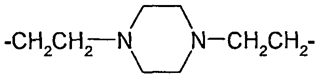 | 2 Na, 5 H₂O | 326-328°C. |
| **41** | H | -NHCOPh-3-O* | CCH₃ | CO₂H | NH₂ | 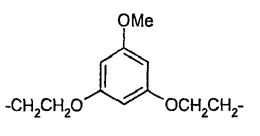 | 2 Na, 5 H₂O + 0,3 acétone | 257-259°C |
| **42** | H | -NHCOPh-3-O* | CCH₃ | CO₂H | NH₂ | -CH₂CH₂NHCO-(CH₂)₂CONHCH₂CH₂- | 2 Na, 5 H₂O | 355-361°C |
| **43** | H | -NHCOPh-3-O* | CCH₃ | CO₂H | NH₂ | -CH₂CH₂NHCONHCH₂CH₂- | 2 Na, 4 H₂O | 354-359°C |
| **44** | H | NHCOPh-3-O* | CCH₃ | CO₂H | NH₂ | -CH₂CONH(CH₂)₂NHCOCH₂- | 2 Na, 5 H₂O | 339-354°C |
| **45** | H | -NHCOPh-3-O* | CCH₃ | CO₂H | NH₂ | -CH₂CONH(CH₂)₃NHCOCH₂- | 2 Na, 4 H₂O | 358-360°C |
| **46** | H | -NHCOPh-3-O^{*} | CCH₃ | CO₂H | NH₂ | -CH₂CONH(CH₂)₄NHCOCH₂- | 2 Na, 5 H₂O | 343°C |
| **47** | H | -NHCOPh-3-O* | CCH₃ | CO₂H | NH₂ | 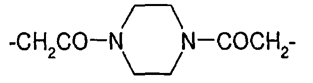 | 2 Na, 6 H₂O | 346-357°C |
| **48** | 7-CONH* | -Ph-3-CO₂H | N | OCH₃ | NH₂ | -CH₂CH₂CH₂(OCH₂CH₂)₄ OCH₂CH₂CH₂- | 2 Na, 8 H₂O | 290-293°C |
| **49** | 7-CONH* | -P h-3-CO₂H | N | OCH₃ | NH₂ | -CH₂CH₂CH₂(OCH₂CH₂)₂ OCH₂CH₂CH₂- | 2 Na, 8,5 H₂O 0,2 acétone | 213°C |
| **50** | 7-CO₂H | -Ph-3-CONH* | N | OCH₃ | NH₂ | -(CH₂)₉- | 2 Na, 4,5 H₂O | 348-355°C |
| **51** | H | -NHCOPh-3-O* | CCH₃ | CO₂H | NH₂ | -(CH₂)₂- | 2 Na, 6,35 H₂O | 286°C |
| **52** | H | -NHCOPh-3-O* | N | CO₂H | NH₂ | -(CH₂)₈- | 2 Na, 4,95 H₂O | 249°C |
| **53** | H | -NHCOPh-3-0* | N | CO₂H | NH₂ | -CH₂CONH(CH₂)₂NHCOCH₂- | 2 Na, 7,5 H₂O | 281-286°C |
| **54** | 7-CO₂H | -NHCOPh-3-O* | N | OCH₃ | NH₂ | -(CH₂)₈- | 2 Lys | RT=15,34 min (Méthode A) |
| **55** | H | -NHCOPh-4-O* | CCH₃ | OCH₃ | NH₂ | -CH₂CONH(CH₂)₂NHCOCH₂- | 2 HCl | 223°C |
| **56** | H | -NHCOPh-3-O* | CCH₃ | OCH₃ | NH₂ | -CH₂CONHCH₂CH(NMe₂)CH₂₋ NHCOCH₂- | 2 HCl 4,5 H₂O | 183°C |
| **57** | H | -NHCOPh-3-O* | CCH₃ | OCH₃ | NH₂ | -CH₂CONHCH₂CH(CH₂NMe₂)-CH₂NHCOCH₂- | 3 HCl 7,5 H₂O | 194°C |
| **58** | H | -NHCOPh-3-O* | CCH₃ | OCH₃ | NH₂ | -CH₂CONHCH₂CH(OH)-CH₂NHCOCH₂- | 2 HCl 7 H₂O | 202°C |
| **59** | H | -NHCOPh-3-O* | CCH₃ | OCH₃ | NH₂ | -CH₂CH₂N(Me)CH₂CH₂N(Me) CH₂CH₂- | 2 HCl 1,5 acétone | 238°C |
| **60** | H | -NHCOPh-3-O* | CCH₃ | OCH₃ | NH₂ | -CH₂CH₂N(Bn)CH₂CH₂N(Bn) CH₂CH₂- | 2 HCl 6,95 H₂O | 217°C |
| **61** | H | -NHCOPh-4-O* | CCH₃ | OCH₃ | NH₂ | -CH₂CH₂N(Me)CH₂CH₂N(Me) CH₂CH₂- | 2 HCl 8 H₂O | 215°C |
| **62** | H | -NHCOPh-3-O* | CCH₃ | OCH₃ | NH₂ | 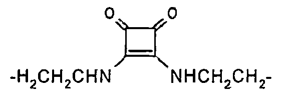 | 2 HCl | 217°C |
| **63** | H | -NHCOPh-3-O* | CCH₃ | OCH₃ | NH₂ | -CH₂CH₂NHCOCONHCH₂CH₂- | - | 225°C |
| **64** | H | -NHCOPh-4-O* | CCH₃ | CO₂H | NH₂ | -CH₂CONH(CH₂)₂NHCOCH₂- | 2 Na, 5 H₂O | 290-297°C |
| **65** | H | -NHCOPh-4-O* | CCH₃ | CO₂H | NH₂ | -CH₂CON(CH₃)CH₂CH₂N (CH₃)COCH₂- | 2 Na, 11 H₂O | 296-300°C |
| **66** | H | -NHCOPh-4-O* | CCH₃ | CO₂H | NH₂ | -(CH₂CH₂O)₃CH₂CH₂- | 2 Na, 7 H₂O | 292-294°C |
| **67** | H | -NHCOPh-4-O* | CCH₃ | CO₂H | NH₂ | -(CH₂CH₂O)₂CH₂CH₂- | 2 Na, 4,5 H₂O | 308-310°C |
| **68** | H | -NHCOPh-4-O* | CCH₃ | CO₂H | NH₂ | -CH₂CH₂OCH₂CH₂- | 2 Na, 5 H₂O | 320-322°C |
| **69** | H | -NHCOPh-4-O* | CCH₃ | CO₂H | NH₂ | -(CH₂)₃- | 2 Na, 7,5 H₂O | 283°C |
| **70** | H | -NHCOPh-4-O* | CCH₃ | CO₂H | NH₂ | -(CH₂)₂- | 2 Na, 4,95 H₂O | 306°C |
| **71** | H | -NHCOPh-3-O* | CCH₃ | CO₂H | H | -CH₂CONH(CH₂)₂NHCOCH₂- | 2 Na, 4,2 H₂O | 330-339°C |
| **72** | H | -NHCOPh-3-O* | CCH₃ | CO₂H | NH₂ | 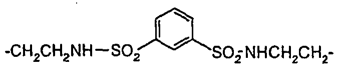 | 2 Na 8 H₂O | 308°C |
| **73** | H | -NHCOPh-3-O* | CCH₃ | CO₂H | NH₂ | -CH₂CH₂NHCOCH₂CONH-CH₂CH₂- | 2 Na 7 H₂O | 289°C |
| **74** | H | -NHCOPh-3-O* | CCH₃ | CO₂H | NH₂ | 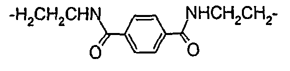 | 2 Lys 6 H₂O | 224°C |
| **75** | H | -NHCOPh-3-O* | CCH₃ | CO₂H | NH₂ | 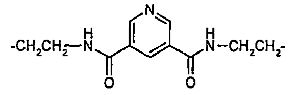 | 2 Lys 8 H₂O | 214°C |
| **76** | H | -NHCOPh-3-O* | CCH₃ | CO₂H | NH₂ | -CH₂CON(Me)CH₂CH₂-N(Me)COCH₂- | 2 Lys | MH+= 1027 RT=11,22 min (Méthode A) |
| **77** | H | -NHCOPh-3-O* | CCH₃ | CO₂H | NH₂ | 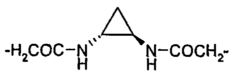 | 2 Na 6,5 H₂O | 305°C |
| **78** | H | -NHCOPh-3-O* | CCH₃ | CO₂H | NH₂ | 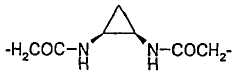 | 2 Na 7,5 H₂O | 297°C |
| **79** | H | -NHCOPh-3-O* | CCH₃ | CO₂H | NH₂ | -CH₂CONHCH₂NHCOCH₂- | 2 Lys 10 H₂O | 209°C |
| **80** | H | -NHCOPh-3-O* | CCH₃ | CO₂H | NH₂ | -CH₂CH₂N(Me)CH₂CH₂N(Me) CH₂CH₂- | 2 Lys 9 H₂O | 198°C |
| **81** | H | NHCO* | CCH₃ | CO₂H | NH₂ | -CH₂CH₂CON(CH₃)CH₂CH₂ N(CH₃)COCH₂CH₂- | 2 Lys | MH⁺ =871,43 RT=1,24 min (Méthode B) |
| **82** | H | NHCO* | CCH₃ | CO₂H | NH₂ | -CH₂CH₂CON(CH₃)CH₂CH₂ (OCH₂CH₂)₂N(CH₃)COCH₂CH₂- | 2 Lys | MH⁺ = 959,51 RT = 1,30 min (Méthode B) |
| **83** | H | NHCO* | CCH₃ | CO₂H | NH₂ | -CH₂CH₂CON(CH₃)CH₂CH₂ OCH₂CH₂N(CH₃)COCH₂CH₂- | 2 Lys | MH⁺ = 915,46 RT = 1,28 min (Méthode B) |
| **84** | H | NHCO* | CCH₃ | CO₂H | NH₂ | 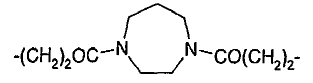 | 2 Lys | MH+= 883,45 RT=1,26 min (Méthode B) |
| **85** | H | NHCO* | CCH₃ | CO₂H | NH₂ | -CH₂CH₂CON(CH₃)(CH₂)₈ N(CH₃)COCH₂CH₂- | 2 Lys | MH⁺ = 955,54 RT = 1,50 min (Méthode B) |
| **86** | H | NHCO* | CCH₃ | CO₂H | NH₉ | -CH₂CH₂CON(CH₃)CH₂CH₂ CH₂N(CH₃)COCH₂CH₂- | 2 Lys | MH+ = 885,50 RT=1,49 min (Méthode B) |
| **87** | H | NHCO* | CCH₃ | COP₂H | NH₂ | -(CH₂)₃CON(CH₃)CH₂CH₂ (OCH₂CH₂)₂N(CH₃)CO(CH₂)₃- | 2 Lys | MH+= 987,58 RT=1,32 min (Méthode B) |
| **88** | H | NHCO* | CCH₃ | CO₂H | NH₂ | -(CH₂)₃CON(CH₃) (CH₂)₈N(CH₃)CO(CH₂)₃- | 2 Lys | MH+= 983,62 RT=1,47 min (Méthode B) |
| **89** | H | NHCO* | CCH₃ | CO₂H | NH₂ | -(CH₂)₃CON(CH₃) (CH₂)₆N(CH₃)CO(CH₂)₃- | 2 Lys | MH+= 955,58 RT=1,39 min (Méthode B) |
| **90** | H | NHCO* | CCH₃ | CO₂H | NH₂ | -(CH₂)₃CON(CH₃)CH₂CH₂ OCH₂CH₂N(CH₃)CO(CH₂)₃- | 2 Lys | MH⁺ = 943,51 RT = 1,35 min (Méthode B) |
| **91** | H | NHCO* | CCH₃ | CO₂H | NH₂ | -(CH₂)₃CON(CH₃) (CH₂)₂N(CH₃)CO(CH₂)₃- | 2 Lys | MH+= 899,52 RT = 1,32 min (Méthode B) |
| **92** | H | NHCO* | CCH₃ | CO₂H | NH₂ | 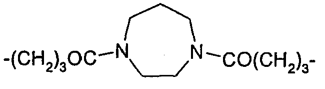 | 2 Lys | MH+= 911,48 RT=1,29 min (Méthode B) |
| **93** | H | NHCO* | CCH₃ | CO₂H | NH₂ | -CH₂OCH₂CON(CH₃)CH₂CH₂ (OCH₂CH₂)₂N(CH₃)COCH₂OCH₂- | 2 Lys | MH⁺ = 991,54 RT=1,33 min (Méthode B) |
| **94** | H | NHCO* | CCH₃ | CO₂H | NH₂ | -CH₂OCH₂CON(CH₃)(CH₂)₈ N(CH₃)COCH₂OCH₂- | 2 Lys | MH+= 987,53 RT=1,50 min (Méthode B) |
| **95** | H | NHCO* | CCH₃ | CO₂H | NH₂ | -CH₂OCH₂CON(CH₃)(CH₂)₆ N(CH₃)COCH₂OCH₂- | 2 Lys | MH+= 959,50 RT = 1,40 min (Méthode B) |
| **96** | H | NHCO* | CCH₃ | CO₂H | NH₂ | -CH₂OCH₂CON(CH₃)(CH₂)₃ N(CH₃)COCH₂OCH₂- | 2 Lys | MH⁺ = 917,57 RT=1,43 min (Méthode B) |
| **97** | H | NHCO* | CCH₃ | CO₂H | NH₂ | 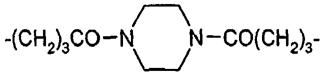 | 2 Lys | MH+= 897,35 RT=1,36 min (Méthode B) |
| **98** | H | NHCO* | CCH₃ | CO₂H | NH₂ | 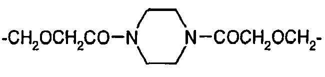 | 2 Lys | MH+= 901,54 RT = 1,43 min (Méthode B) |
| **99** | H | NHCO* | CCH₃ | CO₂H | NH₂ | -(CH₂)₄CON(CH₃) (CH₂)₃N(CH₃)CO(CH₂)₄- | 2 Lys | MH⁺ = 941,65 RT = 1,53 min (Méthode B) |
| **100** | H | NHCO* | CCH₃ | CO₂H | NH₂ | -(CH₂)₂CON(CH₃) (CH₂)₆N(CH₃)CO(CH₂)₂- | 2 Lys | MH+= 926,17 RT = 1,73 min (Méthode C) |
| **101** | H | NHCO* | CCH₃ | CO₂H | NH₂ | -CH₂OCH₂CON(CH₃)CH₂CH₂-OCH₂CH₂N(CH₃)COCH₂OCH₂- | 2 Lys | MH+= 947,37 RT = 1,33 min (Méthode B) |
| **102** | H | NHCO* | CCH₃ | CO₂H | NH₂ | -(CH₂)₄CON(CH₃) (CH₂)₂N(CH₃)CO(CH₂)₄- | 2 Lys | MH+= 927,63 RT=1,45 min (Méthode B) |
| **103** | H | NHCO* | CCH₃ | CO₂H | NH₂ | -(CH₂)₃CON(CH₃) (CH₂)₃N(CH₃)CO(CH₂)₃- | 2 Lys | MH+= 913,61 RT=1,45 min (Méthode B) |
| **104** | H | NHCO* | CCH₃ | CO₂H | NH₂ | -CH₂OCH₂CON(CH₃)CH₂CH₂-N(CH₃)COCH₂OCH₂- | 2 Lys | MH+= 903,56 RT = 1,38 min (Méthode B) |
| **105** | H | -NHCOPh-3-O* | CCH₃ | CO₂H | NH₂ | -CH₂CONHCH₂CONHCH₂CH₂- | 2 Na, 6,2 H₂O | 313-318°C |
| **106** | H | -Ph-4-O* | CCH₃ | CO₂H | NH₂ | -(CH₂CH₂O)₂CH₂CH₂- | 2 Na, 4 H₂O | 257°C |
| **107** | H | -Ph-4-O* | CCH₃ | CO₂H | NH₂ | -(CH₂CH₂0O)₃CH_{Z}CH₂- | 2 Na, 4 H₂O | 245°C |
| **108** | H | -Ph-3-O* | CCH₃ | CO₂H | NH₂ | -(CH₂CH₂O)₂CH₂CH₂- | 2 Na, 4 H₂O | 258°C |
| **109** | H | -Ph-3-O* | CCH₃ | CO₂H | NH₂ | -(CH_{Z}CH₂O)₃CH₂CH₂- | 2 Na, 2,5 H₂O | 236°C |
| **110** | H | -Ph-3-O* | CCH₃ | CO₂H | NH₂ | -CH₂CONH(CH₂)₂NHCOCH₂- | 2 Na, 4 H₂O | 237°C |
| **111** | H | -Ph-3-O* | CCH₃ | CO₂H | NH₂ | -CH₂CONH(CH₂)₆NHCOCH₂- | 2 Na, 5 H₂O | 224°C |
| **112** | H | -Ph-4-O* | CCH₃ | CO₂H | NH₂ | -CH₂CONH(CH₂)₆NHCOCH₂- | 2 Na, 3 H₂O | 241°C |
| **113** | H | -Ph-4-O* | CCH₃ | CO₂H | NH₂ | -CH₂CONH(CH₂)₂NHCOCH₂- | 2 Na, 6 H₂O | 279°C |
| **114** | 6-OCH₂-CH₂NH* | OCH₃ | CCH₃ | CO₂H | NH₂ | -COCH₂OCH₂CONHCH₂-CH₂NHCOCH₂OCH₂CO- | 2 Lys | MH+=1023,41 RT=1,44 min (Méthode B) |
| **115** | 6-OCH₂-CH₂NH* | OCH₃ | CCH₃ | CO₂H | NH₂ | -COCH₂OCH₂CONHCH₂CH₂O CH₂CH₂NHCOCH₂OCH₂CO- | 2 Lys | MH+= 1067,42 RT=1,48 min (Méthode B) |
| **116** | 6-OCH₂-CH₂NH* | OCH₃ | CCH₃ | CO₂H | NH₂ | -COCH₂OCH₂CONH(CH₂CH₂O)₂-CH₂CH₂NHCOCH₂OCH₂CO- | 2 Lys | MH+= 1111,46 RT=1,46min (Méthode B) |

Hétérodimères

| **Ex** | **R** | **R₁** | **X** | **R₃** | **R₄** | **L** | **Sel** | **PF (°C)** |
|---|---|---|---|---|---|---|---|---|
| **117** | H | -NHCOPh-3-O* | CCH₃ | CO₂H | NH₂ | -CH₂CONH(CH₂)₂NHCOCH₂- | 2 Na, 6 H₂O | 278°C |
| | H | -NHCOPh-3-O* | N | CO₂H | NH₂ | | | |
| **118** | H | -NHCOPh-3-O* | CCH₃ | CO₂H | NH₂ | -CH₂CONH(CH₂)₂NHCOCH₂- | 2 Lys, 9,5 H₂O | 213°C |
| | H | -NHCOPh-4-O* | CCH₃ | CO₂H | NH₂ | | | |
| **119** | H | -O* | CCH₃ | CO₂H | NH₂ | -(CH₂CH₂O)₆CH₂CH₂- | 2 Na, 4,5 H₂O | 280°C |
| | 8-O* | -OCH₃ | CCH₃ | CO₂H | NH₂ | | | |
| **120** | 8-O* | -OCH₃ | CCH₃ | CO₂H | NH₂ | -(CH₂CH₂O)₆CH₂CH₂- | 2 Na, 6,4 H₂O 0,1 acétone | 274°C |
| | 6-O* | -OCH₃ | CCH₃ | CO₂H | NH₂ | | | |
| **121** | H | -O* | CCH₃ | CO₂H | NH₂ | -(CH₂CH₂O)₆CH₂CH₂- | 2 Na, 8 H₂O | 196°C |
| | H | -OCH₃ | CPh-4-O* | CO₂H | NH₂ | | | |

Les exemples suivants, donnés à titre non limitatif, illustrent la présente invention.

Dans ce qui suit :
- Les spectres de masses ont été enregistrés sur des appareils de chromatographie (Alliance 2695 Waters, détecteur PDA) et couplés à un spectromètre de masse (ZQ Waters ou Tof Waters). Les séparations chromatographiques sont réalisées en phase inverse C18 à pH=7 ou à pH=3. Les produits détectés en spectrométrie de masse sont ionisés en électrospray positif (ES+).

Les produits caractérisés par leur masse (MH⁺) et leur temps de rétention (RT) ont été analysés soit par :
- la **Méthode A**: les spectres de masse ont été enregistrés sur un appareil de chromatographie (Agilent série 1100) couplé à un spectromètre de masse (MSD Agilent série 1100). Les séparations chromatographiques sont réalisées en phase inverse C18 à pH=7 selon les conditions suivantes :
   ➢ Colonne : X terra MS C18 (2.1x50 mm) 3.5 µm
   ➢ Eluant A : tampon acétate d'ammonium pH=7.0 (10mM)
   ➢ Eluant B : acétonitrile
   ➢ Gradient : 0% à 90% de B en 30 min
   ➢ Débit : 0.4 ml/min
   ➢ Injection : 2 µl - solution à 0.5mg/ml dans le DMSO
   ➢ Détection UV : 220 nm.
   ➢ Température colonne : 30°C

   Les produits détectés en spectrométrie de masse sont ionisés en électrospray positif (ES+).
- la **Méthode B** : les spectres de masse ont été enregistrés sur un appareil de chromatographie (Waters 1525) couplé à un spectromètre de masse (Electrospray time-of flight mass spectrometer Waters LCT). Les séparations chromatographiques sont réalisées en phase inverse C18 selon les conditions suivantes :
   ➢ Colonne : YMC-Pack J'Sphere ODS H80, (33 x 2,1 mm) 4µm, 80Å
   ➢ Eluant A : 0,05% acide trifluoroacétique aqueux
   ➢ Eluant B : 0,05% acide trifluoroacétique dans l'acétonitrile
   ➢ Gradient : 5% B à 95% B en 3,4 minutes
   ➢ Débit : 1 ml/min
   ➢ Injection : 1 µl (solution à 10 mM dans le DMSO)
   ➢ Détection UV : 220 et 254 nm
   ➢ Température colonne : température ambiante
      Les produits détectés en spectrométrie de masse sont ionisés en électrospray positif (ES+).
- la **Méthode C :** les spectres de masse ont été enregistrés sur un appareil de chromatographie (Waters 2795) couplé à un spectromètre de masse (Electrospray quadrupole mass spectrometer (Waters Ultima). Les séparations chromatographiques sont réalisées en phase inverse C18 selon les conditions suivantes :
   ➢ Colonne : YMC-Pack J'Sphere ODS H80, (33 x 2,1 mm) 4µm, 80Å
   ➢ Eluant A : 0,1% acide formique aqueux
   ➢ Eluant B : 0,08% acide formique dans l'acétonitrile
   ➢ Gradient : 5% B à 95% B en 2,5 minutes
   ➢ Débit : 1,3 ml/min
   ➢ Injection : 20 µl (solution à 2 mM dans le DMSO)
   ➢ Détection UV : 220 et 254 nm
   ➢ Température colonne : température ambiante
      Les produits détectés en spectrométrie de masse sont ionisés en électrospray positif (ES+).
- la **Méthode D** : les spectres de masse ont été enregistrés sur un appareil de chromatographie (Agilent série 1100) couplé à un spectromètre de masse (MSD Agilent série 1100). Les séparations chromatographiques sont réalisées en phase inverse C18 selon les conditions suivantes :
   ➢ Colonne : YMC-Pack J'Sphere ODS H80, (20 x 2,1 mm) 4µm
   ➢ Eluant A : 0,05% acide trifluoracétique aqueux
   ➢ Eluant B : acétonitrile
   ➢ Gradient : 96% A à 95% B en 2 minutes, 95% B jusqu'à 2.40 min puis 96 % A à 2.45 min
   ➢ Débit : 1 ml/min
   ➢ Injection : 2 µl - solution à 0.5mg/ml dans le DMSO
   ➢ Détection UV : DAD 220, 254, 324 nm.
   ➢ Température colonne : 30°C
      Les produits détectés en spectrométrie de masse sont ionisés en électrospray positif (ES+).
- Les points de fusion sont déterminés sur l'appareil Büchi B-540.
- Les spectres RMN du proton ont été enregistrés à l'aide d'un spectromètre AVANCE 250 BRUKER et AVANCE 400 BRUKER. Les déplacements chimiques sont exprimés en ppm par rapport au DMSO utilisé comme référence interne. Les abréviations utilisées pour la multiplicité des signaux sont respectivement : s, d, t, q et m pour singulet, doublet, triplet, quadruplet et multiplet. Les spectres RMN-¹H sont effectués dans le (CD₃)₂SO.
- Les purifications par chromatographie flash ont été effectuées à l'aide de silice Merck 60 (15 - 40 µm). Les purifications par chromatographie d'exclusion stérique ont été effectuées à l'aide sur gel Sephadex^{™} LH20 Amersham Biosciences.

### Exemple 1 : Sel disodique du 3,3'-{3,6,9,12,15,18-hexaoxaicosane-1,20-diylbis[oxy(1-méthoxy-2-méthylindolizine-8,3-diyl)carbonyl]}bis(acide 6-aminobenzoïque)

### Etape A : 3-(Benzyloxy)-2-(chlorométhyl)pyridine

On ajoute 25,2 ml de chlorure de thionyle à la solution de 40 g (0,19 mole) de 3-(benzyloxy)-2-(hydroxyméthyl)pyridine (CAS 6059-29-6 ; Desideri, N; Sestili, I; Manarini, S; Cerletti, C; Stein ; Eur. J. Med. Chem. Chim. Ther.; 26 (4) 1991; 455-460) dans 265 ml de dichlorométhane. La solution est agitée à température ambiante sous azote, puis concentrée à sec. Le résidu obtenu est mis en solution dans l'eau. On ajoute du bicarbonate de sodium jusqu'à obtention d'une solution à pH neutre. La solution aqueuse est extraite avec de l'acétate d'éthyle. La phase organique est lavée avec une solution aqueuse saturée de chlorure de sodium, séchée sur sulfate de sodium anhydre puis concentrée à sec. On obtient 41 g (95 %) d'une huile marron.
MH⁺ = 234,2

### Etape B : 3-(Benzyloxy)-2-(méthoxyméthyl)pyridine

A la solution de méthylate de sodium préparée par ajout de 5,2 g (0,23 mole) de sodium à 150 ml de méthanol, on ajoute la solution de 41 g (0,18 mole) de la 3-(benzyloxy)-2-(chlorométhyl)pyridine dans 100 ml de méthanol. Après chauffage à reflux pendant 3 heures sous azote, la solution est concentrée à sec. L'huile obtenue est reprise dans l'eau et extraite à l'acétate d'éthyle. La solution organique est lavée avec une solution aqueuse saturée de chlorure de sodium, séchée sur sulfate de sodium anhydre puis concentrée à sec. On obtient 39 g (97%) d'une huile marron.
¹H-RMN [(CD₃)₂SO, 250 MHz] : 8.15 (1H, d), 7.30-07.55 (7H, m), 5.22 (2H, s), 4.55 (2H, s), 3.31 (3H, s).

### Etape C : 8-(Benzyloxy)-1-méthoxy-2-méthylindolizine

Le mélange de 17,2 g (75,0 m.moles) de 3-(benzyloxy)-2-(méthoxyméthyl)pyridine, 17,4 g (0,19 mole) de chloroacétone et 16,3 g (0,19 mole) de bromure de lithium dans 140 ml d'acétonitrile est chauffé à reflux pendant 24 heures. On verse le milieu réactionnel dans l'eau et on lave la phase aqueuse à deux reprises avec de l'acétate d'éthyle. La phase aqueuse est concentrée à sec pour donner 22,9 g d'une huile marron.

A la solution de la pyridine quaternisée dans 260 ml d'acétonitrile chauffée à reflux, on ajoute 29 ml (0,19 mole) de triéthylamine. La solution est chauffée à reflux pendant 3 heures puis concentrée à sec. On reprend le résidu dans l'eau et on extrait avec de l'acétate d'éthyle. La phase organique est lavée avec une solution aqueuse saturée de chlorure de sodium, séchée sur sulfate de sodium et concentrée sous pression réduite. On obtient 14,3 g d'une huile verte utilisée tel quelle dans l'étape d'acylation.
MH+ = 268,2

### Etape D : 5-{[8-(Benzyloxy)-1-méthoxy-2-méthylindolizin-3-yl]carbonyl}-2-[(trifluoroacétyl)amino]benzoate de méthyle

A la solution de 13,2 g (49,4 m.moles) de 8-(benzyloxy)-1-méthoxy-2-méthylindolizine dans 165 ml de dichlorométhane, on ajoute 4,4 ml (54,3 m.moles) de pyridine puis 15,0 g (48,4 m.moles) de 5-(chlorocarbonyl)-2-[(trifluoroacétyl)amino]benzoate de méthyle (décrit dans la demande de brevet W02003084956) sous argon. Le milieu réactionnel est agité à température ambiante pendant 3 heures. On dilue avec du dichlorométhane et on lave cette solution organique avec une solution aqueuse saturée de bicarbonate de sodium puis une solution aqueuse saturée de chlorure de sodium et on la sèche sur sulfate de sodium. Après concentration sous pression réduite, le solide obtenu est repris dans l'éthanol, filtré et lavé à l'éthanol pour fournir 16,2 g (61 %) d'une poudre orange.
MH⁺ = 541,5 ; point de fusion : 214 °C

### Etape E : 2-Amino-5-{[8-(benzyloxy)-1-méthoxy-2-méthylindolizin-3-yl]carbonyl}benzoate de méthyle

On ajoute 1,15 g (8,33 m.moles) de carbonate de potassium à la solution de 0,90 g (1,67 m.mole) de 5-{[8-(benzyloxy)-1-méthoxy-2-méthylindolizin-3-yl]carbonyl}-2-[(trifluoroacétyl)amino]benzoate de méthyle dans 100 ml de méthanol et 100 ml de dichlorométhane. Le mélange est agité à température ambiante pendant 20 heures. Le précipité jaune formé est filtré, lavé abondamment à l'eau et séché pour fournir 0,61 g (83%) d'une poudre jaune.
MH⁺ = 445,5 ; point de fusion : 174 °C

### Etape F : 2-Amino-5-[(8-hydroxy-1-méthoxy-2-méthylindolizin-3-yl)carbonyl]benzoate de méthyle

Le mélange de 2,0 g (4,5 m.moles) de 2-amino-5-{[8-(benzyloxy)-1-méthoxy-2-méthylindolizin-3-yl]carbonyl}benzoate de méthyle et de 0,42 g (6,75 m.moles) de formiate d'ammonium en présence de 1,0 g de palladium sur charbon (10%) dans 20 ml de N,N-diméthylformamide, est agité pendant 12 heures à température ambiante sous argon. On filtre le mélange sous azote et on le concentre à sec pour obtenir 1,51 g d'une huile rouge. Le produit se dégrade au contact de l'air.
MH+ = 355,3

### Etape G : 3,3'-{3,6,9,12,15,18-hexaoxaicosane-1,20-diylbis[oxy(1-méthoxy-2-méthylindolizine-8,3-diyl)carbonyl]}bis(6-aminobenzoate de méthyle)

A la solution de 1,30 g (3,66 m.moles) de 2-amino-5-[(8-hydroxy-1-méthoxy-2-méthylindolizin-3-yl)carbonyl]benzoate de méthyle dans 12 ml de tétrahydrofurane, on ajoute goutte à goutte 8,0 ml (4,02 m.moles) d'hexaméthyldisilylamidure de potassium (solution 0,5 M dans toluène) à -20 °C sous argon. On observe la formation d'un précipité rouge. On laisse revenir le mélange à température ambiante et on ajoute 6 ml de N,N-diméthylformamide puis 1,0 g (1,83 m.mole) de 1,20-diiodo-3,6,9,12,15,18-hexaoxaicosane (Exemple R1). On agite la solution à 40°C pendant 18 heures. On verse le milieu réactionnel dans une solution d'acide chlorhydrique (1 M) et on extrait avec de l'acétate d'éthyle. La phase organique est lavée avec une solution aqueuse saturée de chlorure de sodium, séchée sur sulfate de sodium et concentrée sous pression réduite. Le résidu obtenu est purifié par flash chromatographie sur gel de silice (dichlorométhane). On obtient 0,92 g (50 %) d'un solide vert.
MH⁺ = 999,8 ; point de fusion : 199°C

### Etape H : Sel disodique du 3,3'-{3,6,9,12,15,18-hexaoxaicosane-1,20-diylbis[oxy(1-méthoxy-2-méthylindolizine-8,3-diyl)carbonyl]}bis(acide 6-aminobenzoïque)

On ajoute 1,35 ml de soude (1 M) à la solution de 0,45 g (0,45 m.mole) du dimère obtenu dans l'étape E ci-dessus dans 4,5 ml de 1-méthyl-2-pyrolidinone. On agite la solution à température ambiante pendant 2 jours. On verse le milieu réactionnel dans 150 ml d'acétone. Le précipité jaune est filtré et séché pour fournir 356 mg d'une poudre jaune (sel disodique, 1,5 H2O).
point de fusion : 265°C
¹H-RMN [(CD₃)₂SO, 250MHz] : 8.61 (2H, d), 8.15 (2H, d), 7.30-8.00 (4H, large s), 7.36 (2H, d), 6.53-6.61 (4H, m), 6.38 (2H, dd), 4.20-4.25 (4H, m), 3.83-3.87 (4H, m), 3.78 (6H, s), 3.63-3.67 (4H, m), 3.47-3.57 (16H, m), 1.94 (6H, s).

### Exemples 2 à 6

En suivant les procédés décrits dans les Etapes G et H de l'**Exemple 1**, on prépare les **Exemples 2 à 6** par dimérisation du 2-amino-5-[(8-hydroxy-1-méthoxy-2-méthylindolizin-3-yl)carbonyl]benzoate de méthyle (Etape F de l'**Exemple 1**) avec les dérivés diiodés adéquats (Exemples R2 à R6).

### Exemnle 7 : Sel disodique du 3,3'-{undécane-1,11-diylbis[imino(2-oxoéthane-2,1-diyl)oxy(1-méthoxy-2-méthylindolizine-8,3-diyl)carbonyl]}bis(acide 6-aminobenzoïque)

### Etape A : 2-Amino-5-{[8-(2-tert-butoxy-2-oxoéthoxy)-1-méthoxy-2-méthylindolizin-3-yl]carbonyl}benzoate de méthyle

A la solution de 1,50 g (4,23 m.moles) de 2-amino-5-[(8-hydroxy-1-méthoxy-2-méthylindolizin-3-yl)carbonyl]benzoate de méthyle (Etape F de l'**Exemple 1**) dans 15 ml de tétrahydrofurane à -20 °C sous argon, on ajoute goutte à goutte 5,4 ml (9,31 m.moles) d'hexaméthyldisilylamidure de potassium (solution 0,5 M dans toluène) puis 0,69 ml (4,66 m.moles) de 2-bromoacétate de *tert-*butyle. On agite pendant 2 heures en laissant revenir le mélange à température ambiante. On verse le milieu réactionnel dans une solution aqueuse saturée d'hydrogénosulfate de potassium et on extrait avec de l'acétate d'éthyle. La phase organique est lavée avec une solution aqueuse saturée de chlorure de sodium, séchée sur sulfate de sodium et concentrée sous pression réduite. L'huile obtenue est purifiée par flash chromatographie sur gel de silice (gradient dichlorométhane/méthanol = 100/0 à 90/10). On obtient 1,26 g (64 %) d'une poudre jaune.
MH⁺ = 469,5 ; point de fusion : 188°C

### Etape B : Acide ({3-[4-amino-3-(méthoxycarbonyl)benzoyl]-1-méthoxy-2-méthylindolizin-8-yl}oxy)acétique

On agite la solution de 0,68 g (1,45 m.mole) du 2-amino-5-{[8-(2-*tert-*butoxy-2-oxoéthoxy)-1-méthoxy-2-méthylindolizin-3-yl]carbonyl}benzoate de méthyle dans 4,44 ml d'acide trifluoroacétique et 7 ml de dichlorométhane. Après 48 heures d'agitation à température ambiante, on concentre le milieu réactionnel à sec et on reprend le solide rouge obtenu dans de l'éther éthylique. On filtre le solide en suspension et on le sèche à 50°C sous vide pour obtenir 0,58 g (95%) d'une poudre orange.
MH⁺ = 413,4 ; point de fusion : 164°C

### Etape C : 3,3'-{Undécane-1,11-diylbis[imino(2-oxoéthane-2,1-diyl)oxy(1-méthoxy-2-méthylindolizine-8,3-diyl)carbonyl]}bis(6-aminobenzoate de méthyle)

A la solution de 0,66 g (1,59 m.mole) de l'acide ({3-[4-amino-3-(méthoxycarbonyl)benzoyl]-1-méthoxy-2-méthylindolizin-8-yl}oxy)acétique dans 8 ml de N,N-diméthylformamide, on ajoute successivement 0,33 ml (2,38 m.moles) de triéthylamine et 0,56 g (1,75 m.mole) de TBTU (O-benzotriazol-1-yl-N,N,N',N'-tétraméthyluronium tétrafluoroborate) puis 0,148 g (0,79 m.mole) de 1,11-diaminoundécane. Le mélange réactionnel est agité à température ambiante sous azote pendant 3 jours, dilué avec de l'acétate d'éthyle, lavé avec une solution de bicarbonate de sodium saturée puis avec une solution de chlorure de sodium saturée. La phase organique est séchée sur sulfate de sodium et concentrée à sec. Le solide obtenu est purifié par chromatographie d'exclusion stérique sur gel Sephadex® LH20 (N,N-diméthylformamide), on obtient 0,18 g (23%) d'une poudre jaune.
MH⁺ = 975,5

### Etape D : 3,3'-{Undécane-1,11-diylbis[imino(2-oxoéthane-2,1-diyl)oxy(1-méthoxy-2-méthylindolizine-8,3-diyl)carbonyl]}bis(acide 6-aminobenzoïque)

On ajoute 0,35 ml de soude (1 M) à la solution de 0,17 g (0,18 m.mole) du dimère obtenu dans l'étape C ci-dessus dans 1 ml de 1-méthyl-2-pyrolidinone. On agite la solution à température ambiante pendant 3 jours. On verse le milieu réactionnel dans une solution aqueuse d'hydrogénosulfate de potassium. Le précipité jaune est filtré,séché et purifié par chromatographie FPLC sur gel C8 Kromasyl 10µ [gradient méthanol / solution aqueuse d'acétate d'ammonium 0.017 M / acétonitrile = 5/95/0 à 45/5/50]. On obtient 28 mg d'une poudre jaune
MH+ = 947,7

### Etape E

A la suspension de 27 mg (0.03 m.mole) du diacide carboxylique obtenu dans l'Etape D ci-dessus dans 3 ml de méthanol, on ajoute 60 µl de soude (1 M). On concentre la solution et on reprend le solide obtenu avec de l'acétone. On filtre l'insoluble et on le sèche sous vide à 50°C pour obtenir 17 mg d'une poudre jaune (sel disodique, 3.5 moles d'eau).
point de fusion : 220°C
¹H-RMN [(CD₃)₂SO, 250MHz] : 8.61 (2H, d), 8.14 (2H, s), 7.98 (2H, t), 7.50-8.00 (4H, large s), 7.37 (2H, d), 6.53-6.60 (4H, m), 6.35 (2H, dd), 4.67 (4H, s), 3.81 (6H, s), 3.18 (4H, dd), 1.96 (6H, s), 1.40-1.50 (4H, m), 1.20-1.30 (14H, m)

### Exemple 8: Sel disodique du diacide 3,3'-(3,6,9,12,15-pentaoxaheptadécane-1,17-diylbis{oxy[3-(4-amino-3-méthoxybenzoyl)imidazo[1,5-a]pyridine-8,1-diyl]})benzoïque

### Etape A : 3,3'-(3,6,9,12,15-Pentaoxaheptadécane-1,17-diylbis{oxy[3-(4-amino-3-méthoxybenzoyl)imidazo[1,5-a]pyridine-8,1-diyl]})dibenzoate de méthyle

A 1,6 g (3,83 m.moles) de 3-[3-(4-amino-3-méthoxybenzoyl)-8-hydroxyimidazo[1,5-*a*]pyridin-1-yl]benzoate de méthyle (décrit dans la demande de brevet W02006097625) en solution dans 15 ml de *N,N*-diméthylformamide à 0°C, on ajoute 0.2 g (4,60 m.moles) d'hydrure de sodium (60% en dispersion dans l'huile) par portion, puis au bout de 10 minutes 0,25 g (1,92 m.mole) du 1,17-diiodo-3,6,9,12,15-pentaoxaheptadécane (Exemple R2). On agite à 60°C pendant 6 heures. Le milieu réactionnel est versé dans une solution d'acide chlorhydrique (0,1 M). Le précipité est filtré, lavé à l'eau puis séché à 50°C sous vide. Après purification par chromatographie sur gel de silice (gradient dichlorométhane/acétone = 95/5 à 75/25), on obtient 0,6 g (29%) d'une poudre jaune.
MH⁺ = 1081,7 ; point de fusion : 80,2°C

### Etape B : Diacide 3,3'-(3,6,9,12,15-pentaoxaheptadécane-1,17-diylbis{oxy[3-(4-amino-3-méthoxybenzoyl)imidazo[1,5-a]pyridine-8,1-diyl]})benzoïque

La solution de 0,4 g (0,37 m.mole) du dimère obtenu dans l'Etape A ci-dessus dans 0,92 ml de soude (1 M) et 7,4 ml de diméthylsulfoxide, est chauffée à 100 °C pendant 5 minutes. Après refroidissement, elle est coulée dans 200 ml d'eau contenant 0,13 g d'hydrogénosulfate de potassium. Le précipité obtenu est filtré, lavé et séché sous vide à 50°C. On obtient 0,36 g (92%) d'une poudre jaune.
MH⁺ = 1053,4

### Etape C

On ajoute 0,60 ml de soude (1 M) à la solution de 0,323 g (0,31 m.mole) du dimère obtenu dans l'étape B ci-dessus dans 30 ml de méthanol. On agite la solution à température ambiante pendant 30 minutes. Le solide obtenu est repris dans l'acétone, filtré et séché sous vide à 50°C pour fournir 0,33 g d'une poudre jaune (sel disodique, 5 moles d'eau).
point de fusion : 209,6°C
¹H-RMN [(CD₃)₂SO, 400MHz] : 9.33 (2H, d), 8.44 (2H, s), 8.19 (2H, s), 8.13 (2H, d), 7.87 (2H, d), 7.82 (2H, d), 7.32 (2H, dd), 7.03 (2H, dd), 6.71 (4H, m), 5.79 (4H, s), 4.20-4.30 (4H, m), 3.90 (6H, s), 3.70-3.85 (4H, dd), 3.30-3.50 (16H, m)

### Exemple 9 :

En suivant les procédés décrits dans les Etapes A à C de l'**Exemple 8**, on prépare l'**Exemple 9** par dimérisation de la (4-amino-3-méthoxyphényl)(8-hydroxy-1-pyridin-4-ylimidazo[1,5-a]pyridin-3-yl)méthanone (composé obtenu en adaptant les protocoles décrits dans la demande de brevet WO2006097625 pour la préparation du 3-[3-(4-amino-3-méthoxybenzoyl)-8-hydroxyimidazo[1,5-*a*]pyridin-1-yl]benzoate de méthyle) avec le dérivé diiodé adéquat (Exemple R2).

### Exemple 10: Chlorhydrate de la [3,6,9,12,15-pentaoxaheptadécane-1,17-diylbis(oxyimidazo[1,5-a]pyridine-8,3-diyl)]bis[(4-amino-3-méthoxyphényl)méthanone]

### Etape A : [3,6,9,12,15-Pentaoxaheptadécane-1,17-diylbis(oxyimidazo[1,5-a]pyridine-8,3-diyl)]bis[(4-amino-3-méthoxyphényl)méthanone]

En suivant le procédé décrit dans l'Etape A de **l'Exemple 8,** on prépare **l'Exemple 10** à partir de (4-amino-3-méthoxyphényl)(8-hydroxyimidazo[1,5-*a*]pyridin-3-yl)méthanone (décrit dans la demande de brevet W02006097625) avec le 1,17-diiodo-3,6,9,12,15-pentaoxaheptadécane (Exemple R2). Après purification par chromatographie flash sur gel de silice (gradient dichlorométhane / acétone = 90/10 à 70/30), on obtient 0,93 g (27%) d'une poudre jaune.
MH⁺ = 813,5,

### Etape B

On ajoute 0,92 ml d'éther éthylique chlorhydrique (1 M) à la suspension de 0,25 g (0,31 m.mole) du dimère obtenu dans l'Etape ci-dessus dans 6 ml de méthanol. On concentre le mélange à sec, on reprend le solide dans l'éther éthylique, on le filtre et on le sèche à 50°C sous vide pour obtenir 0,22 g d'une poudre jaune (dichlorhydrate, 5 moles d'eau).
point de fusion : 209°C
¹H-RMN [(CD₃)₂SO, 400MHz] : 9.23 (2H, d), 8.21 (2H, dd), 7.90 (2H, s), 7.74 (2H, s), 7.03 (2H, dd), 6.80 (2H, d), 6.71 (2H, d), 4.00-5.00 (4H, large s), 4.30-4.35 (4H, m), 3.80-3.90 (8H, m), 3.60-3.70 (4H, m), 3.45-3.60 (12H, m)

### Exemple 11 : Sel disodique du 3,3'-{3,6,9,12,15,18-hexaoxaicosane-1,20-diylbis[oxy(1-méthoxy-2-méthylindolizine-6,3-diyl)carbonyl]}bis(acide 6-aminobenzoïque)

### Etape A : 5-(Benzyloxy)-2-(méthoxyméthyl)pyridine

A la solution de méthylate de sodium préparée par ajout de 6,4 g (0,28 mole) de sodium à 150 ml de méthanol, on ajoute 50 g (0,21 mole) de la 5-benzyloxy-2-chlorométhyl-pyridine (CAS 127590-90-3 ; D. I. Scopes, N. F. Norman, D. E. Bays, D. Belton, J. Brain et a/., J. Med. Chem., 1992, 35, 490-501) en solution dans 150 ml de méthanol. Après chauffage à reflux pendant 3 heures sous azote, la solution est concentrée à sec. L'huile obtenue est reprise dans de l'acétate d'éthyle. La solution est lavée avec une solution aqueuse saturée de chlorure de sodium, séchée sur sulfate de sodium anhydre puis concentrée à sec. On obtient 46 g (94%) d'une huile marron.
MH⁺ = 230,1

### Etape B : 6-(Benzyloxy)-1-méthoxy-2-méthylindolizine

A la suspension de 22 g (0,25 mole) de bromure de lithium et 20 ml (0,25 mole) de chloroacétone dans 250 ml d'éthanol à température ambiante, on ajoute goutte à goutte 22,5 g (0,098 mole) de 5-(benzyloxy)-2-(méthoxyméthyl)pyridine en solution dans 10 ml d'éthanol à température ambiante. Le mélange est porté à reflux pendant 17 heures, puis concentré à sec. Il est repris avec de l'acétate d'éthyle et extrait avec de l'eau. La phase aqueuse qui contient la pyridine quaternisée, est lavée avec de l'acétate d'éthyle puis concentrée à sec.

On obtient une huile marron qui est mise en solution dans un mélange de 400 ml d'acétonitrile et 13 ml d'éthanol. La solution est portée à reflux avant l'ajout goutte à goutte de 41 ml (0.30 mole) de triéthylamine. Le mélange est maintenu 2 heures 30 à reflux. Le milieu réactionnel est de couleur marron foncé homogène. Il est concentré, puis repris avec de l'acétate d'éthyle et lavé avec de l'eau puis avec une solution aqueuse saturée de chlorure de sodium. La phase organique est séchée sur du sulfate de sodium anhydre, puis concentrée à sec. On obtient 12.8 g d'un solide marron vert qui est utilisé tel quel dans l'étape suivante.
MH⁺ = 268,1

### Etape C : 6-{[6-(Benzyloxy)-1-méthoxy-2-méthylindolizin-3-yl]carbonyl}-2-phényl-4H-3,1-benzoxazin-4-one

A la solution de 12,8 g (47,9 m.moles) de 6-(benzyloxy)-1-méthoxy-2-méthylindolizine dans 160 ml de dichlorométhane, on ajoute 10 ml (71,8 m.moles) de triéthylamine et 15 g (52,7 m.moles) de chlorure de 4-oxo-2-phényl-4*H*-3,1-benzoxazine-6-carbonyle par portion à température ambiante et sous azote. Le milieu réactionnel est agité pendant 2 heures puis il est filtré. Le solide obtenu est lavé avec du dichlorométhane puis de l'éther diisopropylique et séché. On obtient 17,8 g (83 %) d'une poudre orange.
MH⁺ = 517,3 ; point de fusion : 224.6°C (décomposition)

### Etape D : Acide 2-Amino-5-[(6-benzyloxy-1-méthoxy-2-méthylindolizin-3-yl)carbonyl] benzoïque

A la suspension de 2,0 g (3,87 m.moles) de 6-{[6-(benzyloxy)-1-méthoxy-2-méthylindolizin-3-yl]carbonyl}-2-phényl-4*H*-3,1-benzoxazin-4-one dans 15 ml de *N-*méthyle-2-pyrrolidone à température ambiante, on ajoute une solution de 2,17 g (38,7 m.moles) d'hydroxyde de potassium dans 6 ml d'eau. Le mélange est porté à reflux pendant 4 heures. Après refroidissement, le milieu réactionnel est versé dans une solution aqueuse d'acide chlorhydrique (0,1 M). Le précipité formé est filtré, lavé abondamment à l'eau puis à l'éther diisopropylique. On obtient 1,15 g (70%) d'une poudre jaune après séchage sous vide à 50°C.
MH⁺ = 431,4 ; point de fusion : 246 °C (décomposition)

### Etape E : 2-Amino-5-[(6-benzyloxy-1-méthoxy-2-méthylindolizin-3-yl)carbonyl]benzoate de méthyle

Au mélange de 1,19 g (2,76 m.moles) de l'acide carboxylique obtenu dans l'Etape E ci-dessus et de 0,5 g (3,59 m.moles) de carbonate de potassium dans 7,0 ml de *N,N*-diméthylformamide, on ajoute 0,34 ml (5,53 m.moles) d'iodure de méthyle. La solution est agitée pendant 4 heures à température ambiante sous azote, puis elle est versée dans une solution aqueuse d'hydrogénocarbonate de sodium saturé. Le précipité formé est filtré et lavé abondamment avec de l'eau puis de l'éther diisopropylique pour fournir 0,96 g (90 %) d'une poudre jaune après séchage.
MH⁺ = 445,2 ; point de fusion : 189°C (décomposition)

### Etape F : 2-Amino-5-[(6-hydroxy-1-méthoxy-2-méthylindolizin-3-yl)carbonyl]benzoate de méthyle

Le mélange de 0,95 g (2,14 m.moles) du 2-amino-5-[(6-benzyloxy-1-méthoxy-2-méthylindolizin-3-yl)carbonyl]benzoate de méthyle, 0,21 g (3,21 m.moles) de formiate d'ammonium et 0,19 g de palladium sur charbon à 10% dans 12 ml de *N,N-*diméthylformamide, est agité pendant 4 heures à température ambiante. Il est ensuite filtré et concentré à sec. Le solide obtenu est repris avec de l'éther diisopropylique, puis filtré pour fournir 0,64 g (85%) d'une poudre jaune.
MH⁺ = 355,3 ; point de fusion : 216 °C

### Etape G : 3,3'-{3,6,9,12,15,18-Hexaoxaicosane-1,20-diylbis[oxy(1-méthoxy-2-méthylindolizine-6,3-diyl)carbonyl]}bis(6-aminobenzoate de méthyle)

A la solution de 0,77 g (1,42 m.mole) du 2-amino-5-[(6-hydroxy-1-méthoxy-2-méthylindolizin-3-yl)carbonyl]benzoate de méthyle dans 30 ml de tétrahydrofurane à - 40°C, on ajoute 5,68 ml (2,84 m.moles) de bis(triméthylsilyl)amidure de potassium (0,5M dans toluène). Un précipité se forme. On laisse le milieu réactionnel revenir à température ambiante puis on ajoute 20 ml de *N,N*-diméthylformamide et 0,64 g (1,41mmol) de 1,20-diiodo-3,6,9,12,15,18-hexaoxaicosane (Exemple R1). La solution est chauffée pendant 24 heures à 60°C. On coule le milieu réactionnel dans une solution de 0,9 g d'hydrogénosulfate de potassium dans 100 ml d'eau et on extrait avec de l'acétate d'éthyle. La phase organique est lavée avec de l'eau puis avec une solution aqueuse saturée de chlorure de sodium, séchée sur sulfate de sodium et concentrée à sec. L'huile marron obtenue est purifiée par chromatographie flash sur gel de silice (gradient toluène/acétate d'éthyle = 100/0 à 0/100). On obtient 0,35 g (24%) d'une poudre jaune.
MH⁺ = 999,4 ; point de fusion : 80°C

### Etape H

On ajoute 0,41 ml de soude (1 M) à la suspension de 0,20 g (0,21 m.mole) du dimère obtenu dans l'étape F ci-dessus dans 0,5 ml de méthanol. On agite la solution à température ambiante pendant 30 minutes puis on concentre la solution à sec. Le solide obtenu est repris dans l'acétone, filtré et séché sous vide à 50 °C pour fournir 0,14 g d'une poudre jaune (sel disodique, 7,4 moles d'eau).
point de fusion : 181°C
¹H-RMN [(CD₃)₂SO, 250MHz] : 8.89 (2H, s), 8.11 (2H, s), 7.49 (2H, d), 7.40-8.10 (4H, large s), 7.35 (2H, dd), 6.86 (2H, dd), 6.58 (2H, dd), 4.00-4.10 (4H, m), 3.81 (6H, s), 3.70-3.08 (4H, m), 3.20-3.65 (20H, m), 1.94 (6H, s)

### Exemples 12 à 16

En suivant les procédés décrits dans les Etapes F à H de **l'Exemple 11**, on prépare les **Exemples 12** à **16** par dimérisation du 2-amino-5-[(6-hydroxy-1-méthoxy-2-méthylindolizin-3-yl)carbonyl]benzoate de méthyle (Etape E de **l'Exemple 11**) avec les dérivés diiodés adéquats (Exemples R2 à R6).

### Exemples 17 à 20

En suivant les procédés décrits dans les Etapes F à H de **l'Exemple 11**, on prépare les **Exemples 17** et **18** par dimérisation du 2-amino-5-{[2-(4-hydroxyphényl)-1-méthoxyindolizin-3-yl]carbonyl}benzoate de méthyle (composé obtenu en adaptant les protocoles décrits dans la demande de brevet WO2003084956) et les **Exemples 19** et **20** par dimérisation du 2-amino-5-{[2-(3-hydroxyphényl)-1-méthoxyindolizin-3-yl]carbonyl}benzoate de méthyle (composé obtenu en adaptant les protocoles décrits dans la demande de brevet WO2003084956) avec les dérivés diiodés adéquats (Exemples R6 et R5).

### Exemple 21 : Sel disodique du 3,3'-{3,6,9,12,15,18-hexaoxaicosane-1,20-diylbis[oxy(2-méthylindolizine-1,3-diyl)carbonyl]}bis(acide 6-aminobenzoïque)

En suivant les procédés décrits dans les Etapes F et G de **l'Exemple 1**, on prépare **l'Exemple 21** par dimérisation du 2-amino-5-[(1-hydroxy-2-méthylindolizin-3-yl)carbonyl]benzoate de méthyle (décrit dans la demande de brevet WO2003084956) avec le 1,20-diiodo-3,6,9,12,15,18-hexaoxaicosane (Exemple R1). On obtient le sel disodique (5,5 H₂O).
point de fusion : 224°C
¹H-RMN [(CD₃)₂SO, 400MHz] : 9.06 (2H, d), 8.14 (2H, s), 7.55 (2H, d), 7.50-8.00 (4H, large s), 7.35 (2H, dd), 6.97 (2H, dd), 6.73 (2H, dd), 6.58 (2H, d), 4.05-4.15 (4H, m), 3.65-3.70 (4H, m), 3.50-3.60 (20H, m), 1.97 (6H, s)

### Exemples 22 : Sel disodique du 3,3'-{3,6,9,12,15-pentaoxaheptadécane-1,17-diylbis[oxy(2-méthylindolizine-1,3-diyl)carbonyl]}bis(acide 6-aminobenzoïque)

### Etape A : 3,3'-{3,6,9,12,15-Pentaoxaheptadécane-1,17-diylbis[oxy(2-méthylindolizine-1,3-diyl)carbonyl]}bis(6-aminobenzoate de méthyle)

A la suspension de 148 mg (3,39 m.moles) d'hydrure de sodium (60% en dispersion dans l'huile) à température ambiante dans 14 ml de *N,N* diméthylformamide sous argon à 0°C, on ajoute 1,10 g (3,39 m.moles) de 2-amino-5-[(1-hydroxy-2-méthylindolizin-3-yl)carbonyl]benzoate de méthyle par portion. Au bout de 5 minutes, on ajoute 0,85 g (1,70 m.mole) du 1,17-diiodo-3,6,9,12,15-pentaoxaheptadécane (Exemple R2) en solution dans 1 ml de N,N-diméthylformamide. On agite le mélange à température ambiante pendant 3 heures. Le milieu réactionnel est versé dans une solution saturée de bicarbonate de sodium et extrait avec de l'acétate d'éthyle. Après décantation, la phase organique est lavée avec de l'eau puis avec une solution aqueuse saturée de chlorure de sodium, séchée sur sulfate de sodium et concentrée sous pression réduite. Le résidu obtenu est purifié par chromatographie flash sur gel de silice (gradient dichlorométhane/acétate d'éthyle = 70/30 à 40/60). On obtient 0,82 g (60 %) d'une poudre jaune.
MH+ = 895,6

### Etape B : 3,3'-{3,6,9,12,15-Pentaoxaheptadécane-1,17-diylbis[oxy(2-méthylindolizine-1,3-diyl)carbonyl]}bis(acide 6-aminobenzoïque)

On ajoute 1,33 ml de soude (1 M) à la solution de 0,50 g (0,56 m.mole) du dimère obtenu dans l'étape A ci-dessus dans 4 ml de méthanol et 2 ml de 1,4-dioxane. On chauffe la solution à 80°C pendant 4 heures. La solution est concentrée à sec. Le résidu obtenu est dissout dans l'eau. On ajoute 0,2 g d'hydrogénosulfate de potassium. Le précipité jaune formé est filtré et séché pour fournir 0,42 g (87%) d'une poudre jaune.
MH⁺ = 867,5

### Etape C

Le produit est salifié par ajout de 0,85 ml de soude (1 M) à la suspension de 0,37 g (0,43 m.mole) du diacide carboxylique obtenu dans l'Etape B ci-dessus dans 50 ml de méthanol. La solution est concentrée à sec puis le solide obtenu est repris dans l'acétone, filtré et séché sous vide à 50°C. On obtient 0,36 g (93%) d'une poudre jaune (sel disodique, 4 H2O).
point de fusion : 165,4 °C
¹H-RMN [(CD₃)₂SO, 250 MHz] : 9.09 (2H, d), 8.16 (2H, s), 7.56 (2H, d), 7.37 (2H, dd), 7.00-8.00 (4H, large s), 6.98 (2H, dd), 6.73 (2H, dd), 6.61 (2H, d), 4.05-4.15 (4H, m), 3.45-3.70 (20H, m), 2.00 (6H, s)

### Exemples 23 à 29

En suivant les procédés décrits dans les Etapes A à C de **l'Exemple 22**, on prépare les **Exemples 23** à **29** par dimérisation du 2-amino-5-[(1-hydroxy-2-méthylindolizin-3-yl)carbonyl]benzoate de méthyle (décrit dans la demande de brevet WO2003084956) avec les agents alkylants adéquats (Exemples R3 à R9).

### Exemple 30: Sel disodique de 3,3'-{éthane-1,2-diylbis[imino(2-oxoéthane-2,1-diyl)oxy-3,1-phénylèneméthylèneoxy(2-méthylindolizine-1,3-diyl)carbonyl]}bis(acide 6-aminobenzoïque)

### Etape A : 2-Amino-5-[(1-{[3-(2-tert-butoxy-2-oxoéthoxy)benzyl]oxy}-2-méthylindolizin-3-yl)carbonyl]benzoate de méthyle

A la solution de 0,80 g (2,47 m.moles) de 2-amino-5-[(1-hydroxy-2-méthylindolizin-3-yl)carbonyl]benzoate de méthyle dans 12 ml de tétrahydrofurane, on ajoute 5,43 ml (2,71 m.moles) de bis(triméthylsilyl)amidure de potassium à -20°C. Après 15 minutes, on ajoute 1,49 g (4,93 m.moles) de [3-(bromométhyl)phénoxy]acétate de tert-butyle (CAS 176673-59-9 décrit dans la demande de brevet W02004014366) en solution dans 2 ml de tétrahydrofurane puis on chauffe le mélange à 40°C pendant 12 heures. On verse le milieu réactionnel dans une solution aqueuse saturée d'hydrogénosulfate de potassium, on extrait avec de l'acétate d'éthyle. La phase organique est lavée avec de l'eau puis avec une solution aqueuse saturée de chlorure de sodium, séchée sur sulfate de sodium et concentrée sous pression réduite. Après purification par chromatographie flash sur gel de silice (gradient toluène / acétate d'éthyle = 100/00 à 60/40), on obtient 0,92 g (61 %) d'une huile jaune.
MH⁺ = 543,3

### Etape B : Acide {3-[({3-[4-amino-3-(méthoxycarbonyl)benzoyl]-2-méthylindolizin-1-yl}oxy)méthyl]phénoxy}acétique

La solution de 0,87 g (1,60 m.mole) de 2-amino-5-[(1-{[3-(2-*tert*-butoxy-2-oxoéthoxy)benzyl]oxy}-2-méthylindolizin-3-yl)carbonyl]benzoate de méthyle dans 3,0 ml d'acide trifluoroacétique et 16 ml de dichlorométhane à température ambiante est agitée pendant 4 heures à température ambiante. On verse le milieu réactionnel dans une solution aqueuse saturée de bicarbonate de sodium et on lave avec de l'acétate d'éthyle. La phase aqueuse est acidifiée avec une solution aqueuse saturée d'hydrogénosulfate de potassium puis extraite avec de l'acétate d'éthyle. La phase organique est lavée avec une solution aqueuse saturée de chlorure de sodium, séchée sur sulfate de sodium et concentrée sous pression réduite. On obtient 0,69 g (88%) d'un solide vert.
MH⁺ = 489,2

### Etape C : 3,3'-{Ehane-1,2-diylbis[imino(2-oxoéthane-2,1-diyl)oxy-3,1-phénylèneméthylèneoxy(2-méthylindolizine-1,3-diyl)carbonyl]}bis(6-aminobenzoate de méthyle)

A la solution de 0,7 g (1,43 m.mole) d'acide {3-[({3-[4-amino-3-(méthoxycarbonyl)benzoyl]-2-méthylindolizin-1-yl)oxy)méthyl]phénoxy}acétique en solution dans 5 ml de N,N-diméthylformamide, on ajoute 0,4 ml (2,87 m.moles) de triéthylamine et 0,67 g (1,50 m.mole) de BOP à 0°C sous argon. Au bout de 30 minutes, on ajoute 50 µl (0,72 m.mole) d'éthane 1,2-diamine. Le milieu réactionnel est agité à température ambiante pendant 48 heures. Le milieu réactionnel est versé dans une solution aqueuse saturée d'hydrogénosulfate de potassium et on extrait avec de l'acétate d'éthyle. La phase organique est lavée avec une solution aqueuse saturée de chlorure de sodium, séchée sur sulfate de sodium et concentrée sous pression réduite. Le solide obtenu est purifié par chromatographie d'exclusion stérique sur gel Sephadex® LH20 (N,N-diméthylformamide), on obtient 262 mg (36%) d'une poudre jaune.
MH⁺ = 1001,4

### Etape D : 3,3'-{Ethane-1,2-diylbis[imino(2-oxoéthane-2,1-diyl)oxy-3,1-phénylèneméthylèneoxy(2-méthylindolizine-1,3-diyl)carbonyl]}bis(acide 6-aminobenzoïque)

On ajoute 0,67 ml de soude (1 M) à la solution de 257 mg (0,26 m.mole) du dimère obtenu dans l'Etape E ci-dessus dans 6 ml de 1-méthyl-2-pyrolidinone. On agite la solution à température ambiante pendant 3 jours. On verse le milieu réactionnel dans 100 ml d'eau contenant 99 mg d'hydrogénosulfate de potassium. Le précipité est isolé par ultrafiltration et séché. Le résidu est purifié par chromatographie d'exclusion stérique sur gel Sephadex® LH20 (*N,N*-diméthylformamide). On obtient 193 mg (76%) d'une poudre jaune.
MH+ = 973,7

### Etape E

Le produit est salifié par ajout de 0,37 ml d'une solution molaire de soude à la suspension de 185 mg (0,19 m.mole) du diacide carboxylique obtenu dans l'Etape ci-dessus dans 20 ml de méthanol. La solution est concentrée à sec puis le solide obtenu est repris dans l'acétone, filtré et séché sous vide à 50°C. On obtient 113 mg d'une poudre jaune (sel disodique , 3 H2O, 1 acétone).
point de fusion : 295,7 °C
¹H-RMN [(CD₃)₂SO, 250 MHz] : 9.61 (2H, large s), 9.02 (2H, d), 8.16 (2H, d), 7.42 (4H, dd), 7.27 (2H, dd), 7.11 (2H, d), 7.02 (2H, s), 7.00-8.00 (4H, large s), 6.89 (4H, ddd), 6.61-6.70 (4H, m), 4.99 (4H, s), 4.51 (4H, s), 3.25-3.35 (4H, m), 1.96 (6H, s)

### Exemples 31 et 32

En suivant les procédés décrits les Etapes C à E de **l'Exemple 30**, on prépare les **Exemples 31** et **32** par dimérisation de l'acide ({3-[4-amino-3-(méthoxycarbonyl)benzoyl]-2-méthylindolizin-1-yl}oxy)acétique (décrit dans la demande de brevet WO2003084956) avec les diamines commerciales adéquates.

### Exemple 33 : Sel disodique du 3,3'-{3,6,9,12-tétraoxatétradécane-1,14-diylbis[imino(2-méthylindolizine-1,3-diyl)carbonyl]}bis(acide 6-aminobenzoïque)

### Etape A

A la suspension de 0,15 g (3,78 m.moles) d'hydrure de sodium (60% en dispersion dans l'huile) dans 5 ml de *N,N*-diméthylformamide à 0°C sous argon, on ajoute 1,60 g (3,78 m.moles) de 2-amino-5-({1-[(tert-butoxycarbonyl)amino]-2-méthylindolizin-3-yl}carbonyl)benzoate de méthyle (décrit dans la demande de brevet WO2003084956) en solution dans 13 ml de N,N-diméthylformamide. Au bout de 10 minutes, on ajoute 0,87 g (1,89 m.mole) de 1,14-diiodo-3,6,9,12-tétraoxatétradécane (Exemple R3) dans 1 ml de N,N-diméthylformamide. On agite le mélange à température ambiante pendant 20 heures. Le milieu réactionnel est dilué avec de l'acétate d'éthyle et lavé avec une solution aqueuse saturée de chlorure de sodium. La phase organique est séchée sur sulfate de sodium et concentrée sous pression réduite. Le résidu obtenu est purifié par chromatographie flash sur gel de silice (gradient dichlorométhane/méthanol = 100/0 à 98/2). On obtient 1,15 g (58 %) d'une poudre jaune.
MH+ = 1049,4 ; point de fusion : 104 °C

### Etape B : 3,3'-{3,6,9,12-Tétraoxatétradécane-1,14-diylbis[imino(2-méthylindolizine-1,3-diyl)carbonyl]}bis(6-aminobenzoate de méthyle)

La solution de 1,06 g (1,01 m.mole) du dimère obtenu dans l'Etape A précédente dans 10 ml de dichlorométhane et 3 ml d'acide trifluoroacétique. La solution est agitée à température ambiante pendant 2 heures. La solution est coulée dans une solution aqueuse saturée de bicarbonate de sodium et extraite avec du dichlorométhane. La phase organique est lavée avec de l'eau et avec une solution aqueuse saturée de chlorure de sodium, séchée sur sulfate de sodium et concentrée sous pression réduite. Le résidu obtenu est purifié par chromatographie flash sur gel de silice (gradient acétate d'éthyle / méthanol = 100/0 à 97/3). On obtient 0,67 g (78%) d'une poudre orange.
MH+ = 849,3 ; point de fusion : 66 °C

### Etape C : 3,3'-{3,6,9,12-Tétraoxatétradécane-1,14-diylbis[imino(2-méthylindolizine-1,3-diyl)carbonyl]}bis(acide 6-aminobenzoïque)

On chauffe à reflux la solution de 0,64 g (0,75 m.mole) du dimère obtenu dans l'Etape B ci-dessus en présence de 1,51 ml de soude (1 M) dans 4 ml de 1,4-dioxane pendant 17 heures. Le mélange réactionnel est concentré à sec, puis dissout dans l'eau. Après ajout de 226 mg d'hydrogénosulfate de potassium, on obtient un précipité orange que l'on filtre et lave abondamment à l'eau puis séché sous vide à 50°C pour fournir 0,54 g (60%) d'une poudre orange.

### Etape D

Le produit final est salifié sous forme de sel disodique par ajout 1,17 ml de soude (1 M) à la suspension de 0,48 g (0,59 m.mole) dans 20 ml de méthanol. La solution est concentrée à sec. Le solide est repris dans l'acétone, filtré et séché pour fournir 0,46 g (61%) d'une poudre rouge (sel disodique, 4 H2O).
point de fusion : 193 °C (décomposition)
¹H-RMN [(CD₃)₂SO, 250 MHz] : 9.11 (2H, d), 8.14 (2H, d), 7.60 (2H, d), 7.36 (2H, dd), 7.00-8.00 (4H, large s), 6.91 (2H, dd), 6.67 (2H, dd), 6.60 (2H, d), 3.90-4.10 (2H, large s), 3.50-3.60 (12H, m), 3.44 (4H, t), 3.05-3.15 (4H, m), 1.97 (6H, s)

### Exemple 34 : Sel disodique de 3,3'-{octane-1,8-diylbis[oxy-3,1-phénylènecarbonylimino(2-méthylindolizine-1,3-diyl)carbonyl]}bis(acide 6-aminobenzoïque)

### Etape A : 5-[(1-{[3-(Acétyloxy)benzoyl]amino}-2-méthylindolizin-3-yl)carbonyl]-2-aminobenzoate de méthyle

A 0,61 g (3,40 m.moles) d'acide 3-acétoxybenzoïque en suspension dans 20 ml de *N,N*-diméthylformamide, on ajoute 0,95 ml (6,80 m.moles) de triéthylamine et 1,50 g (3,40 m.moles) de BOP^{®} à température ambiante. Au bout de 15 minutes, on ajoute 1,0 g (3,09 m.moles) de 2-amino-5-[(1-amino-2-méthylindolizin-3-yl)carbonyl]benzoate de méthyle (décrit dans la demande de brevet WO2003084956) dans 10 ml de *N,N-*diméthylformamide. Le milieu réactionnel est agité pendant 16 heures sous argon puis dilué avec de l'acétate d'éthyle et lavé avec une solution aqueuse saturée de bicarbonate de sodium. La phase organique est lavée avec une solution aqueuse saturée de chlorure de sodium, séchée sur sulfate de sodium et concentrée sous pression réduite. Le solide obtenu est repris dans l'acétone puis filtré et séché. On obtient 0,86 g (57%) d'une poudre jaune verte.
MH⁺ = 486,3 ; point de fusion : 210 °C

### Etape B : 2-Amino-5-({1-[(3-hydroxybenzoyl)amino]-2-méthylindolizin-3-yl}lcarbonyl)benzoate de méthyle

On ajoute 0,46 g (3,30 m.moles) de carbonate de potassium dissous dans 4 ml d'eau à la suspension de 0,80 g (1,65 m.mole) de 5-[(1-{[3-(acétyloxy)benzoyl]amino}-2-méthylindolizin-3-yl)carbonyl]-2-aminobenzoate de méthyle dans 16 ml de méthanol. Le milieu réactionnel est agité à température ambiante pendant 1 heure, il s'homogénéise progressivement. On dilue avec de l'acétate d'éthyle et on acidifie avec une solution molaire d'acide chlorhydrique. Les deux phases sont séparées. La phase organique est lavée avec une solution aqueuse saturée de chlorure de sodium, séchée sur sulfate de sodium et concentrée sous pression réduite. Le solide obtenu est repris dans l'éther éthylique puis filtré et séché. On obtient 0,61 g (84%) d'une poudre jaune-verte.
MH⁺ = 444,3 ; point de fusion : 278 °C

### Etape C : 3,3'-{Octane-1,8-diylbis[oxy-3,1-phénylènecarbonylimino(2-méthylindolizine-1,3-diyl)carbonyl]}bis(6-aminobenzoate de méthyle)

A la suspension de 71 mg (1,63 m.mole) d'hydrure de sodium (60% en dispersion dans l'huile) à température ambiante dans 15 ml de N,N-diméthylformamide, on ajoute 0,72 g (1,63 m.mole) de 2-amino-5-({1-[(3-hydroxybenzoyl)amino]-2-méthylindolizin-3-yl}carbonyl)benzoate de méthyle sous argon. Au bout de 15 minutes, on ajoute 0,30 g (0,82 m.mole) de 1,8-diiodooctane. On chauffe le mélange à 60 °C pendant 72 heures. Le milieu réactionnel est acidifié avec une solution aqueuse saturée d'hydrogénosulfate de potassium puis extrait à l'acétate d'éthyle. La phase organique est lavée avec de l'eau puis avec une solution aqueuse saturée de chlorure de sodium, séchée sur sulfate de sodium et concentrée sous pression réduite. Le résidu obtenu est dissous dans le minimum de N,N-diméthylformamide et purifié par chromatographie d'exclusion stérique sur gel Sephadex® LH20 (N,N-diméthylformamide). On obtient 0,50 g (61 %) d'une poudre jaune.
MH+ = 983,4 ; point de fusion : 213 °C

### Etape D

On ajoute de 0,38 ml de soude (1 M) à la solution de 185 mg (0,19 m.mole) du diacide carboxylique obtenu dans l'étape précédente dans 5 ml de méthanol. La solution est concentrée à sec puis le solide obtenu est repris dans l'acétone, filtré et séché. On obtient 152 mg d'une poudre jaune (sel disodique, 5 H20, 0,35 N,N-diméthylformamide)
point de fusion : 263 °C (décomposition)
1H-RMN [(CD3)2SO, 250MHz] : 9.97 (2H, s), 9.12 (2H, d), 8.20 (2H, s), 7.63 (2H, d), 7.36-7.46 (8H, m), 7.30-8.00 (4H, large s), 7.15 (2H, dd), 7.05 (2H, dd), 6.81 (2H, dd), 6.62 (2H, d), 4.07 (4H, t), 1.96 (6H, s), 1.70-1.85 (4H, m), 1.35-1.55 (8H, m)

### Exemples 35 à 41

En suivant les procédés décrits dans les Etapes E et F de **l'Exemple 34**, on prépare les **Exemples 35 à 41** par dimérisation du 2-amino-5-({1-[(3-hydroxybenzoyl)amino]-2-méthylindolizin-3-yl}carbonyl)benzoate de méthyle (Etape D de **l'Exempte 34**) avec les agents alkylants adéquats (Exemples R2 à R6, R10 et R11).

### Exemple 42 : Sel disodique de 3,3'-{(1,4-dioxobutane-1,4-diyl)bis[iminoéthane-2,1-diyloxy-3,1-phénylènecarbonylimino(2-méthylindolizine-1,3-diyl)carbonyl]})bis(acide 6-aminobenzoïque)

### Etape A : 3-{[(Pyridin-2-ylméthyl)amino]carbonyl}phényl acétate

A 65,0 g (0,36 mole) d'acide 3-acétoxybenzoïque en solution dans 600 ml de dichlorométhane, on ajoute 93 ml (0,66 mole) de triéthylamine et 160 g (0,36 mole) de benzotriazol-1-yloxy-tris(diméthylamino)phosphonium hexafluorophosphate (BOP) à température ambiante sous argon. Au bout de 10 minutes, on ajoute goutte à goutte 32,5 g (0,30 mole) de 2-(aminométhyl)pyridine en solution dans 200 ml dichlorométhane à 0°C. On laisse revenir le milieu réactionnel à température ambiante puis on agite pendant 3 heures.

Le milieu réactionnel est dilué avec de l'acétate d'éthyle et extrait avec une solution aqueuse saturée de bicarbonate de sodium. La phase organique est lavée avec une solution aqueuse saturée de chlorure de sodium, séchée sur sulfate de sodium et concentrée sous pression réduite. Le résidu obtenu est purifié par chromatographie flash sur gel de silice (dichlorométhane). On obtient 36,0 g (44%) d'un sirop marron utilisé tel quel dans l'Etape suivante.
MH+ = 271,1

### Etape B : 3-Hydroxy-N-(pyridin-2-ylméthyl)benzamide

On ajoute 11,7 g (0,85 mole) de carbonate de potassium en solution dans 86 ml d'eau à 11,5 g (0,43 mole) de 3-{[(pyridin-2-ylméthyl)amino]carbonyl}phényl acétate (Etape A) en solution dans 340 ml de méthanol. Le mélange est agité à température ambiante pendant 3 heures puis concentré à sec. Le résidu est repris dans l'acétate d'éthyle et on neutralise avec 85 ml d'une solution molaire d'acide chlorhydrique. Les deux phases sont séparées. La phase aqueuse est extraite avec de l'acétate d'éthyle à trois reprises. Les phases organiques sont rassemblées et lavées avec une solution aqueuse saturée de chlorure de sodium, séchée sur sulfate de sodium. Le solide obtenu après concentration sous vide est lavé à l'éther éthylique et filtré. Après séchage, on obtient 6,3 g (65%) d'un solide blanc.
MH+ = 229,1

### Etape C : [2-(3-{[(Pyridin-2-ylméthyl)amino]carbonyl})phénoxy) éthyl]carbamate de tert-butyle

On ajoute 3,96 g (90,7 m.moles) d'hydrure de sodium (55% dispersion dans l'huile) par portion à la solution de 18,0 g (78,9 m.moles) de 3-hydroxy-N-(pyridin-2-ylméthyl)benzamide dans 160 ml de *N,N*-diméthylformamide à 0 °C sous argon. Après 1 heure, on ajoute 23,0 g (0,10 mole) de (2-bromoéthyl)carbamate de *tert*-butyle et on chauffe le mélange à 90 °C pendant 16 heures. On verse le milieu réactionnel dans de l'eau et on extrait avec de l'acétate d'éthyle. La phase organique est lavée avec une solution aqueuse saturée de chlorure de sodium, séchée sur sulfate de sodium et concentrée sous pression réduite. Le produit est purifié par flash chromatographie sur gel de silice (dichlorométhane / méthanol = 9/1). On obtient 19,0 g (65%) d'une huile incolore.
MH+ =372,2

### Etape D : [2-(3-{[(2-Méthylindolizin-1-yl)amino]carbonyl}phénoxy) éthyl]carbamate de tert-butyle

Le mélange de 19,0 g (51,1 m.moles) de [2-(3-{[(pyridin-2-ylméthyl)amino]carbonyl}phénoxy)éthyl]carbamate de *tert*-butyle, 7,1 g (76,7 m.moles) de chloroacétone et 8,9 g (102 m.moles) de bromure de lithium dans 100 ml d'acétonitrile est chauffé à reflux pendant 16 heures. On verse le mélange dans l'eau. On lave la phase aqueuse à quatre reprises avec de l'acétate d'éthyle. On concentre la phase aqueuse pour obtenir une huile verdâtre.

On chauffe à reflux la solution de la pyridine quaternisée dans 100 ml d'acétonitrile puis on ajoute 17,8 ml (0,13 mole) de triéthylamine. Après 3 heures de reflux sous argon, on concentre à sec. On verse dans l'eau et on extrait avec l'acétate d'éthyle. La phase organique est lavée avec une solution aqueuse saturée de chlorure de sodium, séchée sur sulfate de sodium et concentrée sous pression réduite. L'huile marron obtenue est filtrée sur silice H (toluène) pour donner 8,95 g (43%) d'un solide blanc.
MH+ = 410,5 ; point de fusion : 128 °C

### Etape E : 5-({1-[(3-{2-[(tert-Butoxycarbonyl)amino]éthoxy}benzoyl) amino]-2-méthylindolizin-3-yl}carbonyl)-2-[(trifluoroacétyl)amino]benzoate de benzyle

A la suspension de 9,27 g (24,0 m.moles) de 5-(chlorocarbonyl)-2-[(trifluoroacétyl)amino]benzoate de benzyle dans 180 ml de dichlorométhane, on ajoute 4,43 ml (43,7 m.moles) de pyridine et 8,95 g (21,9 m.moles) [2-(3-{[(2-méthylindolizin-1-yl)amino]carbonyl}phénoxy)éthyl]carbamate de *tert* butyle. Le milieu réactionnel devenu verdâtre est agité à température ambiante pendant 16 heures. On concentre à sec et on reprend le résidu dans de l'éther diisopropylique et l'éthanol. Le solide en suspension est filtré, lavé avec de l'éther diisopropylique et séché. On obtient 6,29 g (38%) d'une poudre jaune.
MH+ = 759,6

### Etape F : 5-[(1-{[3-(2-Aminoéthoxy)benzoyl]amino}-2-méthylindolizin-3-yl)carbonyl]-2-[(trifluoroacétyl)amino]benzoate de benzyle

On ajoute 16 ml (0,20 mole) d'acide trifluoroacétique à la suspension de 6,28 g (8,28 m.moles) de 5-({1-[(3-{2-[(*tert*-butoxycarbonyl)amino]éthoxy}benzoyl)amino]-2-méthylindolizin-3-yl}carbonyl)-2-[(trifluoroacétyl)amino]benzoate de benzyle dans 40 ml de dichlorométhane. Après 30 minutes d'agitation à température ambiante, on verse le milieu réactionnel dans 1 L d'une solution aqueuse saturée de bicarbonate de sodium. On filtre le précipité, on le lave abondamment à l'eau puis à l'éther diisopropylique et on le sèche pour obtenir 4,5 g (82%) d'une poudre jaune.
MH+ = 659,5 ; point de fusion : 219 °C

### Etape G : 3,3'-{(1,4-Dioxobutane-1,4-diyl)bis[iminoéthane-2,1-diyloxy-3,1-phénylènecarbonylimino(2-méthylindolizine-1,3-diyl)carbonyl]}bis{6-[(trifluoroacétyl)amino]benzoate de benzyle}

A 82 mg (0,70 m.mole) d'acide succinimique en solution dans 7 ml de *N,N-*diméthylformamide, on ajoute 0,4 ml (2,94 m.moles) de triéthylamine et 0,62 g (1,47 m.mole) de BOP^{®}. Au bout de 15 minutes, on ajoute 0,92 g (1,40 m.mole) de 5-[(1-{[3-(2-aminoéthoxy)benzoyl]amino}-2-méthylindolizin-3-yl)carbonyl]-2-[(trifluoroacétyl)amino]benzoate de benzyle. Le milieu réactionnel est agité à température ambiante pendant 48 heures. Le milieu réactionnel est dilué avec de l'acétate d'éthyle, filtré et lavé à l'acétate d'éthyle puis séché pour donner 0,43 g (44%) d'une poudre orange.
MH⁺ = 1399,7 ; point de fusion : 281 °C

### Etape H : 3,3'-{(1,4-Dioxobutane-1,4-diyl)bis[iminoéthane-2,1-diyloxy-3,1-phénylènecarbonylimino(2-méthylindolizine-1,3-diyl)carbonyl]}bis(acide 6-[(trifluoroacétyl)amino]benzoique}

On agite le mélange de 0,42 g (0,30 m.mole) de 3,3'-{(1,4-dioxobutane-1,4-diyl)bis[iminoéthane-2,1-diyloxy-3,1-phénylènecarbonylimino(2-méthylindolizine-1,3-diyl)carbonyl]}bis{6-[(trifluoroacétyl)amino]benzoate de benzyle} et de 0,11 g (1,82 m.mole) de formiate d'ammonium en présence de 0,10 g de palladium sur charbon (10%) dans 3 ml de *N,N* diméthylformamide pendant 2 heures à température ambiante. On filtre le mélange et on le concentre à sec pour obtenir 370 mg (quantitatif) d'un solide jaune.
MH+ = 1219,5

### Etape I : 3,3'-{(1,4-Dioxobutane-1,4-diyl)bis[iminoéthane-2,1-diyloxy-3,1-phénylènecarbonylimino(2-méthylindolizine-1,3-diyl)carbonyl]}bis(acide 6-aminobenzoïque)

On ajoute 1,17 ml de soude (1 M) à la suspension de 365 mg (0,30 m.mole) du dimère obtenu dans l'étape B ci-dessus dans 10 ml de méthanol. On agite la solution à température ambiante pendant 1 heure. On verse le milieu réactionnel dans une solution aqueuse saturée d'hydrogénosulfate de potassium. Après filtration et séchage du précipité obtenu, on obtient 270 mg (88%) d'une poudre verte.
MH+ = 1027,7

### Etape J

Le produit final est salifié sous forme de sel de sodium par ajout de 264 mg (0,26 m.mole) du diacide carboxylique obtenu ci-dessus à une solution contenant 0,50 ml de soude (1 M) dans 5 ml d'eau. La solution est lyophilisée. Le lyophilisat est repris dans l'acétone, filtré et séché pour fournir 250 mg d'une poudre jaune (sel disodique, 5 H₂O)
point de fusion : 343 °C (décomposition)
¹H-RMN [(CD₃)₂SO, 250MHz] : 10.00 (2H, s), 9.13 (2H, d), 8.22 (4H, s), 7.65 (2H, s), 7.56-7.59 (4H, m), 7.37-7.47 (4H, m), 7.17 (2H, d), 7.06 (2H, dd), 7.00-8.00 (4H, large s), 6.82 (2H, dd), 6.63 (2H, d), 4.05-4.15 (4H, m), 3.40-3.55 (4H, m), 2.39 (4H, s), 1.97 (6H, s)

### Exemple 43 : Sel disodique de 3,3'-{carbonyl bis[iminoéthane-2,1-diyloxy-3,1-phenylènecarbonylimino(2-méthylindolizine-1,3-diyl)carbonyl]}bis(acide 6-aminobenzoïque)

### Etape A : 3,3'-{Carbonylbis[iminoéthane-2,1-diyloxy-3,1-phénylènecarbonylimino(2-méthylindolizine-1,3-diyl)carbonyl]}bis{6-[(trifluoroacétyl)amino]benzoate de benzyle}

A 0,92 g (1.40 m.mole) de 5-[(1-{[3-(2-aminoéthoxy)benzoyl]amino}-2-méthylindolizin-3-yl)carbonyl]-2-[(trifluoroacétyl)amino]benzoate de benzyle (Etape F de **l'Exemple 42**) en solution dans 10 ml de *N,N*-diméthylformamide, on ajoute 0,18 g (0.70 m.mole) de *N,N*'-disuccinimidylcarbonate. La solution est agitée pendant 3 heures à température ambiante puis elle est diluée avec de l'acétate d'éthyle. Le précipité obtenu est filtré, lavé avec de l'acétate d'éthyle et séché pour donner 0,49 g (53%) d'une poudre jaune.
MH⁺ = 1346,6

### Etape B : 3,3'-{Carbonyl bis[iminoéthane-2,1-diyloxy-3,1-phenylènecarbonylimino(2-méthylindolizine-1,3-diyl)carbonyl]}bis(acide 6-aminobenzoïque)

On ajoute 1,45 ml de soude (1 M) à la solution de 300 mg (0,22 m.mole) du dimère obtenu dans l'étape A ci-dessus dans 1,5 ml de 1-méthyl-2-pyrolidinone. On agite la solution à température ambiante pendant 16 heures. On verse le milieu réactionnel dans 100ml d'eau contenant 120 mg d'hydrogénosulfate de potassium: Le précipité obtenu est filtré, lavé à l'eau et séché pour fournir 155 mg (73%) d'une poudre jaune-orange.
MH+ = 971,5

### Etape C

Le produit final est salifié sous forme de sel disodique. On ajoute par portion 250 mg (0,26 m.mole) du diacide carboxylique obtenu dans l'Etape B ci-dessus à une solution contenant 0,51 ml de soude (1 M) dans 26 ml d'eau. La solution est concentrée à sec. Le solide est repris dans l'acétone, filtré et séché pour fournir 220 mg d'une poudre jaune (sel disodique, 4 H₂O)
point de fusion : 354-359 °C (décomposition)
¹H-RMN [(CD₃)₂SO, 250MHz] : 10.02 (2H, s), 9.14 (2H, d), 8.24 (2H, s), 7.64-7.67 (4H, m), 7.34-7.46 (6H, m), 7.17 (2H, d), 7.05 (2H, dd), 7.00-8.00 (4H, large s), 6.82 (2H, dd), 6.55-6.70 (4H, m), 4.05-4.15 (4H, m), 3.35-3.40 (4H, m), 1.97 (6H, s)

### Exemple 44 : Sel disodique du 3,3'-{éthane-1,2-diylbis[imino(2-oxoéthane-2,1-diyl)oxy-3,1 -phénylènecarbonylimino(2-méthylindolizine-1,3-diyl)carbonyl]}bis(acide 6-aminobenzoïque)

### Etape A : (3-{[(Pyridin-2-ylméthyl)amino]carbonyl}phénoxy)acétate de tert-butyle

A la suspension de 4,5 g (0,10 mole) d'hydrure de sodium (55% dispersion dans l'huile) dans 400 ml de *N,N* diméthylformamide à 0 °C sous argon, on ajoute 23,8 g (0,10 mole) du 3-hydroxy-*N-*(pyridin-2-ylméthyl)benzamide (Etape B de **l'Exemple 42)** par portion suivi après 10 minutes de 15,4 ml (0,10 mole) de 2-bromoacétate de *tert*-butyle. Le mélange est agité à température ambiante pendant 1 heure. Le milieu réactionnel est dilué avec de l'acétate d'éthyle et extrait avec de l'eau. La phase organique est lavée avec une solution aqueuse saturée de chlorure de sodium, séchée sur sulfate de sodium et concentrée sous pression réduite. Le solide blanc est repris dans l'éther diisopropylique, filtré et séché pour donner 26,3 g (74%) d'une poudre beige.
MH⁺ = 343,2

### Etape B : (3-{[(2-Méthylindolizin-1-yl)amino]carbonyl}phénoxy)acétate de tert-butyle

Le mélange de 26,0 g (75,9 m.moles) de (3-{[(pyridin-2-ylméthyl)amino]carbonyl}phénoxy)acétate de *tert*-butyle, 6,65 ml (83,5 m.moles) de chloroacétone et 7,9 g (91,1 m.moles) de bromure de lithium dans 100 ml d'acétonitrile est chauffé à reflux pendant 16 heures. On laisse refroidir à température ambiante et on ajoute 110 ml d'eau et de l'acétate d'éthyle. On sépare les deux phases. On lave la phase aqueuse à deux reprises avec de l'acétate d'éthyle.
On ajoute 26,2 g (0,19 mole) de carbonate de potassium à la phase aqueuse. On chauffe la solution à 90 °C pendant 3 heures sous argon. On extrait avec de l'acétate d'éthyle. La phase organique est lavée avec une solution aqueuse saturée de chlorure de sodium, séchée sur sulfate de sodium et concentrée sous pression réduite. On obtient 7,0 g d'un solide beige utilisé tel quel dans l'étape d'acylation.
MH+ = 381,5

### Etape C : 5-[(1-{[3-(2-tert-Butoxy-2-oxoéthoxy)benzoyl]amino}-2-méthylindolizin-3-yl)carbonyl]-2-[(trifluoroacétyl)amino]benzoate de benzyle

A 13,0 g (34,2 m.moles) du 5-(chlorocarbonyl)-2-[(trifluoroacétyl)amino]benzoate de benzyle (décrit dans la demande de brevet WO2003084956) en suspension dans 60 ml de dichlorométhane, on ajoute goutte à goutte une solution de 12,4 g (32,6 m.moles) de (3-{[(2-méthylindolizin-1-yl)amino]carbonyl}phénoxy)acétate de *tert*-butyle et 2,77 ml de pyridine dans 100 ml de dichlorométhane sous argon. Le milieu réactionnel qui se colore immédiatement en vert, est agité à température ambiante pendant 19 heures. Le milieu réactionnel est dilué avec de l'acétate d'éthyle et versé dans une solution aqueuse saturée de bicarbonate de sodium. Après décantation, la phase organique est lavée avec une solution aqueuse saturée de chlorure de sodium, séchée sur sulfate de sodium et concentrée sous pression réduite. Le solide obtenu est adsorbé sur silice et purifié par flash chromatographie sur gel de silice (dichlorométhane / diéther éthylique = 95/5). On obtient 9,0 g (38%) d'une poudre jaune.
MH⁺ = 730,5 ; point de fusion : 200 °C

### Etape D : Acide (3-{[(3-{3-[(benzyloxy)carbonyl]-4-[(trifluoroacétyl)amino] benzoyl}-2-méthylindolizin-1-yl)amino]carbonyl}phénoxy)acétique

On ajoute 17 ml (0,22 mole) d'acide trifluoroacétique à la suspension de 7,95 g (10,9 m.moles) de 5-[(1-{[3-(2-*tert*-butoxy-2-oxoéthoxy)benzoyl]amino}-2-méthylindolizin-3-yl)carbonyl]-2-[(trifluoroacétyl)amino]benzoate de benzyle dans 55 ml de dichlorométhane à température ambiante. Le mélange s'homogénéise rapidement. On agite la solution pendant 3 heures, on la concentre à sec. On reprend le solide obtenu avec de l'éther éthylique, on le filtre et on le sèche. On obtient 7,25 g (99 %) d'une poudre orange.
MH+ = 674,4 ; point de fusion : 239,5 °C

### Etape E : 3,3'-{Ethane-1,2-diylbis[imino(2-oxoéthane-2,1-diyl)oxy-3,1-phénylènecarbonylimino(2-méthylindolizine-1,3-diyl)carbonyl]}bis{6-[(trifluoroacétyl)amino]benzoate de benzyle}

A 1,4 g (2,08 m.moles) d'acide (3-{[(3-{3-[(benzyloxy)carbonyl]-4-[(trifluoroacétyl)amino]benzoyl}-2-méthylindolizin-1-yl)amino]carbonyl}phénoxy)acétique en solution dans 8,5 ml de *N,N-*diméthylformamide, on ajoute 0,3 ml (2,2 m.moles) de triéthylamine et 0,96 g (2,18 m.moles) de BOP^{®} à 0°C sous argon. Au bout de 15 minutes, on ajoute 70 µl (1,04 m.mole) d'éthane 1,2-diamine. Le milieu réactionnel est agité à température ambiante pendant 24 heures ; il s'épaissit pour donner un gel (au bout de 1 heure, on rajoute 4,5 ml de *N,N*-diméthylformamide). Le milieu réactionnel est dilué avec de l'acétate d'éthyle et lavé avec une solution aqueuse saturée de bicarbonate de sodium. Un précipité se forme, il est filtré et lavé à l'eau et à l'éthanol puis séché. On obtient 0,72 g d'une poudre jaune.
MH⁺ = 1371,6

### Etape F : 3,3'-{Ethane-1,2-diylbis[imino(2-oxoéthane-2,1-diyl)oxy-3,1-phénylènecarbonylimino(2-méthylindolizine-1,3-diyl)carbonyl]}bis(acide 6-aminobenzoïque)

On ajoute 0,52 ml (1,04 m.mole) de soude (2 M) à la solution de 316 mg (0,23 m.mole) du dimère obtenu dans l'étape E ci-dessus dans 4 ml de 1-méthyl-2-pyrolidinone. On agite la solution à température ambiante pendant 3 jours. On verse le milieu réactionnel dans l'acétone. Le précipité obtenu est filtré puis dissout dans 100 ml d'eau auquel on ajoute 140 mg d'hydrogénosulfate de potassium. On lyophilise le mélange. Le résidu obtenu est purifié par chromatographie d'exclusion stérique sur gel Sephadex^{®} LH20 (*N,N*-diméthylformamide). On obtient 153 mg d'une poudre jaune.
MH+ = 999,6

### Etape G

On ajoute de 0,30 ml d'une solution molaire de soude à la suspension de 153 mg (0,15 m.mole) du diacide carboxylique obtenu dans l'étape F précédente dans 1,5 ml de méthanol. La solution est concentrée à sec puis le solide obtenu est repris dans l'acétone, filtré et séché. On obtient 155 mg d'une poudre jaune (sel disodique, 5 H₂O).
point de fusion : 349-354 °C (décomposition)
¹H-RMN [(CD₃)₂SO, 250MHz] : 10.02 (2H, s), 9.13 (2H, d), 8.26 (2H, t), 8.18 (2H, s), 7.67-7.69 (4H, m), 7.42-7.49 (4H, m), 7.37 (2H, d), 7.18 (2H, d), 7.06 (2H, dd), 7.00-8.00 (4H, large s), 6.85 (2H, dd), 6.66 (2H, d), 4.59 (4H, s), 3.10-3.20 (4H, m), 1.97 (6H, s)

### Exemples 45 à 47 :

En suivant les procédés décrits dans les Etapes E à G de **l'Exemple 44**, on prépare les **Exemples 45** à **47** par dimérisation de l'acide (3-{[(3-{3-[(benzyloxy)carbonyl]-4-[(trifluoroacétyl)amino]benzoyl}-2-méthylindolizin-1-yl)amino]carbonyl}phénoxy)acétique (Etape D de **l'Exemple 44**) avec les diamines commerciales adéquates.

### Exemples 48 et 49 :

En suivant les procédés décrits dans les Etapes E à G de **l'Exemple 44**, on prépare les **Exemples 48** et **49** par dimérisation de l'acide 3-(4-amino-3-méthoxybenzoyl)-1-[3-(méthoxycarbonyl)phényl]imidazo[1,5-a]pyridine-7-carboxylique (composé obtenu en adaptant les protocoles décrits dans la demande de brevet W02006097625) avec les diamines commerciales adéquates.

### Exemple 50 :

En suivant les procédés décrits dans les Etapes E à G de **l'Exemple 44**, on prépare l'Exemple 50 par dimérisation de l'acide 3-[7-(éthoxycarbonyl)-3-{3-méthoxy-4-[(trifluoroacétyl)amino]benzoyl)imidazo[1,5-a]pyridin-1-yl]benzoïque (composé obtenu en adaptant les protocoles décrits dans la demande de brevet WO2006097625) avec la diamine commerciale adéquate.

### Exemple 51 : Sel disodique de 3,3'-{éthane-1,2-diylbis[oxy-3,1-phénylènecarbonylimino(2-méhylindolizine-1,3-diyl)carbonyl]})bis(acide benzoïque)

### Etape A : 2-amino-5-[(1-{[3-(2-hydroxyéthoxy)benzoyl]amino}-2-méthylindolizin-3-yl)carbonyl]benzoate de méthyle

On ajoute 0,043 g (0,99 m.mole) d'hydrure de sodium (55% dispersion dans l'huile) par portion à la solution de 0,4 g (0,90 m.mole) de 2-amino-5-({1-[(3-hydroxybenzoyl)amino]-2-méthylindolizin-3-yl}carbonyl)benzoate de méthyle (Etape B de **l'Exemple 35**) dans 4,5 ml de *N,N-*diméthylformamide sous argon à 0°C. Après 30 minutes, on ajoute 0,12 ml (1,08 m.mole) de 2-bromoéthyl acétate et on chauffe le mélange à 70 °C pendant 1 heure.

On verse le milieu réactionnel dans une solution molaire d'acide chlorhydrique et on extrait avec de l'acétate d'éthyle. La phase organique est lavée avec une solution aqueuse saturée de bicarbonate de sodium et une solution aqueuse saturée de chlorure de sodium, séchée sur sulfate de sodium et concentrée sous pression réduite. On obtient 0,35 g (76 %) d'une poudre jaune (MH⁺ = 530,1) que l'on met en suspension dans 100 ml de *N,N-*diméthylformamide et 20 ml de méthanol et auquel on ajoute 0,043 g (0,31 m.mole) de carbonate de potassium. Le mélange est agité à température ambiante pendant 3 jours, et concentré à sec. Le résidu obtenu est lavé à l'eau abondamment et séché pour fournir 0,30g (95%) d'une poudre jaune.
MH⁺ = 488,2

### Etape B : 2-Amino-5-({2-méthyl-1-[(3-{2-[(méthylsulfonyl)oxy]éthoxy}benzoyl)amino]indolizin-3-yl}carbonyl)benzoate de méthyle

A la solution de 0,34 g (0,69 m.mole) du 2-amino-5-[(1-{[3-(2-hydroxyéthoxy)benzoyl]amino}-2-méthylindolizin-3-yl)carbonyl]benzoate de méthyle dans 9 ml de pyridine à -20 °C sous azote, on ajoute 59 µl (0,76 m.mole) de chlorure de mésyle. Le mélange réactionnel est agité pendant 2 heures à -20 °C puis est coulé dans 100 ml d'eau. Le précipité obtenu est filtré, lavé abondamment avec de l'eau et séché sous vide pour donner 268 mg (70%) d'une poudre jaune.
point de fusion : 171 °C, MH+ = 566,1

### Etape C : 3,3'-{éthane-1,2-diylbis[oxy-3,1-phénylènecarbonylimino(2-méthylindolizine-1,3-diyl)carbonyl]}bis(6-aminobenzoate de méthyle)

A la solution de 0,21 g (0,48 m.mole) du 2-amino-5-({1-[(3-hydroxybenzoyl)amino]-2-méthylindolizin-3-yl})carbonyl)benzoate de méthyle (Etape B de l'Exemple 35) dans 15 ml de *N,N*-diméthylformamide à 0 °C sous argon, on ajoute 0,021 g (0,48 m.mole) d'hydrure de sodium (60% dispersion dans l'huile). Le milieu réactionnel devient rouge. Au bout de 15 minutes, on ajoute 0,25 mg (0,44 m.mole) du composé obtenu dans l'Etape B ci-dessus en solution dans 3 ml de *N,N-*diméthylformamide. On chauffe le mélange à 60 °C pendant 12 heures. On laisse le milieu réactionnel revenir à température ambiante puis on le coule dans une solution aqueuse saturée d'hydrogénosulfate de potassium. Le précipité obtenu est filtré, lavé abondamment avec de l'eau, séché sous vide et le résidu obtenu est purifié par chromatographie d'exclusion stérique sur gel Sephadex^{®} LH20 (*N,N-*diméthylformamide). On obtient 220 mg (54%) d'une poudre jaune.
point de fusion : 278 °C (décomposition), MH⁺ = 913,6

### Etape D

A 0,21 g (0,24 m.mole) du dimère obtenu dans l'Etape E ci-dessus dans 5 ml de 1-méthyl-2-pyrolidinone, on ajoute 0,24 ml de soude (2 M) et on agite à température ambiante pendant 72 heures. On verse le milieu réactionnel dans 150 ml d'acétone. Le précipité jaune est filtré et séché pour fournir 73 mg (30%) d'une poudre jaune (sel disodique, 6,35 H₂O).
point de fusion : 286°C
¹H-RMN [(CD₃)₂SO, 250MHz] : 9.97 (2H, s), 9.12 (2H, d), 8.21 (2H, s), 7.65-7.75 (2H, m), 7.35-7.51 (8H, m), 7.00-8.00 (4H, large s), 7.25 (2H, dd), 7.07 (2H, dd), 6.82 (2H, dd), 6.63 (2H, d), 4.49 (4H, s), 1.98 (6H, s)

### Exemple 52 : Sel disodique du 3,3'-[octane-1,8-diylbis(oxy-3,1-phenylènecarbonyliminoimidazo[1,5-a]pyridine-1,3.diylcarbonyl)]bis(acide 6-aminobenzoïque)

### Etape A : 2-amino-5-({1-[(diphénylméthylène)amino]imidazo[1,5-a]pyridin-3-yl}carbonyl)benzoate de méthyle

A la solution de 2,20 g (5,88 m.moles) du 2-amino-5-[(1-bromoimidazo[1,5-a]pyridin-3-yl)carbonyl]benzoate de méthyl (décrit dans la demande de brevet WO2006097625) dans 35 ml de *N,N*-diméthylformamide anhydre sous argon, on ajoute 3,83 g (11,75 m.moles) de carbonate de césium, 0,73 g (1,18 m.mole) de (±)-2,2'-bis(diphénylphosphino)-1,1'-binaptyle [Binap], 4,93 ml (29,4 m.moles) de benzophénoneimine et 0,54 mg (0,59 m.mole) de tris-(dibenzylidèneacétone)dipalladium(0) [Pd₂(dba)₃] puis le mélange est chauffé à 110°C pendant 3 heures. Le milieu réactionnel est ensuite filtré, puis dilué avec de l'acétate d'éthyle. La phase organique est lavée avec de l'eau puis avec une solution aqueuse saturée de chlorure de sodium. Elle est séchée sur sulfate de sodium, filtrée et concentrée à sec pour obtenir un solide rouge. Après purification par chromatographie flash sur gel de silice (gradient toluène / acétate d'éthyle/ triéthylamine = 98/1/1 à 90/9/1), on obtient 1,18 g (42%) d'une poudre rouge-orange.
MH⁺ = 475,3

### Etape B : Chlorhydrate du 2-amino-5-[(1-aminoimidazo[1,5-a]pyridin-3-yl)carbonyl]benzoate de méthyle

A la solution de 1,92 g (4,05 m.moles) de l'imine obtenue dans l'Etape A ci-dessus dans un mélange de 40 ml de dichlorométhane et 8 ml de méthanol, on ajoute 8 ml d'éther éthylique chlorhydrique (1 M). Le milieu orange limpide devient rouge foncé puis un précipité se forme. Après 1 heure à température ambiante, le précipité est filtré puis lavé avec du dichlorométhane et séché sous vide à 50°C pour donner 1,18 g (84%) du chlorhydrate sous forme d'une poudre ocre.
MH+ = 311,2

### Etape C : 3,3'-[Octane-1,8-diylbis(oxy-3,1-phénylènecarbonyliminoimidazo[1,5-a]pyridine-1,3-diylcarbonyl)]bis(6-aminobenzoate de méthyle)

A la solution de 0,20 g (0,48 m.mole) du diacide 3,3'-[octane-1,8-diylbis(oxy)]benzoïque (Exemple **R12**) dans 2,5 ml de *N-*méthylpyrrolidinone, sont ajoutés successivement 0,35 ml (2,5 m.moles) de triéthylamine, 0,63 g (1,2 m.mole) de PyBOP et 0,50 g (1,44 m.mole) du chlorhydrate du 2-amino-5-[(1-aminoimidazo[1,5-a]pyridin-3-yl)carbonyl]benzoate de méthyle sous courant d'azote. Après agitation à température ambiante pendant 3 jours, on ajoute 0,13 g (0,24 m.mole) de PyBOP. Après 24 heures, le milieu réactionnel est versé dans de l'acide chlorhydrique (1M). Le précipité marron formé est lavé à l'eau puis séché sous vide à 50°C. Le brut est dissout dans le minimium de *N,N*-diméthylformamide et purifié par chromatographie d'exclusion stérique sur gel Sephadex^{®} LH20 (*N,N-*diméthylformamide). On obtient 0,34 g (71%) sous forme de poudre marron.
MH+ = 1001,8 ; point de fusion : 187-207°C

### Etape D : 3,3'-[Octane-1,8-diylbis(oxy-3,1-phenylènecarbonyliminoimidazo[1,5-a]pyridine-1,3-diylcarbonyl)]bis(acide 6-aminobenzoïque)

On ajoute 0,53 ml de soude (1 M) à la solution de 0,24 g (0,24 m.mole) du dimère obtenu dans l'étape C ci-dessus dans 1,5 ml de 1-méthyl-2-pyrolidinone. On agite la solution à température ambiante pendant 3 jours. La réaction n'étant pas terminée on ajoute 0,24 ml de soude (1 M) puis au bout de 24 heures, on verse le milieu réactionnel dans une solution d'acide chlorhydrique (0,1 M). Le précipité jaune formé est filtré et séché puis purifié par chromatographie HPLC sur gel Kromasyl C18 10µ [gradient de éluant A (80% eau / 20% solution aqueuse d'acétate d'ammonium 0,1 M) / éluant B (80% acétonitrile / 20% solution aqueuse d'acétate d'ammonium 0,1 M) = 65/35 à 35/65]. On obtient 40 mg (18%) d'une poudre jaune. 0
MH+ = 943,4

### Etape E

A la suspension de 34 mg (0,04 m.mole) du diacide carboxylique obtenu dans l'Etape D ci-dessus dans 7 ml de méthanol, on ajoute 0,07 ml de soude (1 M). On concentre la solution et on reprend le solide obtenu avec de l'acétone. On filtre l'insoluble et on le sèche sous vide à 50°C pour obtenir 33 mg d'une poudre jaune (sel disodique, 4,95 moles d'eau).
point de fusion : 249°C
¹H-RMN [(CD₃)₂SO, 250MHz] : 10.84 (2H, s), 9.66 (2H, d), 8.82 (2H, s), 8.28 (2H, d), 7.63 (2H, d), 7.66-7.74 (4H, m), 7.43 (2H, dd), 7.11-7.22 (6H, m), 7.00-8.00 (4H, large s), 6.55 (2H, d), 4.07 (4H, t), 1.70-1.85 (4H, m), 1.30-1.50 (8H, m)

### Exemple 53 : Sel disodique de 3,3'-{éthane-1,2-diylbis[imino(2-oxoéthane-2,1-diyl)oxy-3,1-phénylènecarbonyliminolmidazo[1,5-a]pyridine-1,3-diylcarbonyl]}bis(acide 6-aminobenzoïque)

En suivant les procédés décrits dans les Etapes C à E de **l'Exemple 52**, on prépare **l'Exemples 53** par dimérisation du 2-amino-5-[(1-amino-imidazo[1,5-*a*]pyridin-3-yl)carbonyl]benzoate de méthyle (décrit dans l'étape B de **l'Exemple 53**) avec le diacide 3,3'-{éthane-1,2-diylbis[imino(2-oxoéthane-2,1-diyl)oxy]}benzoïque (Exemple R13).
MH+ = 973,2 ; point de fusion : 281-286°C

### Exemple 54 : Sel de L-Lysine du 1,1'-[octane-1,8-diylbis(oxy-3,1-phénylènecarbonylimino)]bis[3-(4-amino-3-méthoxybenzoyl)imidazo[acide 1,5-a]pyridine-7-carboxylique]

### Etape A : 1,1'-[octane-1,8-diylbis(oxy-3,1-phénylènecarbonylimino)]bis(3-{3-méthoxy-4-[(trifluoroacétyl)amino]benzoyl}imidazo[1,5-a]pyridine-7-carboxylate) d'éthyle

A la suspension de 1,0 g (2,59 m.moles) du diacide 3,3'-[octane-1,8-diylbis(oxy)]benzoïque (Exemple R12) en suspension dans 26 ml de dichlorométhane, on ajoute 1,24 ml (15,5 m.moles) de pyridine. Le milieu réactionnel devient homogène. On ajoute 0,66 ml (7,77 m.moles) de fluorure de cyanogène. La réaction est exothermique, on observe la formation d'un précipité blanc. Après 3heures d'agitationà température ambiante, le milieu réactionnel est coulé dans une solution aqueuse saturée de bicarbonate de sodium, extrait avec du dichlorométhane. La phase organique est lavée avec une solution aqueuse saturée de chlorure de sodium, séchée sur sulfate de sodium et concentrée à sec. L'huile incolore obtenue cristallise pour donner 0,88 g (83%) d'un solide blanc.

A la solution de 0,6 g (1,33 m.mole) du 1-amino-3-{3-méthoxy-4-[(trifluoroacétyl)amino]benzoyl}imidazo[1,5-*a*]pyridine-7-carboxylate d'éthyle (obtenu en adaptant les protocoles décrits dans la demande de brevet W02006097625) dans 14 ml de *N,N*-diméthylformamide, on ajoute 0,43 ml (5,32 m.moles) de pyridine, puis on refroidit la solution à 0°C avant d'ajouter 0,42 ml (4,0 m.moles) de triméthylchlorosilane. On agite 30 min puis on ajoute 0,25 g (0,67 m.mole) du difluorure d'acide décrit ci-dessus. On agite 5 jours à 50°C. On coule le milieu réactionnel dans une solution aqueuse saturée de sulfate de potassium. Le précipité rouge obtenu est filtré et séché sous vide puis purifié par chromatographie d'exclusion stérique sur gel Sephadex^{®} LH20 (*N,N*-diméthylformamide). On obtient 0.42 g (49%) d'une poudre rouge.

A la solution de 0,40 g (0,32 m.mole) du composé obtenu dans l'Etape A ci-dessus dans 1 ml d'eau et de 1 ml de méthanol, on ajoute 0,26 g (1,91 m.mole) de carbonate de potassium. Le mélange réactionnel est agité à température ambiante pendant 5 jours puis versé dans une solution aqueuse de sulfate de potassium. Le précipité formé est filtré, lavé à l'eau et séché sous vide à 50°C. Le produit est purifié par chromatographie FPLC sur gel C8 Kromasyl 10µ [gradient méthanol / solution aqueuse d'acétate d'ammonium 0,02 M / acétonitrile = 5/95/0 à 45/5/50]. On obtient une cire jaune qu'on dissout dans le minimum de *N,N*-diméthylformamide et on coule cette solution dans 200 ml d'eau. Le précipité obtenu est filtré, lavé à l'eau et séché sous vide à 50°C pour fournir 7 mg d'une poudre rouge.
MH⁺ = 1003,2

### Etape C

A la suspension de 0,01 g (0,01 m.mole) du composé obtenu dans l'Etape C ci-dessus dans 1 ml d'eau, on ajoute 0,003 g (0,02 m.mole) de L-Lysine. La solution est concentrée à sec et le solide est repris dans l'acétone, filtré et séché sous vide pour fournir une poudre orange.
MH⁺ = 1003,2 avec RT = 15,34 min (méthode A)

### Exemple 55: Chlorhydrate du 4,4'-{éthane-1,2-diylbis[imino(2-oxoéthane-2,1-diyl)oxy]}bis{N-[3-(4-amino-3-méthoxybenzoyl)-2-méthylindolizin-1-yl]benzamide}

### Etape A : 4,4'-{Ethane-1,2-diylbis[imino(2-oxoéthane-2,1-diyl)oxy]}bis{N-[3-(4-amino-3-méthoxybenzoyl)-2-méthylindolizin-1-yl]benzamide}

A la suspension de 0,17 g (0,40 m.mole) du diacide 4,4'-{éthane-1,2-diylbis[imino(2-oxoéthane-2,1-diyl)oxy]}benzoïque (Exemple **R14**) dans 4 ml de *N,N-*diméthylformamide, on ajoute successivement 0,12 ml (0,85 m.mole) de triéthylamine et 0,38 g (0,85 m.mole) de BOP^{®}. Au bout de 20 minutes, on ajoute 0,24 g (0,81 m.mole) de (4-amino-3-méthoxyphényl)(1-amino-2-méthylindolizin-3-yl)méthanone (décrit dans la demande de brevet W02003084956) par portion. Le milieu réactionnel est agité à température ambiante pendant 20 heures puis on ajoute 0,38 g (0,85 m.mole) de BOP^{®}. Après 5 heures, le milieu réactionnel est versé dans de l'eau et extrait avec de l'acétate d'éthyle. La phase organique est lavée une solution saturée de chlorure de sodium, séchée sur sulfate de sodium et concentrée à sec. Le résidu obtenu est purifié par chromatographie d'exclusion stérique sur gel Sephadex^{®} LH20 (*N,N*-diméthylformamide). On obtient 41 mg (43%) d'une poudre marron.
MH⁺ = 971,3

### Etape B

On ajoute 0,10 ml d'éther éthylique chlorhydrique (1 M) à la suspension de 40 mg (0,04 m.mole) du dimère obtenu dans l'Etape précédente. La solution est concentrée à sec et le solide obtenu est repris dans l'acétone. L'insoluble est filtré et séché sous vide à 50°C pour fournir 40 mg d'une poudre marron.
point de fusion : 223°C
¹H-RMN [(CD₃)₂SO, 250MHz] : 9.83 (2H, s), 9.30 (2H, d), 8.20-8.40 (2H, m), 8.04 (4H, d), 6.85-7.41 (16H, m), 5.00-6.00 (6H, large s), 4.60 (4H, s), 3.87 (6H, s), 3.25-3.35 (4H, m), 1.89 (6H, s)

### Exemples 56 à 63 :

En suivant les procédés décrits dans les Etapes A et B de **l'Exemple 55**, on prépare **les Exemples 56 à 63** par dimérisation du (4-amino-3-méthoxyphényl)(1-amino-2-méthylindolizin-3-yl)méthanone (décrit dans la demande de brevet W02003084956) avec le diacide carboxylique adéquate (Exemples R14 à R22).

### Exemple 64 : Sel disodique du 3,3'-{Ethane-1,2-diylbis[imino(2-oxoéthane-2,1-diyl)oxy-4,1-phenylènecarbonylimino(2-méthylindolizine-1,3- . diyl)carbonyl]}bis(acide 6-aminobenzoïque)

### Etape A : 3,3'-(Ethane-1,2-diylbis[imino(2-oxoéthane-2,1-diyl)oxy-4,1-phénylènecarbonylimino(2-méthylindolizine-1,3-diyl)carbonyl]}bis{6-[(trifluoroacétyl)amino]benzoate de benzyle}

A 0,40 g (0,96 m.mole) du diacide 4,4'-{éthane-1,2-diylbis[imino(2-oxoéthane-2,1-diyl)oxy]}benzoïque (Exemple **R14**) en solution dans 10 ml de *N,N-*diméthylformamide, on ajoute 0,55 ml (4,0 m.moles) de triéthylamine et 0,93 g (2,1 m.moles) de BOP^{®}. Au bout de 15 minutes, on ajoute 0,95 g (1,92 m.mole) de 5-[(1-amino-2-méthylindolizin-3-yl)carbonyl]-2-[(trifluoroacétyl)amino]benzoate de benzyle (décrit dans Brevet W02003084956). Le milieu réactionnel est agité à température ambiante pendant 7 heures. Le gel obtenu est dilué avec du *N,N-*diméthylformamide et l'insoluble est filtré, lavé avec du *N,N*-diméthylformamide et de l'acétate d'éthyle puis séché sous vide pour fournir 570 mg d'un solide jaune-orange.
MH⁺ = 1371,6 (-1 uma)

### Etape B : 3,3'-{Ethane-1,2-diylbis[imino(2-oxoéthane-2,1-diyl)oxy-4,1-phenylènecarbonylimino(2-méthylindolizine-1,3-diyl)carbonyl]}bis(acide 6-aminobenzoïque)

On ajoute 1,46 ml de soude (1 M) à la solution de 0,57 g (0,42 m.mole) du dimère obtenu dans l'étape A ci-dessus dans 8 ml de 1-méthyl-2-pyrolidinone. On agite la solution à température ambiante pendant 3 jours. On verse le milieu réactionnel dans 200 ml d'eau contenant 400 mg d'hydrogénosulfate de potassium. On filtre l'insoluble et on le sèche avant de le purifier par chromatographie d'exclusion stérique sur gel Sephadex^{®} LH20 (*N,N*-diméthylformamide). On obtient 153 mg d'une poudre jaune.
MH⁺ = 999,6.

### Etape C

Le produit est salifié par ajout de 0,35 ml de soude (1M) à la suspension de 0,18 g (0,15 m.mole) du diacide carboxylique obtenu dans l'étape B ci-dessus dans 2 ml de méthanol. La solution est concentrée à sec puis le solide obtenu est repris dans l'acétone, filtré et séché. On obtient 162 mg d'une poudre jaune (sel disodique, 5 H₂O)
point de fusion : 290-297 °C (décomposition)
¹H-RMN [(CD₃)₂SO, 250MHz] : 9.87 (2H, s), 9.12 (2H, d), 8.37 (2H, large t), 8.21 (2H, s), 8.06 (4H, d), 7.50-8.00 (4H, large s), 7.38-7.44 (4H, m), 7.00-7.12 (6H, m), 6.83 (2H, dd), 6.63 (2H, d), 4.60 (4H, s), 3.25-3.40 (4H, m), 1.96 (6H, s)

### Exemples 65 à 70

En procédant selon les procédés décrits dans les Etapes A à C de **l'Exemple 64**, on prépare les **Exemples 65** à **70** par dimérisation du 2-amino-5-[(1-amino-2-méthylindolizin-3-yl)carbonyl]benzoate de méthyle (décrit dans la demande de brevet W02003084956) avec les diacides carboxyliques adéquates (Exemples **R23 à R28**).

### Exemple 71 :

En procédant selon les procédés décrits dans les Etapes A à C de **l'Exemple 64**, on prépare **l'Exemple 71** par dimérisation du 3-[(1-amino-2-méthylindolizin-3-yl)carbonyl]benzoate de méthyle (composé obtenu en adaptant les protocoles décrits dans la demande de brevet WO2003084956) avec le diacide 3,3'-{éthane-1,2-diylbis[imino(2-oxoéthane-2,1-diyl)oxy]}benzoïque (Exemple R13).
point de fusion :330-339°C
¹H-RMN [(CD₃)₂SO, 400MHz] : 10.10 (2H, s), 9.61 (2H, d), 8.41 (2H, large s), 8.00-8.10 (4H, m), 7.62-7.70 (4H, m), 7.57 (2H, d), 7.38-7.48 (6H, m), 7.23 (2H, dd), 7.15 (2H, dd), 7.00 (2H, dd), 4.56 (4H, s), 3.26 (4H, m), 1.71 (6H, s)

### Exemples 72 : Sel disodique de 3,3'-{1,3-phénylènebis[sulfonyliminoéthane-2,1-diyloxy-3,1-phénylènecarbonylimino(2-méthylindolizine-1,3-diyl)carbonyl]}bis(acide 6-aminobenzoïque)

A température ambiante et sous atmosphère d'azote, 1,35 ml (9,6 m.moles) de triéthylamine est ajouté à une suspension de 0,30 g (0,77 m.mole) de l'hydrogénosulfate de l'acide 2-amino-5-[(1-amino-2-méthylindolizin-3-yl)carbonyl]benzoïque dans 4 ml de tétrahydrofurane. Le milieu réactionnel est hétérogène. Il est ensuite refroidit à 0°C et on ajoute 0,52 ml (4,1 m.mole) de chlorure de triméthylsilyle lentement. Le milieu réactionnel passe de la couleur orange à la couleur verte et reste hétérogène.

En parallèle, à température ambiante et sous atmosphère d'azote, 0,20 ml (1,38 m.mole) de triéthylamine puis 0,40 g (0,76 mmole) de PyBOP sont rajoutés à une suspension de 0,20 g (0,34 m.mole) du diacide 3,3'-[1,3-phénylènebis(sulfonyliminoéthane-2,1-diyloxy)]benzoïque dans 2,7 ml de *N,N-*diméthylformamide. Le milieu réactionnel est agité pendant 40 minutes puis est ajouté *via* une cannule à la suspension de l'amine silylée. Après 20 heures d'agitation, le mélange réactionnel est versé dans 30 ml d'eau et 0,54 ml d'acide sulfurique concentré. Le précipité obtenu est filtré, lavé à l'eau jusqu'à pH neutre. Le produit est purifié par chromatographie FPLC sur gel C8 Kromasyl 10µ [gradient méthanol / solution aqueuse d'acétate d'ammonium 0,02 M / acétonitrile = 5/95/0 à 45/5/50]. On obtient 140 mg d'une cire jaune qu'on dissout dans le minimum de *N,N-*diméthylformamide et on coule cette solution dans 200 ml d'eau. Le précipité obtenu est filtré, lavé à l'eau et séché sous vide à 50°C pour fournir une poudre jaune.

Le diacide carboxylique obtenu est salifié par ajout de 2 équivalents d'une solution molaire de soude, après concentration et séchage de la poudre sous vide à 50°C, on obtient une poudre jaune (sel disodique, 8 moles d'eau).
point de fusion : 308°C
¹H-RMN [(CD₃)₂SO, 400MHz] : 10.02 (2H, large s), 9.10 (2H, d), 8.15-8.19 (4H, m), 7.85 (2H, d), 7.55-7.65 (6H, m), 7.35-7.42 (6H, m), 6.95-7.05 (4H, m), 6.78 (2H, dd), 6.60 (2H, d), 3.98 (4H, large t), 3.13 (4H, large t), 1.94 (6H, s) (les protons des - NH₂ donnent un signal large 6.0-9.0 ppm)

### Exemples 73 à 104

En procédant selon le protocole décrit dans **l'Exemple 72**, on prépare les **Exemple 73** à **104** par dimérisation de l'acide 2-amino-5-[(1-amino-2-méthylindolizin-3-yl)carbonyl]benzoïque (composé obtenu en adaptant les protocoles décrits dans la demande de brevet W02003084956) avec le diacide carboxylique adéquat (Exemples **R31 à R 37, R18 et R38 à R61** respectivement).

### Exemple 105 :

### Etape A : 5-{[1-({3-[2-(glycylamino)éthoxy]benzoyl}amino)-2-méthylindolizin-3-yl]carbonyl}-2-[(trifluoroacétyl)amino]benzoate de benzyle

En suivant le procédé décrit dans l'Etape G de **l'Exemple 42**, on réalise le couplage du 5-[(1-{[3-(2-aminoéthoxy)benzoyl]amino}-2-méthylindolizin-3-yl)carbonyl]-2-[(trifluoroacétyl)amino]benzoate de benzyle (décrit dans Etape F, **Exemple 42**) avec la *N-tert*-butoxycarbonylglycine. Le composé obtenu est ensuite déprotégé avec l'acide trifluoroacétique pour fournir l'amine selon le procédé décrit dans l'Etape F de **l'Exemple 42**.
MH⁺ = 716,1

### Etape B : 5-{[1-({3-[2-({[({3-[(3-{3-[(benzyloxy)carbonyl]-4-[(trifluoroacétyl)amino]benzoyl}-2-méthylindolizin-1-yl)carbamoyl]phénoxy}acétyl)amino]acétyl}amino]éthoxy]benzoyl}amino)-2-méthylindolizin-3-yl]carbonyl}-2-[(trifluoroacétyl)amino]benzoate de benzyle

En suivant le procédé décrit dans l'Etape G de **l**'**Exemple 42**, on réalise le couplage de l'amine obtenue dans l'Etape A ci-dessus avec l'acide (3-{[(3-{3-[(benzyloxy)carbonyl]-4-[(trifluoroacétyl)amino] benzoyl}-2-méthylindolizin-1-yl)amino]carbonyl}phénoxy)acétique (décrit dans l'Etape D de **l'Exemple 44**).
MH⁺ = 1371,3

### Etape C

En suivant les procédés décrits dans les Etape B et C d**e l'Exemple 8**, on réalise la déprotection du composé obtenu dans l'Etape A ci-dessus puis la salification du diacide carboxylique avec la soude (sel disodique, 6,2 moles d'eau).
point de fusion : 313-318°C
¹H-RMN [(CD₃)₂SO, 400MHz] : 9.99 (2H, d), 9.15 (2H, d), 8.40 (1 H, t), 8.24 (1 H, t), 8.15 (2H, d), 7.61-7.69 (4H, m), 7.36-7.49 (6H, m), 7.21 (1H, d), 7.14 (1H, d), 7.07 (2H, dd), 6.83 (2H, dd), 6.69 (2H, d), 4.65 (2H, s), 4.08 (2H, t), 3.81 (2H, d), 3.48 (2H, m), 1.95 (6H, s) (les protons des -NH₂ donnent un signal large 6.0-9.0 ppm)

### Exemple 106 : Sel disodique de 3,3'-{éthane-1,2-diylbis[oxyéthane-2,1-diyloxy-4,1-phénylène(2-méthylindolizine-1,3-diyl)carbonyl]}bis(acide 6-aminobenzoïque)

### Etape A : 3,3'-{Ethane-1,2-diylbis[oxyéthane-2,1-diyloxy-4,1-phénylène(2-méthylindolizine-1,3-diyl)carbonyl]}bis(6-aminobenzoate de méthyle)

A la solution de 0,70 g (1,81 m.mole) du 2-amino-5-[(1-bromo-2-méthylindolizin-3-yl)carbonyl]benzoate de méthyle (décrit dans la demande de brevet WO20055028476) et 0,51 g (0,92 m.mole) du 2,2'-[éthane-1,2-diylbis(oxyéthane-2,1-diyloxy-4,1-phénylène)]bis(4,4,5,5-tétraméthyl-1,3,2-dioxaborolane) (Exemple **R65**) dans 26 ml de diméthoxyéthane dégazée, on ajoute 5,42 ml d'une solution de phosphate de potassium (1 M) et 132 mg (0,18 m.mole) de PdCl₂(dppf). On chauffe le mélange à 100°C pendant 24 heures, on ajoute 66 mg de PdCl₂(dppf) au bout de 6 heures. Le mélange réactionnel est dilué avec du dichlorométhane et filtré. Le filtrat est lavé avec de l'eau et séché sur sulfate de sodium et concentré à sec. Le solide obtenu est purifié par flash chromatographie sur gel de silice (gradient éther diisopropylique / méthanol = 100/0 à 90/10). On obtient 437 mg (53 %) d'un solide jaune.
MH⁺ = 915,3 ; point de fusion : 186°C

### Etape B : 3,3'-{Ethane-1,2-diylbis[oxyéthane-2,1-diyloxy-4,1-phénylène(2-méthylindolizine-1,3-diyl)carbonyl]}bis(acide 6-aminobenzoïque)

On chauffe à reflux la solution de 0,42 g (0,46 m.mole) du dimère obtenu dans l'Etape A ci-dessus en présence de 1,38 ml de soude (1 M) dans 5 ml de méthanol et 5 ml de 1,4-dioxane pendant 5 heures. Le mélange réactionnel est hétérogène, il est dilué avec du méthanol et de l'eau jusqu'à obtention d'une solution qui est coulée dans 150 ml d'eau contenant 182 mg d'hydrogénosulfate de potassium. Le précipité obtenu est filtré et lavé abondamment à l'eau puis séché sous vide à 50°C pour fournie 0,36 g (88%) d'une poudre marron orange.
MH+ = 887,2

### Etape C

On ajoute 0,59 ml de soude (1 M) à la suspension de 0,27 g (0,30 m.mole) du diacide carboxylique obtenu dans l'Etape B ci-dessus dans 50 ml de méthanol. La solution est concentrée à sec. Le solide obtenu est repris dans l'acétone, filtré et séché sous vide à 50 °C pour fournir une poudre jaune 0,25 g (88%) d'une poudre jaune (sel disodique, 4 moles d'eau) point de fusion : 257°C
¹H-RMN [(CD₃)₂SO, 250MHz] : 9.02 (2H, d), 8.22 (2H, s), 7.43 (4H, d), 7.33 (4H, d), 7.00-8.00 (4H, large s), 6.97-7.09 (6H, m), 6.78 (2H, dd), 6.60 (2H, d), 4.17 (4H, t), 3.81 (4H, t), 3.68 (4H, s), 2.03 (6H, s)

### Exemples 107 à 109

En procédant selon les procédés décrits dans les Etapes A à C de **l'Exemple** 106, on prépare les **Exemples 107** à **109** par dimérisation du 2-amino-5-[(1-bromo-2-méthylindolizin-3-yl)carbonyl]benzoate de méthyle (décrit dans la demande de brevet WO20055028476) avec les diesters ou diacides boroniques adéquates (Exemples R66 à R68).

### Exemple 109 : Sel disodique de 3,3'-{éthane-1,2-diylbis[imino(2-oxoéthane-2,1-diyl)oxy-3,1-phénylène(2-méthylindolizine-1,3-diyl)carbonyl]}bis(acide 6-aminobenzoïque)

### Etape A : 2-Amino-5-({1-[3-(2-tert-butoxy-2-oxoéthoxy)phény)]-2méthylindolizin-3-yl}carbonyl)benzoate de méthyle

Le mélange de 2,78 g (7,18 m.moles) de 2-amino-5-[(1-bromo-2-méthylindolizin-3-yl)carbonyl]benzoate de méthyle (décrit dans la demande de brevet WO2005028476), de 3,6 g (10,8 m.moles) de [3-(4,4,5,5-tétraméthyl-1,3,2-dioxaborolan-2-yl)phénoxy]acétate de *tert-*butyle (CAS 769968-18-5 ; décrit dans la demande de brevet WO2004084813.), 0,73 g (1,0 m.mole) de PdCl₂(dppf) dans 22 ml d'une solution molaire de phosphate de potassium et 100 ml de 1,2-diméthoxyéthane est chauffé à 100°C sous argon. Au bout de 2 heures et 4 heures, on ajoute 100 mg de PdCl₂(dppf) supplémentaires. Au bout de 6 heures à 100°C sous argon, on laisse le milieu réactionnel revenir à température ambiante puis on le filtre. La solution organique est lavée à l'eau, avec une solution saturée de chlorure de sodium, séchée sur sulfate de sodium et concentrée à sec. Le résidu obtenu est purifié par chromatographie flash sur gel de silice (gradient dichlorométhane / éther diisopropylique = 100/0 à 0/100) pour donner 2,84 g (77%) d'une poudre jaune.
MH⁺ = 515,2 ; point de fusion : 81 °C

### Etape B : Acide (3-{3-[4-amino-3-(méthoxycarbonyl)benzoyl]-2-méthylindolizin-1-yl}phénoxy)acétique

La solution de 3,68 g (6,65 m.moles) du 2-amino-5-({1-[3-(2-*tert*-butoxy-2-oxoéthoxy)phényl]-2-méthylindolizin-3-yl}carbonyl)benzoate de méthyle dans 11 ml d'acide trifluoroacétique et 35 ml de dichlorométhane à température ambiante pendant 4 heures. On verse le milieu réactionnel dans l'eau et on extrait avec du dichlorométhane. La phase organique est partiellement concentrée, le précipité jaune formé est filtré et lavé avec de l'éther diisopropylique puis séché pour donner 2,75 g (92%) d'une poudre jaune.
MH⁺ = 459,2 ; point de fusion : 160°C

### Etape C : 3,3'-{Ethane-1,2-diylbis[imino(2-oxoéthane-2,1-diyl)oxy-3,1-phénylène(2-méthylindolizine-1,3-diyl)carbonyl]}bis(6-aminobenzoate de méthyle)

A 0,9 g (1,96 m.mole) d'acide 2-amino-5-({1-[3-(carboxyméthoxy)phényl]-2-méthylindolizin-3-yl}carbonyl)benzoïque, on ajoute 0,55 ml (3,93 m.moles) de triéthylamine et 1,12 g (2,16 m.moles) de PyBOP à 0°C sous argon dans 7 ml de *N,N-*diméthylformamide. Au bout de 10 minutes, on ajoute 66 µl (0,98 m.mole) d'éthane 1,2-diamine. Le milieu réactionnel est agité à température ambiante pendant 22 heures puis est versé dans l'eau et extrait avec de l'acétate d'éthyle. La phase organique est lavée avec une solution aqueuse saturée de chlorure de sodium, séchée sur sulfate de sodium et concentrée sous pression réduite. Le solide obtenu est purifié par chromatographie flash sur gel de silice (dichlorométhane / méthanol = 97/3) pour donner 0,68 g (73%) d'une poudre jaune.
MH+ = 941,8 ; point de fusion : 165 °C

### Etape D : 3,3'-{Ethane-1,2-diylbis[imino(2-oxoéthane-2,1-diyl)oxy-3,1-phénylène(2-méthylindolizine-1,3-diyl)carbonyl]}bis(acide 6-aminobenzoïque)

La solution de 0,61 g (0,65 m.mole) du dimère obtenu dans l'Etape E ci-dessus et de 1,6 ml de soude (1 M) dans 11 ml de diméthylsulfoxyde est chauffée à 100°C pendant 10 minutes puis elle est coulée dans 500 ml d'une solution aqueuse contenant 0,22 g d'hydrogénosulfate de potassium. On extrait avec de l'acétate d'éthyle. La phase organique est lavée avec une solution aqueuse saturée de chlorure de sodium, séchée sur sulfate de sodium et concentrée sous pression réduite. Le résidu est purifié par chromatographie d'exclusion stérique sur gel Sephadex^{®} LH20 (*N,N-*diméthylformamide). On obtient 424 mg (72%) d'une poudre jaune.
MH+ = 913,7

### Etape E

On ajoute 0,91 ml de soude (1 M) à la suspension de 424 mg (0,46 m.mole) du diacide carboxylique obtenu dans l'étape D précédente dans 40 ml de méthanol. La solution est concentrée à sec puis le solide obtenu est repris dans l'acétone, filtré et séché sous vide à 50°C. On obtient 405 mg d'une poudre jaune (sel disodique, 4 H₂O).
point de fusion : 237 °C
¹H-RMN [(CD₃)₂SO, 250MHz] : 9.00 (2H, d), 8.50 (2H, large t), 8.23 (2H, s), 7.36-7.54 (6H, m), 6.92-7.08 (8H, m), 7.00-8.00 (4H, large s), 6.80 (2H, dd), 6.62 (2H, d), 4.55 (4H, s), 3.25-3.35 (4H, m), 2.06 (6H, s)

### Exemple 111

En suivant les procédés décrits dans les Etapes C à E de **l'Exemple 110**, on prépare **l'Exemple 111** par dimérisation de l'acide (3-{3-[4-amino-3-(méthoxycarbonyl)benzoyl]-2-méthylindolizin-1-yl}phénoxy)acétique (Etape B de **l'Exemple 110**) avec l'héxane-1,6-diamine.

### Exemples 112 et 113

### Etape A : Acide (4-{3-[4-amino-3-(méthoxycarbonyl)benzoyl]-2-méthylindolizin-1-yl}phénoxy)acétique

En suivant les procédés décrits dans les Etapes A et B de **l'Exemple 110**, on prépare l'acide (4-{3-[4-amino-3-(méthoxycarbonyl)benzoyl]-2-méthylindolizin-1-yl}phénoxy)acétique à partir [4-(4,4,5,5-tétraméthyl-1,3,2-dioxaborolan-2-yl)phénoxy]acétate de *tert*-butyle (CAS 769968-17-4 ; décrit dans la demande de brevet WO2004084813) et de 2-amino-5-[(1-bromo-2-méthylindolizin-3-yl)carbonyl]benzoate de méthyle (décrit dans la demande de brevet WO2005028476).
MH⁺ = 459,1 ; point de fusion : 239°C

### Etape B

On obtient les **Exemples 112 et 113** en suivant les procédés décrits dans les Etapes C à E de **l'Exemple 110** par dimérisation de l'acide (4-{3-[4-amino-3-(méthoxycarbonyl)benzoyl]-2-méthylindolizin-1-yl}phénoxy)acétique avec l'éthane 1,2-diamine et l'héxane-1,6-diamine respectivement.

### Exemples 114 à 116

En adaptant les procédés décrits dans les Etapes A et B de **l'Exemple 64** puis l'Etape C de **l'Exemple 54**, on prépare les **Exemples 114** à **116** par dimérisation du 2-amino-5-{[6-(2-aminoéthoxy)-1-méthoxy-2-méthylindolizin-3-yl]carbonyl}benzoate de méthyle (composé obtenu en adaptant les protocoles décrits dans la demande de brevet WO20055028476) avec les diacides carboxyliques adéquates (Exemples **R62** à **R64**).

### Préparation d'hétérodimères agonistes des FGFRs

### Exemple 117 : Sel disodique du diacide 2-amino-5-[(1-{[3-(2-{[2-({[3-({[3-(4-amino-3-carboxybenzoyl)imidazo[1,5-a]pyridin-1-yl]amino}carbonyl)phénoxy] acétyl}amino)éthyl]amino}-2-oxoéthoxy)benzoyl]amino}-2-méthylindolizin-3-yl)carbonyl]benzoïque

### Etape A : Acide 3-{2-[(2-{[(3-{[(3-{3-[(benzyloxy)carbonyl]-4-[(trifluoroacétyl)amino]benzoyl}-2-méthylindolizin-1-yl)amino]

### carbonyl}phénoxy)acetyl]amino}éthyl)amino]-2-oxoethoxy}benzoïque

A 1,1 g (2,62 m.moles) du diacide 3,3'-{éthane-1,2-diylbis[imino(2-oxoéthane-2,1-diyl)oxy]}benzoïque (Exemple **R13**) mis en suspension dans 26 ml de dichlorométhane, on ajoute successivement 0,73 ml (5,23 m.moles) de triéthylamine, 0,34 g (0,65 m.mole) de PyBOP et après 15 minutes 0,32 g (0,65 m.mole) de la benzyl 5-[(1-amino-2-méthylindolizin-3-yl)carbonyl]-2-[(trifluoroacétyl)amino]benzoate de benzyle (décrit dans la demande de brevet WO03084956). Le mélange réactionnel est agité à température ambiante pendant 4 heures puis versé dans une solution de bicarbonate saturée. Le précipité obtenu est filtré puis dissout dans l'eau. La solution aqueuse est acidifiée et extraite avec de l'acétate d'éthyle. La phase organique est lavée à l'eau puis séchée sur sulfate de sodium et concentrée à sec. Après purification du solide obtenu sur par chromatographie d'exclusion stérique sur gel Sephadex^{®} LH20 (*N,N*-diméthylformamide), on obtient 240 mg (41%) d'une poudre jaune.
MH⁺ = 894,5 ; point de fusion : 196,6°C

### Etape B : 2-Amino-5-({1-[(3-{2-[(2-{[(3-{[(3-{3-[(benzyloxy)carbonyl]-4-[(trifluoroacétyl)amino]benzoyl}-2-méthylindolizin-1-yl)amino]carbonyl} phénoxy)acétyl]amino}éthyl)amino]-2-oxoéthoxy}benzoyl)amino]imidazo [1,5-a]pyridin-3-yl}carbonyl)benzoate de méthyle

A 232 mg (0,26 m.mole) de l'acide carboxylique obtenu dans l'Etape A ci-dessus dissout dans 1,2 ml de N,N-diméthylformamide sous azote, on ajoute successivement 0,12 ml (0,78 m.mole) de triéthylamine, 0,19 g (0,36 m.mole) de PyBOP et après 15 minutes 135 mg (0,39 m.mole) du chlorhydrate du 2-amino-5-[(1-aminoimidazo[1,5-a]pyridin-3-yl)carbonyl]benzoate de méthyle (Etape B de l'Exemple 53) et 6 ml de *N,N*-diméthylformamide. Après 4 heures d'agitation à température ambiante, on ajoute 0,14 g de PyBOP. Après 16 heures, le mélange réactionnel est dilué avec *N,N-*diméthylformamide et filtré.

Au 100 mg de solide obtenu (ester activé) en solution dans 1,5 ml de *N,N-*diméthylformamide, on ajoute 42 mg du chlorhydrate du 2-amino-5-[(1-aminoimidazo[1,5-a]pyridin-3-yl)carbonyl]benzoate de méthyle et 10µl de triéthylamine. A température ambiante la réaction n'évolue pas, on chauffe à 60 °C pendant 48 heures après avoir ajouté 14µl de triéthylamine et 52 mg de PyBOP. Le mélange réactionnel est filtré et le solide obtenu est purifié par chromatographie d'exclusion stérique sur gel Sephadex^{®} LH20 (*N,N*-diméthylformamide). On obtient 5.1 mg (17%) d'une poudre jaune.
MH⁺ = 1187,1

### Etape C : Diacide 3-{2-[(2-{[(3-{[(3-{3-[(benzyloxy)carbonyl]-4-[(trifluoroacétyl)amino]benzoyl}-2-méthylindolizin-1-yl)amino] carbonyl}phénoxy)acetyl]amino}éthyl)amino]-2-oxoethoxy}benzoïque

On ajoute 0,2 ml de soude (1 M) à la solution de 71 mg (0,06 m.mole) de l'hétérodimère obtenu dans l'Etape B ci-dessus dans 1,2 ml de diméthysulfoxide et on agite la solution à température ambiante. Au bout de 48 heures, on rajoute 0,18 ml de soude (1 M) puis on agite 24 heures. La solution est ensuite coulée dans une solution aqueuse saturée d'hydrogénosulfate de potassium, le précipité obtenu est filtré et séché. Le solide obtenu est purifié par chromatographie d'exclusion stérique sur gel Sephadex^{®} LH20 (*N,N*-diméthylformamide) pour fournir 24 mg (41%) d'une poudre jaune.
MH⁺ = 986,8

### Etape D

On ajoute 45,5µl de soude (1 M) à la solution de 23 mg (0,02 m.mole) du dimère obtenu dans l'Etape C ci-dessus dans 30 ml de méthanol. On concentre la solution à sec et on reprend le solide dans de l'acétone. Le solide est filtré et séché sous vide à 50°C pendant 48 heures pour fournir 16 mg (67%) d'une poudre jaune (sel disodique, 6 H₂O).
point de fusion : 278,3°C
¹H-RMN [(CD₃)₂SO, 250MHz] : 10.90 (1H, large s), 10.05 (1H, large s), 9.68 (1 H, d), 9.12 (1 H, d), 8.95 (1 H, large s), 8.35-8.60 (2H, m) 8.28 (1 H, d), 8.19 (1 H, s), 7.00-8.00 (15H, m), 6.81 (1 H, dd), 6.61 (2H, dd), 4.61 (2H, s), 4.57 (2H, s), 3.25-3.42 (4H, m) 1.96 (6H, s).

### Exemple 118 : Sel de L-Lysine de l'acide 2-amino-5-({1-[(3-{2-[(2-{[(4-{[3-(4-amino-3-carboxybenzoyl)-2-méthylindolizin-1-yl]carbamoyl}phénoxy)acétyl]amino}éthyl)amino]-2-oxoéthoxy}benzoyl)amino]-2-méthylindolizin-3-yl}carbonyl)benzoïque

En procédant selon les procédés décrits dans les Etapes A et B de **l'Exemple 72**, on prépare **l'Exemple 118** par dimérisation de l'acide 2-amino-5-[(1-amino-2-méthylindolizin-3-yl)carbonyl]benzoïque (composé obtenu en adaptant les protocoles décrits dans la demande de brevet W02003084956) avec l'acide 3-{2-[(2-{[(4-carboxyphénoxy)acétyl]amino}éthyl)amino]-2-oxoéthoxy}benzoïque (Exemple **R69**).
point de fusion : 213°C
¹H-RMN [(CD₃)₂SO, 400MHz] : 10.05 (1 H, large s), 9.96 (1 H, large s), 9.13 (2H, dd), 9.12 (1H, d), 8.30-8.38 (2H, m), 8.11-8.15 (2H, m), 8.06 (2H, d), 7.70 (2H, d), 7.46-7.50 (4H, m), 7.35 (3H, dd), 7.19 (2H, dd), 7.00-7.09 (4H, m), 6.85-6.77 (2H, m), 6.66 (2H, d), 4.59 (2H, s), 4.56 (2H, s), 3.13 (4H, t), 2.69 (4H, t), 1.95 (3H, s), 1.93 (3H, s) 1.30-1.74 (10H,m).

### Exemple 119: Sel disodique de l'acide 2-amino-5-({1-[(20-{[3-(4-amino-3-carboxybenzoyl)-1-méthoxy-2-méthylindolizin-8-yl]oxy}-3,6,9,12,15,18-hexaoxaicos-1-yl)oxy]-2-méthylindolizin-3-yl}carbonyl)benzoïque

### Etape A : 2-Amino-5-({8-[(20-iodo-3,6,9,12,15,18-hexaoxaicos-1-yl)oxy]-1-méthoxy-2-méthylindolizin-3-yl}carbonyl)benzoate de méthyle

A la solution de 2,0 g (5,64 m.moles) de 2-amino-5-[(8-hydroxy-1-méthoxy-2-méthylindolizin-3-yl)carbonyl]benzoate de méthyle (décrit dans **l'Etape F** de **l'Exemple** 1) dans 56 ml de tétrahydrofurane, on ajoute goutte à goutte 12 ml (6,0 m.moles) d'hexaméthyldisilylamidure de potassium (solution 0,5 M dans toluène) à -20 °C sous azote. On observe la formation d'un précipité rouge. On laisse revenir le mélange à température ambiante et on ajoute 18,5 g (33,9 m.moles) de 1,20-diiodo-3,6,9,12,15,18-hexaoxaicosane (Exemple R1). On agite la solution à température ambiante pendant 18 heures. On verse le milieu réactionnel dans une solution d'acide chlorhydrique (1 M) et on extrait avec un mélange acétate d'éthyle/tétrahydrofurane. La phase organique est lavée avec une solution aqueuse saturée de chlorure de sodium, séchée sur sulfate de sodium et concentrée sous pression réduite. Le résidu obtenu est purifié par flash chromatographie sur gel de silice (gradient dichlorométhane/méthanol : 100/0 à 90/10). On obtient 2,3 g (53 %) d'une huile jaune.
MH⁺ = 773,1

### Etape B : 2-amino-5-({1-[(20-{[3-(4-amino-3-carboxybenzoyl)-1-méthoxy-2-méthylindolizin-8-yl]oxy}-3,6,9,12,15,18-hexaoxaicos-1-yl)oxy]-2-méthylindolizin-3-yl}carbonyl)benzoate de méthyle

A la solution de 0,63 g (1,94 m.mole) de 2-amino-5-[(1-hydroxy-2-méthylindolizin-3-yl)carbonyl]benzoate de méthyle (décrit dans la demande de brevet W02003084956) dans 7 ml de tétrahydrofurane, on ajoute goutte à goutte 3,88 ml (1,94 m.moles) d'hexaméthyldisilylamidure de potassium (solution 0,5 M dans toluène) à -20 °C sous azote. On observe la formation d'un précipité rouge auquel on ajoute 0,5 g (0,65 m.mole) du dérivé iodé obtenu dans **l'Etape A** ci-dessus. On agite la solution à température ambiante pendant 18 heures. On verse le milieu réactionnel dans une solution aqueuse saturée de sulfate de potassium et on extrait avec un mélange acétate d'éthyle/tétrahydrofurane. La phase organique est lavée avec une solution aqueuse saturée de chlorure de sodium, séchée sur sulfate de sodium et concentrée sous pression réduite. Le résidu obtenu est purifié par flash chromatographie sur gel de silice (gradient dichlorométhane/méthanol: 100/0 à 90/10). On obtient 0,42 g (67 %) d'une huile jaune.
MH⁺ = 969,3 avec RT = 22,05 (méthode A)

### Etape C

En procédant selon le procédé décrit dans les Etapes D et E de **l'Exemple 7**, on réalise la saponification du diester méthylique obtenu dans l'Etape précédente et la salification avec la soude (sel disodique, 4,5 moles d'eau).
point de fusion : 280°C
¹H-RMN [(CD₃)₂SO, 400MHz] : 9.07 (1H, d), 8.60 (1H, d), 8.12 (2H, d), 7.55 (1H, d), 7.34 (2H, dd), 6.96 (1H, dd), 6.71 (1H, ddd), 6.52-6.60 (3H, m), 6.36 (1H, d), 4.22 (2H, large t), 4.06 (2H, large t), 3.83 (2H, large t), 3.76 (3H, s), 3.46-3.67 (22H, m), 1.98 (3H, s), 1.91 (3H, s) (les protons des -NH₂ donnent un signal large 6.0-9.0 ppm)

### Exemple 120: Sel disodique de l'acide 2-amino-5-({6-[(20-{[3-(4-amino-3-carboxybenzoyl)-1-méthoxy-2-méthylindolizin-8-yl]oxy}-3,6,9,12,15,18-hexaoxaicos-1-yl)oxy]-1-méthoxy-2-méthylindolizin-3-yl}carbonyl)benzoïque

En procédant selon le procédé décrit dans les Etapes B et C de **l'Exemple 119**, on prépare **l'Exemple 120** à partir du 2-amino-5-({8-[(20-iodo-3,6,9,12,15,18-hexaoxaicos-1-yl)oxy]-1-méthoxy-2-méthylindolizin-3-yl}carbonyl)benzoate de méthyle (décrit dans l'Etape A de **l'Exemple 119**) et du 2-amino-5-[(6-hydroxy-1-méthoxy-2-méthylindolizin-3-yl)carbonyl]benzoate de méthyle (décrit dans l'Etape F de **l'Exemple 11**). (Sel disodique, 6,4 moles d'eau).
point de fusion : 274°C
¹H-RMN [(CD₃)₂SO, 400MHz] : 8.89 (1H, d), 8.60 (1H, d), 8.12 (2H, dd), 7.48 (1 H, dd), 7.34 (2H, m), 6.85 (1 H, dd), 6.53-6.60 (3H, m), 6.37 (1 H, d), 4.21 (2H, large t), 4.03 (2H, large t), 3.83 (2H, large t), 3.80 (3H, s), 3.70-3.77 (5H, m), 3.46-3.65 (20H, m), 1.93 (3H, s), 1.91 (3H, s) (les protons des -NH₂ donnent un signal large 6.0-9.0 ppm)

### Exemple 121: Sel disodique de l'acide 2-amino-5-({1-[(20-{4-[3-(4-amino-3-carboxybenzoyl)-1-méthoxyindolizin-2-yl]phénoxy}-3,6,9,12,15,18-hexaoxaicos-1-yl)oxy]-2-méthylindolizin-3-yl}carb onyl)benzoïque

### Etape A : 2-amino-5-[(2-{4-[(20-iodo-3,6,9,12,15,18-hexaoxaicos-1-yl)oxy]phényl}-1-méthoxyindolizin-3-yl)carbonyl]benzoate de méthyle

En procédant selon le protocole décrit dans l'Etape A de l'**Exemple 119**, on prépare le 2-amino-5-[(2-{4-[(20-iodo-3,6,9,12,15,18-hexaoxaicos-1-yl)oxy]phényl}-1-méthoxyindolizin-3-yl)carbonyl]benzoate de méthyle (huile marron) par alkylation du 2-amino-5-{[2-(4-hydroxyphényl)-1-méthoxyindolizin-3-yl]carbonyl}benzoate de méthyle (composé obtenu en adaptant les protocoles décrits dans la demande de brevet W02003084956) avec le 1,20-diiodo-3,6,9,12,15,18-hexaoxaicosane (**Exemple R1**).
MH⁺ = 835,2 avec RT = 21,15 min (méthode A)

### Etape B : 2-Amino-5-({1-[(20-{4-[3-(4-amino-3-carboxybenzoyl)-1-méthoxyindolizin-2-yl]phénoxy}-3,6,9,12,15,18-hexaoxaicos-1-yl)oxy]-2-méthylindolizin-3-yl}carbonyl)benzoate de méthyle

En procédant selon le protocole décrit dans l'Etape B de l'**Exemple 119**, on prépare le 2-amino-5-({1-[(20-{4-[3-(4-amino-3-carboxybenzoyl)-1-méthoxyindolizin-2-yl]phénoxy}-3,6,9,12,15,18-hexaoxaicos-1-yl)oxy]-2-méthylindolizin-3-yl}carbonyl)benzoate de méthyle (huile jaune) par alkylation du 2-amino-5-[(1-hydroxy-2-méthylindolizin-3-yl)carbonyl]benzoate de méthyle (décrit dans la demande de brevet WO2003084956) avec le dérivé iodé obtenu dans l'**Etape A** ci-dessus.
MH⁺ = 1031,3 avec RT = 22,79 min (méthode A)

### Etape C

Même procédure que dans l'Etape C de l'**Exemple 119** (sel disodique, 8 moles d'eau).
point de fusion : 196°C
¹H-RMN [(CD₃)₂SO, 400MHz] : 9.04 (1 H, d), 8.80 (1 H, d), 8.13 (2H, dd), 7.56 (2H, dd), 7.33 (1H, dd), 7.13 (2H, d),7.04 (1H, dd), 6.93-6.96 (2H, m), 6.70-6.76 (4H, m), 6.56 (1 H, dd), 6.12 (1 H, d), 4.07 (2H, large t), 4.00 (2H, large t), 3.62-3.70 (4H, m), 3.60 (3H, s), 3.45-3.59 (20H, m), 1.98 (3H, s) (les protons des -NH₂ donnent un signal large 6.0-9.0 ppm)

### Préparation et/ou références des réactifs utilisés dans les étapes de dimérisations des Exemples 1 à 121 décrits ci-dessus.

### Exemple R1 : 1,20-Diiodo-3,6,9,12,15,18-hexaoxaicosane

CAS 153399-56-5 ; J.-F. Nierengarten, C. O. Dietrich-Buchecker et J.-P Sauvage, J. Am. Chem. Soc., 1994, 116(1), 375-376.

### Exemple R2 : 1,17-Diiodo-3,6,9,12,15-pentaoxaheptadécane

CAS 118798-05-3 ; C. O. Dietrich-Buchecker et J. P. Sauvage, Angew. Chem., 1989, 101 (2), 192-194.

### Exemple R3 : 1,14-Diiodo-3,6,9,12-tétraoxatétradécane

CAS 76871-59-5 ; L. A. Frederick, T. M. Fyles, N. P. Gurprasad, D. M. Whitfield, Can. J. Chem, 1981, 59, 1724-1733.

### Exemple R4: 1-Iodo-2-{2-[2-(2-iodoéthoxy)éthoxy]éthoxy}ethane

CAS 36839-56-2 ; N. K. Dalley, K. E. Krakowiak, J. S. Bradshaw, M. M. England, X. Kou, R. M. Izatt, Tetrahedron, 1994, 50 (9), 2721-2728.

### Exemple R5 : 1-Iodo-2-[2-(2-iodoéthoxy)éthoxy]éthane

CAS 36839-55-1 : commercial

### Exemple R6 : 1-Bromo-2-(2-bromoéthoxy)éthane

CAS 5414-19-7 : commercial

### Exemple R7 : Ethane-1,2-diylbis(oxyéthane-2,1-diyloxy-3,1-phénylèneméthylène)diméthanesulfonate

A la solution de 0,40 g (1,10 m.mole) de l'[éthane-1,2-diylbis(oxyéthane-2,1-diyloxy-3,1-phénylène)]diméthanol (CAS 197573-04-9; J. E. Kickham,. S. J. Loeb, S. L. Murphy, Chemistry-A European Journal, 1997, 3(8), 1203-1213) dans 8 ml de dichlorométhane à -20°C sous azote, on ajoute 0,46 ml (3,31 m.moles) de triéthylamine et 0,18 ml (2,26 m.moles) de chlorure de mésyle. On agite la solution à - 20°C pendant 3 heures puis on la coule dans de l'acide chlorhydrique (1 M) et on extrait du dichlorométhane. La phase organique est lavée avec une solution aqueuse saturée de chlorure de sodium, séchée sur sulfate de sodium et concentrée sous pression réduite. On obtient 0,50 g (88%) d'une huile incolore.

MNH4+ = 536,3

### Exemnle R8 : 1,1'-[éthane-1,2-diylbis(oxyéthane-2,1-diyloxy)]bis[4-(bromométhyl)benzene]

CAS 110911-60-9 ; B. Cabezon, J. F. Cao, M. Raymo, J. F. Stoddart, A. J. P. White et D. J. Williams, Chemistry-A European Journal, 2000, 6(12), 2262-2273.

### Exemple R9 :1,1'-[octane-1,8-diyioxy)]bis[4-(bromométhyl)benzene]

CAS 263715-25-9 ; C. A. Schalley, G. Silva; Nising, F. Carl; P. Linnartz, Helvetica Chimica Acta, 2002, 85(6), 1578-1596.

### Exemple R10 : Pipérazine-1,4-diyldiéthane-2,1-diyl diméthanesulfonate

CAS 48185-66-6 ; décrit dans Sv. Zikolova, R. Konstantinova, L.Zhelyazkov, G. Sheikova, Trudove na Nauchnoizsledovatelskiya Khimikofarmatsevtichen Institut, 1972, 7, 117-22.

### Exemple R11 : 1,3-bis(2-iodoéthyl)-5-méthoxybenzene

### Etape A : Diéthyl 2,2'-[(5-méthoxy-1,3-phénylène)bis(oxy)]diacétate

A la suspension de 3,1 g (71,4 m.moles) d'hydrure de sodium (60% dispersion dans l'huile) dans 180 ml de 1-méthyl-2-pyrolidinone sous argon, on ajoute 5,0 g (35,7 m.moles) du 5-méthoxybenzene-1,3-diol puis au bout de 10 minutes 8,7 ml (78,5 m.moles) de 2-bromoacétate d'éthyle. On agite le mélange à température ambiante pendant 3 heures. Le milieu réactionnel est dilué avec de l'acétate d'éthyle et lavé avec une solution aqueuse saturée d'hydrogénosulfate de potassium, une solution saturée de chlorure de sodium et séchée sur sulfate de sodium puis concentrée à sec. Le brut est purifié par chromatographie sur gel de silice (dichlorométhane/acétate d'éthyle = 95/5). On obtient 5,9 g (53%) d'une gomme incolore.
MH⁺ = 313,1

### Etape B : 2,2'-[(5-Méthoxy-1,3-phénylène)bis(oxy)]diéthanol

Dans un tricol sous argon, on introduit 10 ml d'hydrure d'aluminium de lithium (1 M dans le tétrahydrofurane). Après avoir refroidi la solution à 0°C, on ajoute 1,0 g (3,2 m.moles) de diéthyl 2,2'-[(5-méthoxy-1,3-phénylène)bis(oxy)]diacétate en solution dans 6 ml de tétrahydrofurane. On observe la formation d'un précipité blanc. Au bout d'une heure d'agitation à température ambiante, on additionne lentement de l'acétate d'éthyle à 0°C. On verse le mélange dans une solution aqueuse saturée d'hydrogénosulfate de potassium et on extrait à l'acétate d'éthyle. La phase organique est lavée ave une solution saturée de chlorure de sodium et séchée sur sulfate de sodium puis concentrée à sec. On obtient 1,0 g (78%) d'une poudre blanche.
MH+ = 229,5

### Etape C

A la solution de 0,43 g (1,88 m.mole) du diol obtenu dans l'Etape précédente dans 3 ml d'acétonitrile, 4,5 ml d'éther éthylique et 2 ml de tétrahydrofurane, on ajoute 1,28 g (4,88 m.moles) de triphénylphosphine et 0,35 g (5,14 m.moles) d'imidazole. Après avoir refroidit la solution à 0°C, on additionne 1,36 g (5,36 m.moles) d'iode par portion. La solution est agitée à 0°C pendant 2 heures puis à température ambiante pendant 16 heures. Elle est versée dans une solution de bisulfite de sodium (10%) et extraite à l'acétate d'éthyle. La phase organique est lavée avec une solution saturée de chlorure de sodium, séchée sur sulfate de sodium et concentrée à sec. Le brut est purifié par chromatographie sur gel de silice (gradient dichlorométhane/acétate d'éthyle = 100/0 à 60/60). On obtient 0,75 g (89%) d'une poudre blanche.
MH+ = 449,0

### Exemple R12 : Diacide 3,3'-[octane-1,8-diylbis(oxy)]benzoïque

CAS 112763-29-8 ; D. Ramprasad, W. K. Lin, K. A. Goldsby, D. H. Busch, J. Am. Chem. Soc., 1988, 110(5), 1480-1487.

### Exemple R13 : Diacide 3,3'-{éthane-1,2-diylbis[imino(2-oxoéthane-2,1-diyl)oxy]}benzoïque

### Etape A : 3-(2-chloro-2-oxoéthoxy)benzoate d'éthyle

A une solution de 20,0 g (89,2 m.moles) d'acide [3-(éthoxycarbonyl)phénoxy]acétique (A. Banerjee, M. M. Adak, S. Das, S. Banerjee, S. Sengupta, Indian Chem. Soc., 1987, 64, 1, 34-37) dans 200 ml de 1,2-dichloroéthane, on ajoute 32,5 ml (0,45 mole) de chlorure de thionyle et 10µl de N,N-diméthylformamide. Le mélange est agité à reflux pendant 1 heure puis est concentré à sec et séché sous vide à 50 °C une nuit et utilisé tel quel dans l'étape suivante.

### Etape B

A la solution de 21,4 g (88,2 m.moles) du chlorure d'acide obtenu dans l'étape précédente dans 170 ml de dichlorométhane, on ajoute goutte à goutte 2,95 ml (44,1 m.moles) d'éthane 1,2-diamine en solution dans 10 ml de dichlorométhane et 12,3 ml (88,2 m.moles) de triéthylamine à température ambiante. La réaction est exothermique (on refroidit à l'aide d'un bain de glace) et on observe la formation d'un précipité blanc. Une fois l'addition terminée, on agite le milieu réactionnel à température ambiante pendant 1 heure. Après dilution avec du dichlorométhane, on lave la phase organique avec une solution aqueuse saturée d'hydrogénosulfate de potassium, une solution de bicarbonate de sodium saturée et on la sèche sur sulfate de sodium avant de la concentrer à sec. Le solide obtenu est repris dans l'heptane, filtré et séché pour fournir 19,2 g (92%) d'une poudre blanche.
MH⁺ = 473,3

### Etape C

A la suspension de 19,5 g (40,3 m.moles) du diester éthylique dans 65 ml de 1,4-dioxane et 65 ml d'éthanol, on ajoute 100 ml de soude (1 M) puis on chauffe le mélange à 100°C pendant 1 heure. La solution limpide est coulée dans 150 ml d'acide chlorhydrique (1 M). Le précipité obtenu est filtré, lavé abondamment à l'eau puis séché sous vide à 50 °C pour fournir 14,4 g (85%) d'une poudre blanche.
MH⁺ = 417,3 ; point de fusion : 282°C

### Exemple R14 : Diacide 4,4'-{éthane-1,2-diylbis[imino(2-oxoéthane-2,1-diyl)oxy]}benzoïque

### Etape A

A 4,6 g (16,1 m.moles) d'acide {4-[(benzyloxy)carbonyl]phénoxy}acétique (décrit dans la demande de brevet WO2001060813) en solution dans 50 ml dichlorométhane, on ajoute 4,9 ml (35,4 m.moles) de triéthylamine et 7,8 g (17,7 m.moles) de BOP^{®} sous argon à 0 °C. Au bout de 30 minutes, on ajoute 0,54 ml (8,03 m.moles) d'éthane 1,2-diamine. Le milieu réactionnel est agité à température ambiante pendant 16 heures. Le milieu réactionnel est dilué avec de l'acétate d'éthyle et extrait avec une solution aqueuse saturée de bicarbonate de sodium. Un précipité se forme, il est filtré, lavé à l'eau puis séché. On obtient 3,0 g d'une poudre blanche qui est utilisée sans purification dans l'étape de saponification suivante.

### Etape D

On ajoute 12,6 ml de soude (1 M) à la solution de 3,0 g (5,03 m.moles) du diester éthylique obtenu dans l'étape C ci-dessus dans 40 ml de 1-méthyl-2-pyrolidinone. On agite la solution à température ambiante pendant 16 heures. On acidifie le milieu réactionnel en ajoutant de l'acide chlorhydrique (1 M). On filtre le précipité et on le lave à l'eau. On obtient après séchage sous vide 1,3 g d'une poudre blanche.
MH⁺ = 417,5 ; point de fusion : 270°C

### Exemple R15: Sel sodique du diacide 3,3'-{[2-(diméthylamino)propane-1,3-diyl]bis[imino(2-oxoéthane-2,1-diyl)oxy]}benzoïque

En suivant les procédés décrits dans les Etapes A à C de l'**Exemple R13**, on prépare l'**Exemple R15** par dimérisation de l'acide [3-(éthoxycarbonyl)phénoxy]acétique avec la *N*²,*N*²-diméthylpropane-1,2,3-triamine. Pour de l'étape C, lorsque la saponification du diester est terminée, le milieu réactionnel est concentré à sec, repris dans du toluène et concentré à sec, puis dissout dans du méthanol et concentré à sec pour donner une gomme jaune. Le produit est dissout dans le méthanol et précipité dans de l'éther diisopropylique. Le précipité est filtré et séché sous vide à 50°C pour donner une poudre blanche.
MH⁺ = 474,4 ; point de fusion : 280°C

### Exemple R16: Sel sodique du diacide 3,3'-({2-[(diméthylamino)méthyl]propane-1,3-diyl}bis[imino(2-oxoéthane-2,1-diyl)oxyl])benzoïque

En suivant les procédés décrits dans l'**Exemple R15**, on prépare l'**Exemple R16** par dimérisation de l'acide [3-(éthoxycarbonyl)phénoxy]acétique avec la 2-(aminométhyl)-*N,N-*diméthylpropane-1,3-diamine. On obtient une poudre blanche.
MH⁺ = 488,4 ; point de fusion : 289°C

### Exemple R17: Diacide 3,3'-{(2-hydroxypropane-1,3-diyl)bis[imino(2-oxoéthane-2,1-diyl)oxy]}benzoïque

En suivant les procédés décrits dans les Etapes A à C de l'**Exemple R13**, on prépare l'**Exemple R15** par dimérisation de l'acide [3-(éthoxycarbonyl)phénoxy]acétique avec le 1,3-diaminopropan-2-ol. On obtient une poudre blanche.
MH⁺ = 447,4 ; point de fusion : 201 °C

### Exemple R18: Sel sodique du diacide 3,3'-{éthane-1,2-diylbis[(méthylimino)éthane-2,1-diyloxy]}benzoïque

### Etape A

Le mélange de 4,0 g (12,5 m.moles) de 3-(2-iodoéthoxy)benzoate d'éthyle (voir préparation de l'analogue ester méthylique CAS 225122-62-3 : P.D. Greenspan, et al., J. Med. Chem., 2001, 44, 4524-4534.), 0,78 ml (6,2 m.moles) de *N,N-*diméthyléthane-1,2-diamine et 1,73 g (12,3 m.moles) de carbonate de potassium dans 23 ml de *N,N-*diméthylformamide, est chauffé à 60°C pendant 3 heures. Le mélange est refroidi à température ambiante, dilué avec de l'acétate d'éthyle et extrait avec de l'eau. La phase organique est lavée avec une solution saturée de chlorure de sodium, séchée sur sulfate de sodium et concentrée à sec pour donner une huile jaune.
MH⁺ = 473,2

### Etape B

On ajoute 6,5 ml de soude (1 M) à la suspension de 1,53 g (3,24 m.moles) du diester éthylique obtenu dans l'étape A ci-dessus dans 20 ml d'éthanol. On agite la solution à température ambiante pendant 17 heures. Le milieu réactionnel est concentré à sec, puis repris dans de l'éthanol et concentré à sec pour donner une poudre blanche à trois reprises. Le produit est mis en suspension dans de l'acétone et filtré puis séché sous vide à 50°C pour donner une poudre blanche.
MH⁺ = 417,2 ; point fusion = 210 °C

### Exemple R19 : Sel sodique du diacide 3,3'-{éthane-1,2-diylbis[(benzylimino)éthane-2,1=diyloxy]}benzoïque

En suivant les procédés décrits dans les Etapes A et B de l'**Exemple R18**, on prépare l'**Exemple R19** par dimérisation du 3-(2-iodoéthoxy)benzoate d'éthyle avec la *N,N-*dibenzyléthane-1,2-diamine. On obtient une poudre blanche.
MH⁺ = 569,3

### Exemple R20 : Sel disodique du diacide 4,4'-{éthane-1,2-diylbis[(méthylimino)éthAne-2,1-diyloxy]}benzoïque

En suivant les procédés décrits dans les Etapes A et B de l'**Exemple R18**, on prépare l'**Exemple R20** par dimérisation du 4-(2-iodoéthoxy)benzoate d'éthyle [CAS 56703-36-7 décrit dans M. Kanao et al., Chem. Pharm. Bull., 1988, 36(8), 2968-76] avec la *N,N-*diméthyléthane-1,2-diamine. On obtient une poudre blanche.
MH⁺ = 417,1

### Exemple R21 : Diacide 3,3'-[(3,4-dioxocyclobut-1-ène-1,2-diyl)bis(iminoéthane-2,1-diyloxy)]benzoïque

### Etape A

Sous atmosphère d'azote et à température ambiante, 0,39 g de 3,4-diéthoxy-3-cyclobutène-1,2-dione (2,27 mmoles) est ajouté à une solution de 1,04 g de 3-(2-aminoéthoxy)benzoate d'éthyle (4,98 mmoles) [voir préparation de l'analogue ester méthylique CAS 153938-41-1 décrit dans C.C. Appeldoorn et al., Tetrahedron Asymm., 2005, 16(2), 361-372.] dans 23 ml de dichlorométhane. Le milieu réactionnel s'épaissit au bout de 24 h de réaction. Après 6 jours d'agitation, le milieu réactionnel est filtré. Le solide obtenu est repris dans de l'éther diisopropylique, filtré et séché sous vide. Après séchage, 1,98 g (87 %) de diester éthylique est obtenu sous forme d'une poudre blanche.
MH⁺ = 497,2 ; point fusion = 144,8 °C

### Etape B

On ajoute 4,2 ml de soude (1 M) à la solution de diester obtenu dans l'étape précédente dans 3,8 ml de diméthylsulfoxide. La solution est agitée à température ambiante pendant 24 heures. Le milieu réactionnel est versé dans une solution aqueuse d'hydrogénosulfate de potassium (2,2 eq.). Le diacide carboxylique précipite, il est filtré, lavé à l'eau jusqu'à pH neutre, séché sous vide pour fournir le diacide carboxylique sous forme d'une poudre blanche.
MH⁺ = 441,3 ; point fusion = 281,5 °C

### Exemple R22 : Sel sodique du diacide 3,3'-[(1,2-dioxoéthane-1,2-diyl)bis(iminoéthane-2,1-diyloxy)]benzoïque

### Etape A

On ajoute 2,82 g (8,70 m.moles) de 1-benzotriazolyle oxalate à une solution de 2,02 g (9,67 mmoles) de 3-(2-aminoéthoxy)benzoate d'éthyle [voir préparation de l'analogue ester méthylique CAS 153938-41-1 décrit dans C.C. Appeldoorn et al., Tetrahedron Asymm., 2005, 16(2), 361-372] dans 97 ml de *N,N-*diméthylformamide. Le mélange est agité à température ambiante pendant 6 jours. Le milieu réactionnel est versé dans une solution aqueuse saturée d'hydrogénocarbonate de sodium puis extrait avec un mélange acétate d'éthyle/tétrahydrofurane (1/1). La phase organique est lavée avec de l'eau jusqu'à pH neutre puis avec une solution aqueuse saturée de chlorure de sodium, séchée avec du sulfate de sodium anhydre et concentrée à sec. Le solide obtenu est repris dans de l'éther diisopropylique, filtré et séché sous vide pour fournir 1,59 g (70 %) de diester sous forme d'une poudre blanche.
MH⁺ = 497,2

### Etape B

On ajoute 5 ml de soude (1 M) à la solution de 1,1 g (2,31 m.moles) du diester dans 35 ml de *N-*méthylpyrrolidinone. Après 21 heures d'agitation, le milieu réactionnel est versé dans 350 ml d'acétone. Le sel de sodium précipite, il est filtré, lavé à l'acétone et séché sous vide pour fournir le diacide carboxylique sous forme d'une poudre blanche.
MH⁺ = 417,1

### Exemple R23 : Diacide 4,4'-{éthane-1,2-diylbis[(méthylimino)(2-oxoéthane-2,1-diyl)oxy]}benzoïque

En suivant les procédés décrits dans les Etapes A et B de l'**Exemple R14**, on prépare l'**Exemple R23** par dimérisation de l'acide {4-[(benzyloxy)carbonyl]phénoxy}acétique (décrit dans la demande de brevet W02001060813) avec la *N,N-*diméthyléthane-1,2-diamine. On obtient une poudre blanche.
MH⁺ = 445,2 ; point de fusion : 178°C

### Exemple R24: Diacide 4,4'-[oxybis(éthane-2,1-diyloxyéthane-2,1-diyloxy)]benzoïque

CAS 111630-85-4 ; K. N. Wiegel, A. C. Griffin,; M. S. Black, D. A. Schiraldi, Journal of Applied Polymer Science, 2004, 92(5), 3097-3106.

### Exemple R25: Diacide 4,4'-[éthane-1,2-diylbis(oxyéthane-2,1-diyloxy)]benzoïque

CAS 101678-92-6 ; B. M. Vogel, S. K. Mallapragada, Biomaterials, 2004, Volume Date 2005, 26 (7), 721-728.

### Exemple R26 : Diacide 4,4'-[oxybis(éthane-2,1-diyloxy)]benzoïque

CAS 69984-27-6 ; D. H. Hua, M. Tamura, M. Masahiro, K.Werbovetz, D. Delfin, M. Salem et P. K. Chiang, Bioorg. Med. Chem., 2003, 11, 20, 4357-4362.

### Exemple R27 : Diacide 4,4'-[éthane-1,2-diylbis(oxy)]benzoïque

CAS 3753-05-7 ; R. van Helden et A. F. Bickel, Recl. Trav. Chim. Pays-Bas, 1961, 80, 1237-1253

### Exemple R28 : Diacide 4,4'-[propane-1,3-diylbis(oxy)]benzoïque

CAS 3753-81-9 ; F. H. McMillan, J. Am. Chem. Soc, 1952, 74, 5229-5230.

### Exemple R29 : Diacide 4,4'-[éthane-1,3-diylbis(oxy)]benzoïque

CAS 3753-05-7; G. Avitabile et a/., J. of Polymer Science, Part B: Polymer Physics, 1999, 37(14), 1687-1701.

### Exemple R30 : Diacide 3,3'-[1,3-phénylènebis(sulfonyliminoéthane-2,1-diyloxy)]benzoïque

### Etape A

On ajoute 0,77 ml (5,5 mmoles) de triéthylamine et 0,61 g (2,2 mmoles) de chlorure de 1,3-benzenedisulfonyle à une solution de 1,01 g (4,84 mmoles) de 3-(2-aminoéthoxy)benzoate d'éthyle [voir préparation de l'analogue ester méthylique CAS 153938-41-1 décrit dans C.C. Appeldoorn et al., Tetrahedron Asymm., 2005, 16(2), 361-372] dans 22 ml de dichlorométhane. Après 5 jours d'agitation à température ambiante, le milieu réactionnel est versé dans une solution aqueuse d'acide chlorydrique (0,1 N) puis extrait à l'acétate d'éthyle, la phase organique est lavée avec une solution aqueuse saturée d'hydrogénocarbonate de sodium puis à l'eau et avec une solution aqueuse saturée de chlorure de sodium, séchée avec du sulfate de sodium anhydre et concentrée à sec. Le solide obtenu est repris dans de l'éther diisopropylique, filtré, séché sous vide pour fournir 1,23 g (90 %) de diester sous forme d'une poudre blanche.
MH+ = 621,2; point de fusion : 124,7 °C

### Etape B

En suivant le procédé décrit dans l'Etape B de l'**Exemple R21**, on obtient le diacide carboxylique sous forme d'une poudre blanche.
MH+ = 565,2; point de fusion : 226,2 °C

### Exemple R31 : Diacide 3,3'-[(1,3-dioxopropane-1,3-diyl)bis(iminoéthane-2,1-diyloxy)]benzoïque

### Etape A

Sous atmosphère d'azote et à température ambiante, l'EDCI (1,02 g / 5,34 mmoles), le 1-hydroxybenzotriazole (0,72 g / 5,34 mmoles), la triéthylamine (0,75 ml / 5,34 mmoles) et l'acide malonique (0,25 g / 2,43 mmoles) sont ajoutés respectivement à une solution du 3-(2-aminoéthoxy)benzoate d'éthyle [voir préparation de l'analogue ester méthylique CAS 153938-41-1 décrit dans C.C. Appeldoorn et al., Tetrahedron Asymm., 2005, 16(2), 361-372] (1,02 g / 4,86 mmoles) dans 24 ml de *N,N-*diméthylformamide. Après 24 heures d'agitation, le milieu réactionnel est versé dans une solution aqueuse saturée d'hydrogénosulfate de potassium puis est extrait avec de l'acétate d'éthyle. La phase organique est lavée à l'eau jusqu'à pH neutre puis avec une solution aqueuse saturée de chlorure de sodium, séchée avec du sulfate de sodium anhydre et concentrée à sec. L'huile obtenue est reprise dans de l'éther diisopropylique, un précipité blanc est obtenu, il est filtré et séché sous vide pour fournir 0,76 g (64%) de diester sous forme d'une poudre blanche.
MH+ = 487,3 ; point de fusion : 98,9 °C

### Etape B

En suivant le procédé décrit dans l'Etape B de l'**Exemple R21**, on obtient le diacide carboxylique sous forme d'une poudre blanche.
MH+ = 431,2 ; point de fusion : 240,8 °C

### Exemple R32 : Diacide 3,3'-[1,4-phénylènebis(carbonyliminoéthane-2,1-diyloxy)]benzoïque

### Etape A

En suivant le procédé décrit dans l'Etape C de l'**Exemple 53**, on réalise le couplage du 3-(2-aminoéthoxy)benzoate d'éthyle [voir préparation de l'analogue ester méthylique CAS 153938-41-1 décrit dans C.C. Appeldoorn et al., Tetrahedron Asymm., 2005, 16(2), 361-372] avec l'acide téréphtalique.
MH+ = 549,3 ; point de fusion : 153,5 °C

### Etape B

En suivant le procédé décrit dans l'Etape B de l'**Exemple R21**, on réalise la saponification du diester pour obtenir le diacide carboxylique sous forme d'une poudre blanche.
MH+ = 493,2 ; point de fusion : 284,5 °C

### Exemple R33 : Diacide 3,3'-[pyridine-3,5-diylbis(carbonyliminoéthane-2,1-diyloxy)]benzoïque

### Etape A

En suivant le procédé décrit dans l'Etape C de l'**Exemple 53**, on réalise le couplage du 3-(2-aminoéthoxy)benzoate d'éthyle [voir préparation de l'analogue ester méthylique CAS 153938-41-1 décrit dans C.C. Appeldoorn et al., Tetrahedron Asymm., 2005, 16(2), 361-372] avec l'acide 3,5 pyridine carboxylique.
MH+ = 550,3; point de fusion : 121 °C

### Etape B

En suivant le procédé décrit dans l'Etape B de l'**Exemple R21**, on réalise la saponification du diester pour obtenir le diacide carboxylique sous forme d'une poudre blanche.
MH⁺ = 494,1 ; point de fusion : 246,5°C

### Exemple R34 : Diacide 3,3'-{éthane-1,2-diylbis[(méthylimino)(2-oxoéthane-2,1-diyl)oxy]}benzoïque

En suivant les procédés décrits dans les Etapes A à C de l'**Exemple R13**, on prépare l'**Exemple R34** par dimérisation de l'acide [3-(éthoxycarbonyl)phénoxy]acétique avec la *N,N-*diméthyléthane-1,2-diamine. On obtient une poudre blanche.
MH⁺ = 445,2 ; point de fusion : 179°C

### Exemple R35 : Diacide 3,3'-{(1R,2R)-cyclopropane-1,2-diylbis[imino(2-oxoéthane-2,1-diyl)oxy]}benzoïque

En suivant les procédés décrits dans les Etapes A à C de l'**Exemple R13**, on prépare l'**Exemple R35** par dimérisation de l'acide [3-(éthoxycarbonyl)phénoxy]acétique avec la 1,2-trans-cyclopropanediamine (CAS 758637-65-9). On obtient une poudre blanche.
MH⁺ = 429,3 ; point de fusion : 121 °C

### Exemple R36 : Diacide 3,3'-{(1R,2S)-cyclopropane-1,2-diylbis[imino(2-oxoethane-2,1-diyl)oxy]}benzoïque

En suivant les procédés décrits dans les Etapes A à C de l'**Exemple R13**, on prépare l'**Exemple R36** par dimérisation de l'acide [3-(éthoxycarbonyl)phénoxy]acétique avec la 1,2-cis-cyclopropanediamine (CAS 365996-16-3). On obtient une poudre blanche.
MH⁺ = 429,3 ; point de fusion : 253°C

### Exemple R37 : Diacide 3,3'-{méthylènebis[imino(2-oxoéthane-2,1-diyl)oxy]} benzoïque

En suivant les procédés décrits dans les Etapes A à C de l'**Exemple R13,** on prépare l'**Exemple R37** par dimérisation de l'acide [3-(éthoxycarbonyl)phénoxy]acétique avec la méthylènediamine. On obtient une poudre blanche.
MH⁺ = 401,4 ; point de fusion : 252°C

### Exemple R38 : N, N'-ethylène-bis-(acide N-méthylsuccinamique)

CAS 62538-62-9 ; décrit dans R.E. Asay et a/., J. Heterocyclic Chem., 1977, 14(1), 85-90

### Exemples R39 à R64

En suivant la procédure décrite pour la préparation de l'**Exemple R38**, on prépare les diacides carboxyliques R39 à R61 avec les diamines et les anhydrides donnés dans le tableau ci-dessous.

| | **diacides carboxyliques** | **diamines** | **anhydrides** | MH⁺/RT (Méthode D) |
|---|---|---|---|---|
| **R39** | HO₂C-CH₂CH₂CON(CH₃)CH₂CH₂-(OCH₂CH₂)₂N(CH₃)COCH₂CH₂CO₂H | (CH₃)HNCH₂CH₂ (OCH₂CH₂)₂NH(CH₃) | 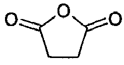 | MH⁺= 377,2 RT= 0.674 min |
| **R40** | HO₂C-CH₂CH₂CON(CH₃)CH₂CH₂-OCH₂CH₂N(CH₃)COCH₂CH₂CO₂H | (CH₃)HNCH₂CH₂ OCH₂CH₂NH(CH₃) | 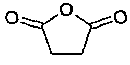 | MH⁺= 333,0 RT= 0,635 min |
| **R41** | 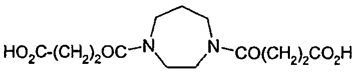 | 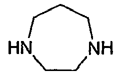 | 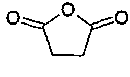 | MH⁺= 301,0 RT= 0,251 min |
| **R42** | HO₂C-CH₂CH₂CON(CH₃)(CH₂)₈-N(CH₃)COCH₂CH₂CO₂H | (CH₃)HN(CH₂)₈ NH(CH₃) | 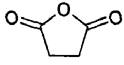 | MH⁺= 373,2 RT= 0,989 min |
| **R43** | HO₂C-CH₂CH₂CON(CH₃)CH₂CH₂-CH₂N(CH₃)COCH₂CH₂CO₂H | (CH₃)HNCH₂CH₂ CH₂NH(CH₃) | 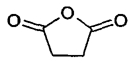 | MH⁺= 303,2 RT= 0,561 min |
| **R44** | HO₂C-(CH₂)₃CON(CH₃)CH₂CH₂-(OCH₂CH₂)₂N(CH₃)CO(CH₂)₃CO₂H | (CH₃)HNCH₂CH₂ (OCH₂CH₂)₂NH(CH₃) | 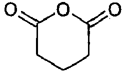 | MH⁺= 405,2 RT= 0,740 min |
| **R45** | HO₂C-(CH₂)₃CON(CH₃) (CH₂)₈N(CH₃)CO(CH₂)₃CO₂H | (CH₃)HN(CH₂)₈NH(CH₃) | 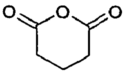 | MH⁺= 401,2 RT= 1,028 min |
| **R46** | HO₂C-(CH₂)₃CON(CH₃) (CH₂)₆N(CH₃)CO(CH₂)₃CO₂H | (CH₃)HN(CH₂)₆NH(CH₃) | 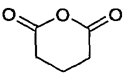 | MH⁺= 373,2 RT= 0,885 min |
| **R47** | HO₂C-(CH₂)₃CON(CH₃)CH₂CH₂-OCH₂CH₂N(CH₃)CO(CH₂)3CO₂H | (CH₃)HNCH₂CH₂ OCH₂CH₂NH(CH₃) | 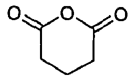 | MH⁺= 361,1 RT= 0,734 min |
| **R48** | HO₂C-(CH₂)₃CON(CH₃) (CH₂)₂N(CH₃)CO(CH₂)₃CO₂H | (CH₃)HN(CH₂)₂NH(CH₃) | 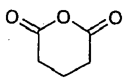 | MH⁺= 317,2 RT= 0,599 min |
| **R49** | 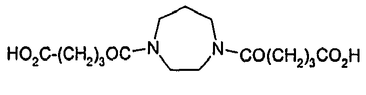 | 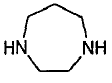 | 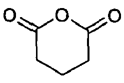 | MH⁺= 329,0 RT= 0,623 min |
| **R50** | HO₂C-CH₂OCH₂CON(CH₃)CH₂CH₂-(OCH₂CH₂)₂N(CH₃)COCH₂OCH₂CO₂H | (CH₃)HNCH₂CH₂ (OCH₂CH₂)₂NH(CH₃) | 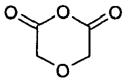 | MH⁺= 409,2 RT= 0,616 min |
| **R51** | HO₂C-CH₂OCH₂CON(CH₃)(CH₂)₈-N(CH₃)COCH₂OCH₂CO₂H | (CH₃)HN(CH₂)₈ NH(CH₃) | 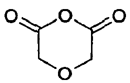 | MH⁺= 405,2 RT= 0,953 min |
| **R52** | HO₂C-CH₂OCH₂CON(CH₃)(CH₂)₆-N(CHa)COCH₂OCHzCOzH | (CH₃)HN(CH₂)₆ NH(CH₃) | 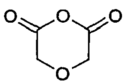 | MH⁺= 377,2 RT= 0,786 min |
| **R53** | HO₂C-CH₂OCH₂CON(CH₃)(CH₂)₃-N(CH₃)COCH₂OCH₂CO₂H | (CH₃)HN(CH₂)₃NH(CH₃) | 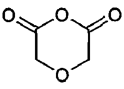 | MH⁺= 355,2 RT= 0,413 min |
| **R54** | 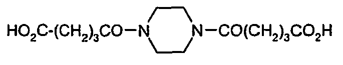 | 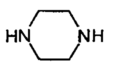 | 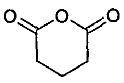 | MH⁺= 315,2 RT= 0,591 min |
| **R55** | 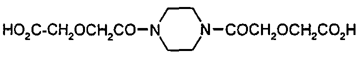 | 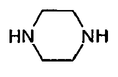 | 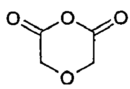 | MH⁺= 319,2 RT= 0,181 min |
| **R56** | HO₂C-(CH₂)₄CON(CH₃) (CH₂)₃N(CH₃)CO(CH₂)₄CO₂H | (CH₃)HN(CH₂)₃NH(CH₃) | 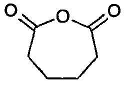 | MH⁺= 359,2 RT= 0,770 min |
| **R57** | HO₂C-(CH₂)₂CON(CH₃) (CH₂)₆N(CH₃)CO(CH₂)₂CO₂H | (CH₃)HN(CH₂)₆NH(CH₃) | 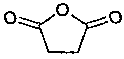 | MH⁺= 345,2 RT= 0,831 min |
| **R58** | HO₂C-CHOCH₂CON(CH₃)CH₂CH₂OCH₂ CH₂N(CH₃)COCH₂OCH₂CO₂H | (CH₃)HNCH₂CH₂OCH₂ CH₂NH(CH₃) | 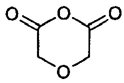 | MH⁺= 365,0 RT= 0,507 min |
| **R59** | HO₂C-(CH₂)₄CON(CH₃) (CH₂)₂N(CH₃)CO(CH₂)₄CO₂H | (CH₃)HN(CH₂)₂NH(CH₃) | 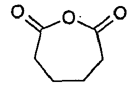 | MH⁺= 345,2 RT= 0,717 min |
| **R60** | HO₂C-(CH₂)₃CON(CH₃) (CH₂).₃N(CH₃)CO(CH₂)₃CO₂H | (CH₃)HN(CH₂)₃NH(CH₃) | 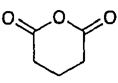 | MH⁺= 331,2 RT= 0,671 min |
| **R61** | HO₂C-CH₂OCH₂CON(CH3)CH₂-CH₂N(CH₃)COCH₂OCH₂CO₂H | (CH₃)HNCH₂-CH₂NH(CH₃) | 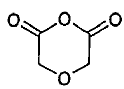 | MH⁺= 321,2 RT= 0,210 min |
| **R62** | HO₂C-CH₂OCH₂CONHCH₂-CH₂NHCOCH₂OCH₂CO₂H | H₂NCH₂CH₂NH₂ | 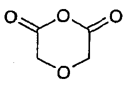 | MH⁺=293,2 RT=0,167 min |
| **R63** | HO₂C-CH₂OCH₂CONHCH₂CH₂O CH₂CH₂NHCOCH₂OCH₂CO₂H | H₂NCH₂CH₂OCH₂CH₂N H₂ | 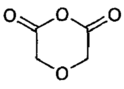 | MH⁺=337,2-RT=0,263 min |
| **R64** | HO₂C-CH₂OCH₂CONH(CH₂CH₂O)₂ CH₂CH₂NHCOCH₂OCH₂-CO₂H | H₂N(CH₂CH₂O)₂-CH₂CH₂NH₂ | 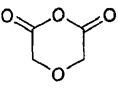 | MH⁺=381,2 RT=0,487 min |

### Exemple R65 : 2,2'-[Ethane-1,2-diylbis(oxyéthane-2,1-diyloxy-4,1-phénylène)] bis(4,4,5,5-tétraméthyl-1,3,2-dioxaborolane)

Le mélange de 3,0 g (13,6 m.moles) de 4-(4,4,5,5-tétramethyl-1,3,2-dioxaborolan-2-yl)phénol, 2,51 g (6,82 m.moles) de 1-iodo-2-[2-(2-iodoéthoxy)éthoxy]éthane (Exemple R5) et 8,9 g (27,3 m.moles) de carbonate de cesium dans 30 ml de *N,N-*diméthylformamide est chauffé à 60°C pendant 5 heures. La solution est coulée dans une solution aqueuse saturée d'hydrogénosulfate de potassium et extraite avec de l'acétate d'éthyle. La phase organique est lavée avec une solution saturée de chlorure de sodium, séchée sur sulfate de sodium et concentrée à sec. Le solide obtenu est purifié par flash chromatographie sur gel de silice (gradient cyclohexane/éther diisopropylique = 90/10 à 0/100) pour fournir 1,8 g (47 %) d'une poudre banche.
MH+ = 555,5 ; point de fusion : 97°C

### Exemple R66 : 2,2'-[Oxybis(éthane-2,1-diyloxyéthane-2,1-diyloxy-4,1-phénylène)] bis(4,4,5,5-teétraméthyl-1,3,2-dioxaborolane)

En suivant le procédé décrit dans l'Exemple R65, on prépare l'Exemple R66 par dimérisation du 4-(4,4,5,5-tétramethyl-1,3,2-dioxaborolan-2-yl)phénol avec 1-iodo-2-{2-[2-(2-iodoéthoxy)éthoxy]éthoxy}éthane (Exemple R4). On obtient une poudre blanche.
MH+ = 599,4 ; point de fusion : 55°C

### Exemple R67 : 2,2'-[Ethane-1,2-diylbis(oxyéthane-2,1 -diyloxy-3,1 -phénytène)] bis(4,4,5,5-tétraméthyl-1,3,2-dioxaborolane)

En suivant le procédé décrit dans l'Exemple R65, on prépare l'Exemple R67 par dimérisation du 3-(4,4,5,5-tétramethyl-1,3,2-dioxaborolan-2-yl)phénol avec 1-iodo-2-[2-(2-iodoéthoxy)éthoxy]éthane (Exemple R5). On obtient une poudre blanche.
MNH₄+ = 572,4

### Exemple R68 : Diacide [oxybis(éthane-2,1-diyloxyéthane-2,1-diyloxy-3,1-phénylène)]boronique

Le mélange de 3,0 g (13,6 m.moles) de 3-(4,4,5,5-tétraméthyl-1,3,2-dioxaborolan-2-yl)phénol, 2,82 g (6,82 m.moles) de 1-iodo-2-{2-[2-(2-iodoéthoxy)éthoxy]éthoxy}éthane (Exemple R4) et 8,88 g (27,3 m.moles) de carbonate de cesium dans 30 ml de *N,N-*diméthylformamide est chauffé à 60°C pendant 5 heures. La solution est coulée dans une solution aqueuse saturée d'hydrogénosulfate de potassium et extraite avec de l'acétate d'éthyle. La phase organique est lavée avec une solution saturée de chlorure de sodium, séchée sur sulfate de sodium et concentrée à sec. On obtient le diester boronique cible en partie hydrolysé en acide boronique.

Le brut réactionnel est mis en solution dans un mélange de 20 ml de 1,2-diméthoxyéthane et de 80 ml d'eau en présence de 3,18 g (30,1 m.moles) de carbonate de sodium. Après 3 jours d'agitation à température ambiante, la solution aqueuse est lavée avec de l'acétate d'éthyle puis acidifiée avec une solution aqueuse saturée d'hydrogénosulfate de potassium et extraite avec de l'acétate d'éthyle. La phase organique est lavée à l'eau, avec une solution saturée de chlorure de sodium, séchée sur sulfate de sodium puis concentrée à sec. On obtient 0,82 g (28%) d'une poudre blanche.
MH+ = 435,3

### Exemple R69 : Acide 3-{2-[(2-{[(4-carboxyphénoxy)acétyl]amino}éthyl)amino]-2-oxoéthoxy}benzoïque

### Etape A : 3-{2-[(2-Aminoéthyl)amino]-2-oxoéthoxy}benzoate d'éthyle

A 2,06 g (9,2 m.moles) d'acide {4-[(benzyloxy)carbonyl]phénoxy}acétique (décrit dans la demande de brevet W02001060813) dans 46 ml de dichlorométhane sous atmosphère d'azote et à température ambiante, on ajoute 1,94 ml (14 m.moles) de triéthylamine, 5,73 g (11 mmoles) de PyBOP et 1,45 ml (9,2 m.moles) de *N-tert-*butyloxycarbonyl-éthylenediamine. Après 22 heures d'agitation, le milieu réactionnel est coulé dans une solution aqueuse d'acide chlorhydrique (0,1 N) puis est extrait à l'acétate d'éthyle. La phase organique est lavée avec une solution aqueuse saturée d'hydrogénocarbonate de sodium, puis avec une solution aqueuse saturée de chlorure de sodium, séchée sur sulfate de sodium anhydre et concentrée à sec. L'huile obtenue est reprise dans de l'éther diisopropylique, le précipité blanc obtenu est filtré, séché sous vide pour fournir 1,95 g (58 %) du carbamate sous forme d'une poudre blanche.
A température ambiante, 5,64 ml (76 m.moles) d'acide trifluoroacétique sont ajoutés à une solution de 1,39 g (3,80 mmoles) du carbamate dans 24 ml de dichlorométhane. Après 4 h 30 d'agitation, 100ml de 1,2-dichloroéthane sont ajoutés au milieu réactionnel qui est ensuite concentré à sec. L'huile obtenue est versée dans une solution aqueuse saturée d'hydrogénocarbonate de sodium et extraite avec un mélange tétrahydrofurane / acétate d'éthyle (1/1). La phase organique est concentrée à sec, l'huile obtenue est séchée sous vide pour fournir 1,09 g (86%) de l'amine sous forme d'une huile jaune.
MH⁺ = 267,3

### Etape B : 3-{2-[(2-{[(4-Carboxyphénoxy)acétyl]amino}éthyl)amino]-2-oxoéthoxy}benzoate d'éthyle

Sous atmosphère d'azote et à température ambiante, 0,86 ml (6,12 m.moles) de triéthylamine, 2,55 g (4,89 m.moles) de PyBOP et 1,09 g (4,08 m.moles) de 3-{2-[(2-aminoéthyl)amino]-2-oxoéthoxy}benzoate d'éthyle sont ajoutés respectivement à une solution de 0,91 g (4,08 m.moles) de {4-[(éthyloxy)carbonyl]phénoxy}acétique (CAS 30893-58-4) dans 20 ml de dichlorométhane. Le milieu réactionnel est hétérogène. Après 5 heures d'agitation, le milieu réactionnel est filtré. Le filtrat obtenu est concentré à sec. Le résidu obtenu est versé dans une solution aqueuse saturée d'hydrogénocarbonate de sodium. Le solide obtenu est filtré, lavé à l'eau et séché sous vide pour fournir 1,22 g (63 %) du diester sous forme d'une poudre blanche.
MH⁺ = 473,2; point de fusion : 117,5 °C

### Etape C

En suivant le procédé décrit dans l'Etape B de l'**Exemple R21**, on réalise la saponification du diester pour obtenir le diacide carboxylique sous forme d'une poudre blanche.
MH⁺ = 417,2 ; point de fusion : 257,1 °C

Les résultats de tests pharmacologiques *in vitro* et *in vivo* effectués en vue de déterminer des propriétés des composés de l'invention sont répertoriés ci-dessous ;

### Modèle de prolifération des Baf/3-FGFR1 β ou FGF-R4α

### Obtention des constructions récepteurs chimériques

Les récepteurs chimériques sont réalisés entre la partie intracellulaire du hMpl (NM_005373) et les domaines transmembranaires et extracellulaires de FGF-R1β ou FGF-R4α.

Pour chaque récepteur du FGF, les fragments FGF-R (BamH/- Sac/) et hMpl (Sac/ - Not/) sont simultanément clonés dans le vecteur pEF6 (Invitrogen) digérés par BamH/ et Not/.

### Transfection des BaF/3

Les cellules murines de type BaF/3 sont transfectées par électroporation. 20 µg de plasmide portant les constructions FGF-R1/β-hMpl ou FGF-R4α-hMpl sont mélangés à 5.10⁶ cellules reprises dans 800 µl de RPMI (invitrogen). Le mélange est soumis à deux chocs électriques de 400 V avec un intervalle de 0,1 ms. Les cellules sont reprises pendant 48h dans un milieu RPMI, 10% SVF (sérum de veau foetal, gibco), glutamine (In vitrogen), NEAA (Non Essential Amino Acids, Invitrogen), NaPyr (pyruvate de sodium, Invitrogen) contenant 10 ng/ml d'IL-3 avant d'être sélectionnées par 10 µg/ml de blasticidine (Cayla).

### Culture des cellules transfectées

Les lignées cellulaires BaF/3 transfectées avec les constructions FGF-FGF-R1β-hMpl ou FGF-R4α-hMpl sont maintenues dans un milieu classique pour BaF/3 contenant 10 µg/ml de blasticidine supplémenté soit par 10 ng/ml d'IL-3 soit par 10 ng/ml de FGF2 associé à 100 µg/ml d'héparine.

### Mesure de prolifération cellulaire

Une culture confluente de cellules BaF/3 portant les récepteurs chimériques est passée au tiers dans du milieu de culture supplémenté en IL-3 (10 ng/ml) pendant 24 h. Puis les cellules sont déprivées en sérum pendant une nuit dans du RPMI avant d'être stimulées. La stimulation est effectuée en plaque 96 puits (microplate krystal, Porvair) et en quadriplicats. Pour chaque condition, sont ajoutés dans l'ordre : 50µl d'une solution de produit concentrée deux fois dans du RPMI et 50µl d'une suspension cellulaire à 200 000 cellules/ml dans du RPMI contenant 0,2% de SVF, du NEAA 2x, du NaPyr 2x et de la glutamine 2x. Les plaques sont incubées 28h à 37°C puis 100µl de Cell Titer Glo (Promega) sont ajoutés et la quantité d'ATP est quantifiée à l'aide d'un luminomètre (Luminoskan Ascent, Labsystems).

Les composés objets de l'invention sont des agonistes des récepteurs FGFs. Notamment ils présentent *in vitro* une activité spécifique envers le FGFR1β et le FGF-R4a comprise en tre 3x10⁻⁵ et 1x10⁻⁶M. A titre d'exemple les composés 44 et 30 sont actifs à une concentration de 30 µM sur les Baf/3-FGFR1β et FGF-R4α.

### Modèle d'angiogenèse in vitro

Les produits sont testés pour induire le réarrangement de cellules endothéliales veineuses humaines (HUVEC) sur du matrigel (Becton dickinson 356230) dilué dans du collagène (rat Tail collagène, type I : Becton dickinson 354236). Après 24 heures, les cellules sont observées au microscope objectif X4 et la longueur des pseudo-tubules est mesurés par l'intermédiaire d'un analyseur d'image (BIOCOM-logiciel Visiolab 2000).

Pour le test d'angiogenèse in vitro, les composés de l'invention ont démontré une activité spécifique comprise entre 10⁻⁶ M et 10⁻¹²M. A titre d'exemple les composés 2, 8 et 50 sont actifs à une concentration de 10 nM sur le modèle d'angiogénèse in vitro.

### Modèle de la revascularisation post ischémique de la patte chez la souris

L'expérience est réalisée sur des souris C57 (IFFA CREDO France).

Les animaux sont anesthésiés par injection intra-péritonéale, sous un volume de 10ml/kg d'une solution de kétamine (Kétamine 1000 Virbac ®, Virbac Carros France) 50 mg/kg et de xylazine (Rompun 2%®, Bayer Pharma Puteaux France) 10 mg/kg L'animal est placé en décubitus dorsal, après rasage et désinfection de la peau par badigeonnage à la Vétédine® solution (Vétoquinol S.A. Lure France), une incision est pratiquée au niveau de la région inguinale. L'artère fémorale est excisée, les collatérales cautérisées et les artères iliaques externe et circonflexe ligaturées. La peau est suturée avec des points séparés à l'aide de fil non résorbable. Les animaux sont placés en salle de réveil dont la température est régulée à 25°C jusqu'au réveil complet.

La perfusion distale est mesurée à l'aide d'un scanner laser Doppler (LDPI Lisca Perimed modèle PIM II, AB Suède). Cette technique permet de mesurer la perfusion cutanée de la partie supérieure des pattes postérieures Ces mesures sont réalisées sous anesthésie (kétamine + xylazine), immédiatement avant l'induction chirurgicale de l'ischémie (T0) et immédiatement après (T1) pour vérifier la sévérité de l'ischémie. Des mesures au niveau de la patte saine et de la patte ischémiée sont réalisées successivement afin d'établir un déficit de perfusion exprimé en %. Des mesures, réalisées dans les mêmes conditions d'anesthésie, sont faites 3, 7 et 14 jours après l'induction de l'ischémie pour évaluer la récupération de perfusion post-ischémique.

Dans cette expérimentation, les composés de l'invention sont actifs à des doses de 1 à 50 mg/kg/jour. A titre d'exemple le composé 21 a une activité significative à une dose de 10 mg/Kg/jour par voie sous-cutanée pendant 7 jours dans le modèle d'ischémie de la patte.

## Revendications

1. Composés agonistes des récepteurs FGFs répondant à la formule générale :
M₁-L-M₂
dans laquelle M₁ ou M₂ identiques ou différents représentent chacun indépendamment l'un de l'autre une unité monomère M et L représente un groupe de liaison qui lie M₁ et M₂ de façon covalente, **caractérisée en ce que**
ladite unité monomère répond à la formule générale M qui suit: 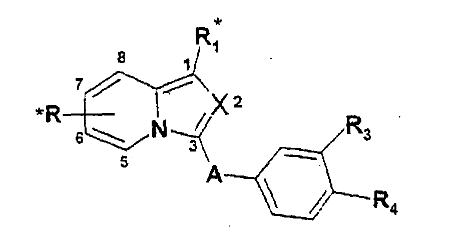 dans laquelle,
X représente N ou C-R₂*,
A représente un radical -CO-,
* indique le site de liaison entre L avec l'unité monomère M₁ d'une part et avec l'unité monomère M₂ d'autre part; ledit site de liaison de chaque unité monomère M₁ ou M₂ étant situé sur l'un des substituants R, R₁ ou R₂,
R représente un atome d'hydrogène, un atome d'halogène, un radical alkyle linéaire ou ramifié de 1 à 5 atomes de carbone, un radical hydroxy, ou un radical de formule:
• -CO₂R₅
• -CO-NR₆R₇
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk
• -NH-CO-Alk, ou
• -NH-CO₂-Alk
dans lesquels :
• R₅ représente un atome d'hydrogène, un radical alkyle linéaire ou ramifié de 1 à 5 atomes de carbone ou un radical benzyle ;
• R₆ et R₇ identiques ou différents représentent chacun un atome d'hydrogène, un radical alkyle linéaire ou ramifié de 1 à 5 atomes de carbone ou un radical benzyle,
• Alk représente un radical alkyle linéaire ou ramifié de 1 à 5 atomes de carbone ou un radical alkylène linéaire ou ramifié de 1 à 5 atomes de carbone ;
• Ph représente un radical phényle éventuellement substitué par un ou plusieurs atomes d'halogène, par un ou plusieurs radicaux hydroxy, par un ou plusieurs radicaux alcoxy linéaires ou ramifiés de 1 à 5 atomes de carbone, ou par un ou plusieurs radicaux -COOR₅;
R₁ représente un atome d'hydrogène, un atome d'halogène, un radical hydroxy, un radical cyano, un radical alkyle linéaire ou ramifié de 1 à 5 atomes de carbone ou un radical de formule :
• -CO₂R₅
• -CO-NR₆R₇
• -CO-NH-Alk-CO₂R₅
• -CO-NH-Ph
• -O-Alk
• -O-Alk-CO₂R₅
• -O-Alk-CO-NR₆R₇
• -O-Alk-NR₆R₇
• -O-Alk-OR₅
• -O-Alk-Ph
• -O-Ph
• -NR₆R₇
• -NH-SO₂-Alk
• -NH-CO-Alk
• -NH-CO-Alk-CO₂R₅
• -NH-CO-Alk-CO-NR₆R₇
• -NH-CO₂-Alk
• -NH-CO-Ph, ou
• un radical aryle ou hétéroaryle de 5 ou 6 atomes choisis parmi C, N, 0, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, ou par un ou plusieurs radicaux -CO₂R₅ ou -CO-NR₆R₇ dans lesquels Alk, Ph, R₅, R₆ et R₇ sont tels que définis au niveau du groupe R;
R₂ représente un radical alkyle linéaire ou ramifié de 1 à 5 atomes de carbone, un radical cycloalkyle de 3 à 6 atomes de carbone ou un radical phényle éventuellement substitué par un ou plusieurs atomes d'halogène, par un ou plusieurs radicaux carboxy, par un ou plusieurs radicaux alcoxy linéaires ou ramifiés de 1 à 5 atomes de carbone, par un ou plusieurs radicaux hydroxy, par un ou plusieurs radicaux benzyloxy, ou par un ou plusieurs radicaux alcoxycarbonyle de 2 à 6 atomes de carbone ;
R₃ et R₄ représentent indépendamment l'un de l'autre un atome d'hydrogène, un radical hydroxy, un radical amino, un radical nitro ou un radical de formule:
• -CO₂R₅
• -CO-NR₆R₇
• -CO-NHOH
• -O-Alk
• -O-Alk-NR₆R₇
• -O-Alk-CO-NR₆R₇
• -NHOH
• -NR₆R₇
• -N(R₈)-CO-Alk
• -N(R₈)-CO-CF₃
• -N(R₈)-CO-Ph
• -N(R₈)-CO₂Alk
• -N(R₈)-SO₂-Alk
• -N(R₈)-CO-Alk-NR₆R₇
• -N(R₈)-SO₂-Alk-NR₆R₇, ou
• -NH-Alk-R₆R₇
dans lesquels R₈ représente un atome d'hydrogène ou un radical -Alk-COOR₅ et Alk, R₅, R₆ et R₇ sont tels que définis au niveau du groupe R;
ou R3 et R4 forment ensemble, avec les atomes de carbone du noyau phényle auquel ils sont attachés, un cycle à 6 chaînons comportant un atome d'azote et un autre hétéroatome tel que l'oxygène; et
L représente :
un radical alkylène linéaire ou ramifié de 2 à 25 atomes de carbone, un radical alkényle linéaire ou ramifié de 2 à 25 atomes de carbone, ou un groupe alkynyle linéaire ou ramifié de 2 à 25 atomes de carbone ;
ou un radical choisi parmi les formules: 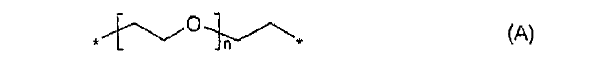 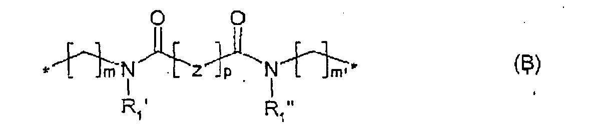 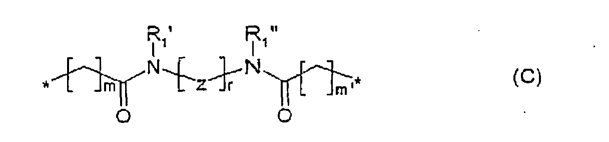 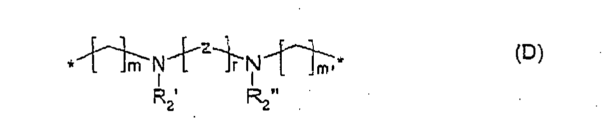 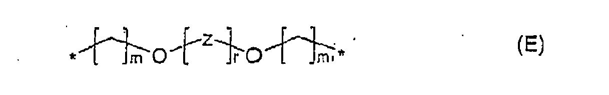 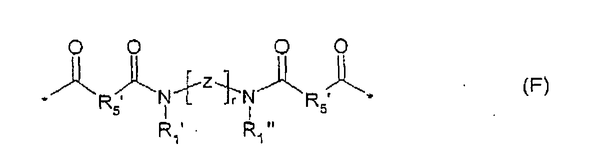 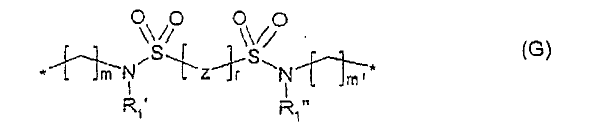 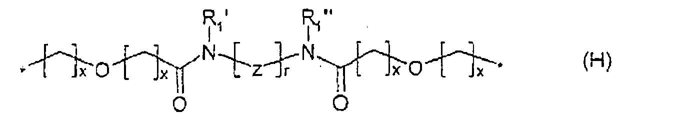
dans lesquelles
* indique l'atome de connexion de L avec l'unité monomère M sur l'un des substituants R, R₁ ou R₂ ;
Z représente une liaison ou un radical carbonyle ou un radical alkylène linéaire, ramifié ou cyclique de 1 à 6 atomes de carbone, éventuellement subsituté par 1 ou 2 radicaux carbonyl, ou bien un radical
• -[CH₂]ₛ-[-CH-(CH₂)_{q}-OR₃']-[CH₂]ₛ-
• -[CH₂]ₛ-[-CH-(CH₂)_{q}-N R₃'R₄']-[CH₂]ₛ-
• -[CH₂-CH₂-O]ₜ-CH₂-CH₂-
• phényle ou alkylphénylalkyle, le groupe phényle étant éventuellement substitué par un ou plusieurs radicaux alcoxy de 1 à 5 atomes de carbone, ou
• hétéroaryle ou alkylhétéroarylalkyle, le groupe hétéroaryle étant éventuellement substitué par un ou plusieurs radicaux alcoxy de 1 à 5 atomes de carbone,
n représente un nombre entier de 1 à 7
m et m' sont identiques ou différents et représentent un nombre entier de 0 à 8
p représente un nombre entier de 0 à 11
r représente un nombre entier de 1 à 11
q représente un nombre entier de 0 à 5
s représente un nombre entier de 0 à 5
t représente un nombre entier de 0 à 5
x représente un nombre entier de 1 à 5
m, m', n, p, r, s, t, x étant tels que le nombre de chaînons du groupe de liaison L n'excède pas 25,
R₁' et R₁" identiques ou différents représentent un atome d'hydrogène, un radical alkyle linéaire ou ramifié de 1 à 5 atomes de carbone,
R₂' et R₂" identiques ou différents représentent un atome d'hydrogène ou un radical alkyle linéaire ou ramifié de 1 à 5 atomes de carbone ou un radical benzyle ou un groupe sulfate,
R₁' et R₁" ainsi que R₂' et R₂" pouvant être éventuellement liés pour former un cycle,
R₃' et R₄' identiques ou différents représentent chacun un atome d'hydrogène, un radical alkyle linéaire ou ramifié de 1 à 5 atomes de carbone ou un radical benzyle, ou un groupe sulfate,
R₅' représente un radical alkyle linéaire ou ramifié de 1 à 5 atomes de carbone.
à l'état de base ou sel d'addition à un acide ou à une base, ainsi qu'à l'état d'hydrate ou de solvat.

2. Composés selon la revendication 1 comprenant l'unité monomère de formule M dans laquelle:
X = C-R₂
R sur les positions 6, 7 ou 8 de l'indolizine représente un atome d'hydrogène, un atome d'halogène, un radical hydroxy ou un radical de formule:
• -COOR₅
• -CO-NR₆R₇
• -O-Alk
• -O-Alk-CO₂R₅
• -O-Alk-NR₆R₇
• -NP₆R₇
• -NH-SO₂-Alk
• NH-CO-Alk, ou
• -NH-CO₂-Alk
dans lesquels:
• R₅ représente un atome d'hydrogène, un radical alkyle linéaire ou ramifié de 1 à 5 atomes de carbone ou un radical benzyle;
• R₆ et R₇ identiques ou différents représentent chacun un atome d'hydrogène, un radical alkyle linéaire ou ramifié de 1 à 5 atomes de carbone ou un radical benzyle,
• Alk représente un radical alkyle linéaire ou ramifié de 1 à 5 atomes de carbone ou un radical alkylène linéaire ou ramifié de 1 à 5 atomes de carbone ;
• Ph représente un radical phényle éventuellement substitué par un ou plusieurs atomes d'halogène, par un ou plusieurs radicaux hydroxy, par un ou plusieurs radicaux alcoxy linéaire ou ramifié de 1 à 5 atomes de carbone, ou par un ou plusieurs radicaux -COOR₅;
R₁ représente un atome d'hydrogène, un atome d'halogène, un radical hydroxy, un radical cyano ou un radical de formule :
• -CO₂R₅
• -CO-NR₆R₇
• CO-NH-Alk-CO₂R₅
• -CO-NH-Ph
• -O-Alk
• -O-Alk-CO₂R₅
• -O-Alk-CO-NR₆R₇
• -O-Alk-NR₆R₇
• -O-Alk-OR₅
• -O-Alk-Ph
• -O-Ph
• -NR₆R₇
• -NH-SO₂-Alk
• -NH-CO-Alk
• -NH-CO-Alk-CO₂R₅
• -NH-CO-Alk-CO-NR₆R₇
• -NH-CO₂-Alk
• -NH-CO-Ph, ou
• un radical aryle ou hétéroaryle de 5 ou 6 atomes choisis parmi C, N, 0, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, ou par un ou plusieurs radicaux -CO₂R₅ ou -CO-NR₆R₇ dans lesquels Alk, Ph, R₅, R₆ et R₇ sont tels que définis au niveau du groupe R;
R₂ représente un radical alkyle linéaire ou ramifié de 1 à 5 atomes de carbone, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone ;
R₃ et R₄ identiques ou différents représentent chacun un atome d'hydrogène, un radical hydroxy, un radical alcoxy de 1 à 5 atomes de carbone, un radical amino, un radical nitro, un radical de formule
• -NR₆R₇
• -NH-CO-Alk
• -NH-SO₂-Alk
• -CO₂R₅
• -CO-NR₆R₇, ou
• -CO-NHOH
dans lesquels Alk, Ph, R₅, R₆ et R₇ sont tels que définis au niveau du groupe R;
à l'état de base ou sel d'addition à un acide ou à une base, ainsi qu'à l'état d'hydrate ou de solvat.

3. Composés selon l'une quelconque des revendications 1 ou 2, comprenant l'unité monomère de formule M dans laquelle :
X = C-R₂
R sur les positions 6, 7 ou 8 de l'indolizine représente un atome d'hydrogène, un radical hydroxy, un radical carboxy ou un radical de formule :
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk, ou
• -NH-CO-Alk
dans lesquels:
• R₅ représente un atome d'hydrogène, un radical alkyle de 1 à 5 atomes de carbone ou un radical benzyle ;
• R₆ et R₇ identiques ou différents représentent chacun un atome d'hydrogène, un radical alkyle linéaire ou ramifié de 1 à 5 atomes de carbone ou un radical benzyle,
• Alk représente un radical alkyle ou un radical alkylène linéaire ou ramifié de 1 à 5 atomes de carbone ;
• Ph représente un radical phényle éventuellement substitué par un ou plusieurs atomes d'halogène, par un ou plusieurs radicaux hydroxy, par un ou plusieurs radicaux alcoxy de 1 à 5 atomes de carbone, ou par un ou plusieurs radicaux -COOR₆;
R₁ représente un atome d'halogène, un radical hydroxy, un radical carboxy ou un radical de formule :
• -O-Alk
• -O-Alk-CO₂R₅
• -O-Alk-Ph
• -O-Ph
• -NR₆R₇
• -NH-CO-Ph, ou
• un radical aryle ou hétéroaryle à 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, par un ou plusieurs radicaux -CO₂R₅ ou -CO-NR₆R₇ dans lesquels Alk, Ph, R₅, R₆ et R₇ sont tels que définis au niveau du groupe R;
R₂ représente un radical alkyle de 1 à 5 atomes de carbone ou un radical cycloalkyle de 3 à 6 atomes de carbone;
R₃ et R₄ identiques ou différents représentent chacun un radical alcoxy linéaire ou ramifié de 1 à 5 atomes de carbone, un radical amino, un radical carboxy, un radical hydroxy, un radical de formule CO-NR₆R₇, ou un radical de formule -NH-SO₂-Alk dans lesquels Alk, R₆ et R₇ sont tels que définis au niveau du groupe R ;
à l'état de base ou sel d'addition à un acide ou à une base, ainsi qu'à l'état d'hydrate ou de solvat.

4. Composés selon la revendication 3 **caractérisés en ce que**,
R sur les positions 6 ou 8 de l'indolizine représente un atome d'hydrogène ou un radical hydroxy ;
R₁ représente un radical hydroxy, ou un radical de formule :
• -O-Alk
• -O-Alk-Ph
• -NR₆R₇, ou
• -NH-CO-Ph
dans lesquels Alk, Ph, R₆ et R₇ sont tels que définis dans la revendication 6.
R₂ représente un radical alkyle de 1 à 5 atomes de carbone ;
R₃ représente un radical alcoxy linéaire ou ramifié de 1 à 5 atomes de carbone ou un radical carboxy ;
R₄ représente un radical amino ;
à l'état de base ou sel d'addition à un acide ou à une base, ainsi qu'à l'état d'hydrate ou de solvat.

5. Composés selon la revendication 1, comprenant l'unité monomère de formule M dans laquelle :
X=N;
R sur les positions 5, 6, 7 ou 8 de l'imidazo[1,5-*a*]pyridine représente un atome d'hydrogène, un atome d'halogène, un radical alkyle linéaire ou ramifié de 1 à 5 atomes de carbone, un radical hydroxy ou un radical de formule:
• -CO₂R₅
• -CO-NR₆R₇
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk
• -NH-CO-Alk, ou
• -NH-CO₂-Alk
dans lesquels:
• R₅ représente un atome d'hydrogène, un radical alkyle linéaire ou ramifié de 1 à 5 atomes de carbone ou un radical benzyle;
• R₆ et R₇ identiques ou différents représentent chacun un atome d'hydrogène, un radical alkyle linéaire ou ramifié de 1 à 5 atomes de carbone ou un radical benzyle,
• Alk représente un radical alkyle linéaire ou ramifié de 1 à 5 atomes de carbone ou un radical alkylène linéaire ou ramifié de 1 à 5 atomes de carbone ;
• Ph représente un radical phényle éventuellement substitué par un ou plusieurs atomes d'halogène, par un ou plusieurs radicaux hydroxy, par un ou plusieurs radicaux alcoxy linéaires ou ramifiés de 1 à 5 atomes de carbone, ou par un ou plusieurs radicaux -COOR₅;
R₁ représente un atome d'hydrogène, un atome d'halogène, un radical cyano ou un radical de formule :
• -CO₂R₅
• -CO-NH-Ph
• -NR₆R₇
• -NH-SO₂-Alk
• -NH-CO-Alk
• -NH-CO₂-Alk
• -NH-CO-Ph, ou
• un radical aryle ou hétéroaryle à 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaires ou ramifiés de 1 à 5 atomes de carbone, par un ou plusieurs radicaux -CO₂R₅ ou -CO-NR₆R₇ dans lesquels Alk, Ph, R₅, R₆ et R₇ sont tels que définis au niveau du groupe R;
R₃ et R₄ identiques ou différents représentent chacun un atome d'hydrogène, un radical hydroxy, un radical alcoxy linéaire ou ramifié de 1 à 5 atomes de carbone, un radical amino, un radical nitro, ou un radical de formule
• -NR₆R₇
• -NH-CO-Alk
• -NH-SO₂-Alk
• -CO₂R₅
• -CO-NR₆R₇, ou
• -CO-NHOH
dans lesquels Alk, Ph, R₅, R₆ et R₇ sont tels que définis au niveau du groupe R;
ou R3 et R4 forment ensemble, avec les atomes de carbone du noyau phényle auquel ils sont rattachés, un cycle carboné à 6 chaînons, comportant un atome d'azote et un autre hétéroatome tel que l'oxygène ;
à l'état de base ou sel d'addition à un acide ou à une base, ainsi qu'à l'état d'hydrate ou de solvat.

6. Composés selon la revendication 5, comprenant l'unité monomère de formule M dans laquelle :
X=N;
R sur les positions 6, 7 ou 8 de l'imidazo[1,5-*a*]pyridine représente un atome d'hydrogène, un atome d'halogène, un radical hydroxy, un radical carboxy ou un radical de formule :
• -CO-NR₆R₇
• -O-Alk
• -O-Alk-CO₂R₅
• -O-Alk-Ph
• -NR₆R₇
• -NH-SO₂-Alk, ou
• -NH-CO-Alk
dans lesquels :
• R₅ représente un atome d'hydrogène; un radical alkyle linéaire ou ramifié de 1 à 5 atomes de carbone ou un radical benzyle ;
• R₆ et R₇ identiques ou différents représentent chacun un atome d'hydrogène, un radical alkyle linéaire ou ramifié de 1 à 5 atomes de carbone ou un radical benzyle,
• Alk représente un radical alkyle linéaire ou ramifié de 1 à 5 atomes de
• carbone ou un radical alkylène linéaire ou ramifié de 1 à 5 atomes de carbone ;
• Ph représente un radical phényle éventuellement substitué par un ou plusieurs atomes d'halogène, par un ou plusieurs radicaux hydroxy, par un ou plusieurs radicaux alcoxy linéaire ou ramifié de 1 à 5 atomes de carbone, ou par un ou plusieurs radicaux -COQR₅;
R₁ représente un atome d'hydrogène, un atome d'halogène, un radical carboxy ou un radical de formule :
• -NR₆R₇
• -NH-SO₂-Alk
• -NH-CO-Alk
• -NH-CO-Ph, ou
• un radical aryle ou hétéroaryle à 5 ou 6 atomes choisis parmi C, N, O, S, éventuellement substitué par un ou plusieurs atomes d'halogène, par un ou plusieurs radicaux alkyles linéaires ou ramifiés de 1 à 5 atomes de carbone, par un ou plusieurs radicaux alcoxy linéaire ou ramifié de 1 à 5 atomes de carbone, ou par un ou plusieurs radicaux -CO₂R₅ ou -CO-NR₆R₇ dans lesquels Alk, Ph, R₅, R₆ et R₇ sont tels que définis au niveau du groupe R;
R₃ et R₄ identiques ou différents représentent chacun un radical alcoxy linéaire ou ramifié de 1 à 5 atomes de carbone, un radical amino, un radical carboxy, un radical hydroxy, ou un radical de formule CO-NR₆R₇ ou -NH-SO₂-Alk à l'état de base ou sel d'addition à un acide ou à une base, ainsi qu'à l'état d'hydrate ou de solvat.

7. Composés selon la revendication 6 **caractérisés en ce que**,
R sur la position 8 de l'imidazo[1,5-*a*]pyridine représente un atome d'hydrogène, un radical hydroxy, ou un radical carboxy,
R₁ représente un atome d'hydrogène, un radical de formule -NH-CO-Ph, ou un radical aryle ou hétéroaryle à 5 ou 6 atomes choisis parmi C, N, O, S, éventuellement substitué par un ou plusieurs radicaux -CO₂R₅,
dans lesquels Alk, Ph, R₅ sont tels que définis dans la revendication 6;
R₃ représente un radical alcoxy linéaire ou ramifié de 1 à 5 atomes de carbone ou un radical carboxy ;
R₄ représente un radical amino ;
à l'état de base ou sel d'addition à un acide ou à une base, ainsi qu'à l'état d'hydrate ou de solvat.

8. Composés agonistes des récepteurs FGF selon la revendication 1 **caractérisés en ce que** M₁ est une unité monomère de formule générale M telle que définie dans l'une des revendications 1 à 4 et M₂ est une unité monomère de formule M telle que définie dans l'une des revendications 5 à 7;
à l'état de base ou sel d'addition à un acide ou à une base, ainsi qu'à l'état d'hydrate ou de solvat.

9. Composés selon l'une quelconque des revendications 1 à 8 **caractérisés en ce que**:
L relie les 2 unités monomères M₁ et M₂ par le radical R₁ ou ;
L relie les 2 unités monomères M₁ et M₂ par le radical R₂ ou ;
L relie les 2 unités monomères M₁ et M₂ par le radical R en sa position 8 ou ;
L relie les 2 unités monomères M₁ et M₂ par le radical R en sa position 7 ou ;
L relie les 2 unités monomères M₁ et M₂ par le radical R en sa position 6 ou ;
L relie les 2 unités monomères M₁ et M₂ d'une part par le radical R en sa position 8 et d'autre part par le radical R en sa position 7 ou 6, ou;
L relie les 2 unités monomères M₁ et M₂ d'une part par le radical R en sa position 7 et d'autre part par le radical R en sa position 6 ou;
L relie les 2 unités monomères M₁ et M₂ d'une part par le radical R₂ et d'autre part par le radical R₁ ou;
L relie les 2 unités monomères M₁ et M₂ d'une part par le radical R₁ et d'autre part par le radical R en sa position 8 ;
à l'état de base ou sel d'addition à un acide ou à une base, ainsi qu'à l'état d'hydrate ou de solvant.

10. Composition pharmaceutique contenant en tant que principe actif un composé répondant à la formule M₁-L-M₂, selon l'une quelconque des revendications 1 à 9 éventuellement en association avec un ou plusieurs excipients inertes et appropriés.

11. Composition pharmaceutique selon la revendication 10 utile dans le traitement de l'ischémie cardiaque, le traitement des maladies associées à un rétrécissement ou à une obstruction des artères ou des artérites, le traitement des angines de poitrine, le traitement de la thromboangéite oblitérante, le traitement de l'athérosclérose, le traitement de l'inhibition de la resténose après angioplastie ou endoartérectomie, le traitement de la cicatrisation, le traitement pour la régénération musculaire, le traitement pour la survie des myoblastes, le traitement de la nociception et le traitement de la douleur chronique, le traitement de la neuropathie périphérique, le traitement pour l'amélioration de la survie du greffon de pancréas bioartificiel chez le patient diabétique, le traitement de la diminution du cholesterol associée à une diminution de l'adiposité, le traitement de l'amélioration de la la revascularisation des greffons et de la survie des greffes, le traitement de la dégénérescence rétinienne, le traitement de la rétinite pigmentaire, le traitement de l'ostéoarthrite, le traitement de la pré-éclampsie, le traitement des lésions vasculaire et du syndrome de détresse respiratoire aiguë, le traitement de la protection osseuse, ou le traitement de la protection des follicules pileux.

12. Composé selon l'une quelconque des revendications 1 à 9 pour son utilisation dans le traitement des maladies nécessitant une activation des récepteurs FGFs.

13. Composé selon l'une quelconque des revendications 1 à 9 pour son utilisation dans le traitement de l'ischémie cardiaque, le traitement des maladies associées à un rétrécissement ou à une obstruction des artères ou des artérites, le traitement des angines de poitrine, le traitement de la thromboangéite oblitérante, le traitement de l'athérosclérose, le traitement de l'inhibition de la resténose après angioplastie ou endoartérectomie, le traitement de la cicatrisation, le traitement pour la régénération musculaire, le traitement pour la survie des myoblastes, le traitement de la nociception et le traitement de la douleur chronique, le traitement de la neuropathie périphérique, le traitement pour l'amélioration de la survie du greffon de pancréas bioartificiel chez le patient diabétique, le traitement de la diminution du cholesterol associée à une diminution de l'adiposité, le traitement de l'amélioration de la la revascularisation des greffons et de la survie des greffes, le traitement de la dégénérescence rétinienne, le traitement de la rétinite pigmentaire, le traitement de l'ostéoarthrite, le traitement de la pré-éclampsie, le traitement des lésions vasculaire et du syndrome de détresse respiratoire aiguë, le traitement de la protection osseuse, ou le traitement de la protection des follicules pileux.

14. Procédé de préparation d'un composé de formule M₁-L-M₂ selon l'une quelconque des revendications 1 à 9, comprenant la réaction d'au moins une unité monomère de formule M-W avec un réactif de formule U-L-U', avec
M et L étant tels que définis dans les revendicatians 1 à 9
U et U' étant identiques ou différents
W et U ainsi que W et U' représentent chacun un groupe fonctionnel capable de réagir l'un sur l'autre pour former, une liaison covalente de type C-C, C-O, C-N, C-C ou C-S, W étant situé sur l'un des substituants R, R₁ ou R₂ tels que définis dans les revendications 1 à 8.

15. Procédé de préparation selon la revendication 14, **caractérisé en ce que** W et U ainsi que W et U' représentent un groupe amino, hydroxyle, carboxy, amido, carbamate, halogène, chlorure de sulfonyle, chlorure d'acide, fluorure d'acide, un ester boronique ou un acide boronique.

16. Procédé de préparation selon la revendication 15, comprenant la réaction desdites unités monomères de formule M-W, avec R, R₁, R₂, R₃ ou R₄ représentant ou posédant un acide carboxylique, et R ou R₁ représentant ou possédant un groupe amino, avec un agent de silylation et une base faible, puis une réaction d'acylation en utilisant un agent diacylant et une base faible, puis hydrolyse en milieu acide.

17. Composé de formule M₁-L-M₂ comme défini dans l'une quelconque des revendications 1 à 9, dont les noms suivent :
- Sel disodique du 3,3'-{3,6,9,12,15-pentaoxaheptadécane-1,17-diylbis[oxy(1-méthoxy-2-méthylindolizine-8,3-diyl)carbonyl]}bis(acide 6-aminobenzoïque)
- Sel disodique du diacide 3,3'-(3,6,9,12,15-pentaoxaheptadécane-1,17-diylbis{oxy[3-(4-amino-3-méthoxybenzoyl)imidazo[1,5-*a*]pyridine-8,1-diyl]})benzoïque
- Sel disodique du 3,3'-{3,6,9,12,15,18-hexaoxaicosane-1,20-diylbis[oxy(1-méthoxy-2-méthylindofizine-6,3-diyl)carbonyl]}bis(acide 6-aminobenzoïque)
- Sel disodique du 3,3'-{3,6,9,12,15-pentaoxaheptadécane-1,17-diylbis[oxy(1-methoxy-2-méthylindolizine-6,3-diyl)carbonyl]}bis(acide 6-aminobenzoïque)
- Sel disodique du 3,3'-{3,6,9,12,15,18-hexaoxaicosane-1,20-diylbis[oxy(2-méthylindolizine-1,3-diyl)carbonyl]}bis(acide 6-aminobenzoïque)
- Sel disodique du 3,3'-{éthane-1,2-diylbis[oxyéthane-2,1-diyloxy-3,1-phénylèneméthylèneoxy(2-méthylindolizine-1,3-diyl)carbonyl]}bis(acide 6-aminobenzoïque)
- Sel disodique de 3,3'-{octane-1,8-diylbis[oxy-3,1-phénylènecarbonylimino(2-méthylindolizine-1,3-diyl)carbonyl]}bis(acide 6-aminobenzoïque)
- Sel disodique du 3,3'-{éthane-1,2-diylbis[oxyéthane-2,1-diyloxy-3,1-phénylènecarbonylimino(2-méthylindolizine-1,3-diyl)carbonyl]}bis(acide 6-aminobenzoïque)
- Sel disodique de 3,3'-{(1,4-dioxobutane-1,4-diyl)bis[iminoéthane-2,1-diyloxy-3,1-phénylènecarbonylimino(2-méthylindolizine-1,3-diyl)carbonyl]}bis(acide 6-aminobenzoïque)
- Sel disodique de 3,3'-{carbonyl bis[iminoéthane-2,1-diyloxy-3,1-phenylènecarbonylimino(2-méthylindolizine-1,3-diyl)carbonyl]}bis(acide 6-aminobenzoïque)
- Sel disodique du 3,3'-{éthane-1,2-diylbis[imino(2-oxoéthane-2,1-diyl)oxy-3,1-phénylènecarbonylimino(2-méthylindolizine-1,3-diyl)carbonyl]}bis(acide 6-aminobenzoïque)
- Sel disodique du 3,3'-{propane-1,3-diylbis[imino(2-oxoéthane-2,1-diyl)oxy-3,1-phénylènecarbonylimino(2-méthylindolizine-1,3-diyl)carbonyl]}bis(acide 6-aminobenzoïque)
- Sel disodique du 3,3'-{butane-1,4-diylbis[imino(2-oxoéthane-2,1-diyl)oxy-3,1-phénylènecarbonylimino(2-méthylindolizine-1,3-diyl)carbonyl]}bis(acide 6-aminobenzoïque)
- Sel disodique du 3,3'-{éthane-1,2-diylbis[imino(2-oxoéthane-2,1-diyl)oxy-4,1-phenylènecarbonylimino(2-méthylindolizine-1,3-diyl)carbonyl]}bis(acide 6-aminobenzoïque)
- Sei disodique du 3,3'-{éthane-1,2-diylbis[oxyéthane-2,1-diyloxy-4,1-phénylènecarbonylimino(2-méthylindolizine-1,3-diyl)carbonyl]}bis(acide 6-aminobenzoïque)
- Sel disodique du 3,3'-{oxybis[éthané-2, 1-diyloxyéthane-2,1-diyloxy-4,1-phényléne(2-méthylindolizine-1,3-diyl)carbonyl]}bis(acide 6-aminobenzoïque)
- Sel disodique du 3,3'-{éthane-1,2-diylbis[oxyéthane-2,1-diyloxy-3,1-phénylène(2-méthylindolizine-1,3-diyl)carbonyl]}bis(acide 6-aminobenzoïque)
- Sel disodique du 3,3'-{oxybis[éthane-2,1-diyloxyéthane-2,1-diyloxy-3,1-phénylène(2-méthylindolizine-1,3-diyl)carbonyl]}bis(acide 6-aminobenzoïque)
- Sel disodique du 3,3'-{hexane-1,6-diylbis[imino(2-oxoéthane-2,1-diyl)oxy-3,1-phénylène{2-méthylindolizine-1,3-diyl)carbonyl]}bis(acide 6-aminobenzoïque)
- Sel disodique du 3,3'-{hexane-1,6-diylbis[imino(2-oxoéthane-2,1-diyl)oxy-4,1-phényène(2-méthylindolizine-1,3-diyl)carbonyl]}bis(acide 6-aminobenzoïque)
- Sel disodique du diacide 2-amino-5-[(1-{[3-(2-{[2-({[3-({[3-(4-amino-3-carboxybenzoyl)imidazo[1,5-*a*]pyridin-1-yl]amino}carbonyl)phénoxy] acétyl}amino)éthyl]amino}-2-oxoéthoxy)benzoyl]amino}-2-méthylindolizin-3-yl)carbonyl]benzoïque

## Claims

1. FGF receptor agonist compounds corresponding to the formula:
M₁-L-M₂
in which M₁ and M₂, which may be identical or different, are each, independently of one another, a monomer unit M, and L is a linker group that links M₁ and M₂ covalently, **characterized in that** said monomer unit corresponds to the formula M which follows: 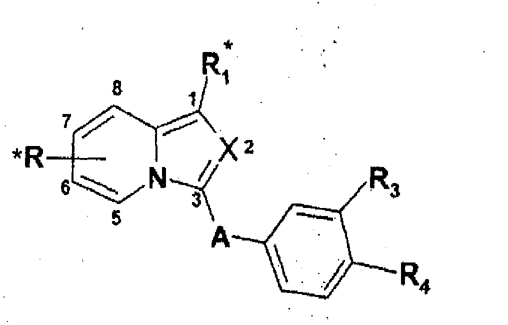 in which,
X is N or C-R₂*,
A is a -CO- radical,
* indicates the linkage site of L with, firstly, the monomer unit M₁ and, secondly, with the monomer unit M₂;
said linkage site of each monomer unit M₁ or M₂ being located on one of the substituents. R, R₁ or R₂;
R is a hydrogen atom, a halogen atom, a linear or branched alkyl radical containing from 1 to 5 carbon atoms, a hydroxyl radical, or a radical of formula:
• -CO₂R₅
• -CO-NR₆R₇
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk
• -NH-CO-Alk or
• -NH-CO₂-Alk
in which:
• R₅ is a hydrogen atom, a linear or branched alkyl radical containing from 1 to 5 carbon atoms or a benzyl radical;
• R₆ and R₇, which may be identical or different, are each a hydrogen atom, a linear or branched alkyl radical containing from 1 to 5 carbon atoms or a benzyl radical;
• Alk is a linear or branched alkyl radical having from 1 to 5 carbon atoms or a linear or branched alkylene radical having from 1 to 5 carbon atoms;
• Ph is a phenyl radical optionally substituted with one or more halogen atoms, with one or more hydroxyl radicals, with one or more linear or branched alkoxy radicals containing from 1 to 5 carbon atoms, or with one or more -COOR₅ radicals;
R₁ is a hydrogen atom, a halogen atom, a hydroxyl radical, a cyano radical, a linear or branched alkyl radical containing from 1 to 5 carbon atoms or a radical of formula:
• -CO₂R₅
• -CO-NR₆R₇
• -CO-NH-Alk-CO₂R₅
• -CO-NH-Ph
• -O-Alk
• -O-Alk-CO₂R₅
• -O-Alk-CO-NR₆R₇
• -O-Alk-NR₆R₇
• -O-Alk-OR₅
• -O-Alk-Ph
• -O-Ph
• -NR₆R₇
• -NH-SO₂-Alk
• -NH-CO-Alk
• -NH-CO-Alk-CO₂R₅
• -NH-CO-Alk-CO-NR₆R₇
• -NH-CO₂-Alk
• -NH-CO-Ph, or
• an aryl or heteroaryl radical containing 5 or 6 atoms selected from C, N, O and S, optionally substituted with one or more halogen atoms, with one or more linear or branched alkyl radicals containing from 1 to 5 carbon atoms, with one or more linear or branched alkoxy radicals containing from 1 to 5 carbon atoms, or with one or more -CO₂R₅ or -CO-NR₆R₇ radicals in which Alk, Ph, R₅, R₆ and R₇ are as defined with respect to the group R;
R₂ is a linear or branched alkyl radical containing from 1 to 5 carbon atoms, a cycloalkyl radical containing from 3 to 6 carbon atoms or a phenyl radical optionally substituted with one or more halogen atoms, with one or more carboxyl radicals, with one or more linear or branched alkoxy radicals containing from 1 to 5 carbon atoms, with one or more hydroxyl radicals, with one or more benzyloxy radicals, or with one or more alkoxycarbonyl radicals containing from 2 to 6 carbon atom;
R₃ and R₄ are, independently of one another, a hydrogen atom, a hydroxyl radical, an amino radical, a nitro radical or a radical of formula:
• -CO₂R₅
• -CO-NR₆R₇
• -CO-NHOH
• -O-Alk
• -O-Alk-NR₆R₇
• -O-Alk-CO-NR₆R₇
• -NHOH
• -NR₆R₇
• -N(R₈)-CO-Alk
• -N(R₈)-CO-CF₃
• -N(R₈)-CO-Ph
• -N(R₈)-CO₂-Alk
• -N(R₆)-SO₂-Alk
• -N(R₈)-CO-Alk-NR₆R₇
• -N(R₈)-SO₂-Alk-NR₆R₇, or
• -NH-Alk-R₆R₇
in which R₈ is a hydrogen atom or an -Alk-COOR₅ radical, and Alk, R₅, R₆ and R₇ are as defined with
respect to the group R;
or else R₃ and R₄ form, together with the carbon atoms of the phenyl ring to which they are attached, a six-membered ring containing a nitrogen atom and another heteroatom such as oxygen; and
L is:
a linear or branched alkylene radical containing from 2 to 25 carbon atoms, a linear or branched alkenyl radical containing from 2 to 25 carbon atoms, or a liner or branched alkynyl group containing from 2 to 25 carbon atoms;
or a radical chosen from the formulae: 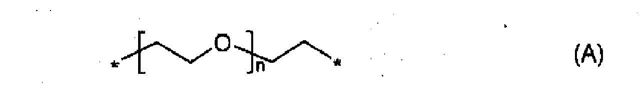 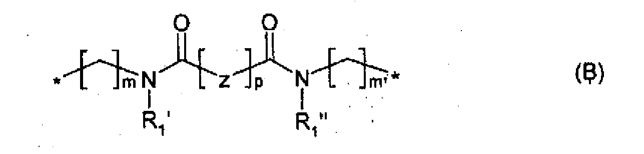 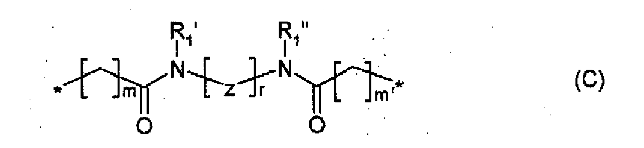 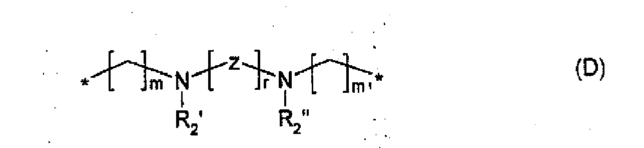 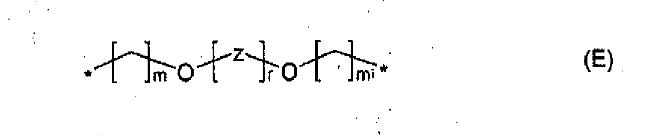 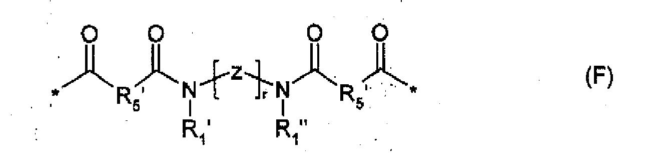 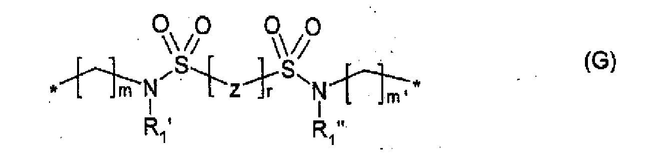 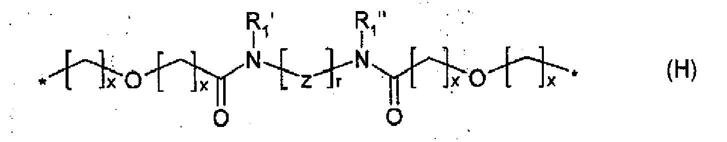 in which
* indicates the atom for connection of L with the monomer unit M on one of the substituents R, R₁ or R₂;
Z is a bond or a carbonyl radical or a linear, branched or cyclic alkylene radical containing from 1 to 6 carbon atoms, optionally substituted with 1 or 2 carbonyl radicals, or else a radical
■ [CH₂]ₛ-[-CH-(CH₂)_{q}-OR₃']-[CH₂]ₛ-
■ - [CH₂]ₛ-[-CH-(CH₂)_{q}-NR₃'R₄']-[CH₂] ₛ-
■ -[CH₂-CH₂-O]ₜ-CH₂-CH₂-
■ phenyl or alkylphenylalkyl, the phenyl group being optionally substituted with one or more alkoxy radicals containing from 1 to 5 carbon atoms, or
■ heteroaryl or alkylheteroarylalkyl, the heteroaryl group being optionally substituted with one or more alkoxy radicals containing from 1 to 5 carbon atoms,
n is an integer from 1 to 7,
m and m' are identical or different and are an integer from 0 to 8,
p is an integer from 0 to 11,
r is an integer from 1 to 11,
q is an integer from 0 to 5,
s is an integer from 0 to 5,
t is an integer from 0 to 5,
x is an integer from 1 to 5,
m, m', n, p, r, s, t and x being such that the number of links of the linker group L does not exceed 25,
R₁' and R₁" , which may be identical or different, are a hydrogen atom, or a linear or branched alkyl radical containing from 1 to 5 carbon atoms,
R₂' and R₂", which may be identical or different, are a hydrogen atom, a linear or branched alkyl radical containing from 1 to 5 carbon atoms, a benzyl radical or a sulphate group,
it being possible for R₁' and. R₁" and also R₂' and R₂" to be optionally linked so as to form a ring,
R₃' and R₄', which may be identical or different, are each a hydrogen atom, a linear or branched alkyl radical containing from 1 to 5 carbon atoms, a benzyl radical or a sulphate group;
R₅' is a linear or branched alkyl radical containing from 1 to 5 carbon atoms,
in the form of a base or an addition salt with an acid or with a base, and also in the form of a hydrate or of a solvate.

2. Compounds according to Claim 1, comprising the monomer unit of formula M in which:
X = C-R₂;
R on the 6-, 7- or 8-position of the indolizine is a hydrogen atom, a halogen atom, a hydroxyl radical or a radicals of formula:
• -COOR₅
• -CO-NR₆R₇
• -O-Alk
• -O-Alk-CO₂R₅
• -O-Alk-NR₆R₇
• -NR₆R₇
• -NH-SO₂-Alk
• -NH-CO-Alk, or
• -NH-CO₂-Alk
in which:
■ R₅ is a hydrogen atom, a linear or branched alkyl radical containing from 1 to 5 carbon atoms or a benzyl radical;
■ R₆ and R₇, which may be identical or different, are each a hydrogen atom, a linear or branched alkyl radical containing from 1 to 5 carbon atoms or a benzyl radical,
■ Alk is a linear or branched alkyl radical containing from 1 to 5 carbon atoms or a linear or branched alkylene radical containing from 1 to 5 carbon atoms;
■ Ph is a phenyl radical optionally substituted with one or more halogen atoms, with one or more hydroxyl radicals, with one or more linear or branched alkoxy radicals containing from 1 to 5 carbon atoms, or with one or more -COOR₅ radicals;
R₁ is a hydrogen atom, a halogen atom, a hydroxyl radical, a cyano radical or a radical of formula:
• -CO₂R₅
• -CO-NR₆R₇
• -CO-NH-Alk-CO₂R₅
• -CO-NH-Ph
• -O-Alk
• -O-Alk-CO₂R₅
• -O-Alk-CO-NR₆R₇
• -O-Al-NR₆R₇
• -O-Alk-OR₅
• -O-Alk-Ph
• -O-Ph
• -NR₆R₇
• -NH-SO₂-Alk
• -NH-CO-Alk
• -NH-CO-Alk-CO₂R₅
• -NH-CO-Alk-CO-NR₆R₇
• -NH-CO₂-Alk
• -NH-CO-Ph, or
■ an aryl or heteroaryl radical containing 5 or 6 atoms selected from C, N, O and S, optionally substituted with one or more halogen atoms, with one or more linear or branched alkyl radicals containing from 1 to 5 carbon atoms, with one or more linear or branched alkoxy radicals containing from 1 to 5 carbon atoms, or with one or more -CO₂R₅ or -CO-NR₆R₇ radicals in which Alk, Ph, R₅, R₆ and R₇ are as defined with respect to the group R;
R₂ is a linear or branched alkyl radical containing from 1 to 5 carbon atoms, a cycloalkyl radical containing from 3 to 6 carbon atoms or a phenyl radical optionally substituted with one or more halogen atoms, with one or more alkoxy radicals containing from 1 to 5 carbon atoms, with one or more carboxyl radicals or with one or more alkoxycarbonyl radicals containing from 2 to 6 carbon atoms;
R₃ and R₄, which may be identical or different are each a hydrogen atom, a hydroxyl radical, an alkoxy radical containing from 1 to 5 carbon atoms, an amino radical, a nitro radical, or a radical of formula:
• -NR₆R₇
• -NH-CO-Alk
• -NH-SO₂-Alk
• -CO₂R₅
• -CO-NR₆R₇, or
• -CO-NHOH
in which Alk, Ph, R₅, R₆ and R₇ are as defined with respect to the group R;
in the form of a base or of an addition salt with an acid or with a base, and also in the form of a hydrate or of a solvate.

3. Compounds according to either of Claims 1 and 2, comprising the monomer unit of formula M in which:
X =C-R₂ ;
R on the 6-, 7- or 8-position of the indolizine is a hydrogen atom, a hydroxyl radical, a carboxyl radical or a radical of formula:
• -O-Alk
• -O-Alk-CO₂R₅
• -O-Alk-CO-NR₅R₇
• -O-Alk-NR₆R₇
• -O-Alk-Ph
• -NR₆R₇
• -NH-SO₂-Alk, or
• -NH-CO-Alk
in which:
■ R₅ is a hydrogen atom, an alkyl radical containing from 1 to 5 carbon atoms or a benzyl radical;
■ R₆ and R₇, which may be identical or different, are each a hydrogen atom, a linear or branched alkyl radical containing from 1 to 5 carbon atoms or a benzyl radical;
■ Alk is a linear or branched alkyl radical or alkylene radical containing from 1 to 5 carbon atoms;
■ Ph is a phenyl radical optionally substituted with one or more halogen atoms, with one or more hydroxyl radicals, with one or more alkoxy radicals containing from 1 to 5 carbon atoms, or with one or more -COOR₅ radicals;
R₁ is a halogen atom, a hydroxyl radical, a carboxyl radical or a radical of formula:
• -O-Alk
• -O-Alk-CO₂R₅
• -O-Alk-Ph
• -O-Ph
• -NR₆R₇
• -NH-CO-Ph, or
• an aryl or heteroaryl radical containing 5 or 6 atoms selected from C, N, O and S, optionally substituted with one or more halogen atoms, with one or more linear or branched alkyl radicals containing from 1 to 5 carbon atoms, with one or more linear or branched alkoxy radicals containing from 1 to 5 carbon atoms, or with one or more -CO₂R₅ or -CO-NR₆R₇ radicals
in which Alk, Ph, R₅, R₆ and R₇ are as defined with respect to the group R;
R₂ is an alkyl radical containing from 1 to 5 carbon atoms or a cycloalkyl radical containing from 3 to 6 carbon atoms;
R₃ and R₄, which may be identical or different, are each a linear or branched alkoxy radical containing from 1 to 5 carbon atoms, an amino radical, a carboxyl radical, a hydroxyl radical, a radical of formula CO-NR₆R₇, or a radical of formula -NH-SO₂-Alk,
in which Alk, R₆ and R₇ are as defined with respect to the group R;
in the form of a base or of an addition salt with an acid.or with a base, and also in the form of a hydrate or of a solvate.

4. Compounds according to Claim. 3, **characterized in that**
R on the 6- or 8-position of the indolizine is a hydrogen atom or a hydroxyl radical;
R₁ is a hydroxyl radical, or a radical of formula:
• -O-Alk
• -O-Alk-Ph
• -NR₆R₇, or
• -NH-CO-Ph
in which Alk, Ph, R₆ and R₇ are as defined in Claim 6;
R₂ is an alkyl radical containing from 1 to 5 carbon atoms;
R₃ is a linear or branched alkoxy radical containing from 1 to 5 carbon atoms or a carboxyl radical;
R₄ is an amino radical;
in the form of a base or of an addition salt with an acid or with a base, and also in the form of a hydrate or of a solvate.

5. Compounds according to Claim 1, comprising the monomer unit of formula M in which:
X = N;
R on the 5-, 6-, 7- or 8-position of the imidazo[1,5-a]pyridine is a hydrogen atom, a halogen atom, a linear or branched alkyl radical containing from 1 to 5 carbon atoms, a hydroxyl radical or a radical of formula:
• -CO₂R₅
• -CO-NR₆R₇
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk
• -NH-CO-Alk, or
• -NH-CO₂-Alk
in which:
■ R₅ is a hydrogen atom, a linear or branched alkyl radical containing from 1 to 5 carbon atoms or a benzyl radical;
■ R₆ and R₇, which may be identical or different, are each a hydrogen atom, a linear or branched alkyl radical containing from 1 to 5 carbon atoms or a benzyl radical;
**■** Alk is a linear or branched alkyl radical containing from 1 to 5 carbon atoms or a linear or branched alkylene radical containing from 1 to 5 carbon atoms;
■ Ph is a phenyl radical optionally substituted with one or more halogen atoms, with one or more hydroxyl radicals, with one or more linear or branched alkoxy radicals containing from 1 to 5 carbon atoms; or with one or more -COOR₅ radicals;
R₁ is a hydrogen atom, a halogen atom, a cyano radical or a radical of formula:
• -CO₂R₅
• -CO-NH-Ph
• -NR₆R₇
• -NH-SO₂-Alk
• -NH-CO-Alk
• -NH-CO₂-Alk
• NH-CO-Ph, or
■ an aryl or heteroaryl radical containing 5 or 6 atoms selected from C, N, O and S, optionally substituted with one or more halogen atoms, with one or more linear or branched alkyl radicals containing from 1 to 5 carbon atoms, with one or more linear or branched alkoxy radicals containing from 1 to 5 carbon atoms, or with one or more -CO₂R₅ or -CO-NR₆R₇ radicals,
in which Alk, Ph, R₅, R₆ and R₇ are as defined with respect to the group R;
R₃ and R₄, which may be identical or different, are each a hydrogen atom, a hydroxyl radical, a linear or branched alkoxy radical containing from 1 to 5 carbon atoms, an amino radical, a nitro radical, or a radical of formula:
• -NR₆R₇
• -NH-CO-Alk
• -NH-SO₂-Alk
• -CO₂R₅
• -CO-NR₆R₇, or
• -CO-NHOH
in which Alk, Ph, R₅, R₆ and R₇ are as defined with respect to the group R;
or R3 and R4 form, together with the carbon atoms of the phenyl ring to which they are attached, a 6-membered carbon-based ring containing a nitrogen atom
and another heteroatom such as oxygen; in the form of a base or of an addition salt with an acid or with a base, and also in the form of a hydrate or of a solvate.

6. Compounds according to Claim 5, comprising the monomer unit of formula M in which:
X = N;
R on the 6-, 7- or 8-position of the imidazo [1,5-a]pyridine is a hydrogen atom, a halogen atom, a hydroxyl radical, a carboxyl radical or a radical of formula:
• -CO-NR₆R₇
• -O-Alk
• -O-Alk-CO₂R₅
• -O-Alk-Ph
• -N₆R₇
• -NH-SO₂-Alk, or
• -NH-CO-Alk
in which:
■ R₅ is a hydrogen atom, a linear or branched alkyl radical containing from 1 to 5 carbon atoms or a benzyl radical;
■ R₆ and R₇, which may be identical or different, are each a hydrogen atom, a linear or branched alkyl radical containing from 1 to 5 carbon atoms or a benzyl radical;
■ Alk is a linear or branched alkyl radical containing from 1 to 5 carbon atoms or a linear or branched alkylene radical containing from 1 to 5 carbon atoms;
**■** Ph is a phenyl radical optionally substituted with one or more halogen atoms, with one or more hydroxyl radicals, with one or more linear or branched alkoxy radicals containing from 1 to 5 carbon atoms, or with one or more -COOR₅ radicals;
R₁ is a hydrogen atom, a halogen atom, a carboxy radical or a radical of formula:
• -NR₆R₇
• -NH-SO₂-Alk
• -NH-CO-Alk
• -NH-CO-Ph, or
• an aryl or heteroaryl radical containing 5 or 6 atoms selected from C, N, O and S, optionally substituted with one or more halogen atoms, with one or more linear or branched alkyl radicals containing from 1 to 5 carbon atoms, with one or more linear or branched alkoxy radicals containing from 1 to 5 carbon atoms, or with one or more -CO₂R₅ or -CO-NR₆R₇ radicals,
in which Alk, Ph, R₅, R₆ and R₇ are as defined with respect to the group R;
R₃ and R₄, which may be identical or different, are each a linear or branched alkoxy radical containing from 1 to 5 carbon atoms, an amino radical, a carboxyl radical, a hydroxyl radical, or a radical of formula CO-NR₆R₇ or -NH-SO₂-Alk;
in the form of a base or of an addition salt with an acid or with a base, and also in the form of a hydrate or of a solvate.

7. Compounds according to Claim 6, **characterized in that**
R on the 8-position of the imidazo [1,5-a] pyridine is a hydrogen atom, a hydroxyl radical or a carboxyl radical,
R₁ is a hydrogen atom, a radical of formula -NH-CO-Ph, or an aryl or heteroaryl radical containing 5 or 6
atoms chosen from C, N, O and S, optionally substituted with one or more -CO₂R₅ radicals,
in which Alk, Ph and R₅ are as defined in Claim 6;
R₃ is a linear or branched alkoxy radical containing from 1 to 5 carbon atoms or a carboxyl radical;
R₄ is an amino radical;
in the form of a base or of an addition salt with an acid or with a base, and also in the form of a hydrate or of a solvate.

8. FGF receptor agonist compounds according to Claim 1, **characterized in that** M₁ is a monomer unit of formula M as defined in one of Claims 1 to 4, and M₂ is a monomer unit of formula M as defined in one of Claims 5 to 7;
in the form of a base or of an addition salt with an acid or with a base, and also in the form of a hydrate or of a solvate.

9. Compounds according to any one of Claims 1 to 8, **characterized in that**:
L connects the 2 monomer units M₁ and M₂ by the radical R₁ or;
L connects the 2 monomer units M₁ and M₂ by the radical R2 or; L connects the 2 monomer units M₁ and M₂ by the radical R in its 8-position or;
L, connects the 2 monomer units M₁ and M₂ by the radical R in its 7-position or;
L connects the 2 monomer units M₁ and M₂ by the radical R in its 6-position or;
L connects the 2 monomer units M₁ and M₂, firstly, by the radical R in its 8-position and, secondly, by the radical R in its 7- or 6-position, or;
L connects the 2 monomer units M₁ and M₂, firstly, by the radical R in its 7-position and, secondly, by the radical R in its 6-position or;
L connects the 2 monomer units M₁ and M₂, firstly, by the radical R₂ and, secondly, by the radical R₁ or;
L connects the 2 monomer units M₁ and M₂, firstly, by the radical R₁ and, secondly, by the radical R in its 8-position;
in the form of a base or of an addition salt with an acid or with a base, and also in the form of a hydrate or of a solvate.

10. Pharmaceutical composition containing, as active ingredient, a compound corresponding to the formula M₁-L-M₂ according to any one of Claims 1 to 9, optionally in combination with one or more inert and suitable excipients.

11. Pharmaceutical composition according to Claim 10, for use in the treatment of cardiac ischaemia, the treatment of diseases associated with narrowing or an obstruction of the arteries, or arteritis, the treatment of angina pectoris, the treatment of thromboangiitis obliterans, the treatment of atherosclerosis, treatment to inhibit post-angioplasty or post-endarterectomy restenosis, wound-healing treatment, treatment for muscle regeneration, treatment for myoblast survival, the treatment of nociception and the treatment, of chronic pain, the treatment of peripheral neuropathy, treatment for improving bioartificial pancreatic graft survival in diabetic patients, treatment to bring about a decrease in cholesterol associated with a decrease in adiposity, treatment for improving graft revascularization and graft survival, the treatment of retinal degeneration, the treatment of pigmentary retinitis, the treatment of osteoarthritis, the treatment of pre-eclampsia, the treatment of vascular lesions and of acute respiratory distress syndrome, bone protection treatment, or treatment for hair follicle protection.

12. Compound according to any one of Claims 1 to 9, for its use in the treatment of diseases requiring FGF receptor activation.

13. Compound according to any one of Claims 1 to 9, for its use in the treatment of cardiac ischaemia, the treatment of diseases associated with narrowing or an obstruction of the arteries, or arteritis, the treatment of angina pectoris, the treatment of thromboangiitis obliterans, the treatment of atherosclerosis, treatment to inhibit post-angioplasty or post-endarterectomy restenosis, wound-healing treatment, treatment for muscle regeneration, treatment for myoblast survival, the treatment of nociception and the treatment of chronic pain, the treatment of peripheral neuropathy, treatment for improving bioartificial pancreatic graft survival in diabetic patients, treatment to bring about a decrease in cholesterol associated with a decrease in adiposity, treatment for improving graft revascularization and graft survival, the treatment of retinal degeneration, the treatment of pigmentary retinitis, the treatment of osteoarthritis, the treatment of pre-eclampsia, the treatment of vascular lesions and of acute respiratory distress syndrome, bone protection treatment, or treatment for hair follicle protection.

14. Process for preparing a compound of formula M₁-L-M₂ according to any one of Claims 1 to 9, comprising the reaction of at least one monomer unit of formula M-W with a reactant of formula U-L-U', with
M and L being as defined in Claims 1 to 9,
U and U' being identical or different,
W and U and also W and U' each being a functional group
capable of reacting with one another so as to form a covalent bond of C-C, C-O, C-N, C-C or C-S type,
W being located on one of the substituents R, R₁ or R₂ as defined in Claims 1 to 8.

15. Preparation process according to Claim 14, **characterized in that** W and U and also W and U' are an amino, hydroxyl, carboxyl, amido, carbamate, halogen, sulphonyl chloride, acid chloride or acid fluoride group, a boronic ester or a boronic acid.

16. Preparation process according to Claim 15, comprising the reaction of said monomer units of formula M-W, with R, R₁, R₂, R₃ or R₄ being or having a Carboxylic acid, and R or R₁ being or having an amino group, with a silylation agent and a weak base, followed by an acylation reaction using a diacylating agent and a weak base, and then hydrolysis in an acidic medium.

17. Compound of formula M₁-L-M₂ as defined in any one of Claims 1 to 9, the names of which follow:
- disodium salt of 3,3'-{3,6,9,12,15-pentaoxaheptadecane-1,17-diylbis[oxy(1-methoxy-2-methylindolizine-8,3-diyl)carbonyl]}bis(6-aminobenzoic acid)
- disodium salt of 3,3'-(3,6,9,12,15-pentaoxaheptadecane-1,17-diylbis{oxy[3-(4-amino-3-methoxybenzoyl)imidazo[1,5-*a*]pyridine-8,1-diyl]})dibenzoic acid
- disodium salt of 3,3'-{3,6,9,12,15,18-hexaoxaicosane-1,20-diylbis[oxy(1-methoxy-2-methylindolizine-6,3-diyl)carbonyl]}bis(6-aminobenzoic acid)
- disodium salt of 3,3'-{3,6,9,12,15-pentaoxaheptadecane-1,17-diylbis[oxy(1-methoxy-2-methylindolizine-6,3-diyl)carbonyl]}bis(6-aminobenzoic acid)
- disodium salt of 3,3'-{3,5,9,12,15,18-hexaoxaicosane-1,20-diylbis[oxy(2-methylindolizine-1,3-diyl)carbonyl]}bis(6-aminobenzoic acid)
- disodium salt of 3,3'-{ethane-1,2-diylbis[oxyethane-2,1-diyloxy-3,1-phenylenemethyleneoxy(2-methylindolizine-1,3-diyl)carbonyl]}bis(6-aminobenzoic acid)
- disodium salt of 3,3'-{octane-1,8-diylbis[oxy-3,1-phenylenecarbonylimino(2-methylindolizine-1,3-diyl)carbonyl]}bis(6-aminobenzoic acid)
- disodium salt of 3,3'-{ethane-1,2-diylbis[oxyethane-2,1-diyloxy-3,1-phenylenecarbonylimino(2-methylindolizine-1,3-diyl)carbonyl]}bis(6-aminobenzoic acid).
- disodium salt of 3,3'-{(1,4-dioxobutane-1,4-diyl)bis[iminoethane-2,1-diyloxy-3,1-phenylenecarbonylimino(2-methylindolizine-1,3-diyl)carbonyl]}bis(6-aminobenzoic acid)
- disodium salt of 3,3'-{carbonyl bis[iminoethane-2,1-diyloxy-3,1-phenylenecarbonylimino(2-methylindolizine-1,3-diyl)carbonyl]}bis(6-aminobenzoic acid)
- disodium salt of 3,3'-{ethane-1,2-diylbis[imino(2-oxoethane-2,1-diyl)oxy-3,1-phenylenecarbonylimino(2-methylindolizine-1,3-diyl)carbonyl]}bis(6-aminobenzoic acid)
- disodium salt of 3,3'-{propane-1,3-diylbis[imino(2-oxoethane-2,1-diyl)oxy-3,1-phenylenecarbonylimino(2-methylindolizine-1,3-diyl)carbonyl]}bis(6-aminobenzoic acid)
- disodium salt of 3,3'-{butane-1,4-diylbis[imino(2-oxoethane-2,1-diyl)oxy-3,1-phenylenecarbonylimino(2-methylindolizine-1,3-diyl)carbonyl]}bis(6-aminobenzoic acid)
- disodium salt of 3,3'-{ethane-1,2-diylbis[imino(2-oxoethane-2,1-diyl)oxy-4,1-phenylenecarbonylimino(2-methylindolizine-1,3-diyl)carbonyl]}bis(6-aminobenzoic acid)
- disodium salt of 3,3'-{ethane-1,2-diylbis[oxyethane-2,1-diyloxy-4,1-phenylenecarbonylimino(2-methylindolizine-1,3-diyl)carbonyl]}bis(6-aminobenzoic acid)
- disodium salt of 3,3'-{oxybis[ethane-2,1-diyloxyethane-2,1-diyloxy-4,1-phenylene(2-methylindolizine-1,3-diyl)carbonyl]}bis(6-aminobenzoic acid)
- disodium salt of 3,3'-{ethane-1,2-diylbis[oxyethane-2,1-diyloxy-3,1-phenylene(2-methylindolizine-1,3-diyl)carbonyl]bis(6-aminobenzoic acid)
- disodium salt of 3,3'-{oxybis[ethane-2,1-diyloxyethane-2,1-diyloxy-3,1-phenylene(2-methylindolizine-1,3-diyl)carbonyl]}bis(6-aminobenzoic acid)
- disodium salt of 3,3'-{hexane-1,6-diylbis[imino(2-oxoethane-2,1-diyl)oxy-3,1-phenylene(2-methylindolizine-1,3-diyl)carbonyl]}bis(6-aminobenzoic acid)
- disodium salt of 3,3'-{hexane-1,6-diylbis[imino(2-oxoethane-2,1-diyl)oxy-4,1-phenylene(2-methylindolizine-1,3-diyl)carbonyl]}bis(6-aminobenzoic acid)
- disodium salt of 2-amino-5-[(1-{[3-(2-{[2-({[3-({[3-(4-amino-3-carboxybenzoyl) imidazo[1,5-*a*]pyridin-1-yl]amino}carbonyl)phenoxy]acetyl}amino)ethyl]amino}-2-oxoethoxy)benzoyl]amino}-2-methylindolizin-3-yl)carbonyl]dibenzoic acid.

## Patentansprüche

1. FGF-Rezeptor-Agonistenverbindungen der allgemeinen Formel:
M₁-L-M₂,
in der M₁ oder M₂, die gleich oder verschieden sind, jeweils unabhängig voneinander eine monomere Einheit M bedeuten und L eine Bindungsgruppe, die M₁ und M₂ kovalent bindet, bedeutet, **dadurch gekennzeichnet, dass** die monomere Einheit die folgende allgemeine Formel M
aufweist: 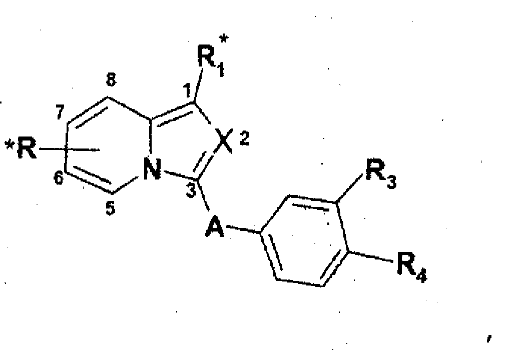 in der
X N oder C-R₂* bedeutet,
A einen Rest -CO- bedeutet,
* die Bindungsstelle zwischen L mit erstens der monomeren Einheit M₁ und zweitens der monomeren Einheit M₂ angibt, wobei sich diese Bindungsstelle von jeder mononeren Einheit M₁ bzw. M₂ an einem der Substituenten R, R₁ oder R₂ befindet,
R ein Wasserstoffatom, ein Halogenatom, einen geradkettigen oder verzweigten Alkylrest mit 1 bis 5 Kohlenstoffatomen, einen Hydroxylrest oder einen Rest der Formel:
• -CO₂R₅
• -CO-NR₆R₇
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk
• -NH-CO-Alk oder
• -NH-CO₂-Alk
worin:
• R₅ ein Wasserstoffatom, einen geradkettigen oder verzweigten Alkylrest mit 1 bis 5 Kohlenstoffatomen oder einen Benzylrest bedeutet;
• R₆ und R₇, die gleich oder verschieden sind, jeweils einen Wasserstoffrest, einen geradkettigen oder verzweigten Alkylrest mit 1 bis 5 Kohlenstoffatomen oder einen Benzylrest bedeuten,
• Alk einen geradkettigen oder verzweigten Alkylrest mit 1 bis 5 Kohlenstoffatomen oder einen geradkettigen oder verzweigten Alkylenrest mit 1 bis 5 Kohlenstoffatomen bedeutet;
• Ph einen Phenylrest, der gegebenenfalls durch 1 oder mehrere Wasserstoffatome, durch ein oder mehrere Hydroxylreste, durch einen oder mehrere geradkettige oder verzweigte Alkoxyreste mit 1 bis 5 Kohlenstoffatomen oder durch einen oder mehrere Reste -COOR₆ substituiert ist, bedeutet;
bedeutet;
R₁ ein Wasserstoffatom, ein Halogenatom, einen Hydroxylrest, einen Cyanorest, einen geradkettigen oder verzweigten Alkylrest mit 1 bis 5 Kohlenstoffatomen oder einen Rest der Formel:
• -CO₂R₅
• -CO-NR₆R₇
• -CO-NH-Alk-CO₂R₅
• -CO-NH-Ph
• -O-Alk
• -O-Alk-CO₂R₅
• -O-Alk-CO-NR₆R₇
• -O-Alk-NR₆R₇
• -O-Alk-OR₅
• -O-Alk-Ph
• -O-Ph
• -NR₆R₇
• -NH-SO₂-Alk
• -NH-CO-Alk
• NH-CO-Alk-CO₂R₅
• -NH-CO-Alk-CO-NR₆R₇
• -NH-CO₂-Alk
• -NH-CO-Ph oder
• einen Aryl- oder Heteroarylrest mit 5 oder 6 Atomen ausgewählt aus der Gruppe C, N, O, S, der gegebenenfalls durch ein oder mehrere Halogenatome, durch einen oder mehrere geradkettige oder verzweigte Alkylreste mit 1 bis 5 Kohlenstoffatomen, durch einen oder mehrere geradkettige oder verzweigte,Alkoxyreste mit 1 bis 5 Kohlenstoffatomen oder durch einen oder mehrere Reste -CO₂R₅ oder -CO-NR₆R₇, worin Alk, Ph, R₅, R₆ und R₇ wie für die Gruppe R definiert sind, substituiert ist
bedeutet;
R₂ einen geradkettigen oder verzweigten Alkylrest mit 1 bis 5 Kohlenstoffatomen, einen Cycloalkylrest mit 3 bis 6 Kohlenstoffatomen oder einen Phenylrest, der gegebenenfalls durch ein oder mehrere Halogenatome, durch einen oder mehrere Carboxylreste, durch einen oder mehrere geradkettige oder verzweigte Alkoxyreste mit 1 bis 5 Kohlenstoffatomen, durch einen oder mehrere Hydroxylreste, durch einen oder mehrere Benzyloxyreste oder durch einen oder mehrere Alkoxycarbonylreste mit 2 bis 6 Kohlenstoffatomen substituiert ist, bedeutet;
R₃ und R₄ unabhängig voneinander ein Wasserstoffatom, einen Hydroxylrest, einen Aminorest, einen Nitrorest oder einen Rest der Formel:
• -CO₂R₅
• -CO-NR₆R₇
• -CO-NHOH
• -O-O-Alk
• -O-Alk-NR₆R₇
• -O-Alk-CO-NR₆R₇
• -NHOH
• -NR₆R₇
• -N(R₈)-CO-Alk
• -N(R₈)-CO-CF₃
• -N(R₈) -CO-Ph
• -N(R₈) -CO₂-Alk
• -N(R₈) -SO₂-Alk
• -N(R₈) -CO-Alk-NR₆R₇
• -N(R₈) -SO₂-Alk-NR₆R₇ oder
• -NH-Alk-R₆R₇
in denen R₈ ein Wasserstoffatom oder einen Rest -Alk-COOR₅ bedeutet und Alk, R₅, R₆ und R₇ wie für die Gruppe R definiert sind, bedeuten;
oder R3 und R4 gemeinsam mit den Kohlenstoffatomen des Phenylrings, an den sie gebunden sind, einen Cyclus mit 6 Ringgliedern umfassend ein Stickstoffatom und ein weiteres Heteroatom wie Sauerstoff bilden; und
L Folgendes bedeutet:
einen geradkettigen oder verzweigten Alkylenrest mit 2 bis 25 Kohlenstoffatomen, einen geradkettigen oder verzweigten Alkenylrest mit 2 bis 25 Kohlenstoffatomen oder eine geradkettige oder verzweigte Alkinylgruppe mit 2 bis 25 Kohlenstoffatomen;
oder einen Rest ausgewählt aus den Formeln: 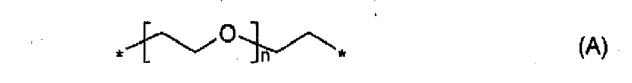 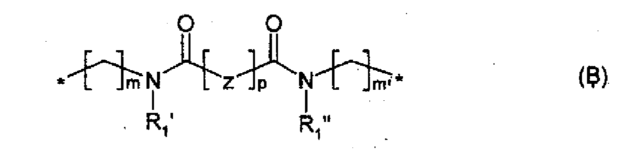 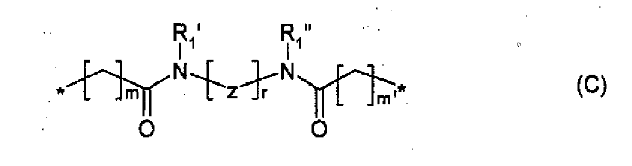 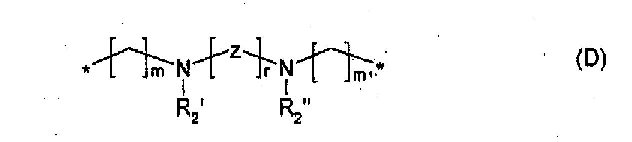 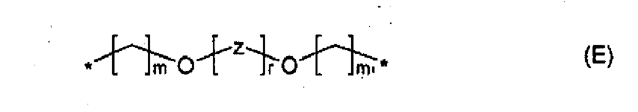 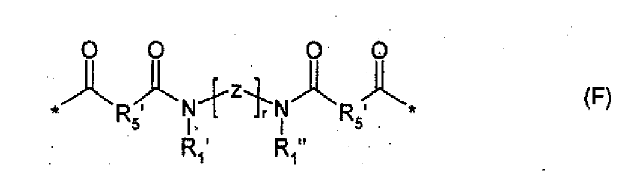 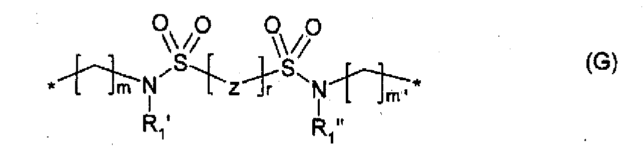 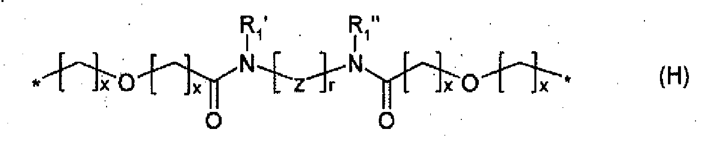 in denen
* das Atom der Verbindung von L mit der monomeren Einheit M an einem der Substituenten R, R₁ oder R₂ angibt;
Z eine Bindung oder einen Carbonylrest oder einen geradkettigen, verzweigten oder cyclischen Alkylenrest mit 1 bis 6 Kohlenstoffatomen, der gegebenenfalls durch
1 oder 2 Carbonylreste substituiert ist, oder einen Rest
• - [CH₂]ₛ-[-CH-(CH₂)_{q}-OR₃']-[CH₂] ₛ-
• - [CH₂]ₛ-[-CH-(CH₂)_{q}-NR₃'R₄']-[CH₂] ₛ-
• - [CH₂-CH₂-O]ₜ-CH₂-CH₂-
• Phenyl oder Alkylphenylalkyl, wobei die Phenylgruppe gegebenenfalls durch einen oder mehrere Alkoxyreste mit 1 bis 5 Kohlenstoffatomen substituiert ist, oder
• Heteroaryl oder Alkylheteroarylalkyl, wobei die Heteroarylgruppe gegebenenfalls durch einen oder mehrere Alkoxyreste mit 1 bis 5 Kohlenstoffatomen substituiert ist, bedeutet,
n eine ganze Zahl von 1 bis 7 bedeutet
m und m' gleich oder verschieden sind und eine ganze Zahl von 0 bis 8 bedeuten
p eine ganze Zahl von 0 bis 11 bedeutet
r eine ganze Zahl von 1 bis 11 bedeutet
q eine ganze Zahl von 0 bis 5 bedeutet
s eine ganze Zahl von 0 bis 5 bedeutet
t eine ganze Zahl von 0 bis 5 bedeutet
x eine ganze Zahl von 1 bis 5 bedeutet
m, m', n, p, r, s, t, x, die der Anzahl der Ringglieder der Bindungsgruppe L entsprechen, nicht mehr als 25 ausmachen,
R₁' und R₁" , die gleich oder verschieden sind, ein Wasserstoffatom, einen geradkettigen oder verzweigten Alkylrest mit 1 bis 5 Kohlenstoffatomen bedeuten,
R₂' und R₂", die gleich oder verschieden sind, ein Wasserstoffatom, einen geradkettigen oder verzweigten Alkylrest mit 1 bis 5 Kohlenstoffatomen oder einen Benzylrest oder eine Sulfatgruppe bedeuten,
R₁' und R₁" wie auch R₂' und R₂" gegebenenfalls unter Bildung eines Cyclus gebunden sein können,
R₃' und R₄', die gleich oder verschieden sind, jeweils ein Wasserstoffatom, einen geradkettigen oder verzweigten Alkylrest mit 1 bis 5 Kohlenstoffatomen oder einen Benzylrest oder eine Sulfatgruppe bedeuten,
R₅' einen geradkettigen oder verzweigten Alkylrest mit 1 bis 5 Kohlenstoffatomen bedeutet;
als Base oder Säure- oder Basenadditionssalz sowie als Hydrat oder Solvat.

2. Verbindungen nach Anspruch 1, umfassend die monomere Einheit der Formel M, in der:
X = C-R₂
R an den Positionen 6, 7 oder 8 des Indolizins ein Wasserstoffatom, ein Halogenatom, einen Hydroxylrest oder einen Rest der Formel:
• -COOR₅
• -CO-NR₆R₇
• -O-Alk
• -O-Alk-CO₂R₅
• -O-Alk-NR₆R₇
• -NR₆R₇
• -NH-SO₂-Alk
• -NH-CO-Alk oder
• -NH-CO₂-Alk
bedeutet, in denen:
• R₅ ein Wasserstoffatom, einen geradkettigen oder verzweigten Alkylrest mit 1 bis 5 Kohlenstoffatomen oder einen Benzylrest bedeutet;
• R₆ und R₇, die gleich oder verschieden sind, jeweils einen Wasserstoffrest, einen geradkettigen oder verzweigten Alkylrest mit 1 bis 5 Kohlenstoffatomen oder einen Benzylrest bedeuten,
• Alk einen geradkettigen oder verzweigten Alkylrest mit 1 bis 5 Kohlenstoffatomen oder einen geradkettigen oder verzweigten Alkylenrest mit 1 bis 5 Kohlenstoffatomen bedeutet;
• Ph einen Phenylrest, der gegebenenfalls durch 1 oder mehrere Halogenatome, durch ein oder mehrere Hydroxylreste, durch einen oder mehrere geradkettige oder verzweigte Alkoxyreste mit 1 bis 5 Kohlenstoffatomen oder durch einen oder mehrere Reste -COOR₅ substituiert ist,
bedeutet;
R₁ ein Wasserstoffatom, ein Halogenatom, einen Hydroxylrest, einen Cyanorest oder einen Rest der Formel:
• -CO₂R₅
• -CO-NR₆R₇
• -CO-NH-Alk-CO₂R₅
• -CO-NH-Ph
• -O-Alk
• -O-Alk-CO₂R₅
• -O-Alk-CO-NR₆R₇
• -O-Alk-NR₆R₇
• -O-Alk-OR₅
• -O-Alk-Ph
• O-Ph
• -NR₆R₇
• -NH-SO₂-Alk
• -NH-CO-Alk
• -NH-CO-Alk-CO₂R₅
• -NH-CO-Alk-CO-NR₆R₇
• -NH-CO₂₋Alk
• -NH-CO-Ph oder
• einen Aryl- oder Heteroarylrest mit 5 oder 6 Atomen ausgewählt aus der Gruppe C, N, O, S, der gegebenenfalls durch ein oder mehrere halogenatome, durch einen oder mehrere geradkettige oder verzweigte Alkylreste mit 1 bis 5 Kohlenstoffatomen, durch einen oder mehrere geradkettige oder verzweigte Alkoxyreste mit 1 bis 5 Kohlenstoffatomen oder durch einen oder mehrere Reste -CO₂R₅ oder -CO-NR₆R₇, worin Alk, Ph, R₅, R₆ und R₇ wie für die Gruppe R definiert sind, substituiert ist
bedeutet;
R₂ einen geradkettigen oder verzweigten Alkylrest mit 1 bis 5 Kohlenstoffatomen, einen Cycloalkylrest mit 3 bis 6 Kohlenstoffatomen oder einen Phenylrest, der gegebenenfalls durch ein oder mehrere Halogenatome, durch einen oder mehrere Alkoxyreste mit 1 bis 5 Kohlenstoffatomen, durch einen oder mehrere Carboxylreste oder durch einen oder mehrere Alkoxycarbonylreste mit 2 bis 6 Kohlenstoffatomen substituiert ist, bedeutet;
R₃ und R₄, die gleich oder verschieden sind, jeweils ein Wasserstoffatom, einen Hydroxylrest, einen Alkoxyrest mit 1 bis 5 Kohlenstoffatomen, einen Aminorest, einen Nitrorest, einen Rest der Formel
• -NR₆R₇
• -NH-CO-Alk
• -NH-SO₂-Alk
• -CO₂R₅
• -CO-NR₆R₇ oder
• -CO-NHOH
worin Alk, Ph, R₅, R₆ und R₇ wie für die Gruppe R definiert sind, bedeuten;
als Base oder Säure- oder Basenadditionssalz sowie als Hydrat oder Solvat.

3. Verbindungen nach einem der Ansprüche 1 oder 2, umfassend die monomere Einheit der Formel M, in der:
X = C-R₂
R an den Positionen 6, 7 oder 8 des Indolizins ein Wasserstoffatom, einen Hydroxylrest, einen Carboxylrest oder einen Rest der Formel:
• -O-Alk
• -O-Alk-CO₂R₅
• -O-Alk-CO-NR₅R₇
• -O-Alk-NR₆R₇
• -O-Alk-Ph
• -NR₅R₇
• -NH-SO₂-Alk oder
• -NH-CO-Alk
bedeutet, in denen:
• R₅ ein Wasserstoffatom, einen Alkylrest mit 1 bis 5 Kohlenstoffatomen oder einen Benzylrest bedeutet;
• R₆ und R₇, die gleich oder verschieden sind, jeweils ein Wasserstoffatom, einen geradkettigen oder verzweigten Alkylrest mit 1 bis 5 Kohlenstoffatomen oder einen Benzylrest bedeuten,
• Alk einen Alkylrest oder einen geradkettigen oder verzweigten Alkylenrest mit 1 bis 5 Kohlenstoffatomen bedeutet;
• Ph einen Phenylrest, der gegebenenfalls durch ein oder mehrere Halogenatome, durch einen oder mehrere Hydroxylreste, durch einen oder mehrere Alkoxyreste mit 1 bis 5 Kohlenstoffatomen oder durch einen oder mehrere Reste -COOR₅ substituiert ist, bedeutet;
R₁ ein Halogenatom, einen Hydroxylrest, einen Carboxylrest oder einen Rest der Formel:
• -O-Alk
• -O-Alk-CO₂R₅
• -O-Alk-Ph
• -O-Ph
• -NR₆R₇
• -NH-CO-Ph oder
• einen Aryl- oder Heteroarylrest mit 5 oder 6 Atomen ausgewählt aus der Gruppe C, N, O, S, der gegebenenfalls durch ein oder mehrere halogenatome, durch einen oder mehrere geradkettige oder verzweigte Alkylreste mit 1 bis 5 Kohlenstoffatomen, durch einen oder mehrere geradkettige oder verzweigte Alkoxyreste mit 1 bis 5 Kohlenstoffatomen oder durch einen oder mehrere Reste -CO₂R₅ oder -CO-NR₆R₇, worin Alk, Ph, R₅, R₆ und R₇ wie für die Gruppe R definiert sind, substituiert ist
bedeutet;
R₂ einen geradkettigen oder verzweigten Alkylrest mit 1. bis 5 Kohlenstoffatomen oder einen Cycloalkylrest mit 3 bis 6 Kohlenstoffatomen bedeutet;
R₃ und R₄, die gleich oder verschieden sind, jeweils einen geradkettigen oder verzweigten Alkoxyrest mit 1 bis. 5 Kohlenstoffatomen, einen Aminorest, einen Carboxylrest, einen Hydroxylrest, einen Rest der Formel CO-NR₆R₇ oder einen Rest der Formel -NH-SO₂-Alk, worin Alk, R₆ und R₇ wie für die Gruppe R definiert sind, bedeuten;
als Base oder Säure- oder Basenadditionssalz sowie als Hydrat oder Solvat.

4. Verbindungen nach Anspruch 3, **dadurch** gekenntzeichnet, dass
R an den Positionen 6 oder 8. des Indolizins ein Wasserstoffatom oder einen Hydroxylrest bedeutet;
R₁ einen Hydroxylrest oder einen Rest der Formel:
• -O-Alk
• -O-Alk-Ph
• -NR₆R₇ oder
• -NH-CO-Ph
bedeutet, worin Alk, Ph, R₆ und R₇ wie für Anspruch 6 definiert sind;
R₂ einen Alkylrest mit 1 bis 5 Kohlenstoffatomen bedeutet;
R₃ einen geradkettigen oder verzweigten Alkoxyrest mit 1 bis 5 Kohlenstoffatomen oder einen Carboxylrest bedeutet;
R₄ einen Aminorest bedeutet;
als Base oder als Säure- oder Basenadditionssalz sowie als Hydrat oder Solvat.

5. Verbindungen nach Anspruch 1, umfassend die monomere Einheit der Formel M, worin:
X = N;
R an den Positionen 5, 6, 7 oder 8 des Imidazo[1,5-*a*]pyridins ein Wasserstoffatom, ein Halogenatom, einen geradkettigen oder verzweigten Alkylrest mit 1 bis 5 Kohlenstoffatomen, einen Hydroxylrest oder einen Rest der Formel:
• -CO₂R₅
• -CO-NR₆R₇
• -O-Alk
• -O-Alk-CO₂R₅
• -O-Alk-CO-NR₆R₇
• -O-Alk-NR₆R₇
• -O-Alk-Ph
• -NR₆R₇
• -NH-SO₂-Alk
• -NH-CO-Alk oder
• -NH-CO₂-Alk
bedeutet, worin:
• R₅ ein Wasserstoffatom, einen geradkettigen oder verzweigten Alkylrest mit 1 bis 5 Kohlenstoffatomen oder einen Benzylrest bedeutet;
• R₆ und R₇, die gleich oder verschieden sind, jeweils ein Wasserstoffatom, einen geradkettigen oder verzweigten Alkylrest mit 1 bis 5 Kohlenstoffatomen oder einen Benzylrest bedeuten;
• Alk einen geradkettigen oder verzweigten Alkylrest mit 1 bis 5 Kohlenstoffatomen oder einen geradkettigen oder verzweigten Alkylenrest mit 1 bis 5 Kohlenstoffatomen bedeutet;
• Ph einen Phenylrest, der gegebenenfalls durch ein oder mehrere Halogenatome, durch einen oder mehrere Hydroxylreste, durch einen oder mehrere geradkettige oder verzweigte Alkoxyreste mit 1 bis 5 Kohlenstoffatomen oder durch einen oder mehrere Reste -COOR₅ substituiert ist, bedeutet;
R₁ ein Wasserstoffatom, ein Halogenatom, einen Cyanorest oder einen Rest der Formel:
• -CO₂R₅
• -CO-NH-Ph
• -NR₆R₇
• -NH-SO₂-Alk
• -NH-CO-Alk
• -NH-CO₂-Alk
• -NH-CO-Ph oder
• einen Aryl- oder Heteroarylrest mit 5 oder 6 Atomen ausgewählt aus der Reihe C, N, 0, S, der gegebenenfalls durch ein oder mehrere Halogenatome, durch einen oder mehrere geradkettige oder verzweigte Alkylreste mit 1 bis 5 Kohlenstoffatomen, die durch einen oder mehrere geradkettige oder verzweigte Alkoxyreste mit 1 bis 5 Kohlenstoffatomen, durch einen oder mehrere Reste -CO₂R₅ oder -CO-NR₆R₇, in denen Alk, Ph, R₅, R₆ und R₇ wie für die Gruppe R definiert sind, substituiert ist, bedeutet;
R₃ und R₄, die gleich oder verschieden sind, jeweils ein Wasserstoffatom, einen Hydroxylrest, einen geradkettigen oder verzweigten Alkoxyrest mit 1 bis 5 Kohlenstoffatomen, einen Aminorest, einen Nitrorest oder einen Rest der Formel
• -NR₆R₇
• -NH-CO-Alk
• -NH-SO₂-Alk
• -CO₂R₅
• -CO-NR₆R₇ oder
• -CO-NHOH
in denen Alk, Ph, R₅, R₆ und R₇ wie für die Gruppe R definiert sind,
bedeuten;
oder R₃ und R₄ gemeinsam mit den Kohlenstoffatomen des Phenylrings, an den sie gebunden sind, einen Kohlenstoffcyclus mit 6 Ringgliedern, der ein Stickstoffatom und ein weiteres Heteroatom wie Sauerstoff umfasst, bilden;
als Base oder als Säure- oder Basenadditionssalz sowie als Hydrat oder Solvat.

6. Zusammensetzungen nach Anspruch 5, umfassend die monomere Einheit der Formel M, in der:
X = N;
R an den Positionen 6, 7 oder 8 des Imidazo[1,5-*a*]pyridins ein Wasserstoffatom, ein Halogenatom, einen Hydroxylrest, einen Carboxylrest oder einen Rest der Formel:
• -CO-NR₆R₇
• -O-Alk
• -O-Alk-CO₂R₅
• -O-Alk-Ph
• -NR₆R₇
• -NH-SO₂-Alk oder
• -NH-CO-Alk
bedeutet, in denen:
• R₅ ein Wasserstoffatom, einen geradkettigen oder verzweigten Alkylrest mit 1 bis 5 Kohlenstoffatomen oder einen Benzylrest bedeutet;
• R₆ und R₇, die gleich oder verschieden sind, jeweils ein Wasserstoffatom, einen geradkettigen oder verzweigten Alkylrest mit 1 bis 5 Kohlenstoffatomen oder einen Benzylrest bedeuten;
• Alk einen geradkettigen oder verzweigten Alkylrest mit 1 bis 5 Kohlenstoffatomen oder einen geradkettigen oder verzweigten Alkylenrest mit 1 bis 5 Kohlenstoffatomen bedeutet;
• Ph einen Phenylrest, der gegebenenfalls durch ein oder mehrere Halogenatome, durch einen oder mehrere Hydroxylreste, durch einen oder mehrere geradkettige oder verzweigte Alkoxyreste mit 1 bis 5 Kohlenstoffatomen oder durch einen oder mehrere Reste -COOR₅ substituiert ist, bedeutet;
R₁ ein Wasserstoffatom, ein Halogenatom, einen Carboxylrest oder einen Rest der Formel:
• -NR₆R₇
• -NH-SO₂-Alk
• -NH-CO-Alk
• -NH-CO-Ph oder
• einen Aryl- oder Heteroarylrest mit 5 oder 6 Atomen ausgewählt aus der Reihe C, N, O, S, der gegebenenfalls durch ein oder mehrere Halogenatome, durch einen oder mehrere geradkettige oder verzweigte Alkylreste mit 1 bis 5 Kohlenstoffatomen, die durch einen oder mehrere geradkettige oder verzweigte Alkoxyreste mit 1 bis 5 Kohlenstoffatomen, durch einen oder mehrere Reste -CO₂R₅ oder -CO-NR₆R₇, in denen Alk, Ph, R₅, R₆ und R₇ wie für die Gruppe R definiert sind, substituiert ist, bedeutet;
R₃ und R₄, die gleich oder verschieden sind, jeweils einen geradkettigen oder verzweigten Alkoxyrest mit 1 bis 5 Kohlenstoffatomen, einen Aminorest, einen Carboxylrest, einen Hydroxylrest oder einen Rest der Formel CO-NR₆R₇ oder -NH-SO₂-Alk bedeuten;
als Base oder als Säure; oder Basenadditionssalz sowie als Hydrat oder Solvat.

7. Verbindungen nach Anspruch 6, **dadurch gekennzeichnet, dass**
R in Postition 8 des Imidazol [1,5-a] pyridin ein Wasserstoffatom, einen Hydroxylrest oder einen Carboxylrest bedeutet,
R₁ ein Wasserstoffatom, einen Rest der Formel -NH-CO-Ph oder einen Aryl- oder Heteroarylrest mit 5 oder 6 Atomen ausgewählt aus der Reihe C, N, O, S, der gegebenenfalls durch einen oder mehrere Reste -CO₂R₅ substituiert ist, bedeutet,
in denen Alk, Ph, R₅ wie in Anspruch 6 definiert sind; R₃ einen geradkettigen oder verzweigten Alkoxyrest mit 1 bis 5 Kohlenstoffatomen oder einen Carboxylrest bedeutet;
R₄ einen Aminorest bedeutet;
als Base oder als Säure- oder Basenadditionssalz sowie als Hydrat oder Solvat.

8. FGF-Rezeptor-Agonistenverbindungen nach Anspruch 1, **dadurch gekennzeichnet, dass** M₁ eine monomere Einheit der allgemeinen Formel M wie in einem der Ansprüche 1 bis 4 definiert ist und M₂ eine monomere Einheit der Formel M wie in einem der Ansprüche 5 bis 7 definiert ist;
als Base oder als Säure- oder Basenadditionssalz sowie als Hydrat oder Solvat.

9. Zusammensetzungen nach einem der Ansprüche 1 bis 8, **dadurch gekennzeichnet, dass**:
L die 2 monomeren Einheiten-M₁ und M₂ über den Rest R₁ bindet oder;
L die 2 monomeren Einheiten M₁ und M₂ über den Rest R₂ bindet oder;
L die 2 monomeren Einheiten M₁ und M₂ über den Rest R in dessen Position 8 bindet oder; L die 2 monomeren Einheiten M₁ und M₂ über den Rest
R in dessen Position 7 bindet oder; L die 2 monomeren Einheiten M₁ und M₂ über den Rest R in dessen Position 6 bindet oder;
L die 2 monomeren Einheiten M₁ und M₂ erstens durch den Rest R in dessen Position 8 und zweitens durch den Rest R in dessen Position 7 oder 6 bindet oder;
L die 2 monomeren Einheiten M₁ und M₂ erstens durch den Rest R in dessen Position 7 und zweitens durch den Rest R in dessen Position 6 bindet oder;
L die 2 monomeren Einheiten M₁ und M₂ erstens durch den Rest R₂ und zweitens durch den Rest R₁ bindet oder;
L die 2 monomeren Einheiten M₁ und M₂ erstens durch den Rest R₁ und zweitens durch den Rest R in dessen Position 8 bindet;
als Base oder als Säure- oder Basenadditionssalz sowie als Hydrat oder Solvat.

10. Pharmazeutische Zusammensetzung, die als Wirkstoff eine Verbindung der Formel M₁-L-M₂ nach einem der Ansprüche 1 bis 9 gegebenenfalls gemeinsam mit einem oder mehreren inerten und geeigneten Grundstoffen enthält.

11. Pharmazeutische Zusammensetzung nach Anspruch 10, die sich für die Behandlung von ischämischer Herzerkrankung, die Behandlungen von Krankheiten, die mit einer Verengung oder Obstruktion von Arterien oder Arteritiden assoziiert sind, die Behandlung von Angina Pectoris, die Behandlung von obliterierender Thromboangiitis, die Behandlung von Atherosklerose, die Behandlung der Hemmung der Restenose nach Angioplastie oder Endarterektomie, die Behandlung der Vernarbung, die Behandlung für die Muskelregeneration, die Behandlung für das Überleben der Myoblasten, die Behandlung der Nociception und die Behandlung von chronischen Schmerzen, die Behandlung der peripheren Neuropathie, die Behandlung für die Verbesserung des Überlebens des bioartifiziellen Pankreastransplantats beim Diabetespatienten, die Behandlung der Cholesterinreduktion in Assoziation mit der Adipositasreduktion, die Behandlung der Verbesserung der Revaskularisierung von Transplantaten und des Überlebens der Transplantate, die Behandlung von Retinadegeneration, die Behandlung von Retinitis pigmentosa, die Behandlung von Osteoarthritis, die Behandlung von Preeclampsia, die Behandlung von Gefäßläsionen und akuter Atemnot, die Knochenschutzbehandlung oder die Haarfollikelschutzbehandlung eignet.

12. Verbindung nach einem der Ansprüche 1 bis 9 zur Verwendung in der Behandlung von Krankheiten, die einer Aktivierung der FGF-Rezeptoren bedürfen.

13. Verbindung nach einem der Ansprüche 1 bis 9 zur Verwendung in der Behandlung von ischämischer Herzerkrankung, der Behandlung von Krankheiten, die mit einer Verengung oder Obstruktion von Arterien oder Arteritiden assoziiert sind, der Behandlung von Angina Pectoris, der Behandlung von obliterierender Thromboangiitis, der Behandlung von Atherosklerose, der Behandlung der Hemmung der Restenose nach Angioplastie oder Endarterektomie, der Behandlung der Vernarbung, der Behandlung für die Muskelregeneration, der Behandlung für das Überleben der Myoblasten, der Behandlung der Nociception und der Behandlung von chronischen Schmerzen, der Behandlung der peripheren Neuropathie, der Behandlung für die Verbesserung des Überlebens des bioartifiziellen Pankreastransplantats beim Diabetespatienten, der Behandlung der Cholesterinreduktion in Assoziation mit der Adipositasreduktion, der Behandlung der Verbesserung der Revaskularisierung von Transplantaten und des Überlebens der Transplantate, der Behandlung von Retinadegeneration, der Behandlung von Retinitis pigmentosa, der Behandlung von Osteoarthritis, der Behandlung von Preeclampsia, der Behandlung von Gefäßläsionen und akuter Atemnot, der Knochenschutzbehandlung oder der Haarfollikelschutzbehandlung.

14. Verfahren zur Herstellung einer Verbindung der Formel M₁-L-M₂ nach einem der Ansprüche 1 bis 9, umfassend das Umsetzen von mindestens einer monomeren Einheit der Formel M-W mit einem Reaktionspartner der Formel U-L-U', wobei
M und L wie in den Ansprüchen 1 bis 9 definiert sind,
U und U' gleich oder verschieden sind
W und U sowie W und U' jeweils eine funktionelle Gruppe bedeuten, die fähig ist, miteinander unter Bildung einer kovalenten Bindung des Typs C-C, C-O, C-N, C-C oder C-S zu reagieren, wobei sich W an einem der Substituenten R, R₁ oder R₂ wie in den Ansprüchen 1 bis 8. definiert befindet.

15. Herstellungsverfahren nach Anspruch 14, **dadurch gekennzeichnet, dass** W und U sowie W und U' eine Amino-, Hydroxyl-, Carboxyl-, Amido-, Carbamat-, Halogen-, Sulfonylchlorid-, Säurechlorid-, Säurefluorid-, Boronester- oder Boronsäuregruppe bedeuten.

16. Herstellungsverfahren nach Anspruch 15, umfassend das Umsetzen der monomeren Einheiten der Formel M-W, wobei R, R₁, R₂, R₃ oder R₄ eine Carbonsäuregruppe aufweisen oder darstellen und oder R₁ eine Aminogruppe aufweisen oder darstellen, mit einem Silylierungsmittel und einer schwachen Base, gefolgt von einer Acylierung unter Verwendung eines Diacylierungsmittels und einer schwachen Base, gefolgt von einer Hydrolyse im sauren Milieu.

17. Verbindung der Formel M₁-L-M₂ wie in einem der ansprüche 1 bis 9 definiert, mit der folgenden Bezeichnung:
- Dinatriumsalz der 3,3'-{3,6,9,12,15-Pentaoxaheptadecan-1,17-diylbis[oxy(1-methoxy-2-methylindolizin-8,3-diyl)carbonyl]}bis(6-aminobenzoesäure)
- Dinatriumsalz der 3,3'-{3,6,9,12,15-Pentaoxaheptadecan-1,17-diylbis{oxy[3-(4-amino-3-methyloxybenzoyl)imidazo[1,5-a]pyridin-8,1-diyl)]}benzoesäure
- Dinatriumsalz der 3,3'-{3,6,9,12,15,18-Hexaoxaicosan-1,20-diylbis[oxy(1-methoxy-2-methylindolizin-6,3-diyl)carbonyl]bis(6-aminobenzoesäure)
- Dinatriumsalz der. 3,3'-{3,6,9,12,15-Pentaoxaheptadecan-1,17-diylbis[oxy(1-methoxy-2-methylindolizin-6,3-diyl)carbonyl]}bis(6-aminobenzoesäure)
- Dinatriumsalz der 3,3'-{3,6,9,12,15,18-Hexaoxaicosan-1,20-diylbis[oxy(2-methylindolizin-1,3-diyl)carbonyl]}bis(6-aminobenzoesäure)
- Dinatriumsalz der 3,3'-(Ethan-1,2-diylbis[oxyethan-2,1-diyloxy-3,1-phenylenmethylendxy(2-methylindolizin-1,3-diyl)carbonyl]bis(6-aminobenzoesäure)
- Dinatriumsalz der 3,3'-(Octan-1,8-diylbis[oxy-3,1-phenylencarbonylimino(2-methylindolizin-1,3-diyl)carbonyl]}bis(6-aminobenzoesäure)
- Dinatriumsalz der 3,3'-{Ethan-1,2-diylbis[oxyethan-2,1-diyloxy-3,1-phenylencarbonylimino(2-methylindolizin-1,3-diyl)carbonyl]}bis(6-aminobenzoesäure
- Dinatriumsalz der 3,3'-{[1,4-Dioxobutan-1,4-diyl)bis[iminoethan-2,1-diyloxy-3,1-phenylencarbonylimino(2-methylindolizin-1,3-diyl)carbonyl]}bis(6-aminobenzoesäure)
- Dinatriumsalz der 3,3'-(Carbonylbis[iminoethan-2,1-diyloxy-3,1-phenylencarbonylimino(2-methylindolizin-1,3-diyl)carbonyl]}bis(6-aminobenzoesäure)
- Dinatriumsalz der 3,3'-(Ethan-1,2-diylbis[imino(2-oxoethan-2,1-diyl)oxy-3,1-phenylencarbonylimino(2-methylindolizin-1,3-diyl)carbonyl]}bis(6-aminobenzoesäure)
- Dinatriumsalz der 3,3'-(Propan-1,3-diylbis[imino(2-oxoethan-2,1-diyl)oxy-3,1-phenylencarbonylimino(2-methylindolizin-1,3-diyl) carbonyl]}bis(6-aminobenzoesäure)
- Dinatriumsalz der 3,3'-(Butan-1,4-diylbis[imino(2-oxoethan-2,1-diyl)oxy-3,1-phenylencarbonylimino(2-methylindolizin-1,3-diyl)carbonyl]}bis(6-aminobenzoesäure)
- Dinatriumsalz der 3,3'-{Ethan-1,2-diylbis[imino(2-oxoethan-2,1-diyl)oxy-4,1-phenylencarbonylimino(2-methylindolizin-1,3-diyl)carbonyl])bis(6-aminobenzoesäure)
- Dinatriumsalz der 3,3'-(Ethan-1,2-diylbis[oxyethan-2,1-diyloxy-4,1-phenylencarbonylimino(2-methylindolizin-1,3-diyl)carbonyl]}bis(6-aminobenzoesäure)
- Dinatriumsalz der 3;3'-{Oxybis[ethan-2,1-diyloxyethan-2,1-diyloxy-4,1-phenylen(2-methylindolizin-1,3-diyl) carbonyl]}bis(6-aminobenzoesäure}
- Dinatriumsalz der 3,3'-{ethan-1,2-diylbis[oxyethan-2,1-diyloxy-3,1-phenylen(2-methylindolizin-1,3-diyl)carbonyl]}bis(6-aminobenzoesäure)
- Dinatriumsalz der 3,3'-{Oxybis[ethan-2,1-diyloxyethan-2,1-diyloxy-3,1-phenylen(2-methylindolizin-1,3-diyl)carbonyl]}bis(6-aminobenzoesäure)
- Dinatriumsalz der 3,3'-{Hexan-1,6-diylbis[imino(2-oxoethan-2,1-diyl)oxy-3,1-phenylen(2-methylindolizin-1,3-diyl)carbonyl]}bis(6-aminobenzoesäure)
- Dinatriumsalz der 3,3'{Hexan-1,6-diylbis[imino(2-oxoethan-2,1-diyl)oxy-4,1-phenylen(2-methylindolizin-1,3-diyl)carbonyl]}bis(6-aminobenzoesäure)
- Dinatriumsalz der 2-Amino-5-[(1-{[3-(2-{[2-({[3-({[3-(4-amino-3-carboxybenzoyl)imidazo[1,5-a] pyridin-1-yl]amino}carbonyl)phenoxy]acetyl}amino)ethyl]amino}-2-oxoethoxy)benzoyl]amino}-2-methylindolizin-3-ylcarbonyl]dibenzoesäure
